# EUROPEAN PATENT APPLICATION

(11) **EP 3 202 419 A1**
(43) Date of publication of application: **09.08.2017**
(21) Application number: 16204809.4
(22) Date of filing: 06.06.2013
(51) Int. Cl.: A61K 39/395, A61K 38/00, A61P 35/00, C12Q 1/02, C07K 16/28, A61K 38/17, A61K 45/06

(54) **PVRIG BINDING AGENTS THAT MODULATE THE HIPPO PATHWAY AND USES THEREOF**

(30) Priority: 06.06.2012 US 201261656249 P; 14.12.2012 US 201261737390 P; 14.03.2013 US 201361783190 P
(62) Divisional of application: 13801023.6
(71) Applicant: OncoMed Pharmaceuticals, Inc., Redwood City, CA 94063 (US)
(72) Inventor: GURNEY, Austin L., San Francisco, CA 94114 (US); CHARTIER-COURTAUD, Cecile, Palo Alto, CA 94301 (US)
(74) Representative: Sutcliffe, Nicholas Robert

(57) **Abstract**

The present invention relates to agents that modulate the Hippo pathway and Hippo pathway signaling, such as antibodies, soluble receptors, polypeptides, peptides, and small molecules. The invention relates to methods of screening for and/or identifying such agents and methods of using the agents for the treatment of diseases such as cancer.

## Description

### CROSS-REFERENCE TO RELATED APPLICATIONS

This application claims priority benefit of U.S. Provisional Application No. 61/656,249, filed June 6, 2012, U.S. Provisional Application No. 61/737,390, filed December 14, 2012, and U.S. Provisional Application No. 61/783,190, filed March 14, 2013, each of which is hereby incorporated by reference herein in its entirety.

### FIELD OF THE INVENTION

This invention generally relates to agents that modulate the Hippo pathway and Hippo pathway signaling, such as antibodies and soluble receptors, as well as to methods of using the agents for the treatment of diseases such as cancer.

### BACKGROUND OF THE INVENTION

The Hippo pathway is a signaling pathway that regulates cell proliferation and cell death and determines organ size. The pathway is believed to play a role as a tumor suppressor in mammals and disorders of the pathway are often detected in human cancers. The pathway is involved in and/or may regulate the self-renewal and differentiation of stem cells and progenitor cells. In addition, the Hippo pathway may be involved in wound healing and tissue regeneration. Furthermore, it is believed that as the Hippo pathway cross-talks with other signaling pathways such as Wnt, Notch, and Hedgehog, it may influence a wide variety of biological events and that its dysfunction could be involved in many human diseases in addition to cancer. For reviews, see, for example, Halder et al., 2011, Development 138:9-22; Zhao et al., 2011, Nature Cell Biology 13:877-883; Bao et al., 2011, J. Biochem. 149:361-379; Zhao et al., 2010, J. Cell Sci. 123:4001-4006.

The Hippo pathway is composed of a kinase cascade that is highly conserved across *Drosophila* and mammalian systems. In *Drosophilia*, the atypical cadherins, Dachsous (Ds) and Fat (Ft) have been reported to function as receptors for the Hippo pathway (see, for example, Bennett et al., 2006, Curr. Biol., 16:2101-2110; Matakatsu et al., 2006, Development, 133:2315-2324). Recently, it was reported that the *Drosophilia* cell adhesion molecule Echinoid (Ed) interacts with Salvador (Sav) and may be involved in Hippo signaling (Yue et al, 2012, Dev. Cell, 22:255-267). However, a cell surface receptor has not been identified for the mammalian Hippo pathway.

Two of the core components of the mammalian Hippo pathway are Lats1 and Lats2, which are nuclear Dbf2-related (NDR) family protein kinases homologous to *Drosophila* Warts (Wts). The Lats1/2 proteins are activated by association with the scaffold proteins Mob1A/B (Mps one binder kinase activator-like 1A and 1B), which are homologous to *Drosophila* Mats. Lats1/2 proteins are also activated by phosphorylation by the STE20 family protein kinases Mst1 and Mst2, which are homologous to *Drosophila* Hippo. Lats1/2 kinases phosphorylated the downstream effectors YAP (Yes-associated protein) and TAZ (transcriptional coactivator with PDZ-binding motif; WWTR1), which are homologous to *Drosophila* Yorkie. The phosphorylation of YAP and TAZ by Lats1/2 are crucial events within the Hippo signaling pathway. Lats1/2 phosphorylates YAP at multiple sites, but phosphorylation of Ser127 is critica.l for YAP inhibition. Phosphorylation of YAP generates a protein-binding motif for the 14-3-3 family of proteins, which upon binding of a 14-3-3 protein, leads to retention and/or sequestration of YAP in the cell cytoplasm. Likewise, Lats1/2 phosphorylate TAZ at multiple sites, but phosphorylation of Ser89 is critical for TAZ inhibition. Phosphorylation of TAZ leads to retention and/or sequestration of TAZ in the cell cytoplasm. Similar to YAP, phosphorylation of TAZ also generates a 14-3-3 binding site. In addition, phosphorylation of YAP and TAZ is believed to destabilize these proteins by activating phosphorylation-dependent degradation catalyzed by YAP or TAZ ubiquitination. Thus, when the Hippo pathway is "on", YAP and/or TAZ is phosphorylated, inactive, and generally sequestered in the cytoplasm; in contrast, when the Hippo pathway is "off", YAP and/or TAZ is non-phosphorylated, active, and generally found in the nucleus.

Non-phosphorytated, activated YAP is translocated into the cell nucleus where its major target transcription factors are the four proteins of the TEA-domain-containing family (TEAD1-TEAD4, collectively "TEAD"). YAP together with TEAD (or other transcription factors such as Smad1, RUNX, ErbB4 and p73) has been shown to induce the expression of a variety of genes, including connective tissue growth factor (CTGF), Gli2, Birc5, Birc2, fibroblast growth factor 1 (FGF1), and amphiregulin (AREG). Like YAP, non-phosphorylated TAZ is translocated into the cell nucleus where it interacts with multiple DNA-binding transcription factors, such as peroxisome proliferator-activated receptor γ (PPARγ), thyroid transcription factor-1 (TTF-1), Pax3, TBX5, RUNX, TEAD1 and Smad2/3/4. Many of the genes activated by YAP/TAZ-transcription factor complexes mediate cell survival and proliferation. Therefore, under some conditions YAP and/or TAZ acts as an oncogene and the Hippo pathway acts as a tumor suppressor. Thus, targeting the Hippo pathway may be another area for therapeutic intervention for cancer and other diseases.

### BRIEF SUMMARY OF THE INVENTION

The present invention provides binding agents, such as antibodies, soluble receptors, and small molecules that modulate the Hippo pathway. The invention also provides compositions, such as pharmaceutical compositions, comprising the binding agents. In certain embodiments, the binding agents that modulate the Hippo pathway are novel polypeptides, such as antibodies and fusion proteins. In certain embodiments, the binding agents that modulate the Hippo pathway are novel small peptides or small molecules. In certain embodiments, the binding agents are antibodies or fragments thereof that specifically bind human cell adhesion molecules of the immunoglobulin superfamily (IgCAM). The invention further provides methods of inhibiting the growth of a tumor by administering the binding agents that modulate the Hippo pathway to a subject with a tumor. The invention further provides methods of treating cancer by administering the binding agents that modulate the Hippo pathway to a subject in need thereof. The invention further provides methods of inhibiting tumor or cancer metastases by administering the binding agents that modulate the Hippo pathway to a subject in need thereof. In some embodiments, the methods of treating cancer, inhibiting tumor growth, or inhibiting metastases comprise activating or stimulating the Hippo pathway. In some embodiments, the methods of treating cancer, inhibiting tumor growth, or inhibiting metastases comprise inhibiting YAP activity. In some embodiments, the methods of treating cancer, inhibiting tumor growth, or inhibiting metastases comprise inhibiting TAZ activity. In certain embodiments, the methods of treating cancer, inhibiting tumor growth, or inhibiting metastases comprise reducing the tumorigenicity of a tumor.

In one aspect, the invention provides a binding agent, such as an antibody or a soluble receptor, that specifically binds one or more human IgCAMs. These binding agents are referred to herein as "IgCAM-binding agents". In some embodiments, the binding agent specifically binds the extracellular domain of at least one human IgCAM. In some embodiments, the binding agent specifically binds the extracellular domain of at least one human IgCAM selected from the JAM (junctional adhesion molecule) family, the PVR (poliovirus receptor) family, and/or the CADM (cell adhesion molecule) family. In some embodiments, the binding agent specifically binds the extracellular domain of one or more IgCAMs selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In some embodiments, the binding agent specifically binds the extracellular domain of CLMP, VSIG4, VSIG8, JAM2, JAM3, CADM1, CADM3, CADM4, PVRL1, PVRL3, PVRL4, TIGIT, TMIGD1, and/or TMEM25. In some embodiments, the binding agent specifically binds the extracellular domain of CADM1, CADM3, PVRL1 and/or PVRL3. In some embodiments, the binding agent specifically binds the extracellular domain of CADM3, PVRL1 and/or PVRL3. In some embodiments, the binding agent specifically binds the extracellular domain of CADM3.

In some embodiments, the IgCAM-binding agent modulates Hippo pathway activity. In some embodiments, the IgCAM-binding agent increases Hippo pathway signaling. In some embodiments, the IgCAM-binding agent is an agonist of Hippo pathway activity and/or enhances Hippo pathway signaling. In some embodiments, the IgCAM-binding agent inhibits YAP activity, enhances phosphorylation of YAP, enhances degradation of YAP, and/or inhibits activation of YAP. In some embodiments, the IgCAM-binding agent inhibits TAZ activity, enhances phosphorylation of TAZ, enhances degradation of TAZ, and/or inhibits activation of TAZ.

In some embodiments, the IgCAM-binding agent inhibits Hippo pathway signaling. In some embodiments, the IgCAM-binding agent is an antagonist of Hippo pathway activity and/or decreases Hippo pathway signaling. In some embodiments, the IgCAM-binding agent increases YAP activity, decreases phosphorylation of YAP, decreases degradation of YAP, and/or increases activation of YAP. In some embodiments, the IgCAM-binding agent increases TAZ activity, decreases phosphorylation of TAZ, decreases degradation of TAZ, and/or increases activation of TAZ.

In certain embodiments, the IgCAM-binding agent is a polypeptide. In certain embodiments, the IgCAM-binding agent is isolated. In certain embodiments, the IgCAM-binding agent is substantially pure.

In some embodiments of each of the aforementioned aspects and embodiments, as well as other aspects and embodiments described herein, the IgCAM-binding agent is an antibody. In some embodiments, the antibody is a monoclonal antibody. In some embodiments, the antibody is a monovalent antibody. In some embodiments, the antibody is a bispecific antibody. In some embodiments, the antibody is an antibody fragment. In some embodiments, the antibody binds at least one human IgCAM. In some embodiments, the antibody binds at least one human IgCAM and at least one mouse IgCAM. In some embodiments, the antibody binds at least one IgCAM with a K_{D} of less than 10nM.

In certain embodiments of each of the aforementioned aspects and embodiments, as well as other aspects and embodiments described herein, the IgCAM-binding agent is a soluble receptor. In some embodiments, the soluble receptor comprises the extracellular domain of an IgCAM. In some embodiments, the soluble receptor comprises a portion or fragment of the extracellular domain of an IgCAM. In some embodiments, the soluble receptor is a fusion protein. In some embodiments, the soluble receptor comprises a human Fc region. In some embodiments, the soluble receptor binds at least one human IgCAM. In some embodiments, the soluble receptor binds at least one human IgCAM and at least one mouse IgCAM. In some embodiments, the soluble receptor binds at least one IgCAM with a K_{D} of less than 10nM.

In another aspect, the invention provides isolated polynucleotide molecules comprising a polynucleotide that encodes a binding agent of the invention. The invention further provides expression vectors that comprise these polynucleotides, as well as cells that comprise the expression vectors and/or the polynucleotides. In some embodiments, the cell is a hybridoma cell line.

In other aspects, the invention provides methods of inhibiting growth of a tumor, comprising contacting the tumor with an effective amount of an IgCAM-binding agent that modulates the Hippo pathway, including each of the binding agents described herein. In some embodiments, the IgCAM-binding agent is an agonist of Hippo pathway activity, enhances Hippo pathway signaling, inhibits YAP activity, enhances phosphorylation of YAP, enhances degradation of YAP, inhibits activation of YAP, inhibits TAZ activity, enhances phosphorylation of TAZ, enhances degradation of TAZ, and/or inhibits activation of TAZ.

In another aspect, the invention provides a method of inhibiting the growth of a tumor in a subject, comprising administering to the subject a therapeutically effective amount of an IgCAM-binding agent that modulates the Hippo pathway, including each of the binding agents described herein. In some embodiments, the IgCAM-binding agent is an agonist of Hippo pathway activity, enhances Hippo pathway signaling, inhibits YAP activity, enhances phosphorylation of YAP, enhances degradation of YAP, inhibits activation of YAP, inhibits TAZ activity, enhances phosphorylation of TAZ, enhances degradation of TAZ, and/or inhibits activation of TAZ.

In another aspect, the invention provides a method of modulating Hippo pathway signaling in a cell, comprising contacting the cell with an effective amount of an IgCAM-binding agent, including any of those described herein. In some embodiments, the cell is a tumor cell. In some embodiments, the tumor is a colorectal tumor. In some embodiments, the tumor is a breast tumor. In some embodiments, the tumor is an ovarian tumor. In some embodiments, the tumor is a pancreatic tumor. In some embodiments, the tumor is a lung tumor. In some embodiments, the tumor is a melanoma tumor. In some embodiments, the tumor expresses elevated levels of YAP. In some embodiments, the tumor expresses elevated levels of non-phosphorylated YAP. In some embodiments, the tumor contains elevated levels of YAP in the cell nucleus. In some embodiments, the tumor has elevated expression levels of YAP-dependent genes. In some embodiments, the tumor expresses elevated levels of TAZ. In some embodiments, the tumor expresses elevated levels of non-phosphorylated TAZ. In some embodiments, the tumor contains elevated levels of TAZ in the cell nucleus. In some embodiments, the tumor has elevated expression levels of TAZ-dependent genes. In certain embodiments, the IgCAM-binding agent inhibits growth of the tumor.

In another aspect, the invention provides methods of treating cancer in a subject. In some embodiments, the method comprises administering to a subject a therapeutically effective amount of any of the binding agents that modulate Hippo pathway signaling described herein. In some embodiments, the binding agent is an agonist of Hippo pathway activity, enhances Hippo pathway signaling, inhibits YAP activity, enhances phosphorylation of YAP, enhances degradation of YAP, and/or inhibits activation of YAP. In some embodiments, the binding agent inhibits TAZ activity, enhances phosphorylation of TAZ, enhances degradation of TAZ, and/or inhibits activation of TAZ. In some embodiments, the cancer is pancreatic cancer. In some embodiments, the cancer is colorectal cancer. In some embodiments, the cancer is breast cancer. In some embodiments, the cancer is lung cancer. In some embodiments, the cancer is melanoma. In some embodiments, the cancer is ovarian cancer. In some embodiments, the cancer expresses elevated levels of YAP. In some embodiments, the cancer expresses elevated levels of non-phosphorylated YAP. In some embodiments, the cancer has elevated expression levels of YAP-dependent genes. In some embodiments, the cancer expresses elevated levels of TAZ. In some embodiments, the cancer expresses elevated levels of non-phosphorylated TAZ. In some embodiments, the cancer has elevated expression levels of TAZ-dependent genes.

In another aspect, the invention provides methods of treating a disease in a subject wherein the disease is associated with activation of YAP and/or aberrant YAP activity, wherein the methods comprise administering a therapeutically effective amount of a binding agent that modulates the Hippo pathway, including any of the binding agents described herein. In some embodiments, the modulation of the Hippo pathway comprises increasing Hippo pathway signaling. In some embodiments, the modulation of the Hippo pathway comprises increasing YAP phosphorylation. In some embodiments, the modulation of the Hippo pathway comprises increasing YAP degradation. In some embodiments, the modulation of the Hippo pathway comprises increasing retention of YAP in the cytoplasm. In some embodiments, the modulation of the Hippo pathway comprises reducing YAP translocation to the nucleus. In some embodiments, the modulation of the Hippo pathway comprises reducing the expression of YAP-dependent genes.

In another aspect, the invention provides methods of treating a disease in a subject wherein the disease is associated with activation of TAZ and/or aberrant TAZ activity, wherein the methods comprise administering a therapeutically effective amount of a binding agent that modulates the Hippo pathway, including each of the binding agents described herein. In some embodiments, the modulation of the Hippo pathway comprises increasing Hippo pathway signaling. In some embodiments, the modulation of the Hippo pathway comprises increasing TAZ phosphorylation. In some embodiments, the modulation of the Hippo pathway comprises increasing TAZ degradation. In some embodiments, the modulation of the Hippo pathway comprises increasing retention of TAZ in the cytoplasm. In some embodiments, the modulation of the Hippo pathway comprises reducing TAZ translocation to the nucleus. In some embodiments, the modulation of the Hippo pathway comprises reducing the expression of TAZ-dependent genes.

In some embodiments, invention provides a method of modulating Hippo pathway signaling in a cell, comprising contacting the cell with an effective amount of an IgCAM-binding agent that inhibits Hippo pathway signaling. In some embodiments, the modulation of the Hippo pathway comprises decreasing Hippo pathway signaling. In some embodiments, the modulation of the Hippo pathway comprises decreasing YAP phosphorylation. In some embodiments, the modulation of the Hippo pathway comprises decreasing YAP degradation. In some embodiments, the modulation of the Hippo pathway comprises decreasing retention of YAP in the cytoplasm. In some embodiments, the modulation of the Hippo pathway comprises increasing YAP translocation to the nucleus. In some embodiments, the modulation of the Hippo pathway comprises increasing the expression of YAP-dependent genes. In some embodiments, the modulation of the Hippo pathway comprises decreasing TAZ phosphorylation. In some embodiments, the modulation of the Hippo pathway comprises decreasing TAZ degradation. In some embodiments, the modulation of the Hippo pathway comprises decreasing retention of TAZ in the cytoplasm. In some embodiments, the modulation of the Hippo pathway comprises increasing TAZ translocation to the nucleus. In some embodiments, the modulation of the Hippo pathway comprises increasing the expression of TAZ-dependent genes.

In another aspect, the invention provides methods of treating a wound. In some embodiments the methods promote and/or enhance wound healing in a subject. In some embodiments, the methods comprise administering to a subject a therapeutically effective amount of a binding agent that modulates Hippo pathway signaling, such as a binding agent described herein. In some embodiments, the administered binding agent is an antagonist of Hippo pathway activity, suppresses Hippo pathway signaling, increases YAP activity, suppresses phosphorylation of YAP, suppresses degradation of YAP, and/or promotes activation of YAP. In some embodiments, the administered binding agent increases TAZ activity, suppresses phosphorylation of TAZ, suppresses degradation of TAZ, and/or promotes activation of TAZ. In some embodiments, the wound is an acute cutaneous wound. In some embodiments, the wound is a chronic cutaneous wound. In some embodiments, the wound is a surgical wound.

In another aspect, the invention provides methods of enhancing tissue regeneration. In some embodiments, the methods comprise contacting cells with an effective amount of a binding agent that modulates Hippo pathway signaling, such as a binding agent described herein. In some embodiments, the methods comprise administering to a subject a therapeutically effective amount of a binding agent that modulates Hippo pathway signaling, such as a binding agent described herein. In some embodiments, the modulation of Hippo pathway signaling comprises antagonizing the Hippo pathway. In some embodiments, the modulation of Hippo pathway signaling comprises reducing signaling of the Hippo pathway. In some embodiments, the method involves the treatment of tissue damage caused by immune related disorders (such as autoimmune disorders); inflammation (including both acute and chronic inflammatory disorders); ischemia (such as myocardial infarction); traumatic injury (such as bums, lacerations, and abrasions); infection (such as bacterial, viral, and fungal infections); and chronic damage (such as cirrhosis of the liver).

In certain embodiments of each of the aforementioned aspects, as well as other aspects and/or embodiments described elsewhere herein, the methods comprise a step of determining the location of YAP and/or TAZ (e.g., nuclear or cytoplasmic) in the cells of the tumor or cancer. In certain embodiments, of each of the aforementioned aspects, as well as other aspects and/or embodiments described elsewhere herein, the methods comprise a step of determining the expression level of YAP-dependent genes or proteins encoded by these genes. In certain embodiments, the methods comprise a step of determining the expression level of TAZ-dependent genes or proteins encoded by these genes.

In another aspect, the invention provides a method of identifying a human subject or selecting a human subject for treatment with a binding agent that modulates Hippo pathway signaling, including but not limited to, each of the binding agents described herein. In some embodiments, the method comprises determining if the subject has a tumor that has an elevated expression level of YAP as compared to the expression of YAP in a reference sample. In some embodiments, the method comprises determining if the subject has a tumor that has an elevated expression level of YAP as compared to a pre-determined level of YAP. In some embodiments, the method comprises determining if the subject has a tumor that has an elevated expression level of YAP as compared to the expression of YAP in normal tissue. In some embodiments, the method comprises determining if the subject has a tumor that has an elevated level of YAP in the nucleus of tumor cells as compared to the level of YAP in the nucleus of cells from normal tissue. In some embodiments, the method comprises determining if the subject has a tumor that has an elevated level of non-phosphorylated YAP in tumor cells as compared to the level of non-phosphorylated YAP in cells from normal tissue. In some embodiments, the method comprises determining if the subject has a tumor that has an elevated expression level of TAZ as compared to the expression of TAZ in normal tissue. In some embodiments, the method comprises determining if the subject has a tumor that has an elevated level of TAZ in the nucleus of tumor cells as compared to the level of TAZ in the nucleus of cells from normal tissue. In some embodiments, the method comprises determining if the subject has a tumor that has an elevated level of non-phosphorylated TAZ in tumor cells as compared to the level of non-phosphorylated TAZ in cells from normal tissue. In some embodiments, the "normal tissue" is a tissue of the same type as the tumor, e.g., a lung tumor and normal lung tissue.

In another aspect, the invention provides methods of targeting tumor cells with an IgCAM-binding agent, such as the agents described herein. In some embodiments, the methods comprise administering to a subject an effective amount of an IgCAM-binding agent. In some embodiments, the binding agent is an antibody. In some embodiments, the IgCAM-binding agent comprises a soluble receptor. In some embodiments, the IgCAM-binding agent is a soluble receptor. In some embodiments, the IgCAM-binding agent is conjugated to or complexed with a cytotoxic agent.

Compositions comprising an IgCAM-binding agent as described herein and cell lines that produce the binding agents are provided. Moreover, pharmaceutical compositions comprising an IgCAM-binding agent described herein and a pharmaceutically acceptable carrier are further provided. Methods of treating cancer and/or inhibiting tumor growth in a subject comprising administering to the subject an effective amount of a composition comprising a binding agent such as the IgCAM-binding agents described herein are also provided. Methods of enhancing wound healing and/or tissue regeneration in a subject comprising administering to the subject an effective amount of a composition comprising a binding agent such as the IgCAM-binding agents described herein are also provided.

In another aspect, the invention provides methods of screening and/or identifying an agent that modulates the Hippo pathway. In some embodiments, a method of screening for an agent that modulates the Hippo pathway comprises: screening for an agent that specifically binds the extracellular domain of an IgCAM, wherein the agent modulates the Hippo pathway. In some embodiments, a method of screening for and/or identifying an agent that modulates the Hippo pathway comprises contacting cells with a test agent and determining the cellular localization of YAP and/or TAZ using a bimolecular fluorescence complementation (BiFC) assay. In some embodiments, a method of screening and/or identifying an agent that modulates the Hippo pathway comprises: (a) contacting a first set of cells, but not a second set of cells, with a test agent, and (b) determining the amount of phosphorylated YAP and/or phosphorylated TAZ in the first set of cells as compared to the second set of cells. In some embodiments, a method of screening and/or identifying an agent that modulates the Hippo pathway comprises: (a) contacting a first set of cells, but not a second set of cells, with a test agent, and (b) determining the amount of non-phosphorylated YAP and/or non-phosphorylated TAZ in the first set of cells as compared to the second set of cells. In some embodiments, a method of screening and/or identifying an agent that modulates the Hippo pathway comprises: (a) contacting a first set of cells, but not a second set of cells, with a test agent, and (b) determining the expression of at least one YAP-dependent gene and/or at least one TAZ-dependent gene in the first set of cells as compared to the expression of the YAP-dependent gene and/or the TAZ-dependent gene in the second set of cells. In some embodiments, the at least one YAP-dependent gene or the at least one TAZ-dependent gene is selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GLI2, BIRC2, BIRC5 (survivin), FGF1, IL33, GRB2, IGFBP3, AREG, FN1, ADAMTS1, AXL, ADAMTS5, MET, CYR61, IL8, ZEB1, FOXC2, N-cadherin, and SNAIL. In some embodiments, the test agent is an antibody, a soluble receptor, a small molecule, a polypeptide, a peptide, a peptidomimetic, or a drug compound.

In another aspect, the invention provides an agent identified by any of the screening methods described herein.

Where aspects or embodiments of the invention are described in terms of a Markush group or other grouping of alternatives, the present invention encompasses not only the entire group listed as a whole, but also each member of the group individually and all possible subgroups of the main group, and also the main group absent one or more of the group members. The present invention also envisages the explicit exclusion of one or more of any of the group members in the claimed invention.

### BRIEF DESCRIPTION OF THE FIGURES

Figure 1. Microarray gene expression analysis of YAP in patient-derived tumors from the OncoMed tumor bank.

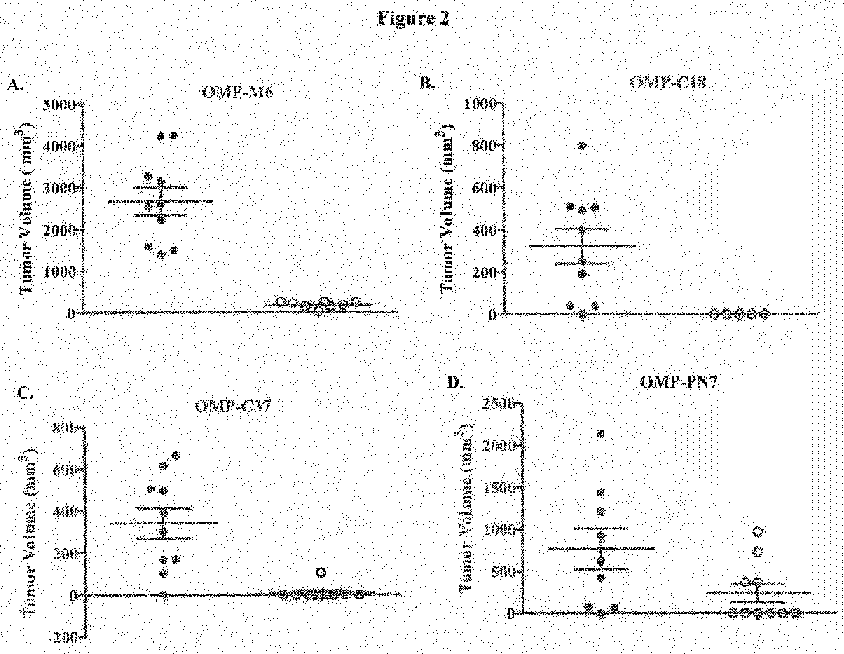

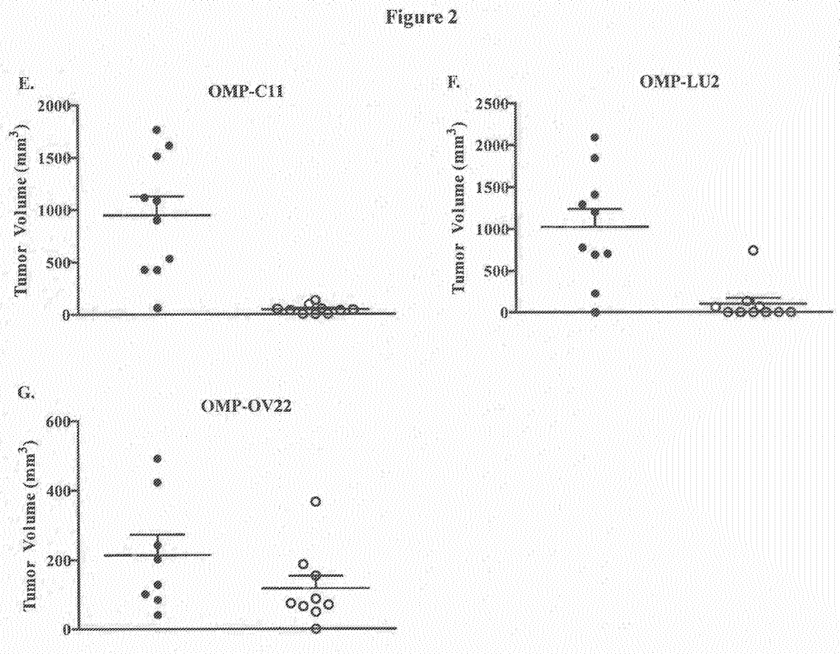

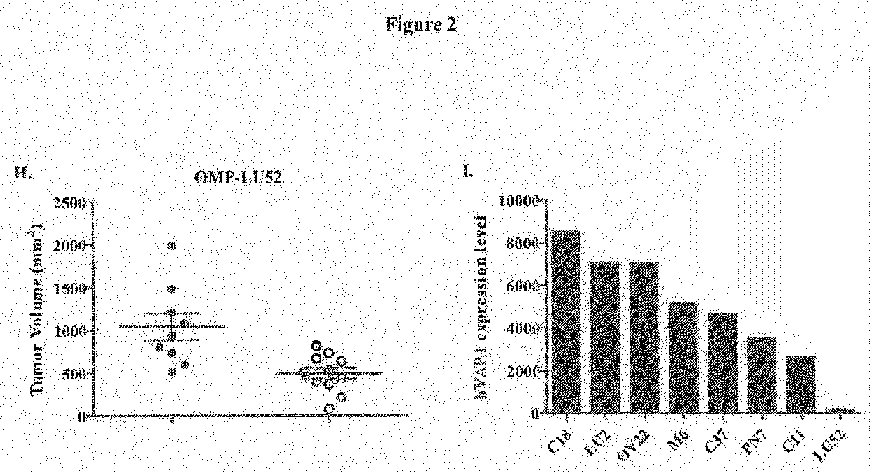

Figure 2. Inhibition of tumor growth by the expression of dnYAP. Tumor cells were transduced with dnYAP-lentivirus (-o-) or GFP-lentivirus (-•-) and incubated 72 hours. Transduced cells, identified as GFP positive and DAPI negative (GFP⁺/DAPI⁻), were sorted by flow cytometry and subcutaneously injected into NOD/SCID mice. Tumor growth was monitored for 3 months and data is shown as tumor volume (mm³). (A) OMP-M6 melanoma tumors; (B) OMP-C18 colon tumors; (C) OMP-C37 colon tumors; (D) OMP-PN7 pancreatic tumors; (E) OMP-C11 colon tumors; (F) OMP-L2 lung tumors; (G) OMP-OV22 ovarian tumors; (H) OMP-LU52 lung tumors. (1) YAP expression in tumors as determined by microarray analysis.
Figure 3. Effect of expression of dnYAP on YAP activity.
Figure 4. Effect of cell detachment on Hippo pathway components as determined by Western blot analysis.
Figure 5. Family tree of candidate Hippo pathway receptors.
Figure 6. Diagram of candidate Hippo pathway receptor, membrane-bound decoy receptor, and soluble receptor.
Figure 7. Expression of membrane-anchored decoy receptor. (A) Cell surface expression of GFP-tagged IgCAM decoy receptor as viewed by fluorescent microscopy. (B) Flow cytometry histogram of GFP-tagged IgCAM decoy receptor.
Figure 8. Effect of IgCAM decoy receptors on Hippo pathway components as determined by Western blot analysis.
Figure 9. Bimolecular fluorescence complementation assay for determination of YAP nuclear translocation. (A) Diagrams of YFP constructs with YAP, TEAD2, and TEAD3. (B) Nuclear YFP fluorescence of low density cells as viewed by fluorescent microscopy and phase contrast. (C) FACS analysis of transduced HeLa cells.
Figure 10. Tetracycline-inducible HeLa cell line NC12 expressing GFP-YAP. (A) Expression of GFP-YAP in response to Tet activation. (B) Western blot analysis of GFP-YAP expression in response to Tet activation.
Figure 11. Diagrams of Cre-dependent EmGFP expression construct and YAP-Cre constructs for use in determination of YAP nuclear translocation.
Figure 12. Effect on tumor growth by over-expression of membrane-anchored IgCAM decoy receptors in colon tumor OMP-C18. (A) CADM2, CADM4, CD226, PVR; (B) CADM3, TMEM25, JAM3, TIGIT; (C) CADM3, TMEM25, JAM3, TIGIT; (D) JAM2, PVRL1, ESAM; (E) VSIG1, VSIG4, PVRL4, CD200; (F) PVRL3, JAM1, CADM1, VSIG2; and (G) CLMP, VSIG8, TMIGD1, VSIG3.
Figure 13. Effect on tumor growth by over-expression of membrane-anchored IgCAM decoy receptors in colon tumor OMP-LU2. (A) CADM2; (B) CADM3, PVRL4, VSIG4; (C) CD226, JAM2, JAM3, CADM1; (D) CADM4, TIGIT, PVRL1, PVRL3.
Figure 14. Effect on tumor growth by over-expression of membrane-anchored IgCAM decoy receptors in colon tumor OMP-LU40. (A) CADM1, CADM2, CADM3, CADM4, CD226, PVR; (B) PVRL4, VSIG4, CD226; (C) PVRL3, ESAM, VSIG2.
Figure 15. Western blot analysis of IgCAM protein complexes using cells transfected with FLAG-tagged full-length CADM1, CADM3, PVR, PVRL1, and PVRL3. CADM3-CD4TM-GFP and LGR5-CD4TM-GFP constructs were used as negative controls. Protein complexes were analyzed for (A) Afadin, DCAF7, DLG1, INADL, LIN7, LIN7C; (B) MPDZ, MPP5?, PARD3, FLAG control; (C) YAP, phospho-YAP. Tubulin and actin were used as controls.
Figure 16. Western blot analysis of IgCAM1 protein complexes using cells transfected with FLAG-tagged tagged full-length CADM1 and c-myc-tagged YAP. Protein complexes were analyzed for myc-tagged YAP and FLAG-tagged CADM1.

### DETAILED DESCRIPTION OF THE INVENTION

The present invention provides novel agents, including, but not limited to, polypeptides such as antibodies and soluble receptors that modulate the Hippo pathway. The agents include agonists and antagonists of Hippo pathway signaling. Related polypeptides and polynucleotides, compositions comprising the agents, and methods of making the agents are also provided. Methods of screening for agents that modulate the Hippo pathway are provided. Methods of using the novel agents, such as methods of inhibiting tumor growth, methods of treating cancer, methods of activating and/or enhancing Hippo pathway signaling, methods of inhibiting or reducing Hippo pathway signaling, and/or methods of identifying and/or selecting subjects for treatment, are further provided.

### I. Definitions

To facilitate an understanding of the present invention, a number of terms and phrases are defined below.

The terms "agonist" and "agonistic" as used herein refer to or describe an agent that is capable of, directly or indirectly, substantially inducing, activating, promoting, increasing, or enhancing the biological activity of a target and/or a signaling pathway (e.g., the Hippo pathway). The term "agonist" is used herein to include any agent that partially or fully induces, activates, promotes, increases, or enhances the activity of a protein. Suitable agonists specifically include, but are not limited to, agonist antibodies or fragments thereof, soluble receptors, and other fusion proteins.

The terms "antagonist" and "antagonistic" as used herein refer to or describe an agent that is capable of, directly or indirectly, partially or fully blocking, inhibiting, reducing, or neutralizing a biological activity of a target and/or signaling pathway (e.g., the Hippo pathway). The term "antagonist" is used herein to include any agent that partially or fully blocks, inhibits, reduces, or neutralizes the activity of a protein. Suitable antagonist agents specifically include, but are not limited to, antagonist antibodies or fragments thereof soluble receptors, and other fusion proteins.

The terms "modulation" and "modulate" as used herein refer to a change or an alteration in a biological activity. Modulation includes, but is not limited to, stimulating or inhibiting an activity. Modulation may be an increase or a decrease in activity (e.g., a decrease in Hippo pathway signaling; an increase in Hippo pathway signaling), a change in binding characteristics, or any other change in the biological, functional, or immunological properties associated with the activity of a protein, pathway, or other biological point of interest.

The term "antibody" as used herein refers to an immunoglobulin molecule that recognizes and specifically binds a target, such as a protein, polypeptide, peptide, carbohydrate, polynucleotide, lipid, or combinations of the foregoing, through at least one antigen recognition site within the variable region of the immunoglobulin molecule. As used herein, the term encompasses intact polyclonal antibodies, intact monoclonal antibodies, antibody fragments (such as Fab, Fab', F(ab')2, and Fv fragments), single chain Fv (scFv) antibodies, multispecific antibodies such as bispecific antibodies generated from at least two intact antibodies, bispecific antibodies, monospecific antibodies, monovalent antibodies, chimeric antibodies, humanized antibodies, human antibodies, fusion proteins comprising an antigen-binding site of an antibody, and any other modified immunoglobulin molecule comprising an antigen-binding site as long as the antibodies exhibit the desired biological activity. An antibody can be any of the five major classes of immunoglobulins: IgA, IgD, IgE, IgG, and IgM, or subclasses (isotypes) thereof (e.g., IgG1, IgG2, IgG3, IgG4, IgAl and IgA2), based on the identity of their heavy-chain constant domains referred to as alpha, delta, epsilon, gamma, and mu, respectively. The different classes of immunoglobulins have different and well-known subunit structures and three-dimensional configurations. Antibodies can be naked or conjugated to other molecules, including but not limited to, toxins and radioisotopes.

The term "antibody fragment" refers to a portion of an intact antibody and refers to the antigenic determining variable regions of an intact antibody. Examples of antibody fragments include, but are not limited to, Fab, Fab', F(ab')2, and Fv fragments, linear antibodies, single chain antibodies, and multispecific antibodies formed from antibody fragments. "Antibody fragment" as used herein comprises an antigen-binding site or epitope-binding site.

The term "variable region" of an antibody refers to the variable region of the antibody light chain, or the variable region of the antibody heavy chain, either alone or in combination. The variable regions of the heavy and light chain each consist of four framework regions (FR) connected by three complementarity determining regions (CDRs), also known as "hypervariable regions". The CDRs in each chain are held together in close proximity by the framework regions and, with the CDRs from the other chain, contribute to the formation of the antigen-binding sites of the antibody. There are at least two techniques for determining CDRs: (1) an approach based on cross-species sequence variability (i.e., Kabat et al., 1991, Sequences of Proteins of Immunological Interest, 5th Edition, National Institutes of Health, Bethesda MD.), and (2) an approach based on crystallographic studies of antigen-antibody complexes (Al-Lazikani et al., 1997, J. Mol. Biol., 273:927-948). In addition, combinations of these two approaches are sometimes used in the art to determine CDRs.

The term "monoclonal antibody" as used herein refers to a homogenous antibody population involved in the highly specific recognition and binding of a single antigenic determinant or epitope. This is in contrast to polyclonal antibodies that typically include a mixture of different antibodies directed against different antigenic determinants. The term "monoclonal antibody" encompasses both intact and full-length "monoclonal antibodies as well as antibody fragments (e.g., Fab, Fab', F(ab')2, Fv), single chain (scFv) antibodies, fusion proteins comprising an antibody portion or fragment, and any other modified immunoglobulin molecule comprising an antigen recognition site (antigen-binding site). Furthermore, "monoclonal antibody" refers to such antibodies made by any number of techniques, including but not limited to, hybridoma production, phage selection, recombinant expression, and transgenic animals.

The term "humanized antibody" as used herein refers to forms of non-human (e.g., murine) antibodies that are specific immunoglobulin chains, chimeric immunoglobulins, or fragments thereof that contain minimal non-human sequences. Typically, humanized antibodies are human immunoglobulins in which residues of the CDRs are replaced by residues from the CDRs of a non-human species (e.g., mouse, rat, rabbit, or hamster) that have the desired specificity, affinity, and/or binding capability (Jones et al., 1986, Nature, 321:522-525; Riechmann et al., 1988, Nature, 332:323-327; Verhoeyen et al., 1988, Science, 239:1534-1536). In some instances, the Fv framework region residues of a human immunoglobulin are replaced with the corresponding residues in an antibody from a non-human species that has the desired specificity, affinity, and/or binding capability. The humanized antibody can be further modified by the substitution of additional residues either in the Fv framework region and/or within the replaced non-human residues to refine and optimize antibody specificity, affinity, and/or binding capability. The humanized antibody may comprise substantially all of at least one, and typically two or three, variable domains containing all or substantially all of the CDRs that correspond to the non-human immunoglobulin whereas all or substantially all of the framework regions are those of a human immunoglobulin consensus sequence. The humanized antibody can also comprise at least a portion or fragment of an immunoglobulin constant region or domain (Fc), typically that of a human immunoglobulin.

The term "human antibody" as used herein refers to an antibody produced by a human or an antibody having an amino acid sequence corresponding to an antibody produced by a human made using any of the techniques known in the art. This definition of a human antibody specifically excludes a humanized antibody comprising non-human antigen-binding residues.

The term "chimeric antibody" as used herein refers to an antibody wherein the amino acid sequence of the immunoglobulin molecule is derived from two or more species. Typically, the variable region of both light and heavy chains corresponds to the variable region of antibodies derived from one species of mammals (e.g., mouse, rat, rabbit, etc.) with the desired specificity, affinity, and/or binding capability, while the constant regions are homologous to the sequences in antibodies derived from another species (usually human) to avoid eliciting an immune response in that species.

The phrase "affinity matured antibody" as used herein refers to an antibody with one or more alterations in one or more CDRs thereof that result in an improvement in the affinity of the antibody for antigen as compared to a parent antibody that does not possess those alterations(s). Preferred affinity matured antibodies will have nanomolar or even picomolar affinities for the target antigen. Affinity matured antibodies are produced by procedures known in the art. For example, Marks et al., 1992, Bio/Technology 10:779-783, describes affinity maturation by VH and VL domain shuffling. Random mutagenesis of CDR and/or framework residues is described by Barbas et al., 1994, PNAS, 91:3809-3813; Schier et al., 1995, Gene, 169:147-155; Yelton et al., 1995, J. Immunol. 155:1994-2004; Jackson et al., 1995, J. lmmunol., 154:3310-9; and Hawkins et al., 1992, J. Mol. Biol., 226:889-896.

As used herein the term "soluble receptor" refers to an extracellular fragment of a receptor protein that can be secreted from a cell in soluble form. The term "soluble receptor" encompasses a molecule comprising the entire extracellular domain, or a portion or fragment of the extracellular domain.

The terms "epitope" and "antigenic determinant" are used interchangeably herein and refer to that portion of an antigen capable of being recognized and specifically bound by a particular antibody. When the antigen is a polypeptide, epitopes can be formed both from contiguous amino acids and noncontiguous amino acids juxtaposed by tertiary folding of a protein. Epitopes formed from contiguous amino acids (also referred to as linear epitopes) are typically retained upon protein denaturing, whereas epitopes formed by tertiary folding (also referred to as conformational epitopes) are typically lost upon protein denaturing. An epitope typically includes at least 3, and more usually, at least 5, 6, 7, or 8-10 amino acids in a unique spatial conformation.

The terms "selectively binds" or "specifically binds" mean that a binding agent reacts or associates more frequently, more rapidly, with greater duration, with greater affinity, or with some combination of the above to the epitope, protein, or target molecule than with alternative substances, including unrelated proteins. In certain embodiments "specifically binds" means, for instance, that a binding agent binds a protein or target with a K_{D} of about 0.1mM or less, but more usually less than about 1µM. In certain embodiments, "specifically binds" means that a binding agent binds a target with a K_{D} of at least about 0.1µM or less, at least about 0.01µM or less, or at least about 1nM or less. Because of the sequence identity between homologous proteins in different species, specific binding can include a binding agent that recognizes a protein or target in more than one species. Likewise, because of homology within certain regions of polypeptide sequences of different proteins, specific binding can include a binding agent that recognizes more than one protein or target. It is understood that, in certain embodiments, a binding agent that specifically binds a first target may or may not specifically bind a second target. As such, "specific binding" does not necessarily require (although it can include) exclusive binding, i.e. binding to a single target. Thus, a binding agent may, in certain embodiments, specifically bind more than one target. In certain embodiments, multiple targets may be bound by the same antigen-binding site on the binding agent. For example, an antibody may, in certain instances, comprise two identical antigen-binding sites, each of which specifically binds the same epitope on two or more proteins. In certain alternative embodiments, an antibody may be bispecific and comprise at least two antigen-binding sites with differing specificities. By way of non-limiting example, a bispecific antibody may comprise one antigen-binding site that recognizes an epitope on one protein and further comprise a second, different antigen-binding site that recognizes a different epitope on a second protein. Generally, but not necessarily, reference to binding means specific binding.

The terms "polypeptide" and "peptide" and "protein" are used interchangeably herein and refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids), as well as other modifications known in the art. It is understood that, because the polypeptides of this invention may be based upon antibodies, in certain embodiments, the polypeptides can occur as single chains or associated chains.

The terms "polynucleotide" and "nucleic acid" and "nucleic acid molecule" are used interchangeably herein and refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase.

"Conditions of high stringency" may be identified by those that: (1) employ low ionic strength and high temperature for washing, for example 15mM sodium chloride/1.5mM sodium citrate (1x SSC) with 0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 in 5x SSC (0.75M NaCl, 75mM sodium citrate) at 42°C; or (3) employ 50% formamide, 5x SSC, 50mM sodium phosphate (pH 6.8), 0.1% sodium pyrophosphate, 5x Denhardt's solution, sonicated salmon sperm DNA (50µg/ml), 0.1% SDS, and 10% dextran sulfate at 42°C, with washes in 0.2x SSC containing 50% formamide at 55°C, followed by a high-stringency wash consisting of 0.1x SSC containing EDTA at 55°C.

The terms "identical" or percent "identity" in the context of two or more nucleic acids or polypeptides, refer to two or more sequences or subsequences that are the same or have a specified percentage of nucleotides or amino acid residues that are the same, when compared and aligned (introducing gaps, if necessary) for maximum correspondence, not considering any conservative amino acid substitutions as part of the sequence identity. The percent identity may be measured using sequence comparison software or algorithms or by visual inspection. Various algorithms and software that may be used to obtain alignments of amino acid or nucleotide sequences are well-known in the art. These include, but are not limited to, BLAST, ALIGN, Megalign, BestFit, GCG Wisconsin Package, and variants thereof. In some embodiments, two nucleic acids or polypeptides of the invention are substantially identical, meaning they have at least 70%, at least 75%, at least 80%, at least 85%, at least 90%, and in some embodiments at least 95%, 96%, 97%, 98%, 99% nucleotide or amino acid residue identity, when compared and aligned for maximum correspondence, as measured using a sequence comparison algorithm or by visual inspection. In some embodiments, identity exists over a region of the sequences that is at least about 10, at least about 20, at least about 40-60 residues, at least about 60-80 residues in length or any integral value there between. In some embodiments, identity exists over a longer region than 60-80 residues, such as at least about 80-100 residues, and in some embodiments the sequences are substantially identical over the full length of the sequences being compared, such as the coding region of a nucleotide sequence.

A "conservative amino acid substitution" is one in which one amino acid residue is replaced with another amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). For example, substitution of a phenylalanine for a tyrosine is a conservative substitution. Preferably, conservative substitutions in the sequences of the polypeptides and/or antibodies of the invention do not abrogate the binding of the polypeptide or antibody containing the amino acid sequence, to the antigen(s), i.e., the one or more IgCAM protein(s) to which the polypeptide or antibody binds. Methods of identifying nucleotide and amino acid conservative substitutions which do not eliminate antigen binding are well-known in the art.

The term "vector" as used herein means a construct, which is capable of delivering, and usually expressing, one or more gene(s) or sequence(s) of interest in a host cell. Examples of vectors include, but are not limited to, viral vectors, naked DNA or RNA expression vectors, plasmid, cosmid, or phage vectors, DNA or RNA expression vectors associated with cationic condensing agents, and DNA or RNA expression vectors encapsulated in liposomes.

A polypeptide, antibody, polynucleotide, vector, cell, or composition which is "isolated" is a polypeptide, antibody, polynucleotide, vector, cell, or composition which is in a form not found in nature. Isolated polypeptides, antibodies, polynucleotides, vectors, cells, or compositions include those which have been purified to a degree that they are no longer in a form in which they are found in nature. In some embodiments, a polypeptide, antibody, polynucleotide, vector, cell, or composition which is isolated is substantially pure.

The term "substantially pure" as used herein refers to material which is at least 50% pure (i.e., free from contaminants), at least 90% pure, at least 95% pure, at least 98% pure, or at least 99% pure.

The terms "cancer" and "cancerous" as used herein refer to or describe the physiological condition in mammals in which a population of cells are characterized by unregulated cell growth. Examples of cancer include, but are not limited to, carcinoma, blastoma, sarcoma, and hematologic cancers such as lymphoma and leukemia.

The terms "tumor" and "neoplasm" as used herein refer to any mass of tissue that results from excessive cell growth or proliferation, either benign (noncancerous) or malignant (cancerous) including pre-cancerous lesions.

The term "metastasis" as used herein refers to the process by which a cancer spreads or transfers from the site of origin to other regions of the body with the development of a similar cancerous lesion at a new location. A "metastatic" or "metastasizing" cell is one that loses adhesive contacts with neighboring cells and migrates via the bloodstream or lymph from the primary site of disease to invade neighboring body structures.

The terms "cancer stem cell" and "CSC" and "tumor stem cell" and "tumor initiating cell" are used interchangeably herein and refer to cells from a cancer or tumor that: (1) have extensive proliferative capacity; 2) are capable of asymmetric cell division to generate one or more types of differentiated cell progeny wherein the differentiated cells have reduced proliferative or developmental potential; and (3) are capable of symmetric cell divisions for self-renewal or self-maintenance. These properties confer on the cancer stem cells the ability to form or establish a tumor or cancer upon serial transplantation into an immunocompromised host (e.g., a mouse) compared to the majority of tumor cells that fail to form tumors. Cancer stem cells undergo self-renewal versus differentiation in a chaotic manner to form tumors with abnormal cell types that can change over time as mutations occur.

The terms "cancer cell" and "tumor cell" refer to the total population of cells derived from a cancer or tumor or pre-cancerous lesion, including both non-tumorigenic cells, which comprise the bulk of the cancer cell population, and tumorigenic stem cells (cancer stem cells). As used herein, the terms "cancer cell" or "tumor cell" will be modified by the term "non-tumorigenic" when referring solely to those cells lacking the capacity to renew and differentiate to distinguish those tumor cells from cancer stem cells.

The term "tumorigenic" as used herein refers to the functional features of a cancer stem cell including the properties of self-renewal (giving rise to additional tumorigenic cancer stem cells) and proliferation to generate all other tumor cells (giving rise to differentiated and thus non-tumorigenic tumor cells).

The term "tumorigenicity" as used herein refers to the ability of a random sample of cells from the tumor to form palpable tumors upon serial transplantation into immunocompromised hosts (e.g., mice).

The term "subject" refers to any animal (e.g., a mammal), (including, but not limited to, humans, non-human primates, canines, felines, rodents, and the like, which is to be the recipient of a particular treatment. Typically, the terms "subject" and "patient" are used interchangeably herein in reference to a human subject.

The term "pharmaceutically acceptable" refers to a product or compound approved (or approvable) by a regulatory agency of the Federal or a state government or listed in the U.S. Pharmacopeia or other generally recognized pharmacopeia for use in animals, including humans.

The terms "pharmaceutically acceptable excipient, carrier or adjuvant" or "acceptable pharmaceutical carrier" refer to an excipient, carrier or adjuvant that can be administered to a subject, together with at least one binding agent (e.g., an antibody) of the present disclosure, and which does not destroy the pharmacological activity thereof and is nontoxic when administered in doses sufficient to deliver a therapeutic effect.

The terms "effective amount" or "therapeutically effective amount" or "therapeutic effect" refer to an amount of a binding agent, an antibody, polypeptide, polynucleotide, small organic molecule, or other drug effective to "treat" a disease or disorder in a subject such as, a mammal. In the case of cancer, the therapeutically effective amount of a drug (e.g., an antibody) has a therapeutic effect and as such can reduce the number of cancer cells; decrease tumorigenicity, tumorigenic frequency or tumorigenic capacity; reduce the number or frequency of cancer stem cells; reduce the tumor size; reduce the cancer cell population; inhibit or stop cancer cell infiltration into peripheral organs including, for example, the spread of cancer into soft tissue and bone; inhibit and stop tumor or cancer cell metastasis; inhibit and stop tumor or cancer cell growth; relieve to some extent one or more of the symptoms associated with the cancer; reduce morbidity and mortality; improve quality of life; or a combination of such effects. To the extent the agent, for example an antibody, prevents growth and/or kills existing cancer cells, it can be referred to as cytostatic and/or cytotoxic.

The terms "treating" or "treatment" or "to treat" or "alleviating" or "to alleviate" refer to both (1) therapeutic measures that cure, slow down, lessen symptoms of, and/or halt progression of a diagnosed pathologic condition or disorder and (2) prophylactic or preventative measures that prevent or slow the development of a targeted pathologic condition or disorder. Thus those in need of treatment include those already with the disorder; those prone to have the disorder; and those in whom the disorder is to be prevented. In some embodiments, a subject is successfully "treated" according to the methods of the present invention if the patient shows one or more of the following: a reduction in the number of or complete absence of cancer cells; a reduction in the tumor size; inhibition of or an absence of cancer cell infiltration into peripheral organs including the spread of cancer cells into soft tissue and bone; inhibition of or an absence of tumor or cancer cell metastasis; inhibition or an absence of cancer growth; relief of one or more symptoms associated with the specific cancer; reduced morbidity and mortality; improvement in quality of life; reduction in tumorigenicity; reduction in the number or frequency of cancer stem cells; or some combination of effects.

As used in the present disclosure and claims, the singular forms "a", "an" and "the" include plural forms unless the context clearly dictates otherwise.

It is understood that wherever embodiments are described herein with the language "comprising" otherwise analogous embodiments described in terms of "consisting of" and/or "consisting essentially of" are also provided. It is also understood that wherever embodiments are described herein with the language "consisting essentially of" otherwise analogous embodiments described in terms of "consisting of" are also provided.

The term "and/or" as used in a phrase such as "A and/or B" herein is intended to include both A and B; A or B; A (alone); and B (alone). Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

### II. Agents that modulate the Hippo pathway

The present invention provides agents that modulate the Hippo pathway. The Hippo pathway is modulated by cell-cell contact, implying that there are cell surface receptors that direct and/or control Hippo pathway signaling events. The identity of these receptors in mammalian cells has not been elucidated. A central characteristic of epithelial cell biology is that epithelial cells exist in single-cell layers. As such, they have three distinct surfaces, an apical surface exposed to the lumen, a basolateral membrane that interacts with the basement membrane, and an "intercellular surface" forming the interaction region between adjacent cells. We hypothesized that putative Hippo pathway receptors would generally be restricted to this third surface, the intercellular surface, as this would be the likely region to enable direct cell-cell communication. Many proteins, including cell adhesion molecules (CAMs) are involved in interactions of cells with each other and with their microenvironment. Some of these proteins are known to reside within the intercellular membrane region, including cadherins which contribute to adherens junctions, connexins which contribute to gap junctions, and claudins and occludin which contribute to tight junctions. In addition to these proteins, other proteins are thought to reside in the apical junctional complex created by the tight junctions and adherens junctions.

Interestingly, many of these intercellular surface proteins have been identified as receptors for a variety of viruses. It is likely that viruses have evolved to exploit the localization of these receptors within the intercellular interface, which may allow for enhanced viral spreading between cells without exposure of the viruses to the lumen where they would be more vulnerable to attack and clearance by the immune system. Among these proteins are CAR (or CXAR) which is a receptor for coxsackie virus and adenovirus, PVR which is the poliovirus receptor, PVRL1 which is a receptor for herpes virus, PVRL4 which is a receptor for measles virus, and junction adhesion molecule (JAM1 or JAM-A) which is a receptor for reovirus. See, for example, Barton et al., 2001, Cell 104:441-451; Gonzalez-Mariscal et al., 2009, Front. Biosci., 14:731-768; Muhlebach et at., 2011, Nature 480:530-533.

Many of the intercellular surface receptors are members of the immunoglobulin superfamily of cell adhesion molecules (IgCAMs) and are generally related in both structure and function. Most of these proteins are type 1 transmembrane proteins, which typically consist of an extracellular domain (ECD) which contains one or more Ig-like domains, a single transmembrane domain, and a cytoplasmic tail. IgCAM members mediate interactions through their N-terminal Ig-like domains, which commonly bind other Ig-like domains on an opposing cell surface (homophilic adhesion), and also interact with integrins and carbohydrates (heterophilic adhesion) (Wong et al., 2012, Int. J. Cell Biol.*;* epub). Interestingly, many of the IgCAMs molecules are also expressed on immune cells suggesting that immunosurveillance mechanisms might be triggered if their normal localization within the intercellular interface is altered. In seeking to identify receptors that modulate the Hippo pathway, we hypothesized that this particular subset of the immunoglobulin superfamily would be well-positioned to mediate intercellular signaling in the Hippo pathway.

Thus, in some embodiments, agents that modulate the Hippo pathway specifically bind the extracellular domain of a cell adhesion molecule of the immunoglobulin superfamily (IgCAM). These agents are referred to herein as "IgCAM-binding agents". In some embodiments, the IgCAM-binding agents are antibodies. In some embodiments, the IgCAM-binding agents are soluble receptors. In some embodiments, the IgCAM-binding agents comprise soluble receptors. In some embodiments, the IgCAM-binding agents are polypeptides. In some embodiments, the IgCAM-binding agents are fusion proteins. In some embodiments, the IgCAM-binding agents are small molecules. In some embodiments, the IgCAM-binding agents are small peptides. In certain embodiments, the IgCAM-binding agent binds at least one IgCAM selected from the JAM family, the PVR family, and/or the CADM family. In certain embodiments, the IgCAM-binding agent binds at least one IgCAM selected from the JAM family. In certain embodiments, the IgCAM-binding agent binds at least one IgCAM selected from the PVR family. In certain embodiments, the IgCAM-binding agent binds at least one IgCAM selected from the CADM family. In certain embodiments, the IgCAM-binding agent binds at least one IgCAM selected from the group consisting of AMICA, CAR, CLMP, ESAM, GPA33, VSIG1, VSIG2, VSIG3, VSIG4. VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In certain embodiments, the IgCAM-binding agent binds at least one IgCAM selected from the group consisting of: AMICA, CAR, CLMP, ESAM, GPA33, VSIG1, VSIG2, VSIG3, VSIG4, VSIG8, JAM1, JAM2, JAM3, CADM2, CADM3, CADM4, CRTAM, TMIGD1, PVR, PVRL1, PVRL2, PVRL3, PVRL4, PRIG, CD200, CD200R1, CD200R1L, CD226, CD96, TIGIT, and TMEM25. In some embodiments, the IgCAM-binding agent binds CLMP, VSIG4, VSIG8, JAM2, JAM3, CADM1, CADM3, CADM4, PVRL1, PVRL3, PVRL4, TIGIT, TMIGD1, and/or TMEM25. In some embodiments, the IgCAM-binding agent binds CADM1, CADM3, PVRL1 and/or PVRL3. In some embodiments, the IgCAM-binding agent binds CADM3, PVRL1 and/or PVRL3. In some embodiments, the IgCAM-binding agent binds CADM3. In some embodiments, the IgCAM-binding agent does not bind CADM1. In certain embodiments, the IgCAM-binding agent specifically binds more than one IgCAM. The full-length amino acid (aa) sequences for many human IgCAMs are known in the art and several are provided herein as SEQ ID NO:1 (AMICA), SEQ ID NO:2 (CAR), SEQ ID NO:3 (CLMP), SEQ ID NO:4 (ESAM), SEQ ID NO:5 (GPA33), SEQ ID NO:6 (JAM1), SEQ ID NO:7 (JAM2), SEQ ID NO:8 (JAM3), SEQ ID NO:9 (VSIG1), SEQ ID NO:10 (VSIG2), SEQ ID NO:11 (VSIG3), SEQ ID NO:12 (VSIG4), SEQ ID NO:13 (VSIG8), SEQ ID NO:14 (CADM1), SEQ ID NO:15 (CADM2), SEQ ID NO:16 (CADM3), SEQ ID NO:17 (CADM4), SEQ ID NO:18 (CRTAM), SEQ ID NO:19 (TMIGD1), SEQ ID NO:20 (CD96), SEQ ID NO:21 (CD200), SEQ ID NO:22 (CD200R1), SEQ ID NO:23 (CD200R1L), SEQ ID NO:24 (CD226), SEQ ID NO:25 (PVRIG), SEQ ID NO:26 (PVR), SEQ ID NO:27 (PVRL1), SEQ ID NO:28 (PVRL2), SEQ ID NO:29 (PVRL3), SEQ ID NO:30 (PVRL4), SEQ ID NO:31 (TIGIT), and SEQ ID NO:32 (TMEM25).

In some embodiments, the IgCAM-binding agent is an agonist of at least one IgCAM. In certain embodiments, the IgCAM-binding agent is an agonist of at least one IgCAM selected from the JAM family, the PVR family, and/or the CADM family. In certain embodiments, the IgCAM-binding agent is an agonist of at least one IgCAM selected from the JAM family. In certain embodiments, the IgCAM-binding agent is an agonist of at least one IgCAM selected from the PVR family. In certain embodiments, the IgCAM-binding agent is an agonist of at least one IgCAM selected from the CADM family. In certain embodiments, the IgCAM-binding agent is an agonist of at least one IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In certain embodiments, the IgCAM-binding agent is an agonist of at least one IgCAM selected from the group consisting of: AMICA, CAR, CLMP, ESAM, GPA33, VSIG1, VSIG2, VSIG3, VSIG4, VSIG8, JAM1, JAM2, JAM3, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In some embodiments, the IgCAM-binding agent is an agonist of CLMP, VSIG4, VSIG8, JAM2, JAM3, CADM3, CADM4, PVRL1, PVRL3, PVRL4, TIGIT, TMIGD1, and/or TMEM25. In some embodiments, the IgCAM-binding agent is an agonist of CADM1, CADM3, PVRL1 and/or PVRL3. In some embodiments, the IgCAM-binding agent is an agonist of CADM3, PVRL1 and/or PVRL3. In some embodiments, the IgCAM-binding agent is an agonist of CADM3.

In certain embodiments, the IgCAM-binding agent is an agonist of the Hippo pathway. In some embodiments, the IgCAM-binding agent activates Hippo pathway signaling. In some embodiments, the IgCAM-binding agent induces Hippo pathway signaling. In some embodiments, the IgCAM-binding agent enhances Hippo pathway signaling. In some embodiments, the IgCAM-binding agent inhibits YAP activation. In some embodiments, the IgCAM-binding agent inhibits YAP translocation to the nucleus. In some embodiments, the IgCAM-binding agent enhances or increases YAP phosphorylation. In some embodiments, the IgCAM-binding agent enhances or increases YAP degradation. In some embodiments, the IgCAM-binding agent enhances or increases YAP retention or sequestration in the cell cytoplasm. In some embodiments, the IgCAM-binding agent reduces expression of YAP-dependent genes or proteins encoded by YAP-dependent genes. The term "YAP-dependent gene(s)" refers to genes whose expression is modulated (e.g., increased or decreased) by YAP. This term is used to reflect either the collective group of genes, subsets, or individual genes whose expression is modulated by the combination of YAP and a transcription factor. In some embodiments, the IgCAM-binding agent inhibits TAZ activation. In some embodiments, the IgCAM-binding agent inhibits TAZ translocation to the nucleus. In some embodiments, the IgCAM-binding agent enhances or increases TAZ phosphorylation. In some embodiments, the IgCAM-binding agent enhances or increases TAZ degradation. In some embodiments, the IgCAM-binding agent enhances or increases TAZ retention in the cell cytoplasm. In some embodiments, the IgCAM-binding agent reduces expression ofTAZ-dependent genes or proteins encoded by TAZ-dependent genes. The term "TAZ-dependent gene(s)" refers to genes whose expression is modulated (e.g., increased or decreased) by TAZ. This term is used to reflect either the collective group of genes, subsets, or individual genes whose expression is modulated by the combination of TAZ and a transcription factor. In some embodiments, the IgCAM-binding agent reduces expression of at least one gene selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GLI2, BIRC2, BIRC5 (survivin), FGF1, IL33, GRB2, IGFBP3, AREG, FN1, ADAMTS1, ADAMTS5, AXL, MET, CYR61, IL8, ZEB1, FOXC2, N-eadherin, and SNAIL.

In certain embodiments, the IgCAM-binding agent enhances contact-dependent growth inhibition. In certain embodiments, the IgCAM-binding agent reduces cell proliferation. In certain embodiments, the IgCAM-binding agent inhibits anchorage-independent growth.

In some embodiments, the IgCAM-binding agent is an antagonist of at least one IgCAM. In certain embodiments, the IgCAM-binding agent is an antagonist of at least one IgCAM selected from the JAM family, the PVR family, and/or the CADM family. In certain embodiments, the IgCAM-binding agent is an antagonist of at least one IgCAM selected from the JAM family. In certain embodiments, the IgCAM-binding agent is an antagonist of at least one IgCAM selected from the PVR family. In certain embodiments, the IgCAM-binding agent is an antagonist of at least one IgCAM selected from the CADM family. In certain embodiments, the IgCAM-binding agent is an antagonist of at least one IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In certain embodiments, the IgCAM-binding agent is an antagonist of at least one IgCAM selected from the group consisting of: AMICA, CAR, CLMP, ESAM, GPA33, VSIG1, VSIG2, VSIG3, VSIG4, VSIG8, JAM1, JAM2, JAM3, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In some embodiments, the IgCAM-binding agent is an antagonist of CLMP, VSIG4, VSIG8, JAM2, JAM3, CADM3, CADM4, PVRL1, PVRL3, PVRL4, TIGIT, TMIGD1, and/or TMEM25. In some embodiments, the IgCAM-binding agent is an antagonist of CADM1, CADM3, PVRL1 and/or PVRL3. In some embodiments, the IgCAM-binding agent is an antagonist of CADM3, PVRL1 and/or PVRL3. In some embodiments, the IgCAM-binding agent is an antagonist of CADM3.

In some embodiments, the IgCAM-binding agent is an antagonist of the Hippo pathway. In some embodiments, the IgCAM-binding agent decreases Hippo pathway signaling. In some embodiments, the IgCAM-binding agent inhibits Hippo pathway signaling. In some embodiments, the IgCAM-binding agent suppresses Hippo pathway signaling. In some embodiments, the IgCAM-binding agent increases YAP activation. In some embodiments, the IgCAM-binding agent promotes YAP translocation to the nucleus. In some embodiments, the IgCAM-binding agent suppresses or decreases YAP phosphorylation. In some embodiments, the IgCAM-binding agent suppresses or decreases YAP degradation. In some embodiments, the IgCAM-binding agent suppresses or decreases YAP retention in the cell cytoplasm. In some embodiments, the IgCAM-binding agent increases TAZ activation. In some embodiments, the IgCAM-binding agent promotes TAZ translocation to the nucleus. In some embodiments, the IgCAM-binding agent suppresses or decreases TAZ phosphorylation. In some embodiments, the IgCAM-binding agent suppresses or decreases TAZ degradation. In some embodiments, the IgCAM-binding agent suppresses or decreases TAZ retention in the cell cytoplasm. In some embodiments, the IgCAM-binding agent increases expression of genes suppressed by the Hippo signaling pathway. In some embodiments, the IgCAM-binding agent increases expression of at least one gene selected from the group consisting of: CD44, CD47, CD133, TDGF1, EPHB2, CA12, LRP4, GPC4, CLDN2, CTGF, PAG1, SEMA4D, RHEB, MAGI I, ITPR3, CD168, NRP2, GLI2, BIRC2, BIRC5 (survivin), FGF1, IL33, GRB2, IGFBP3, AREG, FN1, ADAMTS1, AXL, ADAMTS5, MET, CYR61, IL8, ZEB1, FOXC2, N-cadherin, and SNAIL.

In some embodiments, the IgCAM-binding agent binds at least one IgCAM and interferes with the interaction of the IgCAM with a second protein. In some embodiments, the IgCAM-binding agent binds at least one IgCAM and interferes with the interaction of the IgCAM with a second IgCAM. In some embodiments, the IgCAM-binding agent is an antibody that interferes with the interaction of at least one IgCAM with at least one second IgCAM. In some embodiments, the IgCAM-binding agent comprises an antibody that interferes with the interaction of at least one IgCAM with at least one second IgCAM. In some embodiments, the IgCAM-binding agent is a soluble receptor that interferes with the interaction of at least one IgCAM with at least one second IgCAM. In some embodiments, the IgCAM-binding agent comprises a soluble receptor that interferes with the interaction of at least one IgCAM with at least one second IgCAM. In some embodiments, the IgCAM-binding agent is a small molecule that interferes with the interaction of at least one IgCAM with at least one second IgCAM. In some embodiments, the IgCAM-binding agent is a small peptide that interferes with the interaction of at least one IgCAM with at least one second IgCAM.

In some embodiments, the IgCAM-binding agent binds at least one IgCAM protein with a dissociation constant (K_{D}) of about 1µM or less, about 100nM or less, about 40nM or less, about 20nM or less, about 10nM or less, about 1nM or less, or about 0.1nM or less. In some embodiments, an IgCAM-binding agent binds an IgCAM with a K_{D} of about 1nM or less. In some embodiments, an IgCAM-binding agent binds an IgCAM with a K_{D} of about 0.1nM or less. In certain embodiments, an IgCAM-binding agent described herein binds at least one additional IgCAM. In some embodiments, an IgCAM-binding agent binds a human IgCAM with a K_{D} of about 0.1nM or less. In some embodiments, the IgCAM-binding agent binds both a human IgCAM and a mouse IgCAM with a K_{D} of about 10nM or less. In some embodiments, an IgCAM-binding agent binds both a human IgCAM and a mouse IgCAM with a K_{D} of about 1nM or less. In some embodiments, an IgCAM-binding agent binds both a human IgCAM and a mouse IgCAM with a K_{D} of about 0.1nM or less. In some embodiments, the dissociation constant of the binding agent to an IgCAM is the dissociation constant determined using an IgCAM fusion protein comprising at least a portion or fragment of the IgCAM immobilized on a Biacore chip.

In some embodiments, the IgCAM-binding agent binds a human IgCAM with a half maximal effective concentration (EC₅₀) of about 1µM or less, about 100nM or less, about 40nM or less, about 20nM or less, about 10nM or less, about 1nM or less, or about 0.1nM or less. In certain embodiments, an IgCAM-binding agent also binds at least one additional IgCAM with an EC₅₀ of about 40nM or less, about 20nM or less, about 10nM or less, about 1nM or less or about 0.1nM or less.

In some embodiments, the IgCAM-binding agent is an antibody. In some embodiments, the antibody is a recombinant antibody. In some embodiments, the antibody is a monoclonal antibody. In some embodiments, the antibody is a chimeric antibody. In some embodiments, the antibody is a humanized antibody. In some embodiments, the antibody is a human antibody. In certain embodiments, the antibody is an IgG1 antibody. In certain embodiments, the antibody is an IgG2 antibody. In certain embodiments, the antibody is an antibody fragment comprising an antigen-binding site. In some embodiments, the antibody is monovalent. In some embodiments, the antibody is bivalent. In some embodiments, the antibody is monospecific. In some embodiments, the antibody is bispecific or multispecific. In some embodiments, the antibody is conjugated to a cytotoxic moiety. In some embodiments, the antibody is isolated. In some embodiments, the antibody is substantially pure.

In some embodiments, the IgCAM-binding agents are polyclonal antibodies. Polyclonal antibodies can be prepared by any known method. In some embodiments, polyclonal antibodies are raised by immunizing an animal (e.g., a rabbit, rat, mouse, goat, or donkey) by multiple subcutaneous or intraperitoneal injections of the relevant antigen (e.g., a purified peptide fragment, full-length recombinant protein, or fusion protein). The antigen can be optionally conjugated to a carrier such as keyhole limpet hemocyanin (KLH) or serum albumin. The antigen (with or without a carrier protein) is diluted in sterile saline and usually combined with an adjuvant (e.g., Complete or Incomplete Freund's Adjuvant) to form a stable emulsion. After a sufficient period of time, polyclonal antibodies are recovered from blood, ascites, and the like, of the immunized animal. The polyclonal antibodies can be purified from serum or ascites according to standard methods in the art including, but not limited to, affinity chromatography, ion-exchange chromatography, gel electrophoresis, and dialysis.

In some embodiments, the IgCAM-binding agents are monoclonal antibodies. Monoclonal antibodies can be prepared using hybridoma methods known to one of skill in the art (see e.g., Kohler and Milstein, 1975, Nature, 256:495-497). In some embodiments, using the hybridoma method, a mouse, hamster, or other appropriate host animal, is immunized as described above to elicit from lymphocytes the production of antibodies that will specifically bind the immunizing antigen. In some embodiments, lymphocytes can be immunized *in vitro.* In some embodiments, the immunizing antigen can be a human protein or a portion or fragment thereof. In some embodiments, the immunizing antigen can be a mouse protein or a portion or fragment thereof.

Following immunization, lymphocytes are isolated and fused with a suitable myeloma cell line using, for example, polyethylene glycol, to form hybridoma cells that can then be selected away from unfused lymphocytes and myeloma cells. Hybridomas that produce monoclonal antibodies directed specifically against a chosen antigen may be identified by a variety of methods including, but not limited to, immunoprecipitation, immunoblotting, and *in vitro* binding assay (e.g., flow cytometry, FACS, ELISA, and radioimmunoassay). The hybridomas can be propagated either in *in vitro* culture using standard methods (J.W. Goding, 1996, Monoclonal Antibodies: Principles and Practice, 3rd Edition, Academic Press, San Diego, CA) or *in vivo* as ascites tumors in an animal. The monoclonal antibodies can be purified from the culture medium or ascites fluid according to standard methods in the art including, but not limited to, affinity chromatography, ion-exchange chromatography, gel electrophoresis, and dialysis.

In certain embodiments, monoclonal antibodies can be made using recombinant DNA techniques as known to one skilled in the art (see e.g., U.S. Patent No. 4,816,567). The polynucleotides encoding a monoclonal antibody are isolated from mature B-cells or hybridoma cells, such as by RT-PCR using oligonucleotide primers that specifically amplify the genes encoding the heavy and light chains of the antibody, and their sequence is determined using conventional techniques. The isolated polynucleotides encoding the heavy and light chains are then cloned into suitable expression vectors which produce the monoclonal antibodies when transfected into host cells such as E. coli, simian COS cells, Chinese hamster ovary (CHO) cells, or myeloma cells that do not otherwise produce immunoglobulin proteins. In other embodiments, recombinant monoclonal antibodies, or fragments thereof, can be isolated from phage display libraries expressing CDRs and/or variable regions of the desired species (see e.g., McCafferty et al., 1990, Nature, 348:552-554; Clackson et al., 1991, Nature, 352:624-628; and Marks et al., 1991, J. Mol. Biol., 222:581-597).

The polynucleotide(s) encoding a monoclonal antibody can further be modified in a number of different manners using recombinant DNA technology to generate alternative antibodies. In some embodiments, the constant domains of the light and heavy chains of, for example, a mouse monoclonal antibody can be substituted for those regions of, for example, a human antibody to generate a chimeric antibody, or for a non-immunoglobulin polypeptide to generate a fusion antibody. In some embodiments, the constant regions are truncated or removed to generate the desired antibody fragment of a monoclonal antibody. Site-directed or high-density mutagenesis of the variable region(s) can be used to optimize specificity, affinity, etc. of a monoclonal antibody.

In some embodiments, the monoclonal antibody against a human IgCAM is a humanized antibody. Typically, humanized antibodies are human immunoglobulins in which residues from the CDRs are replaced by residues from a CDR of a non-human species (e.g., mouse, rat, rabbit, hamster, etc.) that have the desired specificity, affinity, and/or binding capability using methods known to one skilled in the art. In some embodiments, the Fv framework region residues of a human immunoglobulin are replaced with the corresponding residues in an antibody from a non-human species that has the desired specificity, affinity, and/or binding capability. In some embodiments, the humanized antibody can be further modified by the substitution of additional residues either in the Fv framework region and/or within the replaced non-human residues to refine and optimize antibody specificity, affinity, and/or capability. The humanized antibody may comprise substantially all of at least one, and typically two or three, variable domain regions containing all, or substantially all, of the CDRs that correspond to the non-human immunoglobulin whereas all, or substantially all, of the framework regions are those of a human immunoglobulin consensus sequence. In some embodiments, the humanized antibody can also comprise at least a portion or fragment of an immunoglobulin constant region or domain (Fc), typically that of a human immunoglobulin. In certain embodiments, such humanized antibodies are used therapeutically because they may reduce antigenicity and HAMA (human anti-mouse antibody) responses when administered to a human subject. One skilled in the art would be able to obtain a functional humanized antibody with reduced immunogenicity following known techniques (see e.g., U.S. Patent Nos. 5,225,539; 5,585,089; 5,693,761; and 5,693,762).

In some embodiments, the IgCAM-binding agent is a human antibody. Human antibodies can be directly prepared using various techniques known in the art. In some embodiments, immortalized human B lymphocytes immunized *in vitro* or isolated from an immunized individual that produces an antibody directed against a target antigen can be generated (see, e.g., Cole et al., 1985, Monoclonal Antibodies and Cancer Therapy, Alan R. Liss, p. 77; Boemer et al., 1991, J. Immunol., 147:86-95; and U.S. Patent Nos. 5,750,373; 5,567,610 and 5,229,275). In some embodiments, the human antibody can be selected from a phage library, where that phage library expresses human antibodies (Vaughan et al., 1996, Nature Biotechnology, 14:309-314; Sheets et al., 1998, PNAS, 95:6157-6162; Hoogenboom and Winter, 1991, J. Mol. Biol., 227:381; Marks et al., 1991, J. Mol. Biol., 222:581). Alternatively, phage display technology can be used to produce human antibodies and antibody fragments *in vitro,* from immunoglobulin variable domain gene repertoires from unimmunized donors. Techniques for the generation and use of antibody phage libraries are also described in U.S. Patent Nos. 5,969,108; 6,172,197; 5,885,793; 6,521,404; 6,544,731; 6,555,313; 6,582,915; 6,593,081; 6,300,064; 6,653,068; 6,706,484; and 7,264,963; and Rothe et al., 2008, J. Mol. Bio., 376:1182-1200. Affinity maturation strategies including, but not limited to, chain shuffling (Marks et al., 1992, Bio/Technology, 10:779-783) and site-directed mutagenesis, are known in the art and may be employed to generate high affinity human antibodies.

In some embodiments, human antibodies can be made in transgenic mice that contain human immunoglobulin loci. These mice are capable, upon immunization, of producing the full repertoire of human antibodies in the absence of endogenous immunoglobulin production. This approach is described in U.S. Patent Nos. 5,545,807; 5,545,806; 5,569,825; 5,625,126; 5,633,425; and 5,661,016.

This invention also encompasses bispecific antibodies that specifically recognize at least one human IgCAM protein. Bispecific antibodies are capable of specifically recognizing and binding at least two different epitopes. The different epitopes can either be within the same molecule (e.g., two epitopes on one human IgCAM) or on different molecules (e.g., one epitope on a human IgCAM and one epitope on a second molecule). In some embodiments, the bispecific antibodies are monoclonal human or humanized antibodies. In some embodiments, the antibodies can specifically recognize and bind a first antigen target, (e.g., an IgCAM) as well as a second antigen target, such as an effector molecule on a leukocyte (e.g., CD2, CD3, CD28, CD80, or CD86) or a Fc receptor (e.g., CD64, CD32, or CD16) so as to focus cellular defense mechanisms to the cell expressing the first antigen target. In some embodiments, the antibodies can be used to direct cytotoxic agents to cells which express a particular target antigen. These antibodies possess an antigen-binding arm and an arm which binds a cytotoxic agent or a radionuclide chelator, such as EOTUBE, DPTA, DOTA, or TETA.

Techniques for making bispecific antibodies are known by those skilled in the art, see for example, Millstein et al., 1983, Nature, 305:537-539; Brennan et al., 1985, Science, 229:81; Suresh et al., 1986, Methods in Enzymol., 121:120; Traunecker et al., 1991, EMBO J., 10:3655-3659; Shalaby et al., 1992, J. Exp. Med., 175:217-225; Kostelny et al., 1992, J. Immunol., 148:1547-1553; Gruber et al., 1994, J Immunol., 152:5368; U.S. Patent No. 5,731,168; and U.S. Patent Publication No. 2011/0123532. Bispecific antibodies can be intact antibodies or antibody fragments. Antibodies with more than two valencies are also contemplated. For example, trispecific antibodies can be prepared (Tutt et al., 1991, J. Immunol., 147:60). Thus, in certain embodiments the antibodies to IgCAMs are multispecific.

In certain embodiments, the antibodies (or other polypeptides) described herein may be monospecific. For example, in certain embodiments, each of the one or more antigen-binding sites that an antibody contains is capable of binding (or binds) a homologous epitope on more than one IgCAM. In certain embodiments, an antigen-binding site of a monospecific antibody described herein is capable of binding (or binds), for example, PVR and PVRL1 (i.e., the same epitope is found on both PVR and PVRL1 proteins).

In certain embodiments, the IgCAM-binding agent is an antibody fragment. Antibody fragments may have different functions or capabilities than intact antibodies; for example, antibody fragments can have increased tumor penetration. Various techniques are known for the production of antibody fragments including, but not limited to, proteolytic digestion of intact antibodies. In some embodiments, antibody fragments include a F(ab')2 fragment produced by pepsin digestion of an antibody molecule. In some embodiments, antibody fragments include a Fab fragment generated by reducing the disulfide bridges of an F(ab')2 fragment. In other embodiments, antibody fragments include a Fab fragment generated by the treatment of the antibody molecule with papain and a reducing agent. In certain embodiments, antibody fragments are produced recombinantly. In some embodiments, antibody fragments include Fv or single chain Fv (scFv) fragments. Fab, Fv, and scFv antibody fragments can be expressed in and secreted from E. coli or other host cells, allowing for the production of large amounts of these fragments. In some embodiments, antibody fragments are isolated from antibody phage libraries as discussed herein. For example, methods can be used for the construction of Fab expression libraries (Huse et al., 1989, Science, 246:1275-1281) to allow rapid and effective identification of monoclonal Fab fragments with the desired specificity for an IgCAM or derivatives, fragments, analogs or homologs thereof. In some embodiments, antibody fragments are linear antibody fragments. In certain embodiments, antibody fragments are monospecific or bispecific. In certain embodiments, the IgCAM-binding agent is a scFv. Various techniques can be used for the production of single-chain antibodies specific to one or more human IgCAMs and are known to those of skill in the art.

It can further be desirable, especially in the case of antibody fragments, to modify an antibody in order to increase (or decrease) its serum half-life. This can be achieved, for example, by incorporation of a salvage receptor binding epitope into the antibody fragment by mutation of the appropriate region in the antibody fragment or by incorporating the epitope into a peptide tag that is then fused to the antibody fragment at either end or in the middle (e.g., by DNA or peptide synthesis).

Heteroconjugate antibodies are also within the scope of the present invention. Heteroconjugate antibodies are composed of two covalently joined antibodies. Such antibodies have, for example, been proposed to target immune cells to unwanted cells (U.S. Patent No. 4,676,980). It is also contemplated that the heteroconjugate antibodies can be prepared *in vitro* using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate.

For the purposes of the present invention, it should be appreciated that modified antibodies can comprise any type of variable region that provides for the association of the antibody with the target (i.e., a human IgCAM). In this regard, the variable region may comprise or be derived from any type of mammal that can be induced to mount a humoral response and generate immunoglobulins against the desired tumor associated antigen. As such, the variable region of the modified antibodies can be, for example, of human, murine, non-human primate (e.g. cynomolgus monkeys, macaques, etc.), or rabbit origin. In some embodiments, both the variable and constant regions of the modified immunoglobulins are human. In other embodiments, the variable regions of compatible antibodies (usually derived from a non-human source) can be engineered or specifically tailored to improve the binding properties or reduce the immunogenicity of the molecule. In this respect, variable regions useful in the present invention can be humanized or otherwise altered through the inclusion of imported amino acid sequences.

In certain embodiments, the variable domains in both the heavy and light chains are altered by at least partial replacement of one or more CDRs and, if necessary, by partial framework region replacement and sequence modification and/or alteration. Although the CDRs may be derived from an antibody of the same class or even subclass as the antibody from which the framework regions are derived, it is envisaged that the CDRs will be derived from an antibody of different class and preferably from an antibody from a different species. It may not be necessary to replace all of the CDRs with all of the CDRs from the donor variable region to transfer the antigen binding capacity of one variable domain to another. Rather, it may only be necessary to transfer those residues that are necessary to maintain the activity of the antigen-binding site.

Alterations to the variable region notwithstanding, those skilled in the art will appreciate that the modified antibodies of this invention will comprise antibodies (e.g., full-length antibodies or immunoreactive fragments thereof) in which at least a fraction of one or more of the constant region domains has been deleted or otherwise altered so as to provide desired biochemical characteristics such as increased tumor localization or increased serum half-life when compared with an antibody of approximately the same immunogenicity comprising a native or unaltered constant region. In some embodiments, the constant region of the modified antibodies will comprise a human constant region. Modifications to the constant region compatible with this invention comprise additions, deletions or substitutions of one or more amino acids in one or more domains. The modified antibodies disclosed herein may comprise alterations or modifications to one or more of the three heavy chain constant domains (CH1, CH2 or CH3) and/or to the light chain constant domain. In some embodiments, one or more domains are partially or entirely deleted from the constant regions of the modified antibodies. In some embodiments, the modified antibodies will comprise domain deleted constructs or variants wherein the entire CH2 domain has been removed (ΔCH2 constructs). In some embodiments, the omitted constant region domain is replaced by a short amino acid spacer (e.g., 10 amino acid residues) that provides some of the molecular flexibility typically imparted by the absent constant region.

In some embodiments, the modified antibodies are engineered to fuse the CH3 domain directly to the hinge region of the antibody. In other embodiments, a peptide spacer is inserted between the hinge region and the modified CH2 and/or CH3 domains. For example, constructs may be expressed wherein the CH2 domain has been deleted and the remaining CH3 domain (modified or unmodified) is joined to the hinge region with a 5-20 amino acid spacer. Such a spacer may be added to ensure that the regulatory elements of the constant domain remain free and accessible or that the hinge region remains flexible. However, it should be noted that amino acid spacers may, in some cases, prove to be immunogenic and elicit an unwanted immune response against the construct. Accordingly, in certain embodiments, any spacer added to the construct will be relatively non-immunogenic so as to maintain the desired biological qualities of the modified antibodies.

In some embodiments, the modified antibodies may have only a partial deletion of a constant domain or substitution of a few or even a single amino acid. For example, the mutation of a single amino acid in selected areas of the CH2 domain may be enough to substantially reduce Fc binding and thereby increase cancer cell localization and/or tumor penetration. Similarly, it may be desirable to simply delete the part of one or more constant region domains that controls a specific effector function (e.g. complement C1q binding) to be modulated. Such partial deletions of the constant regions may improve selected characteristics of the antibody (e.g., serum half-life) while leaving other desirable functions associated with the constant region intact. Moreover, as alluded to above, the constant regions of the disclosed antibodies may be modified through the mutation or substitution of one or more amino acids that enhances the function of the resulting construct. In this respect it may be possible to disrupt the activity provided by a conserved binding site (e.g., Fc binding) while substantially maintaining the configuration and immunogenic profile of the modified antibody. In certain embodiments, the modified antibodies comprise the addition of one or more amino acids to the constant region to enhance desirable characteristics such as decreasing or increasing effector function or provide for more cytotoxin or carbohydrate attachment sites.

It is known in the art that the constant region mediates several effector functions. For example, binding of the C1 component of complement to the Fc region of IgG or IgM antibodies (when the antibodies are bound to antigen) activates the complement system. Activation of complement is important in the opsonization and lysis of cell pathogens. The activation of complement also stimulates the inflammatory response and can be involved in autoimmune hypersensitivity. In addition, the Fc region of an antibody can bind a cell expressing a Fc receptor (FcR). There are a number of Fc receptors which are specific for different classes of antibody, including IgG (gamma receptors), IgE (epsilon receptors), IgA (alpha receptors) and IgM (mu receptors). Binding of antibody to Fc receptors on cell surfaces triggers a number of important and diverse biological responses including engulfment and destruction of antibody-coated particles, clearance of immune complexes, lysis of antibody-coated target cells by killer cells (called antibody-dependent cell cytotoxicity or ADCC), release of inflammatory mediators, placental transfer, and control of immunoglobulin production.

In certain embodiments, the IgCAM-binding antibodies provide for altered effector functions that, in turn, affect the biological profile of the administered antibody. For example, in some embodiments, the deletion or inactivation (through point mutations or other means) of a constant region domain may reduce Fc receptor binding of the circulating modified antibody thereby increasing cancer cell localization and/or tumor penetration. In other embodiments, the constant region modifications increase or reduce the serum half-life of the antibody. In some embodiments, the constant region is modified to eliminate disulfide linkages or oligosaccharide moieties. Modifications to the constant region in accordance with this invention may easily be made using biochemical or molecular engineering techniques well-known to the skilled artisan.

In certain embodiments, an IgCAM-binding agent that is an antibody does not have one or more effector functions. For instance, in some embodiments, the antibody has no ADCC activity, and/or no complement-dependent cytotoxicity (CDC) activity. In certain embodiments, the antibody does not bind an Fc receptor and/or complement factors. In certain embodiments, the antibody has no effector function.

The present invention further embraces variants and equivalents which are substantially homologous to the chimeric, humanized, and human antibodies, or antibody fragments thereof, set forth herein. These can contain, for example, conservative substitution mutations, i.e. the substitution of one or more amino acids by similar amino acids. For example, conservative substitution refers to the substitution of an amino acid with another within the same general class such as, for example, one acidic amino acid with another acidic amino acid, one basic amino acid with another basic amino acid, or one neutral amino acid by another neutral amino acid. What is intended by a conservative amino acid substitution is well known in the art and described herein.

Thus, the present invention provides methods for producing an antibody that binds at least one IgCAM. In some embodiments, the method for producing an antibody that binds at least one IgCAM comprises using hybridoma techniques. In some embodiments, a method for producing an antibody that binds the extracellular domain of a human IgCAM is provided. In some embodiments, a method for producing an antibody that binds the extracellular domain of a human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25 is provided. In some embodiments, a method for producing an antibody that binds the extracellular domain of a human IgCAM selected from the group consisting of: AMICA, CAR, CLMP, ESAM, GPA33, VSIG1, VSIG2, VSIG3, VSIG4, VSIG8, JAM1, JAM2, JAM3, CADM2, CADM3, CADM4, CRTAM, TMIGDI, PVR, PVRL1, PVRL2, PVRL3, PVRL4, PVRIG, CD200, CD200R1, CD200R1L, CD226, CD96, TIGIT, and TMEM25 is provided. In some embodiments, the method comprises using SEQ ID NO:33 or a portion or fragment thereof as an immunogen. In some the method comprises using SEQ ID NO:34 or a portion or fragment thereof as an immunogen. In some embodiments, the method comprises using SEQ ID NO:35 or a portion or fragment thereof as an immunogen. In some embodiments, the method comprises using SEQ ID NO:36 or a portion or fragment thereof as an immunogen. In some embodiments, the method comprises using SEQ ID NO:37 or a portion or fragment thereof as an immunogen. In some embodiments, the method comprises using SEQ ID NO:38 or a portion or fragment thereof as an immunogen. In some embodiments, the method comprises using SEQ ID NO:39 or a portion or fragment thereof as an immunogen. In some embodiments, the method comprises using SEQ ID NO:40 or a portion or fragment thereof as an immunogen. In some embodiments, the method comprises using SEQ ID NO:41 or a portion or fragment thereof as an immunogen. In some embodiments, the method comprises using SEQ ID NO:42 or a portion or fragment thereof as an immunogen. In some embodiments, the method comprises using SEQ ID NO:43 or a portion or fragment thereof as an immunogen. In some embodiments, the method comprises using SEQ ID NO:44 or a portion or fragment thereof as an immunogen. In some embodiments, the method comprises using SEQ ID NO:45 or a portion or fragment thereof as an immunogen. In some embodiments, the method comprises using SEQ ID NO:46 or a portion or fragment thereof as an immunogen. In some embodiments, the method comprises using SEQ ID NO:47 or a portion or fragment thereof as an immunogen. In some embodiments, the method comprises using SEQ ID NO:48 or a portion or fragment thereof as an immunogen. In some embodiments, the method comprises using SEQ ID NO:49 or a portion or fragment thereof as an immunogen. In some embodiments, the method comprises using SEQ ID NO:50 or a portion or fragment thereof as an immunogen. In some embodiments, the method comprises using SEQ ID NO:51 or a portion or fragment thereof as an immunogen. In some embodiments, the method comprises using SEQ ID NO:52 or a portion or fragment thereof as an immunogen. In some embodiments, the method comprises using SEQ ID NO:53 or a portion or fragment thereof as an immunogen. In some embodiments, the method comprises using SEQ ID NO:54 or a portion or fragment thereof as an immunogen. In some embodiments, the method comprises using SEQ ID NO:55 or a portion or fragment thereof as an immunogen. In some embodiments, the method comprises using SEQ ID NO:56 or a portion or fragment thereof as an immunogen. In some embodiments, the method comprises using SEQ ID NO:57 or a portion or fragment thereof as an immunogen. In some embodiments, the method comprises using SEQ ID NO:58 or a portion or fragment thereof as an immunogen. In some embodiments, the method comprises using SEQ ID NO:59 or a portion or fragment thereof as an immunogen. In some embodiments, the method comprises using SEQ ID NO:60 or a portion or fragment thereof as an immunogen. In some embodiments, the method comprises using SEQ ID NO:61 or a portion or fragment thereof as an immunogen. In some embodiments, the method comprises using SEQ ID NO:62 or a portion or fragment thereof as an immunogen. In some embodiments, the method comprises using SEQ ID NO:63 or a portion or fragment thereof as an immunogen. In some embodiments, the method comprises using SEQ ID NO:64 or a portion or fragment thereof as an immunogen.

In some embodiments, the method of generating an antibody that binds at least one human IgCAM comprises screening a human phage library. The present invention further provides methods of identifying an antibody that binds at least one IgCAM. In some embodiments, the antibody is identified by screening using FACS for binding to an IgCAM or a portion or fragment thereof. In some embodiments, the antibody is identified by screening using ELISA for binding to an IgCAM or a portion or fragment thereof. In some embodiments, the antibody is identified by screening for the effect on cell morphology in a clonogenic assay. In some embodiments, the antibody is identified by screening for the effect on cell growth and/or proliferation in a clonogenic assay. In some embodiments, the antibody is identified by screening for activation or enhancement of Hippo pathway signaling. In some embodiments, the antibody is identified by screening for inhibition of YAP activation. In some embodiments, the antibody is identified by screening for translocation of YAP. In some embodiments, the antibody is identified by screening for YAP phosphorylation. In some embodiments, the antibody is identified by screening for inhibition of TAZ activation. In some embodiments, the antibody is identified by screening for translocation of TAZ. In some embodiments, the antibody is identified by screening for TAZ phosphorylation.

In some embodiments, a method of generating an antibody to a human IgCAM comprises immunizing a mammal with a polypeptide comprising the extracellular domain of a human IgCAM. In some embodiments, a method of generating an antibody to a human IgCAM comprises immunizing a mammal with a polypeptide comprising at least a portion or fragment of the extracellular domain from AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, or TMEM25. In some embodiments, a method of generating an antibody to a human IgCAM comprises immunizing a mammal with a polypeptide comprising at least a portion or fragment of SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64. In some embodiments, the method further comprises isolating antibodies or antibody-producing cells from the mammal.

In some embodiments, a method of generating a monoclonal antibody which binds a human IgCAM comprises: (a) immunizing a mammal with a polypeptide comprising at least a portion or fragment of the extracellular domain from AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, or TMEM25; (b) isolating antibody-producing cells from the immunized mammal; (c) fusing the antibody-producing cells with cells of a myeloma cell line to form hybridoma cells. In some embodiments, the method further comprises (d) selecting a hybridoma cell expressing an antibody that binds at least one IgCAM.

In some embodiments, the antibody generated by the methods described herein is a Hippo pathway agonist. In some embodiments, the antibody generated by the methods described herein activates, increases, and/or enhances Hippo pathway signaling. In some embodiments, the antibody generated by the methods described herein increases YAP phosphorylation. In some embodiments, the antibody generated by the methods described herein increases YAP degradation. In some embodiments, the antibody generated by the methods described herein increases retention of YAP in the cytoplasm. In some embodiments, the antibody generated by the methods described herein inhibits YAP activation. In some embodiments, the antibody generated by the methods described herein inhibits YAP translocation to the nucleus. In some embodiments, the antibody generated by the methods described herein increases TAZ phosphorylation. In some embodiments, the antibody generated by the methods described herein increases TAZ degradation. In some embodiments, the antibody generated by the methods described herein increases retention of TAZ in the cytoplasm. In some embodiments, the antibody generated by the methods described herein inhibits TAZ activation. In some embodiments, the antibody generated by the methods described herein inhibits TAZ translocation to the nucleus.

In some embodiments, the antibody generated by the methods described herein is a Hippo pathway antagonist. In some embodiments, the antibody generated by the methods described herein inhibits, decreases, and/or suppresses Hippo pathway signaling. In some embodiments, the antibody generated by the methods described herein decreases YAP phosphorylation. In some embodiments, the antibody generated by the methods described herein decreases YAP degradation. In some embodiments, the antibody generated by the methods described herein decreases retention of YAP in the cytoplasm. In some embodiments, the antibody generated by the methods described herein increases or enhances YAP activation. In some embodiments, the antibody generated by the methods described herein increases or enhances YAP translocation to the nucleus. In some embodiments, the antibody generated by the methods described herein decreases TAZ phosphorylation. In some embodiments, the antibody generated by the methods described herein decreases TAZ degradation. In some embodiments, the antibody generated by the methods described herein decreases retention of TAZ in the cytoplasm. In some embodiments, the antibody generated by the methods described herein increases or enhances TAZ activation. In some embodiments, the antibody generated by the methods described herein increases or enhances TAZ translocation to the nucleus.

In some embodiments, a method of producing an antibody to at least one human IgCAM comprises identifying an antibody using a membrane-bound heterodimeric molecule comprising a single antigen-binding site. In some non-limiting embodiments, the antibody is identified using methods and polypeptides described in International Publication WO 2011/100566, which is incorporated by reference herein in its entirety.

In some embodiments, a method of producing an antibody to at least one human IgCAM comprises screening an antibody-expressing library for antibodies that bind at least one human IgCAM. In some embodiments, the antibody-expressing library is a phage library. In some embodiments, the antibody-expressing library is a mammalian cell display library. In some embodiments, the screening comprises panning. In some embodiments, the antibody-expressing library is screened using at least a portion or fragment of the extracellular domain of a human IgCAM. In some embodiments, the antibody-expressing library is screened using at least a portion or fragment of the extracellular domain of a human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In some embodiments, antibodies identified in the first screening, are screened again using a different IgCAM thereby identifying an antibody that binds more than one IgCAM.

In certain embodiments, the antibodies described herein are isolated. In certain embodiments, the antibodies described herein are substantially pure.

In certain embodiments, the IgCAM-binding agent is a soluble receptor. In certain embodiments, the IgCAM-binding agent comprises the extracellular domain of an IgCAM. In some embodiments, the IgCAM-binding agent comprises a fragment of the extracellular domain of an IgCAM (e.g., the N-terminal domain of an IgCAM). In some embodiments, soluble receptors comprising a fragment of the extracellular domain of an IgCAM can demonstrate altered biological activity (e.g., increased protein half-life) compared to soluble receptors comprising the entire IgCAM ECD. Protein half-life can be further increased by covalent modification with polyethylene glycol (PEG) or polyethylene oxide (PEO). In certain embodiments, the IgCAM is a human IgCAM. In certain embodiments, the IgCAM ECD or a fragment of the IgCAM ECD is a human IgCAM ECD selected from the JAM family, the PVR family, or the CADM family. In certain embodiments, the IgCAM ECD is a human IgCAM ECD selected from the JAM family. In certain embodiments, the IgCAM ECD is a human IgCAM ECD selected from the PVR family. In certain embodiments, the IgCAM ECD is a human IgCAM ECD selected from the CADM family. In certain embodiments, the human IgCAM ECD is an IgCAM ECD from AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, or TMEM25. In certain embodiments, the human IgCAM ECD is an IgCAM ECD from AMICA, CAR, CLMP, ESAM, GPA33, VSIG1, VSIG2, VSIG3, VSIG4, VSIG8, JAM1, JAM2, JAM3, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, or TMEM25. In some embodiments, the human IgCAM ECD is an IgCAM ECD from CLMP, VSIG4, VSIG8, JAM2, JAM3, CADM3, CADM4, PVRL1, PVRL3, PVRL4, TIGIT, TMIGD1, and/or TMEM25. In some embodiments, the human IgCAM ECD is an IgCAM ECD from CADM I, CADM3, PVRL1 and/or PVRL3. In some embodiments, the human IgCAM ECD is an IgCAM ECD from CADM3, PVRL1 and/or PVRL3. In some embodiments the human IgCAM ECD is an IgCAM ECD from CADM3. In some embodiments, the human IgCAM ECD is not from CADM1.

The predicted ECD domains for AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25 are provided as SEQ ID NOs:33-64. Those of skill in the art may differ in their understanding of the exact amino acids corresponding to the various ECD domains. Thus, the N-terminus and/or C-terminus of the ECDs described herein may extend or be shortened by 1, 2, 3, 4, 5, 6, 7, 8, 9, 10 or more amino acids.

In some embodiments, the IgCAM-binding agent comprises a sequence selected from the group consisting of: SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO-61, SEQ ID NO:62, SEQ ID NO:63, and SEQ ID NO:64. In some embodiments, the IgCAM-binding agent comprises a fragment of a sequence selected from the group consisting of: SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, and SEQ ID NO:64.

In certain embodiments, the IgCAM-binding agent comprises a variant of any one of the aforementioned IgCAM ECD sequences that comprises one or more (e.g., one, two, three, four, five, six, seven, eight, nine, ten, etc.) conservative substitutions and is capable of binding IgCAM(s).

In some embodiments, the IgCAM-binding agent, such as a soluble receptor comprising the ECD of a human IgCAM, is a fusion protein. As used herein, a "fusion protein" is a hybrid protein expressed by a nucleic acid molecule comprising nucleotide sequences of at least two genes. In certain embodiments, the IgCAM-binding agent, such as a soluble receptor comprising the ECD of a human IgCAM, further comprises a non-IgCAM polypeptide. In some embodiments, IgCAM soluble receptors may include an IgCAM ECD linked to other non-IgCAM functional and structural polypeptides including, but not limited to, a human Fc region, protein tags (e.g., myc, FLAG, GST), other endogenous proteins or protein fragments, or any other useful protein sequence including any linker region between an IgCAM ECD and a second polypeptide. In certain embodiments, the non-IgCAM polypeptide is a human Fc region. The Fc region can be obtained from any of the classes of immunoglobulin, IgG, IgA, IgM, IgD and IgE. In some embodiments, the Fc region is a human IgG1 Fc region. In some embodiments, the Fc region is a human IgG2 Fc region. In some embodiments, the Fc region is a wild-type Fc region. In some embodiments, the Fc region is a natural variant of a wild-type Fc region. In some embodiments, the Fc region is a mutated Fc region. In some embodiments, the Fc region is truncated at the N-terminal end by 1, 2, 3, 4, 5, 6, 7, 8, 9, or 10 amino acids, (e.g., in the hinge domain). In some embodiments, the Fc region is truncated at the C-terminal end (e.g., lysine is absent). In some embodiments, an amino acid in the hinge domain is changed to hinder undesirable disulfide bond formation. In some embodiments, a cysteine is replaced with a different amino acid to hinder undesirable disulfide bond formation. In some embodiments, a cysteine is replaced with a serine to hinder undesirable disulfide bond formation. In certain embodiments, the non-IgCAM polypeptide comprises SEQ ID NO:65, SEQ ID NO:66, or SEQ ID NO:67. In certain embodiments, the non-IgCAM polypeptide consists essentially of SEQ ID NO:65, SEQ ID NO:66, or SEQ ID NO:67.

In certain embodiments, an IgCAM-binding agent is a fusion protein comprising at least a or fragment of an IgCAM ECD and a Fc region. In some embodiments, the C-terminus of the IgCAM ECD is linked to the N-terminus of the immunoglobulin Fc region. In some embodiments, the IgCAM ECD is directly linked to the Fc region (i.e. without an intervening peptide linker). In some embodiments, the IgCAM ECD is linked to the Fc region via a peptide linker.

As used herein, the term "linker" refers to a linker inserted between a first polypeptide (e.g., a IgCAM ECD or portion or fragment thereof) and a second polypeptide (e.g., a Fc region). In some embodiments, the linker is a peptide linker. Linkers should not adversely affect the expression, secretion, or bioactivity of the fusion protein. Linkers should not be antigenic and should not elicit an immune response. Suitable linkers are known to those of skill in the art and often include mixtures of glycine and serine residues and often include amino acids that are sterically unhindered. Other amino acids that can be incorporated into useful linkers include threonine and alanine residues. Linkers can range in length, for example from 1-50 amino acids in length, 1-22 amino acids in length, 1-10 amino acids in length, 1-5 amino acids in length, or 1-3 amino acids in length. Linkers may include, but are not limited to, SerGly, GGSG, GSGS, GGGS, S(GGS)n where n is 1-7, GRA, poly(Gly), poly(Ala), ESGGGGVT (SEQ ID NO:74), LESGGGGVT (SEQ ID NO:75), GRAQVT (SEQ ID NO:76), WRAQVT (SEQ ID NO:77), and ARGRAQVT (SEQ ID NO:78). As used herein, a linker is an intervening peptide sequence that does not include amino acid residues from either the C-terminus of the first polypeptide (e.g., an IgCAM ECD) or the N-terminus of the second polypeptide (e.g., the Fc region).

In some embodiments, the IgCAM-binding agent comprises a first polypeptide comprising SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64; and a second polypeptide comprising SEQ ID NO:65, SEQ ID NO:66, or SEQ ID NO:67, wherein the first polypeptide is directly linked to the second polypeptide.

In some embodiments, the IgCAM-binding agent comprises a first polypeptide comprising SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64; and a second polypeptide comprising SEQ ID NO:65, SEQ ID NO:66, or SEQ ID NO:67, wherein the first polypeptide is connected to the second polypeptide by a linker.

In some embodiments, the IgCAM-binding agent comprises a first polypeptide comprising a portion or fragment of SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64; and a second polypeptide comprising SEQ ID NO:65, SEQ ID NO:66, or SEQ ID NO:67, wherein the first polypeptide is directly linked to the second polypeptide.

In some embodiments, the IgCAM-binding agent comprises a first polypeptide comprising a portion or fragment of SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64; and a second polypeptide comprising SEQ ID NO:65, SEQ ID NO:66, or SEQ ID NO:67, wherein the first polypeptide is connected to the second polypeptide by a linker.

In some embodiments, the IgCAM-binding agent comprises a first polypeptide that is at least 80% identical to SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64; and a second polypeptide comprising SEQ ID NO:65, SEQ ID NO:66, or SEQ ID NO:67, wherein the first polypeptide is directly linked to the second polypeptide. In some embodiments, the first polypeptide is at least 85%, at least 90%, or at least 95% identical to SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64.

In some embodiments, the IgCAM-binding agent comprises a first polypeptide that is at least 80% identical to SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64; and a second polypeptide comprising SEQ ID NO:65, SEQ ID NO:66, or SEQ ID NO:67, wherein the first polypeptide is connected to the second polypeptide by a linker. In some embodiments, the first polypeptide is at least 85%, at least 90%, or at least 95% identical to SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, or SEQ ID NO:64.

IgCAMs generally contain a signal sequence that directs the transport of the proteins. Signal sequences (also referred to as signal peptides or leader sequences) are located at the N-terminus of nascent polypeptides. They target the polypeptide to the endoplasmic reticulum and the proteins are sorted to their destinations, for example, to the inner space of an organelle, to an interior membrane, to the cell outer membrane, or to the cell exterior via secretion. Most signal sequences are cleaved from the protein by a signal peptidase after the proteins are transported to the endoplasmic reticulum. The cleavage of the signal sequence from the polypeptide usually occurs at a specific site in the amino acid sequence and is dependent upon amino acid residues within the signal sequence. Although there is usually one specific cleavage site, more than one cleavage site may be recognized and/or used by a signal peptidase resulting in a non-homogenous N-terminus of the polypeptide. For example, the use of different cleavage sites within a signal sequence can result in a polypeptide expressed with different N-terminal amino acids. Accordingly, in some embodiments, the polypeptides as described herein may comprise a mixture of polypeptides with different N-termini. In some embodiments, the N-termini differ in length by 1, 2, 3, 4, 5, 6, 7, 8, 9, 10, or more amino acids. In some embodiments, the N-termini differ in length by 1, 2, 3, 4, or 5 amino acids. In some embodiments, the polypeptide is substantially homogeneous, i.e., the polypeptides have the same N-terminus. In some embodiments, the signal sequence of the polypeptide comprises one or more (e.g., one, two, three, four, five, six, seven, eight, nine, ten, etc.) amino acid substitutions and/or deletions. In some embodiments, the signal sequence of the polypeptide comprises amino acid substitutions and/or deletions that allow one cleavage site to be dominant, thereby resulting in a substantially homogeneous polypeptide with one N-terminus. In some embodiments, the signal sequence of the polypeptide is not a native IgCAM signal sequence.

In certain embodiments, an IgCAM-binding agent comprises a Fc region of an immunoglobulin. Those skilled in the art will appreciate that some of the binding agents of this invention will comprise fusion proteins in which at least a portion of the Fc region has been deleted or otherwise altered so as to provide desired biochemical characteristics, such as increased cancer cell localization, increased tumor penetration, reduced serum half-life, or increased serum half-life, when compared with a fusion protein of approximately the same immunogenicity comprising a native or unaltered constant region. Modifications to the Fc region may include additions, deletions, or substitutions of one or more amino acids in one or more domains. The modified fusion proteins disclosed herein may comprise alterations or modifications to one or more of the two heavy chain constant domains (CH2 or CH3) or to the hinge region. In other embodiments, the entire CH2 domain may be removed (ΔCH2 constructs). In some embodiments, the omitted constant region domain is replaced by a short amino acid spacer (e.g., 10 aa residues) that provides some of the molecular flexibility typically imparted by the absent constant region domain.

In some embodiments, the modified fusion proteins are engineered to link the CH3 domain directly to the hinge region or to the first polypeptide. In other embodiments, a peptide spacer is inserted between the hinge region or the first polypeptide and the modified CH2 and/or CH3 domains. For example, constructs may be expressed wherein the CH2 domain has been deleted and the remaining CH3 domain (modified or unmodified) is joined to the hinge region or first polypeptide with a 5-20 amino acid spacer. Such a spacer may be added to ensure that the regulatory elements of the constant domain remain free and accessible or that the hinge region remains flexible. However, it should be noted that amino acid spacers may, in some cases, prove to be immunogenic and elicit an unwanted immune response against the construct. Accordingly, in certain embodiments, any spacer added to the construct will be relatively non-immunogenic so as to maintain the desired biological qualities of the fusion protein.

In some embodiments, the modified fusion proteins may have only a partial deletion of a constant domain or substitution of a few or even a single amino acid. For example, the mutation of a single amino acid in selected areas of the CH2 domain may be enough to substantially reduce Fc binding and thereby increase cancer cell localization and/or tumor penetration. Similarly, it may be desirable to simply delete that part of one or more constant region domains that control a specific effector function (e.g., complement C1q binding). Such partial deletions of the constant regions may improve selected characteristics of the binding agent (e.g., serum half-life) while leaving other desirable functions associated with the subject constant region domain intact. Moreover, as alluded to above, the constant regions of the disclosed fusion proteins may be modified through the mutation or substitution of one or more amino acids that enhances the profile of the resulting construct. In this respect it may be possible to disrupt the activity provided by a conserved binding site (e.g., Fc binding) while substantially maintaining the configuration and immunogenic profile of the modified fusion protein. In certain embodiments, the modified fusion proteins comprise the addition of one or more amino acids to the constant region to enhance desirable characteristics such as decreasing or increasing effector function, or provide for more cytotoxin or carbohydrate attachment sites.

It is known in the art that the constant region mediates several effector functions. For example, binding of the C1 component of complement to the Fc region of IgG or IgM antibodies (bound to antigen) activates the complement system. Activation of complement is important in the opsonization and lysis of cell pathogens. The activation of complement also stimulates the inflammatory response and can also be involved in autoimmune hypersensitivity. In addition, the Fc region can bind to a cell expressing a Fc receptor (FcR). There are a number of Fc receptors which are specific for different classes of antibody, including IgG (gamma receptors), IgE (epsilon receptors), IgA (alpha receptors) and IgM (mu receptors).

In some embodiments, the modified fusion proteins provide for altered effector functions that, in turn, affect the biological profile of the administered agent. For example, in some embodiments, the deletion or inactivation (through point mutations or other means) of a constant region domain may reduce Fc receptor binding of the circulating modified agent, thereby increasing cancer cell localization and/or tumor penetration. In other embodiments, the constant region modifications increase or reduce the serum half-life of the agent. In some embodiments, the constant region is modified to eliminate disulfide linkages or oligosaccharide moiety attachment sites.

In certain embodiments, a modified fusion protein does not have one or more effector functions normally associated with an Fc region. In some embodiments, the agent has no ADCC activity, and/or no CDC activity. In certain embodiments, the agent does not bind to the Fc receptor and/or complement factors. In certain embodiments, the agent has no effector function.

The IgCAM-binding agents (e.g., antibodies or soluble receptors) of the present invention can be assayed for specific binding by any method known in the art. The immunoassays which can be used include, but are not limited to, competitive and non-competitive assay systems using techniques such as Biacore analysis, FACS analysis, immunofluorescence, immunocytochemistry, Western blots, radioimmunoassays, ELISA, "sandwich" immunoassays, immunoprecipitation assays, precipitation reactions, gel diffusion precipitin reactions, immunodiffusion assays, agglutination assays, complement-fixation assays, immunoradiometric assays, fluorescent immunoassays, and protein A immunoassays. Such assays are routine and well-known in the art (see, e.g., Ausubel et al., Editors, 1994-present, Current Protocols in Molecular Biology, John Wiley & Sons, Inc., New York, NY).

For example, the specific binding of an IgCAM-binding agent (e.g., an antibody or a soluble receptor) to a human IgCAM such as PVR may be determined using ELISA. An ELISA assay comprises preparing antigen, coating wells of a 96 well microtiter plate with antigen, adding the IgCAM-binding agent conjugated to a detectable compound such as an enzymatic substrate (e.g. horseradish peroxidase or alkaline phosphatase) to the well, incubating for a period of time and detecting the presence of the antibody bound to the antigen. In some embodiments, the IgCAM-binding agent is not conjugated to a detectable compound, but instead a second conjugated antibody that recognizes the IgCAM-binding agent is added to the well. In some embodiments, instead of coating the well with the antigen, the IgCAM-binding agent can be coated to the well and a second antibody conjugated to a detectable compound can be added following the addition of the antigen to the coated well. One of skill in the art would be knowledgeable as to the parameters that can be modified to increase the signal detected as well as other variations of ELISAs known in the art.

In another example, the specific binding of an IgCAM-binding agent (e.g., an antibody or a soluble receptor) to a human IgCAM may be determined using FACS. A FACS screening assay may comprise generating a cDNA construct that expresses an antigen as a fusion protein (e.g., PVR-CD4TM), transfecting the construct into cells, expressing the antigen on the surface of the cells, mixing the IgCAM-binding agent with the transfected cells, and incubating for a period of time. The cells bound by the IgCAM-binding agent may be identified by using a secondary antibody conjugated to a detectable compound (e.g., PE-conjugated anti-Fc antibody) and a flow cytometer. One of skill in the art would be knowledgeable as to the parameters that can be modified to optimize the signal detected as well as other variations of FACS that may enhance screening (e.g., screening for blocking antibodies).

The binding affinity of an IgCAM-binding agent (e.g., an antibody or a soluble receptor) to an antigen/target (e.g., an IgCAM) and the off-rate of a binding agent-antigen/target interaction can be determined by competitive binding assays. One example of a competitive binding assay is a radioimmunoassay comprising the incubation of labeled antigen/target (e.g., ³H or ¹²⁵I), or fragment or variant thereof, with the binding agent of interest in the presence of increasing amounts of unlabeled antigen/target followed by the detection of the binding agent bound to the labeled antigen/target. The affinity of the binding agent for an antigen/target (e.g., an IgCAM) and the binding off-rates can be determined from the data by Scatchard plot analysis. In some embodiments, Biacore kinetic analysis is used to determine the binding on and off rates of binding agents that bind an antigen/target (e.g., an IgCAMs). Biacore kinetic analysis comprises analyzing the binding and dissociation of binding agents from chips with immobilized antigen/target (e.g., an IgCAM) on the chip surface.

In certain embodiments, the IgCAM-binding agent (e.g., an antibody or a soluble receptor) described herein binds at least one human IgCAM and modulates Hippo pathway activity. In some embodiments, the IgCAM-binding agent is an agonist of the Hippo pathway and activates and/or increases Hippo pathway signaling. In some embodiments, the IgCAM-binding agent is an agonist of the Hippo pathway and increases YAP phosphorylation. In some embodiments, the IgCAM-binding agent is an agonist of the Hippo pathway and increases YAP degradation. In some embodiments, the IgCAM-binding agent is an agonist of the Hippo pathway and reduces expression of YAP-dependent genes and/or proteins encoded by YAP-dependent genes. In some embodiments, the expression of the YAP-dependent gene is modulated by the combination of YAP and a transcription factor selected from the group consisting of: TEAD1, TEAD2, TEAD3, TEAD4, Smad1, Smad4, RUNX, ErbB4, and p73. As described herein, examples of YAP-dependent genes include, but are not limited to, CD44, CD47, CD133, TDGF1, EPHB2, CA12, LRP4, GPC4, CLDN2, GTGF, PAG1, SEMA4D, RHEB, MAG11, ITPR3, CD168, NRP2, Gli2, BIRC2, BIRC5, FGF1, IL33, GRB2, IGFBP3, and AREG. In some embodiments, the IgCAM-binding agent is an agonist of the Hippo pathway and increases TAZ phosphorylation. In some embodiments, the IgCAM-binding agent is an agonist of the Hippo pathway and increases TAZ degradation. In some embodiments, the IgCAM-binding agent is an agonist of the Hippo pathway and reduces expression of TAZ-dependent genes. In some embodiments, the expression of the TAZ-dependent gene is modulated by the combination of TAZ and a transcription factor selected from the group consisting of: TEAD1, TEAD2, TEAD3, TEAD4, RUNX, ERBB4, SMAD2/3/4, PAX3, TBX5, TTF-1, PPARγ, and p73. Examples of TAZ-dependent genes include, but are not limited to, CTGF, CD44, FN1, BIRC5 (survivin), ADAMTSI, GLI2, AXL, ADAMTS5, MET, CYR61, IL8, ZEB1, FOXC2, N-cadherin, and SNAIL. In some embodiments, the IgCAM-binding agent is an agonist of the Hippo pathway and modulates (e.g., increases or decreases) expression of at least one gene selected from the group consisting of: CD44, CD47, CD133, TDGF1, EPHB2, CA12, LRP4, GPC4, CLDN2, CTGF, PAG1, SEMA4D, RHEB, MAG11, ITPR3, CD168, NRP2, GLI2, BIRC2, BIRC5 (survivin), FGF1, IL33, GRB2, IGFBP3, AREG, FN1, ADAMTS1, AXL, ADAMTS5, MET, CYR61, IL8, ZEB1, FOXC2, N-cadherin, and SNAIL.

In certain embodiments, the IgCAM-binding agent (e.g., an antibody or a soluble receptor) is an agonists of at least one human IgCAM. In some embodiments, the IgCAM-binding agent is an agonist of at least one IgCAM and activates and/or increases Hippo pathway activity. In certain embodiments, the IgCAM-binding agent increases Hippo pathway activity by at least about 10%, at least about 20%, at least about 30%, at least about 50%, at least about 75%, at least about 90%, or about 100%, In some embodiments, the IgCAM-binding agent increases activity of one, two, three, four, or more IgCAMs. In some embodiments, the IgCAM-binding agent increases activity of at least one of AMIGA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, or TMEM25.

In certain embodiments, the IgCAM-binding agent (e.g., an antibody or a soluble receptor) inhibits cell growth or cell proliferation. In certain embodiments, the IgCAM-binding agent inhibits cell growth or cell proliferation by at least about 10%, at least about 20%, at least about 30%, at least about 50%, at least about 75%, at least about 90%, or about 100%. In certain embodiments, the IgCAM-binding agent (e.g., an antibody or a soluble receptor) affects cell growth or cell morphology in a clonogenic assay. In some embodiments, the IgCAM-binding agent is selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGHT, or TMEM25.

In certain embodiments, the IgCAM-binding agent is an antagonist of the Hippo pathway. In some embodiments, the IgCAM-binding agent decreases Hippo pathway signaling. In some embodiments, the IgCAM-binding inhibits Hippo pathway signaling. In some embodiments, the IgCAM-binding agent suppresses Hippo pathway signaling. In some embodiments, the IgCAM-binding agent increases YAP activation. In some embodiments, the IgCAM-binding agent increases YAP translocation to the nucleus. In some embodiments, the IgCAM-binding agent suppresses or decreases YAP phosphorylation. In some embodiments, the IgCAM-binding agent suppresses or decreases YAP degradation. In some embodiments, the IgCAM-binding agent suppresses or decreases YAP retention in the cell cytoplasm. In some embodiments, the IgCAM-binding agent increases TAZ activation. In some embodiments, the IgCAM-binding agent increases TAZ translocation to the nucleus. In some embodiments, the IgCAM-binding agent suppresses or decreases TAZ phosphorylation. In some embodiments, the IgCAM-binding agent suppresses or decreases TAZ degradation. In some embodiments, the IgCAM-binding agent suppresses or decreases TAZ retention in the cell cytoplasm. In some embodiments, the IgCAM-binding agent reduces expression of genes in the Hippo signaling pathway. In some embodiments, the IgCAM-binding agent is an antagonist of the Hippo pathway and modulates (e.g., increases or decreases) expression of at least one gene selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GL12, BIRC2, BIRC5 (survivin), FGFI, IL33, GRB2, IGFBP3, AREG, FN1, ADAMTSI, AXL, ADAMTS5, MET, CYR61, IL8, ZEB1, FOXC2, N-cadherin, and SNAIL.

In certain embodiments, the IgCAM-binding agent (e.g., an antibody or a soluble receptor) is an antagonist of at least one human IgCAM. In some embodiments the IgCAM-binding agent is an antagonist of at least one IgCAM and suppresses and/or inhibits Hippo pathway activity. In certain embodiments, the IgCAM-binding agent decreases Hippo pathway activity by at least about 10%, at least about 20%, at least about 30%, at least about 50%, at least about 75%, at least about 90%, or about 100%. In some embodiments, the IgCAM-binding agent decreases activity of one, two, three, four, or more IgCAM. In some embodiments, the IgCAM-binding agent decreases activity of at least one of AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, or TMEM25.

In certain embodiments, the IgCAM-binding agent (e.g., an antibody or a soluble receptor) increases cell growth or cell proliferation. In certain embodiments, the IgCAM-binding agent increases cell growth or cell proliferation by at least about 10%, at least about 20%, at least about 30%, at least about 50%, at least about 75%, at least about 90%, or about 100%. In some embodiments, the IgCAM-binding agent is selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, or TMEM25.

In certain embodiments, the IgCAM-binding agent (e.g., an antibody or a soluble receptor) is an antagonist of YAP activity. In some embodiments, the IgCAM-binding agent is an antagonist of YAP activity by increasing phosphorylation of YAP. In some embodiments, the IgCAM-binding agent is an antagonist of YAP activity by increasing cytoplasmic retention or sequestration of YAP. In some embodiments, the IgCAM-binding agent is an antagonist of YAP activity by decreasing nuclear translocation of YAP. In some embodiments, the IgCAM-binding agent is an antagonist of YAP activity by increasing degradation of YAP. In certain embodiments, the IgCAM-binding agent inhibits YAP activity by at least about 10%, at least about 20%, at least about 30%, at least about 50%, at least about 75%, at least about 90%, or about 100%.

In certain embodiments, the IgCAM-binding agent (e.g., an antibody or a soluble receptor) is an antagonist of TAZ activity. In some embodiments, the IgCAM-binding agent is an antagonist of TAZ activity by increasing phosphorytation of TAZ. In some embodiments, the IgCAM-binding agent is an antagonist of TAZ activity by increasing cytoplasmic retention or sequestration of TAZ. In some embodiments, the IgCAM-binding agent is an antagonist of TAZ activity by decreasing nuclear translocation of TAZ. In some embodiments, the IgCAM-binding agent is an antagonist of TAZ activity by increasing degradation of TAZ. In certain embodiments, the IgCAM-binding agent inhibits TAZ activity by at least about 10%, at least about 20%, at least about 30%, at least about 50%, at least about 75%, at least about 90%, or about 100%. In certain embodiments, the IgCAM-binding agent (e.g., an antibody or a soluble receptor) increases Hippo pathway signaling. In certain embodiments, Hippo pathway signaling by one or more IgCAMs selected from the group consisting of: AMICA, CAR, CLMP, ESAM, GPA33, VSIG1, VSIG2, VSIG43, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25 is increased. In certain embodiments, the increase of Hippo pathway signaling by an IgCAM-binding agent results in an increase in the level of Hippo pathway signaling of at least about 10%, at least about 25%, at least about 50%, at least about 75%, at least about 90%, or at least about 95%. In certain embodiments, the increase of Hippo pathway signaling by an IgCAM-binding agent results in an increase in the amount of phosphorylated YAP of at least about 10%, at least about 25%, at least about 50%, at least about 75%, at least about 90%, or at least about 95%. In certain embodiments, the increase of Hippo pathway signaling by an IgCAM-binding agent results in a decrease in the amount of non-phosphorylated YAP of at least about 10%, at least about 25%, at least about 50%, at least about 75%, at least about 90%, or at least about 95%.

In certain embodiments, the increase of Hippo pathway signaling by an IgCAM-binding agent results in an increase in the amount of phaspharylated TAZ of at least about 10%, at least about 25%, at least about 50%, at least about 75%, at least about 90%, or at least about 95%. In certain embodiments, the increase of Hippo pathway signaling by an IgCAM-binding agent results in a decrease in the amount of non-phosphorylated TAZ of at least about 10%, at least about 25%, at least about 50%, at least about 75%, at least about 90%, or at least about 95%.

In certain embodiments, the IgCAM-binding agent (e.g., an antibody or soluble receptor) inhibits activation of YAP and/or TAZ. It is understood that an IgCAM-binding agent that inhibits activation of YAP and/or TAZ may, in certain embodiments, inhibit activation of YAP and/or TAZ by one or more IgCAMs, but not necessarily inhibit activation of YAP and/or TAZ by all IgCAMs. In certain embodiments, activation of YAP and/or TAZ is inhibited by one or more IgCAMs selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25. In certain embodiments, the inhibition of activation of YAP by an IgCAM-binding agent results in a reduction in the level of activation of YAP and/or TAZ of at least about 10%, at least about 25%, at least about 50%, at least about 75%, at least about 90%, or at least about 95%. In certain embodiments, the inhibition of activation of YAP and/or TAZ by an IgCAM-binding agent results in an increase in the amount of phosphorylated YAP and/or TAZ of at least about 10%, at least about 25%, at least about 5 0%, at least about 75%, at least about 90%, or at least about 95%. In certain embodiments, the inhibition of activation of YAP and/or TAZ by an IgCAM-binding agent results in a decrease in the amount of non-phosphorylated YAP of at least about 10%, at least about 25%, at least about 50%, at least about 75%, at least about 90%, or at least about 95%.

In certain embodiments, the IgCAM-binding agent (e.g., an antibody or a soluble receptor) reduces expression of at least one YAP-dependent gene. In certain embodiments, the IgCAM-binding agent reduces expression of at least one protein encoded by a YAP-dependent gene. In certain embodiments, the IgCAM-binding agent (e.g., an antibody or a soluble receptor) reduces expression of at least one TAZ-dependent gene. In certain embodiments, the IgCAM-binding agent reduces expression of at least one protein encoded by a TAAZ-dependent gene. In certain embodiments, one or more IgCAMs selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25 reduces expression of at least one YAP-dependent gene or reduces expression of at least one TAZ-dependent gene. In some embodiments, the IgCAM-binding agent reduces expression of at least one gene selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GLI2, BIRC2, BIRC5 (survivin), FGF1, IL33, GRB2, IGFBP3, and AREG, FN1, ADAMTS1, AXL, ADAMTS5, MET, CYR61, IL8, ZEB1, FOXC2, N-cadherin, and SNAIL. In certain embodiments, an IgCAM-binding agent results in a reduction in the level of expression of at least one YAP-dependent gene of at least about 10%, at least about 25%, at least about 50%, at least about 75%, at least about 90%, or at least about 95%. In certain embodiments, an IgCAM-binding agent results in a reduction in the level of expression of at least one TAZ-dependent gene of at least about 10%, at least about 25%, at least about 50%, at least about 75%, at least about 90%, or at least about 95%.

*In vivo* and *in vitro* assays for determining whether an IgCAM-binding agent (or candidate IgCAM-binding agent) modulates Hippo pathway signaling are known in the art or are being developed. In some embodiments, the effect of an agent on Hippo pathway signaling is evaluated by YAP and/or TAZ activity. If the Hippo pathway is inhibited, activated, non-phosphorylated YAP and/or TAZ translocates to the nucleus of the cell where it binds with a DNA-binding transcription factor, such as TEAD leading to transcription of a variety of target genes. Therefore, for example, a cell-based, luciferase reporter assay utilizing a reporter vector containing multiple copies of the GT-IIC motif from the SV40 enhancer, which serves as a TEAD binding site upstream of a firefly luciferase reporter gene (TBS-Luc reporter) may be used to measure YAP and/or TAZ activity *in vitro.* If an IgCAM-binding agent activates the Hippo pathway, thereby inhibiting YAP and/or TAZ activity, luciferase activity will be inhibited. In addition to the TBS-Luc reporter assay, the effect of an IgCAM-binding agent (or candidate agent) on Hippo pathway signaling may be measured *in vitro* or *in vivo* by measuring the effect of the agent on the level of expression of YAP-dependent genes and/or TAZ-dependent genes, such as CD44, CD47, CD133, TDGF1, EPHB2, CA12, LRP4, GPC4, CLDN2, CTGF, PAG1, SEMA4D, RHEB, MAGI1, ITPR3, CD168, NRP2, GLI2, BIRC2, BIRC5, FGF1, IL33, GRB2, IGFBP3, and AREG. In certain embodiments, the effect of an IgCAM-binding agent on Hippo pathway signaling may also be assessed by measuring the effect of the agent on the phosphorylation state of Mst1, Mst2, Lats1, Lats2, YAP, and/or TAZ.

In some embodiments, modulation of the Hippo pathway signaling is evaluated by translocation of YAP. As described above, when the Hippo pathway is inhibited, activated non-phosphorylated YAP translocates to the nucleus. By tagging the YAP protein, for example with GFP, the location of YAP can be determined. Therefore, for example, a cell-based assay using a YAP-GFP fusion protein may be used to determine YAP activation by observing the location of YAP after treatment with an agent. Another assay to determine YAP activation comprises using a YAP-Cre fusion protein with a Cre-dependent fluorescent protein. For example, a monomeric form of fluorescent protein DsRed is flanked by loxP sites 5' to a green fluorescent protein and serves as a reporter protein. YAP is fused to the coding region of Cre. When YAP is inactive and cytoplasmic, the fluorescent protein DsRed is expressed in the nucleus; however, when YAP is activated, the YAP-Cre protein translocates to the nucleus of the cell, the DsRed is removed by Cre/loxP recombination and the green fluorescent protein is expressed in the nucleus.

In some embodiments, modulation of the Hippo pathway signaling is evaluated by translocation of TAZ. As with YAP, when the Hippo pathway is inhibited, activated non-phosphorylated TAZ translocates to the nucleus. By tagging the TAZ protein, for example with GFP, the location of TAZ can be determined. Therefore, for example, a cell-based assay using a TAZ-GFP fusion protein may be used to determine TAZ activation by observing the location of TAZ after treatment with an agent. Another assay to determine TAZ activation comprises using a TAZ-Cre fusion protein with a Care-dependent fluorescent protein. For example, a monomeric form of fluorescent protein DsRed is flanked by loxP sites 5' to a green fluorescent protein. TAZ is fused to the coding region of Cre. When TAZ is inactive and cytoplasmic, the fluorescent protein DsRed is expressed in the nucleus; however, when TAZ is activated, the TAZ-Cre protein translocates to the nucleus of the cell, the DsRed is removed by Cre/loxP recombination and the green fluorescent protein is expressed in the nucleus.

In some embodiments, YAP or TAZ translocation is evaluated using a novel bimolecular fluorescence complementation (BiFC) assay. The BiFC assay is based upon the discovery that two non-fluorescent fragments of a fluorescent protein can associate to form a fluorescent complex and that the association of the fragments can be facilitated by fusing them to two proteins that interact with each other. In some embodiments, the C-terminus of YAP (or TAZ) is fused to the C-terminus of a yellow fluorescent protein (YFP) to generate YAP-YFPC. The N-terminus ofYFP is fused to the N-terminus of TEAD2 or TEAD3 to generate YFPN-TEAD2 or YFPN-TEAD3. These constructs are transfected into cells. Under conditions where the Hippo pathway is inhibited, the YAP-YFPC construct will translocate into the nucleus of the cell where it will complex with the YFPN-TEAD construct, leading to a functional fluorescent protein that can be detected by epifluorescence microscopy.

In certain embodiments, the IgCAM-binding agents have one or more of the following effects: inhibit proliferation of tumor cells, inhibit tumor growth, reduce the tumorigenicity of a tumor, reduce the tumorigenicity of a tumor by reducing the frequency of cancer stem cells in the tumor, trigger cell death of tumor cells, increase cell contact-dependent growth inhibition, increase tumor cell apoptosis, reduce epithelial mesenchymal transition (EMT), or decrease survival of tumor cells.

In certain embodiments, the IgCAM-binding agents are capable of inhibiting tumor growth. In certain embodiments, the IgCAM-binding agents are capable of inhibiting tumor growth *in vivo* (e.g., in a xenograft mouse model, and/or in a human having cancer).

In certain embodiments, the IgCAM-binding agents are capable of reducing the tumorigenicity of a tumor. In certain embodiments, the IgCAM-binding agent is capable of reducing the tumorigenicity of a tumor in an animal model, such as a mouse xenograft model. In certain embodiments, the IgCAM-binding agent is capable of reducing the tumorigenicity of a tumor comprising cancer stem cells in an animal model, such as a mouse xenograft model. In certain embodiments, the number or frequency of cancer stem cells in a tumor is reduced by at least about two-fold, about three-fold, about five-fold, about ten-fold, about 50-fold, about 100-fold, or about 1000-fold. In certain embodiments, the reduction in the number or frequency of cancer stem cells is determined by limiting dilution assay using an animal model. Additional examples and guidance regarding the use of limiting dilution assays to determine a reduction in the number or frequency of cancer stem cells in a tumor can be found, e.g., in International Publication Number WO 2008/042236; U.S. Patent Publication No. 2008/0064049; and U.S. Patent Publication No. 2008/0178305.

In certain embodiments, the IgCAM-binding agents increase proliferation of cells, increase wound healing, and/or increase tissue regeneration.

In certain embodiments, the IgCAM-binding agents described herein have a circulating half-life in mice, cynomolgus monkeys, or humans of at least about 5 hours, at least about 10 hours, at least about 24 hours, at least about 3 days, at least about 1 week, or at least about 2 weeks. In certain embodiments, the IgCAM-binding agent is an IgG (e.g., IgG1 or IgG2) antibody that has a circulating half-life in mice, cynomolgus monkeys, or humans of at least about 5 hours, at least about 10 hours, at least about 24 hours, at least about 3 days, at least about 1 week, or at least about 2 weeks. Methods of increasing (or decreasing) the half-life of agents such as polypeptides and antibodies are known in the art. For example, known methods of increasing the circulating half-life of IgG antibodies include the introduction of mutations in the Fc region which increase the pH-dependent binding of the antibody to the neonatal Fc receptor (FcRn) at pH 6.0 (see, e.g., U.S. Patent Publication Nos. 2005/0276799, 2007/0148164, and 2007/0122403). Known methods of increasing the circulating half-life of antibody fragments lacking the Fc region include such techniques as PEGylation.

In some embodiments of the present invention, the IgCAM-binding agents are polypeptides. In some embodiments of the present invention, the IgCAM-binding agents are small peptides. The polypeptides can be recombinant polypeptides, natural polypeptides, or synthetic polypeptides that bind at least one human IgCAM. It will be recognized in the art that some amino acid sequences of the invention can be varied without significant effect of the structure or function of the protein. Thus, the invention further includes variations of the polypeptides which show substantial activity against a human IgCAM. In some embodiments, amino acid sequence variations of IgCAM-binding polypeptides include deletions, insertions, inversions, repeats, and/or other types of substitutions.

The polypeptides, analogs and variants thereof, can be further modified to contain additional chemical moieties not normally part of the polypeptide. The derivatized moieties can improve the solubility, the biological half-life, and/or absorption of the polypeptide. The moieties can also reduce or eliminate undesirable side effects of the polypeptides and variants. An overview for chemical moieties can be found in Remington: The Science and Practice of Pharmacy, 21st Edition, 2005, University of the Sciences, Philadelphia, PA.

The polypeptides described herein can be produced by any suitable method known in the art. Such methods range from direct protein synthesis methods to constructing a DNA sequence encoding polypeptide sequences and expressing those sequences in a suitable host. In some embodiments, a DNA sequence is constructed using recombinant technology by isolating or synthesizing a DNA sequence encoding a wild-type protein of interest. Optionally, the sequence can be mutagenized by site-specific mutagenesis to provide functional analogs thereof. See, e.g., Zoeller et al., 1984, PNAS, 81 :5662-5066 and U.S. Patent No. 4,588,585.

In some embodiments, a DNA sequence encoding a polypeptide of interest may be constructed by chemical synthesis using an oligonucleotide synthesizer. Oligonucleotides can be designed based on the amino acid sequence of the desired polypeptide and selecting those codons that are favored in the host cell in which the recombinant polypeptide of interest will be produced. Standard methods can be applied to synthesize a polynucleotide sequence encoding an isolated polypeptide of interest. For example, a complete amino acid sequence can be used to construct a back-translated gene. Further, a DNA oligomer containing a nucleotide sequence coding for the particular isolated polypeptide can be synthesized. For example, several small oligonucleotides coding for portions of the desired polypeptide can be synthesized and then ligated. The individual oligonucleotides typically contain 5' or 3' overhangs for complementary assembly.

Once assembled (by synthesis, site-directed mutagenesis, or another method), the polynucleotide sequences encoding a particular polypeptide of interest can be inserted into an expression vector and operatively linked to an expression control sequence appropriate for expression of the protein in a desired host. Proper assembly can be confirmed by nucleotide sequencing, restriction enzyme mapping, and/or expression of a biologically active polypeptide in a suitable host. As is well-known in the art, in order to obtain high expression levels of a transfected gene in a host, the gene must be operatively linked to transcriptional and translational expression control sequences that are functional in the chosen expression host.

In certain embodiments, recombinant expression vectors are used to amplify and express DNA encoding binding agents (e.g., antibodies or soluble receptors) against at least one human IgCAM. For example, recombinant expression vectors can be replicable DNA constructs which have synthetic or cDNA-derived DNA fragments encoding a polypeptide chain of an IgCAM-binding agent, an anti-IgCAM antibody, or fragment thereof, operatively linked to suitable transcriptional and/or translational regulatory elements derived from mammalian, microbial, viral or insect genes. A transcriptional unit generally comprises an assembly of (1) a genetic element or elements having a regulatory role in gene expression, for example, transcriptional promoters or enhancers, (2) a structural or coding sequence which is transcribed into mRNA and translated into protein, and (3) appropriate transcription and translation initiation and termination sequences. Regulatory elements can include an operator sequence to control transcription. The ability to replicate in a host, usually conferred by an origin of replication, and a selection gene to facilitate recognition of transformants can additionally be incorporated. DNA regions are "operatively linked" when they are functionally related to each other. For example, DNA for a signal peptide (secretory leader) is operatively linked to DNA for a polypeptide if it is expressed as a precursor which participates in the secretion of the polypeptide; a promoter is operatively linked to a coding sequence if it controls the transcription of the sequence; or a ribosome binding site is operatively linked to a coding sequence if it is positioned so as to permit translation. In some embodiments, structural elements intended for use in yeast expression systems include a leader sequence enabling extracellular secretion of translated protein by a host cell. In other embodiments, where recombinant protein is expressed without a leader or transport sequence, it can include an N-terminal methionine residue. This residue can optionally be subsequently cleaved from the expressed recombinant protein to provide a final product.

The choice of an expression control sequence and an expression vector depends upon the choice of host. A wide variety of expression host/vector combinations can be employed. Useful expression vectors for eukaryotic hosts include, for example, vectors comprising expression control sequences from SV40, bovine papilloma virus, adenovirus, and cytomegalovirus. Useful expression vectors for bacterial hosts include known bacterial plasmids, such as plasmids from E. coli, including pCR1, pBR322, pMB9 and their derivatives, and wider host range plasmids, such as M 13 and other filamentous single-stranded DNA phages.

Suitable host cells for expression of an IgCAM-binding polypeptide or IgCAM-binding antibody (or an IGCAM protein to use as an antigen) include prokaryotes, yeast cells, insect cells, or higher eukaryotic cells under the control of appropriate promoters. Prokaryotes include gram-negative or gram-positive organisms, for example E. coli or Bacillus. Higher eukaryotic cells include established cell lines of mammalian origin as described below. Cell-free translation systems may also be employed. Appropriate cloning and expression vectors for use with bacterial, fungal, yeast, and mammalian cellular hosts are described by Pouwels et al. (1985, Cloning Vectors: A Laboratory Manual, Elsevier, New York, NY). Additional information regarding methods of protein production, including antibody production, can be found, e.g., in U.S. Patent Publication No. 2008/0187954; U.S. Patent Nos. 6,413,746 and 6,660,501; and International Patent Publication No. WO 2004/009823.

Various mammalian or insect cell culture systems are used to express recombinant polypeptides. Expression of recombinant proteins in mammalian cells can be preferred because such proteins are generally correctly folded, appropriately modified, and biologically functional. Examples of suitable mammalian host cell lines include COS-7 (monkey kidney-derived), L-929 (murine fibroblast-derived), C127 (murine mammary tumor-derived), 3T3 (murine fibroblast-derived), CHO (Chinese hamster ovary-derived), HeLa (human cervical cancer-derived), BHK (hamster kidney fibroblast-derived), and HEK-293 (human embryonic kidney-derived) cell lines and variants thereof. Mammalian expression vectors can comprise non-transcribed elements such as an origin of replication, a suitable promoter and enhancer linked to the gene to be expressed, and other 5' or 3' flanking non-transcribed sequences, and 5' or 3' non-translated sequences, such as necessary ribosome binding sites, a polyadenylation site, splice donor and acceptor sites, and transcriptional termination sequences. Baculovirus systems for production of heterologous proteins in insect cells are well-known to those of skill in the art (see, e.g., Luckow and Summers, 1988, Bio/Technology, 6:47).

Thus, the present invention provides cells comprising the IgCAM-binding agents described herein. In some embodiments, the cells produce the IgCAM-binding agents described herein. In certain embodiments, the cells produce an antibody. In certain embodiments, the cells produce a fusion protein. In some embodiments, the cells produce a soluble receptor.

The proteins produced by a transformed host can be purified according to any suitable method. Standard methods include chromatography (e.g., ion exchange, affinity, and sizing column chromatography), centrifugation, differential solubility, or by any other standard technique for protein purification. Affinity tags such as hexa-histidine, maltose binding domain, influenza coat sequence, and glutathione-S-transferase can be attached to the protein to allow easy purification by passage over an appropriate affinity column. Isolated proteins can also be physically characterized using such techniques as proteolysis, mass spectrometry (MS), nuclear magnetic resonance (NMR), high performance liquid chromatography (HPLC), and x-ray crystallography.

In some embodiments, supernatants from expression systems which secrete recombinant protein into culture media can be first concentrated using a commercially available protein concentration filter, for example, an Amicon or Millipore Pellicon ultrafiltration unit. Following the concentration step, the concentrate can be applied to a suitable purification matrix. In some embodiments, an anion exchange resin can be employed, for example, a matrix or substrate having pendant diethylaminoethyl (DEAE) groups. The matrices can be acrylamide, agarose, dextran, cellulose, or other types commonly employed in protein purification. In some embodiments, a cation exchange step can be employed. Suitable cation exchangers include various insoluble matrices comprising sulfopropyl or carboxymethyl groups. In some embodiments, a hydroxyapatite media can be employed, including but not limited to, ceramic hydroxyapatite (CHT). In certain embodiments, one or more reverse-phase HPLC steps employing hydrophobic RP-HPLC media, e.g., silica gel having pendant methyl or other aliphatic groups, can be employed to further purify an IgCAM-binding agent. Some or all of the foregoing purification steps, in various combinations, can also be employed to provide a homogeneous recombinant protein.

In some embodiments, recombinant protein produced in bacterial culture can be isolated, for example, by initial extraction from cell pellets, followed by one or more concentration, salting-out, aqueous ion exchange, or size exclusion chromatography steps. HPLC can be employed for final purification steps. Microbial cells employed in expression of a recombinant protein can be disrupted by any convenient method, including freeze-thaw cycling, sonication, mechanical disruption, or use of cell lysing agents.

Methods known in the art for purifying antibodies and other proteins also include, for example, those described in U.S. Patent Publication Nos. 2008/0312425, 2008/0177048, and 2009/0187005.

In certain embodiments, the IgCAM-binding agent is a polypeptide that is not an antibody or does not comprise an antibody Fc region. In certain embodiments, the polypeptide comprises a protein scaffold of a type selected from the group consisting of protein A, protein G, a lipocalin, a fibronectin domain, an ankyrin consensus repeat domain, and thioredoxin. A variety of methods for identifying and producing non-antibody polypeptides that bind with high affinity to a protein target are known in the art. See, e.g., Skerra, 2007, Curr. Opin. Biotechnol., 18:295-304; Hosse et al., 2006, Protein Science, 15:14-27; Gill et al., 2006, Curr. Opin. Biotechnol., 17:653-658; Nygren, 2008, FEBS J., 275:2668-76; and Skerra, 2008, FEBS J., 275:2677-83. In certain embodiments, phage display technology may be used to produce and/or identify an IgCAM-binding polypeptide. In certain embodiments, mammalian cell display technology may be used to produce and/or identify an IgCAM-binding polypeptide.

In certain embodiments, the IgCAM-binding agents can be used in any one of a number of conjugated (i.e. an immunoconjugate or radioconjugate) or non-conjugated forms. In certain embodiments, the binding agents can be used in a non-conjugated form to harness the subject's natural defense mechanisms including CDC and ADCC to eliminate malignant or cancer cells.

In some embodiments, the IgCAM-binding agent (e.g., an antibody, a soluble receptor, or a polypeptide) is conjugated to a cytotoxic agent. In some embodiments, the cytotoxic agent is a chemotherapeutic agent including, but not limited to, methotrexate, adriamicin, doxorubicin, melphalan, mitomycin C, chlorambucil, daunorubicin or other intercalating agents. In some embodiments, the cytotoxic agent is an enzymatically active toxin of bacterial, fungal, plant, or animal origin, or fragments thereof, including, but not limited to, diphtheria A chain, nonbinding active fragments of diphtheria toxin, exotoxin A chain, ricin A chain, abrin A chain, modeccin A chain, alpha-sarcin, Aleurites fordii proteins, dianthin proteins, Phytolaca americana proteins (PAPI, PAPII, and PAP-S), Momordica charantia inhibitor, curcin, crotin, Sapaonaria officinalis inhibitor, gelonin, mitogellin, restrictocin, phenomycin, enomycin, and the tricothecenes. In some embodiments, the cytotoxic agent is a radioisotope to produce a radioconjugate or a radioconjugated binding agent. A variety of radionuclides are available for the production of radioconjugated binding agents including, but not limited to, ⁹⁰Y, ¹²⁵I, ¹³¹I, ¹²³I, ¹¹¹In, ¹³¹In, ¹⁰⁵Rh, ¹⁵³Sm, ⁶⁷Cu, ⁶⁷Ga, ¹⁶⁶Ho, ¹⁷⁷Lu, ¹⁸⁶Re, ¹⁸⁸Re and ²¹²Bi. Conjugates of a binding agent and one or more small molecule toxins, such as a calicheamicin, maytansinoids, a trichothene, and CC1065, and the derivatives of these toxins that have toxin activity, can also be used. In some embodiments, the IgCAM-binding agent (e.g., an antibody, a soluble receptor, or a polypeptide) is conjugated to a maytansinoid. Conjugates of a binding agent and cytotoxic agent are made using a variety of bifunctional protein-coupling agents such as N-succinirtridyl-3-(2-pyridyidithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis(p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene).

### III. Polynucleotides

In certain embodiments, the invention encompasses polynucleotides comprising polynucleotides that encode a polypeptide that specifically binds at least one human IgCAM or a fragment of such a polypeptide. The term "polynucleotides that encode a polypeptide" encompasses a polynucleotide which includes only coding sequences for the polypeptide as well as a polynucleotide which includes additional coding and/or non-coding sequences. The polynucleotides of the invention can be in the form of RNA or in the form of DNA. DNA includes cDNA, genomic DNA, and synthetic DNA; and can be doublestranded or single-stranded, and if single stranded can be the coding strand or non-coding (anti-sense) strand.

In certain embodiments, the polynucleotide comprises a polynucleotide encoding a polypeptide comprising an amino acid sequence selected from the group consisting of: SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, and SEQ ID NO:64.

In certain embodiments, the polynucleotide comprises a polynucleotide having a nucleotide sequence at least 80% identical, at least 85% identical, at least 90% identical, at least 95% identical, and in some embodiments, at least 96%, 97%, 98% or 99% identical to a polynucleotide encoding an amino acid sequence selected from the group consisting of: SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50 SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO.60, SEQ ID NO:61, SEQ ID NO:62, SEQ ID NO:63, and SEQ ID NO:64. Also provided is a polynucleotide that comprises a polynucleotide that hybridizes to a polynucleotide or to a polynucleotide complementary to a polynucleotide encoding an amino acid sequence selected from the group consisting of: SEQ ID NO:33, SEQ ID NO:34, SEQ ID NO:35, SEQ ID NO:36, SEQ ID NO:37, SEQ ID NO:38, SEQ ID NO:39, SEQ ID NO:40 SEQ ID NO:41, SEQ ID NO:42, SEQ ID NO:43, SEQ ID NO:44, SEQ ID NO:45, SEQ ID NO:46 SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, and SEQ ID NO:64. In certain embodiments, the hybridization is under conditions of high stringency.

In certain embodiments, the polynucleotides comprise the coding sequence for the mature polypeptide fused in the same reading frame to a polynucleotide which aids, for example, in expression and secretion of a polypeptide from a host cell (e.g., a leader sequence which functions as a secretory sequence for controlling transport of a polypeptide from the cell). The polypeptide having a leader sequence is a preprotein and can have the leader sequence cleaved by the host cell to form the mature form of the polypeptide. The polynucleotides can also encode for a proprotein which is the mature protein plus additional 5' amino acid residues. A mature protein having a prosequence is a proprotein and is an inactive form of the protein. Once the prosequence is cleaved an active mature protein remains.

In certain embodiments, the polynucleotides comprise the coding sequence for the mature polypeptide fused in the same reading frame to a marker sequence that allows, for example, for purification of the encoded polypeptide. For example, the marker sequence can be a hexa-histidine tag supplied by a pQE-9 vector to provide for purification of the mature polypeptide fused to the marker in the case of a bacterial host, or the marker sequence can be a hemagglutinin (HA) tag derived from the influenza hemagglutinin protein when a mammalian host (e.g., COS-7 cells) is used. In some embodiments, the marker sequence is a FLAG-tag, a peptide of sequence DYKDDDDK (SEQ ID NO:73) which can be used in conjunction with other affinity tags.

The present invention further relates to variants of the hereinabove described polynucleotides encoding, for example, fragments, analogs, and/or derivatives.

In certain embodiments, the present invention provides polynucleotides comprising polynucleotides having a nucleotide sequence at least about 80% identical, at least about 85% identical, at least about 90% identical, at least about 95% identical, and in some embodiments, at least about 96%, 97%, 98% or 99% identical to a polynucleotide encoding a polypeptide comprising an IgCAM-binding agent (e.g., an antibody or a soluble receptor) described herein.

As used herein, the phrase a polynucleotide having a nucleotide sequence at least, for example, 95% "identical" to a reference nucleotide sequence is intended to mean that the nucleotide sequence of the polynucleotide is identical to the reference sequence except that the polynucleotide sequence can include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence can be deleted or substituted with another nucleotide, or a number of nucleotides up to 5% of the total nucleotides in the reference sequence can be inserted into the reference sequence. These mutations of the reference sequence can occur at the 5' or 3' terminal positions of the reference nucleotide sequence or anywhere between those terminal positions, interspersed either individually among nucleotides in the reference sequence or in one or more contiguous groups within the reference sequence.

The polynucleotide variants can contain alterations in the coding regions, non-coding regions, or both. In some embodiments, the polynucleotide variants contain alterations which produce silent substitutions, additions, or deletions, but do not alter the properties or activities of the encoded polypeptide. In some embodiments, nucleotide variants are produced by silent substitutions due to the degeneracy of the genetic code. Polynucleotide variants can be produced for a variety of reasons, for example, to optimize codon expression for a particular host (i.e., change codons in the human mRNA to those preferred by a bacterial host such as E. coli).

In certain embodiments, the polynucleotides are isolated. In certain embodiments, the polynucleotides are substantially pure.

Vectors and cells comprising the polynucleotides described herein are also provided. In some embodiments, an expression vector comprises a polynucleotide molecule. In some embodiments, a host cell comprises an expression vector comprising the polynucleotide molecule. In some embodiments, a host cell comprises a polynucleotide molecule.

### IV. Methods of use and pharmaceutical compositions

The IgCAM-binding agents (e.g., antibodies and soluble receptors) of the invention are useful in a variety of applications including, but not limited to, therapeutic treatment methods, such as the treatment of cancer. In certain embodiments, the binding agents are useful for activating and/or enhancing Hippo pathway signaling, increasing cell contact-dependent growth inhibition, inhibiting YAP activation, inhibiting TAZ activation, inhibiting tumor growth, reducing tumor volume, increasing tumor cell apoptosis, and/or reducing the tumorigenicity of a tumor. The methods of use may be *in vitro, ex vivo,* or *in vivo* methods. In some embodiments, an IgCAM-binding agent is an agonist of the Hippo pathway. In some embodiments, an IgCAM-binding agent inhibits YAP activity. In some embodiments, an IgCAM-binding agent inhibits TAZ activity.

In some embodiments, the IgCAM-binding agents (e.g., an antibody or a soluble receptor) are used in the treatment of a disease associated with activation of YAP, activation of TAZ, decreased Hippo pathway signaling, and/or aberrant Hippo pathway signaling. In some embodiments, the IgCAM-binding agents are used in the treatment of a disease associated with aberrant expression or aberrant exposure of IgCAMs. In some embodiments, the disease is a disease dependent upon YAP activation. In some embodiments, the disease is a disease dependent upon TAZ activation. In some embodiments, the IgCAM-binding agents are used in the treatment of disorders characterized by increased levels of stem cells and/or progenitor cells. In some embodiments, the methods comprise administering a therapeutically effective amount of an IgCAM-binding agent to a subject. In some embodiments, the subject is human.

The present invention provides methods for inhibiting growth of a tumor using the IgCAM-binding agents described herein. In certain embodiments, the method of inhibiting growth of a tumor comprises contacting a cell with an IgCAM-binding agent (e.g., an antibody or a soluble receptor) *in vitro.* For example, an immortalized cell line or a cancer cell line is cultured in medium to which is added an IgCAM-binding agent to inhibit tumor growth. In some embodiments, tumor cells are isolated from a patient sample such as, for example, a tissue biopsy, pleural effusion, or blood sample and cultured in medium to which is added an IgCAM-binding agent to inhibit tumor growth.

In some embodiments, the method of inhibiting growth of a tumor comprises contacting the tumor or tumor cells with an IgCAM-binding agent (e.g., an antibody or a soluble receptor) *in vivo.* In certain embodiments, contacting a tumor or tumor cell with an IgCAM-binding agent is undertaken in an animal model. For example, an IgCAM-binding agent may be administered to immunocompromised mice (e.g. NOD/SCID mice) which have xenografts. In some embodiments, tumor cells are isolated from a patient sample such as, for example, a tissue biopsy, pleural effusion, or blood sample and injected into immunocompromised mice that are then administered an IgCAM-binding agent to inhibit tumor cell growth. In some embodiments, the IgCAM-binding agent is administered at the same time or shortly after introduction of tumorigenic cells into the animal to prevent tumor growth ("preventative model"). In some embodiments, the IgCAM-binding agent is administered as a therapeutic after tumors have grown to a specified size ("therapeutic model"). In some embodiments, the IgCAM-binding agent is an antibody. In some embodiments, the IgCAM-binding agent is a soluble receptor.

In certain embodiments, the method of inhibiting growth of a tumor comprises administering to a subject a therapeutically effective amount of an IgCAM-binding agent. In certain embodiments, the subject is a human. In certain embodiments, the subject has a tumor or has had a tumor which was removed. In some embodiments, the subject has a tumor with an elevated expression level of YAP. In some embodiments, the "elevated" expression level is in comparison to the expression level of YAP in normal tissue of the same tissue type. In some embodiments, the "elevated" expression level is in comparison to the expression level of YAP in other tumors of the same tissue type. In some embodiments, the "elevated" expression level is in comparison to the expression level of YAP in a reference sample. In some embodiments, the "elevated" expression level is in comparison to a pre-determined level of YAP. These comparisons may also be applied to the terms "increased", "decreased", and similar terms. In some embodiments, the subject has a tumor with an increased level of non-phosphorylated YAP. In some embodiments, the subject has a tumor with a decreased level of phosphorylated YAP. In some embodiments, the subject has a tumor with increased expression of YAP-dependent genes. In some embodiments, the subject has a tumor with an elevated expression level of TAZ. In some embodiments, the subject has a tumor with an increased level of non-phosphorylated TAZ. In some embodiments, the subject has a tumor with a decreased level of phosphorylated TAZ. In some embodiments, the subject has a tumor with increased expression of TAZ-dependent genes. In some embodiments, the IgCAM-binding agent is an antibody. In some embodiments, the IgCAM-binding agent is a soluble receptor.

In addition, the invention provides a method of inhibiting growth of a tumor in a subject, comprising administering a therapeutically effective amount of an IgCAM-binding agent to the subject. In certain embodiments, the tumor comprises cancer stem cells. In certain embodiments, the frequency of cancer stem cells in the tumor is reduced by administration of the IgCAM-binding agent. The invention also provides a method of reducing the frequency of cancer stem cells in a tumor, comprising contacting the tumor with an effective amount of an IgCAM-binding agent. In some embodiments, a method of reducing the frequency of cancer stem cells in a tumor in a subject, comprising administering to the subject a therapeutically effective amount of an IgCAM-binding agent is provided. In some embodiments, the IgCAM-binding agent is an antibody. In some embodiments, the IgCAM-binding agent is a soluble receptor.

In some embodiments, the tumor is a solid tumor. In certain embodiments, the tumor is a tumor selected from the group consisting of colorectal tumor, pancreatic tumor, lung tumor, ovarian tumor, liver tumor, breast tumor, kidney tumor, prostate tumor, gastrointestinal tumor, melanoma, cervical tumor, bladder tumor, glioblastoma, and head and neck tumor. In certain embodiments, the tumor is a colorectal tumor. In certain embodiments, the tumor is an ovarian tumor. In some embodiments, the tumor is a lung tumor. In certain embodiments, the tumor is a pancreatic tumor. In certain embodiments, the tumor is a melanoma tumor.

In certain embodiments, the tumor is a tumor in which Hippo pathway signaling is inactive or at low levels. In some embodiments, the tumor is a tumor in which Hippo pathway signaling is aberrant. In some embodiments, the tumor or tumor cells have lost contact-dependent growth inhibition. In some embodiments, the tumor or tumor cells have enhanced anchorage-independent growth. In some embodiments, the tumor or tumor cells increased cell proliferation.

In certain embodiments, the tumor has elevated expression levels of YAP or over-expresses YAP. In general, the phrase "a tumor has elevated expression levels of" a protein (or similar phrases) refers to expression levels of a protein in a tumor as compared to expression levels of the same protein in normal tissue. In certain embodiments, the tumor has elevated levels of non-phosphorylated YAP. In some embodiments, the tumor has elevated levels of YAP in the nucleus. In general, the phrase "a tumor has elevated levels of" a protein (or similar phrases) refers to levels of a protein in a tumor as compared to levels of the same protein in normal tissue. In certain embodiments, the tumor has elevated expression levels ofTAZ or over-expresses TAZ. In certain embodiments, the tumor has elevated levels of non-phosphorylated TAZ. In some embodiments, the tumor has elevated levels of TAZ in the nucleus.

The present invention further provides methods for treating cancer comprising administering a therapeutically effective amount of an IgCAM-binding agent to a subject. In certain embodiments, the cancer is characterized by cells expressing elevated levels of YAP as compared to expression levels in normal tissue. In certain embodiments, the cancer is characterized by cells expressing elevated levels of non-phosphorylated YAP as compared to expression levels in normal tissue. In certain embodiments, the cancer is characterized by cells expressing decreased levels of phosphorylated YAP as compared to expression levels in normal tissue. In some embodiments, the cancer is characterized by cells expressing increased levels of at least one YAP-dependent gene. In certain embodiments, the cancer is characterized by cells expressing elevated levels of TAZ as compared to expression levels in normal tissue. In certain embodiments, the cancer is characterized by cells expressing elevated levels of non-phosphorylated TAZ as compared to expression levels in normal tissue. In certain embodiments, the cancer is characterized by cells expressing decreased levels of phosphorylated TAZ as compared to expression levels in normal tissue. In some embodiments, the cancer is characterized by cells expressing increased levels of at least one TAZ-dependent gene. In some embodiments, the IgCAM-binding agent binds the extracellular domain of at least one human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25, and inhibits or reduces growth of the cancer. In some embodiments, the IgCAM-binding agent binds the extracellular domain of at least one human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25, inhibits YAP and/or TAZ translocation to the nucleus, and inhibits or reduces growth of the cancer. In some embodiments, the IgCAM-binding agent binds the extracellular domain of at least one human IgCAM selected from the group consisting of: AMICA, CAR, CLMP, ESAM, GPA33, VSIC1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25, enhances phosphorylation of YAP and/or TAZ, and inhibits or reduces growth of the cancer. In some embodiments, the IgCAM-binding agent binds the extracellular domain of at least one human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25, enhances YAP and/or TAZ retention in the cytoplasm, and inhibits or reduces growth of the cancer. In some embodiments, the IgCAM-binding agent binds the extracellular domain of at least one human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25, enhances YAP and/or TAZ degradation, and inhibits or reduces growth of the cancer. In some embodiments, the IgCAM-binding agent binds the extracellular domain of at least one human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25, inhibits YAP and/or TAZ activation, and inhibits or reduces growth of the cancer. In some embodiments, the IgCAM-binding agent binds the extracellular domain of at least one human IgCAM selected from the group consisting of: AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25, inhibits YAP and/or TAZ activation, and reduces or inhibits the expression of at least one YAP-dependent gene or at least one TAZ-dependent gene. In some embodiments, the IgCAM-binding agent is an antibody. In some embodiments, the IgCAM-binding agent is a soluble receptor. In some embodiments, the IgCAM-binding agent does not bind CADM1.

The present invention provides for methods of treating cancer comprising administering a therapeutically effective amount of an IgCAM-binding agent to a subject (e.g., a subject in need of treatment). In certain embodiments, the subject is a human. In certain embodiments, the subject has a cancerous tumor. In certain embodiments, the subject has had a tumor removed. In some embodiments, a method of treating cancer comprises administering a therapeutically effective amount of an IgCAM-binding agent to a subject, wherein the subject has a tumor that has elevated expression of YAP and/or TAZ.

In certain embodiments, the cancer is a cancer selected from the group consisting of colorectal cancer, pancreatic cancer, lung cancer, ovarian cancer, liver cancer, breast cancer, kidney cancer, prostate cancer, gastrointestinal cancer, melanoma, cervical cancer, bladder cancer, glioblastoma, and head and neck cancer. In certain embodiments, the cancer is pancreatic cancer. In certain embodiments, the cancer is ovarian cancer. In certain embodiments, the cancer is colorectal cancer. In certain embodiments, the cancer is breast cancer. In certain embodiments, the cancer is prostate cancer. In certain embodiments, the cancer is lung cancer. In certain embodiments, the cancer is melanoma.

In addition, the invention provides a method of reducing the tumorigenicity of a tumor in a subject, comprising administering to a subject a therapeutically effective amount of an IgCAM-binding agent. In certain embodiments, the tumor comprises cancer stem cells. In some embodiments, the tumorigenicity of a tumor is reduced by reducing the frequency of cancer stem cells in the tumor. In some embodiments, the methods comprise using the IgCAM-binding agents described herein. In certain embodiments, the frequency of cancer stem cells in the tumor is reduced by administration of an IgCAM-binding agent.

In certain embodiments, the methods further comprise a step of determining the level of YAP expression in the tumor or cancer. In some embodiments, the step of determining the level of YAP expression in the tumor or cancer comprises determining the level of expression of cytoplasmic and/or nuclear YAP. In some embodiments, the level of expression of YAP in a tumor or cancer is compared to the level of expression of YAP in a reference sample. As used herein, a "reference sample" includes but is not limited to, normal tissue, non-cancerous tissue of the same tissue type, tumor tissue of the same tissue type, and tumor tissue of a different tissue type. In some embodiments, the level of expression of YAP in a tumor or cancer is compared to a pre-determined level of YAP. In some embodiments, the level of expression of YAP in a tumor or cancer is compared to a pre-determined level of expression of YAP. In some embodiments, the level of expression of YAP in the nucleus of cells in a tumor or cancer is compared to the level of expression of YAP in the nucleus of cells in a reference sample or to a pre-determined level of YAP. In some embodiments, the level of expression of phosphorylated YAP in cells in a tumor or cancer is compared to the level of expression of phosphorylated YAP in a reference sample or to a pre-determined level of phosphorylated YAP. In some embodiments, the level of expression of non-phosphorylated YAP in cells in a tumor or cancer is compared to the level of expression of non-phosphorylated YAP in a reference sample or to a pre-determined level of non-phosphorylated YAP. In some embodiments, determining the level of YAP expression is done prior to treatment. In some embodiments, the subject is administered an IgCAM-binding agent described herein if the tumor or cancer has an elevated level of YAP expression as compared to the expression of the YAP in a reference sample or to a pre-determined level of YAP. In some embodiments, the subject is administered an IgCAM-binding agent describe herein if the tumor or cancer has an elevated level of non-phosphorylated YAP expression as compared to the expression of non-phosphorylated YAP in a reference sample or to a pre-determined level of non-phosphorylated YAP.

In certain embodiments, the methods further comprise a step of determining the level of TAZ expression in the tumor or cancer. In some embodiments, the step of determining the level of TAZ expression in the tumor or cancer comprises determining the level of expression of cytoplasmic and/or nuclear TAZ. In some embodiments, the level of expression of TAZ in a tumor or cancer is compared to the level of expression ofTAZ in a reference sample. In some embodiments, the level of expression of TAZ in a tumor or cancer is compared to a pre-determined level of TAZ. In some embodiments, the level of expression of TAZ in the nucleus of cells in a tumor or cancer is compared to the level of expression of TAZ in the nucleus of cells in a reference sample or to a pre-determined level of TAZ. In some embodiments, the level of expression of phosphorylated TAZ in cells in a tumor or cancer is compared to the level of expression of phosphorylated TAZ in a reference sample or to a pre-determined level of phosphorylated TAZ. In some embodiments, the level of expression of non-phosphorylated TAZ in cells in a tumor or cancer is compared to the level of expression of non-phosphorylated TAZ in a reference sample or to a pre-determined level of non-phosphorylated TAZ. In some embodiments, determining the level of TAZ expression is done prior to treatment. In some embodiments, the subject is administered an IgCAM-binding agent described herein if the tumor or cancer has an elevated level of TAZ expression as compared to the expression of the TAZ in a reference sample or to a pre-determined level of TAZ. In some embodiments, the subject is administered an IgCAM-binding agent describe herein if the tumor or cancer has an elevated level of non-phosphorylated TAZ expression as compared to the expression of non-phosphorylated TAZ in a reference sample or to a pre-determined level of non-phosphorylated TAZ.

In certain embodiments, the methods further comprise a step of determining the level of expression of YAP-dependent genes in the tumor or cancer. In some embodiments, the level of expression of YAP-dependent genes in a tumor or cancer is compared to the level of expression of YAP-dependent genes in a reference sample or to a pre-determined level. In some embodiments, the methods further comprise a step of determining the level of expression of at least one gene selected from the group consisting of: CD44, CD47, CD 133, TDGF1, EPHB2, CA12, LRP4, GPC4, CLDN2, CTGF, PAG1, SEMA4D, RHEB, MAGI1, ITPR3, CD168, NRP2, GL12, BIRC2, B1RC5 (survivin), FGF1, IL33, GRB2, IGFBP3, AREG, FN1, ADAMTS1, AXL, ADAMTS5, MET, CYR61, IL8, ZEB1, FOXC2, N-cadherin, and SNAIL.

In certain embodiments, the methods further comprise a step of determining the level of expression of TAZ-dependent genes in the tumor or cancer. In some embodiments, the level of expression of TAZ-dependent genes in a tumor or cancer is compared to the level of expression of TAZ-dependent genes in a reference sample or to a pre-determined level. In some embodiments, the methods comprise a step of determining the level of expression of at least one gene selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GLI2, BIRC2, BIRC5 (survivin), FGF1, IL33, GRB2, IGFBP3, AREG, FN1, ADAMTS1, AXL, ADAMTS5, MET, CYR61, IL8, ZEB1, FOXC2, N-cadherin, and SNAIL.

In addition, the present invention provides methods of identifying a human subject for treatment with an IgCAM-binding agent, comprising determining if the subject has a tumor that has an elevated level of non-phosphorylated YAP as compared to expression of non-phosphorylated YAP in a reference sample or to a pre-determined level of non-phosphorylated YAP. In some embodiments, if the tumor has an elevated level of non-phosphorylated YAP, the subject is selected for treatment with an IgCAM-binding agent that specifically binds at least one IgCAM. In some embodiments, if selected for treatment, the subject is administered an IgCAM-binding agent described herein. In certain embodiments, the subject has had a tumor removed. In some embodiments, methods of identifying a human subject for treatment with an IgCAM-binding agent, comprising determining if the subject has a tumor that has an elevated level of YAP activity as compared to expression of YAP activity in a reference sample or to a pre-determined level of YAP activity are provided. In some embodiments, methods of identifying a human subject for treatment with an IgCAM-binding agent, comprising determining if the subject has a tumor that has increased expression of YAP-dependent genes as compared to expression of YAP-dependent genes in a reference sample or to a pre-determined level are provided.

Additionally, the present invention provides methods of identifying a human subject for treatment with an IgCAM-binding agent, comprising determining if the subject has a tumor that has an elevated level of non-phosphorylated TAZ as compared to expression of non-phosphorylated TAZ in a reference sample or to a pre-determined level of non-phosphorylated TAZ. In some embodiments, if the tumor has an elevated level of non-phosphorylated TAZ, the subject is selected for treatment with an IgCAM-binding agent that specifically binds at least one IgCAM. In some embodiments, if selected for treatment, the subject is administered an IgCAM-binding agent described herein. In certain embodiments, the subject has had a tumor removed. In some embodiments, methods of identifying a human subject for treatment with an IgCAM-binding agent, comprising determining if the subject has a tumor that has an elevated level of TAZ activity as compared to expression of TAZ activity in a reference sample or to a pre-determined level of TAZ activity are provided. In some embodiments, methods of identifying a human subject for treatment with an IgCAM-binding agent, comprising determining if the subject has a tumor that has increased expression of TAZ-dependent genes as compared to expression of TAZ-dependent genes in a reference sample or to a pre-determined level are provided. The present invention also provides methods of treating cancer in a human subject, comprising: (a) selecting a subject for treatment based, at least in part, on the subject having a cancer that has an elevated level of non-phosphorylated YAP and/or has an elevated level of non-phosphorylated TAZ, and (b) administering to the subject a therapeutically effective amount of an IgCAM-binding agent described herein.

Methods for determining the level of YAP and/or TAZ expression in a cell, tumor, or cancer are known by those of skill in the art. For nucleic acid expression these methods include, but are not limited to, PCR-based assays, microarray analyses, and nucleotide sequencing (e.g., NextGen sequencing). For protein expression, these methods include, but are not limited, Western blot analysis, protein arrays, immunohistochemistry (IHC) assays, ELISAs, and FACS.

Methods for determining whether a tumor or cancer has an elevated level of YAP and/or TAZ expression can use a variety of samples. In some embodiments, the sample is taken from a subject having a tumor or cancer. In some embodiments, the sample is a fresh tumor/cancer sample. In some embodiments, the sample is a frozen tumor/cancer sample. In some embodiments, the sample is a formalin-fixed paraffin-embedded sample. In some embodiments, the sample is processed to a cell lysate. In some embodiments, the sample is processed to DNA or RNA.

Methods of treating a disease or disorder in a subject, wherein the disease or disorder is associated with aberrant (e.g., decreased levels) Hippo pathway signaling are further provided. In some embodiments, the treatment methods comprise administering a therapeutically effective amount of an IgCAM-binding agent to the subject. In some embodiments, the IgCAM-binding agent is an antibody. In some embodiments, the IgCAM-binding agent is a soluble receptor.

The invention also provides a method of activating or enhancing Hippo pathway signaling in a cell comprising contacting the cell with an effective amount of an IgCAM-binding agent. In certain embodiments, the cell is a tumor cell. In certain embodiments, the method is an *in vivo* method wherein the step of contacting the cell with the IgCAM-binding agent comprises administering a therapeutically effective amount of the IgCAM-binding agent to the subject. In some embodiments, the method is an *in vitro* or *ex vivo* method. In certain embodiments, the IgCAM-binding agent activates, induces, enhances, and/or increases Hippo pathway signaling. In some embodiments, the IgCAM-binding agent inhibits activation of YAP. In some embodiments, the IgCAM-binding agent inhibits activation of TAZ. In some embodiments, the IgCAM-binding agent is an antibody. In some embodiments, the IgCAM-binding agent is a soluble receptor.

The invention also provides a method of inactivating or inhibiting Hippo pathway signaling in a cell comprising contacting the cell with an effective amount of an IgCAM-binding agent. In certain embodiments, the method is an *in vivo* method wherein the step of contacting the cell with the IgCAM-binding agent comprises administering a therapeutically effective amount of the IgCAM-binding agent to the subject. In some embodiments, the method is *an in vitro* or *ex vivo* method. In certain embodiments, the IgCAM-binding agent inhibits, suppresses, and/or decreases Hippo pathway signaling. In some embodiments, the IgCAM-binding agent increases activation of YAP. In some embodiments, the IgCAM-binding agent increases activation of TAZ. In some embodiments, the IgCAM-binding agent is an antibody. In some embodiments, the IgCAM-binding agent is a soluble receptor.

In another aspect, the invention provides methods of treating a wound, and/or promoting or enhancing wound healing in a subject. In some embodiments, the methods comprise administering to a subject a therapeutically effective amount of an IgCAM-binding agent that modulates Hippo pathway signaling. In some embodiments, the IgCAM-binding agent is an antagonist of Hippo pathway activity, suppresses Hippo pathway signaling, increases YAP activity, suppresses phosphorylation of YAP, suppresses degradation of YAP, and/or promotes activation of YAP. In some embodiments, the administered binding agent increases TAZ activity, suppresses phosphorylation of TAZ, suppresses degradation of TAZ, and/or promotes activation of TAZ. In some embodiments, the wound is an acute wound. In some embodiments, the wound is a surgical wound. In some embodiments, the wound is a chronic cutaneous wound.

In another aspect, the invention provides methods of regenerating tissue. In some embodiments, the methods comprise contacting a cell with an effective amount of an IgCAM-binding agent that modulates Hippo pathway signaling. In some embodiments, the methods comprise administering to a subject a therapeutically effective amount of an IgCAM-binding agent that modulates Hippo pathway signaling. In some embodiments, the IgCAM-binding agent is an antagonist of Hippo pathway activity, suppresses Hippo pathway signaling, increases YAP activity, suppresses phosphorylation of YAP, suppresses degradation of YAP, and/or promotes activation of YAP. In some embodiments, the IgCAM-binding agent increases TAZ activity, suppresses phosphorylation of TAZ, suppresses degradation of TAZ, and/or promotes activation of TAZ. In some embodiments, the method involves the treatment of tissue damage caused by: immune related disorders (such as autoimmune disorders); inflammation (including both acute and chronic inflammatory disorders); ischemia (such as myocardial infarction); traumatic injury (such as bums, lacerations, and abrasions); infection (such as bacterial, viral, and fungal infections); and chronic disease (such as cirrhosis of the liver).

In another aspect, the invention provides methods of targeting tumor cells with an IgCAM-binding agent, such as an agent described herein. Over-expression of IgCAMs on tumor cells or aberrant exposure of IgCAMs on tumor cells, may allow the IgCAMs to serve as targets for surveillance or immunosurveillance by the binding agents described herein. For example, within most normal cellular architecture IgCAMs would be expressed at the intercellular surfaces and would not be detected by IgCAM-binding agents. However, a tumor which has lost normal cellular architecture may comprise cells with aberrant exposure of IgCAMs, making these cells detectable by surveillance with IgCAM-binding agents. As the IgCAM-binding agents can be conjugated to cytotoxic agents, the aberrant exposure and/or expression of IgCAMs on tumor cells may also allow for targeted delivery of cytotoxins to the tumor cells. Thus, in some embodiments, the methods comprise administering to a subject an effective amount of an IgCAM-binding agent. In some embodiments, the binding agent is an antibody. In some embodiments, the IgCAM-binding agent is a soluble receptor. In some embodiments, the IgCAM-binding agent is conjugated to or complexed with a cytotoxic agent.

The present invention further provides pharmaceutical compositions comprising the IgCAM-binding agents described herein. In certain embodiments, the pharmaceutical compositions further comprise a pharmaceutically acceptable vehicle. These pharmaceutical compositions find use in inhibiting tumor growth and treating cancer in a subject (e.g., a human patient).

In certain embodiments, formulations are prepared for storage and use by combining a purified binding agent of the present invention with a pharmaceutically acceptable vehicle (e.g., a carrier or excipient). Suitable pharmaceutically acceptable vehicles include, but are not limited to, nontoxic buffers such as phosphate, citrate, and other organic acids; salts such as sodium chloride; antioxidants including ascorbic acid and methionine; preservatives such as octadecyldimethylbenzyl ammonium chloride, hexamethonium chloride, benzalkonium chloride, benzethonium chloride, phenol, butyl or benzyl alcohol, alkyl parabens, such as methyl or propyl paraben, catechol, resorcinol, cyclohexanol, 3-pentanol, and m-cresol; low molecular weight polypeptides (e.g., less than about 10 amino acid residues); proteins such as serum albumin, gelatin, or immunoglobulins; hydrophilic polymers such as polyvinylpyrrolidone; amino acids such as glycine, glutamine, asparagine, histidine, arginine, or lysine; carbohydrates such as monosaccharides, disaccharides, glucose, mannose, or dextrins; chelating agents such as EDTA; sugars such as sucrose, mannitol, trehalose or sorbitol; salt-forming counter-ions such as sodium; metal complexes such as Zn-protein complexes; and non-ionic surfactants such as TWEEN or polyethylene glycol (PEG). (Remington: The Science and Practice of Pharmacy, 21st Edition, 2005, University of the Sciences in Philadelphia, PA).

The pharmaceutical compositions of the present invention can be administered in any number of ways for either local or systemic treatment. Administration can be topical by epidermal or transdermal patches, ointments, lotions, creams, gels, drops, suppositories, sprays, liquids and powders; pulmonary by inhalation or insufflation of powders or aerosols, including by nebulizer, intratracheal, and intranasal; oral; or parenteral including intravenous, intraarterial, intratumoral, subcutaneous, intraperitoneal, intramuscular (e.g., injection or infusion), or intracranial (e.g., intrathecal or intraventricular).

The therapeutic formulation can be in unit dosage form. Such formulations include tablets, pills, capsules, powders, granules, solutions or suspensions in water or non-aqueous media, or suppositories. In solid compositions such as tablets the principal active ingredient is mixed with a pharmaceutical carrier. Conventional tableting ingredients include corn starch, lactose, sucrose, sorbitol, talc, stearic acid, magnesium stearate, dicalcium phosphate or gums, and diluents (e.g., water). These can be used to form a solid preformulation composition containing a homogeneous mixture of a compound of the present invention, or a non-toxic pharmaceutically acceptable salt thereof. The solid preformulation composition is then subdivided into unit dosage forms of a type described above. The tablets, pills, etc. of the formulation or composition can be coated or otherwise compounded to provide a dosage form affording the advantage of prolonged action. For example, the tablet or pill can comprise an inner composition covered by an outer component. Furthermore, the two components can be separated by an enteric layer that serves to resist disintegration and permits the inner component to pass intact through the stomach or to be delayed in release. A variety of materials can be used for such enteric layers or coatings, such materials include a number of polymeric acids and mixtures of polymeric acids with such materials as shellac, cetyl alcohol and cellulose acetate.

The IgCAM-binding agents described herein can also be entrapped in microcapsules. Such microcapsules are prepared, for example, by coacervation techniques or by interfacial polymerization, for example, hydroxymethylcellulose or gelatin-microcapsules and poly-(methylmethacylate) microcapsule, respectively, in colloidal drug delivery systems (for example, liposomes, albumin microspheres, microemulsions, nanoparticles and nanocapsules) or in macroemulsions as described in Remington: The Science and Practice of Pharmacy, 21st Edition, 2005, University of the Sciences in Philadelphia, PA.

In certain embodiments, pharmaceutical formulations include an IgCAM-binding agent of the present invention complexed with liposomes. Methods to produce liposomes are known to those of skill in the art. For example, some liposomes can be generated by reverse phase evaporation with a lipid composition comprising phosphatidylcholine, cholesterol, and PEG-derivatized phosphatidylethanolamine (PEG-PE). Liposomes can be extruded through filters of defined pore size to yield liposomes with the desired diameter.

In certain embodiments, sustained-release preparations can be produced. Suitable examples of sustained-release preparations include semi-permeable matrices of solid hydrophobic polymers containing an IgCAM-binding agent, where the matrices are in the form of shaped articles (e.g., films or microcapsules). Examples of sustained-release matrices include polyesters, hydrogels such as poly(2-hydroxyethyl-methacrylate) or poly(vinyl alcohol), polylactides, copolymers of L-glutamic acid and 7 ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), sucrose acetate isobutyrate, and poly-D-(-)-3-hydroxybutyric acid.

In certain embodiments, in addition to administering an IgCAM-binding agent, the method or treatment further comprises administering at least one additional therapeutic agent. An additional therapeutic agent can be administered prior to, concurrently with, and/or subsequently to, administration of the IgCAM-binding agent. Pharmaceutical compositions comprising an IgCAM-binding agent and the additional therapeutic agent(s) are also provided. In some embodiments, the at least one additional therapeutic agent comprises 1, 2, 3, or more additional therapeutic agents.

Combination therapy with two or more therapeutic agents often uses agents that work by different mechanisms of action, although this is not required. Combination therapy using agents with different mechanisms of action may result in additive or synergetic effects. Combination therapy may allow for a lower dose of each agent than is used in monotherapy, thereby reducing toxic side effects and/or increasing the therapeutic index of the agent(s). Combination therapy may decrease the likelihood that resistant cancer cells will develop. In some embodiments, combination therapy comprises a therapeutic agent that affects (e.g., inhibits or kills) non-tumorigenic cells and a therapeutic agent that affects (e.g., inhibits or kills) tumorigenic CSCs.

In some embodiments, the combination of an IgCAM-binding agent and at least one additional therapeutic agent results in additive or synergistic results. In some embodiments, the combination therapy results in an increase in the therapeutic index of the IgCAM-binding agent. In some embodiments, the combination therapy results in an increase in the therapeutic index of the additional agent(s). In some embodiments, the combination therapy results in a decrease in the toxicity and/or side effects of the IgCAM-binding agent. In some embodiments, the combination therapy results in a decrease in the toxicity and/or side effects of the additional agent(s).

Useful classes of therapeutic agents include, for example, antitubulin agents, auristatins, DNA minor groove binders, DNA replication inhibitors, alkylating agents (e.g., platinum complexes such as cisplatin, mono(platinum), bis(platinum) and tri-nuclear platinum complexes and carboplatin), anthracyclines, antibiotics, antifolates, antimetabolites, chemotherapy sensitizers, duocarmycins, etoposides, fluorinated pyrimidines, ionophores, lexitropsins, nitrosoureas, platinols, purine antimetabolites, puromycins, radiation sensitizers, steroids, taxanes, topoisomerase inhibitors, vinca alkaloids, or the like. In certain embodiments, the second therapeutic agent is an alkylating agent, an antimetabolite, an antimitotic, a topoisomerase inhibitor, or an angiogenesis inhibitor. In some embodiments, the second therapeutic agent is a platinum complex such as carboplatin or cisplatin. In some embodiments, the additional therapeutic agents are a platinum complex and a taxane.

Therapeutic agents that may be administered in combination with the IgCAM-binding agents include chemotherapeutic agents. Thus, in some embodiments, the method or treatment involves the administration of an IgCAM-binding agent of the present invention in combination with a chemotherapeutic agent or in combination with a cocktail of chemotherapeutic agents. Treatment with an IgCAM-binding agent can occur prior to, concurrently with, or subsequent to administration of chemotherapies. Combined administration can include co-administration, either in a single pharmaceutical formulation or using separate formulations, or consecutive administration in either order but generally within a time period such that all active agents can exert their biological activities simultaneously. Preparation and dosing schedules for such chemotherapeutic agents can be used according to manufacturers' instructions or as determined empirically by the skilled practitioner. Preparation and dosing schedules for such chemotherapy are also described in The Chemotherapy Source Book, 4th Edition, 2008, M. C. Perry, Editor, Lippincott, William & Wilkins, Philadelphia, PA.

Chemotherapeutic agents useful in the instant invention include, but are not limited to, alkylating agents such as thiotepa and cyclosphosphamide (CYTOXAN); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamime; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytosine arabinoside, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenishers such as folinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK; razoxane; sizofuran; spirogermanium; tenuazonic acid; triaziquone; 2,2',2"-trichlorotriethylamine; urethan; vindesine; dacarbazine; mannomustine; mitobronitol; mitolactol; pipobroman; gacytosine; arabinoside (Ara-C); taxoids, e.g. paclitaxel (TAXOL) and docetaxel (TAXOTERE); chlorambucil; gemcitabine; 6-thioguanine; mercaptopurine; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; ibandronate; CPT11; topoisomerase inhibitor RFS 2000; difluoromethylornithine (DMFO); retinoic acid; esperamicins; capecitabine (XELODA); and pharmaceutically acceptable salts, acids or derivatives of any of the above. Chemotherapeutic agents also include anti-hormonal agents that act to regulate or inhibit hormone action on tumors such as antiestrogens including for example tamoxifen, raloxifene, aromatase inhibiting 4(5)-imidazoles, 4-hydroxylamoxifen, trioxifene, keoxifene, LY117018, onapristone, and toremifene (FARESTON); and anti-androgens such as flutamide, nilutamide, bicalutamide, leuprolide, and goserelin; and pharmaceutically acceptable salts, acids or derivatives of any of the above. In certain embodiments, the additional therapeutic agent is cisplatin. In certain embodiments, the additional therapeutic agent is carboplatin.

In certain embodiments, the chemotherapeutic agent is a topoisomerase inhibitor. Topoisomerase inhibitors are chemotherapy agents that interfere with the action of a topoisomerase enzyme (e.g., topoisomerase I or II). Topoisomerase inhibitors include, but are not limited to, doxorubicin HCl, daunorubicin citrate, mitoxantrone HCl, actinomycin D, etoposide, topotecan HCl, teniposide (VM-26), and irinotecan, as well as pharmaceutically acceptable salts, acids, or derivatives of any of these. In some embodiments, the additional therapeutic agent is irinotecan.

In certain embodiments, the chemotherapeutic agent is an anti-metabolite. An anti-metabolite is a chemical with a structure that is similar to a metabolite required for normal biochemical reactions, yet different enough to interfere with one or more normal functions of cells, such as cell division. Anti-metabolites include, but are not limited to, gemcitabine, fluorouracil, capecitabine, methotrexate sodium, ralitrexed, pemetrexed, tegafur, cytosine arabinoside, thioguanine, 5-azacytidine, 6-mercaptopurine, azathioprine, 6-thioguanine, pentostatin, fludarabine phosphate, and cladribine, as well as pharmaceutically acceptable salts, acids, or derivatives of any of these. In certain embodiments, the additional therapeutic agent is gemcitabine.

In certain embodiments, the chemotherapeutic agent is an antimitotic agent, including, but not limited to, agents that bind tubulin. In some embodiments, the agent is a taxane. In certain embodiments, the agent is paclitaxel or docetaxel, or a pharmaceutically acceptable salt, acid, or derivative of paclitaxel or docetaxel. In certain embodiments, the agent is paclitaxel (TAXOL), docetaxel (TAXOTERE), albumin-bound paclitaxel (ABRAXANE), DHA-paclitaxel, or PG-paclitaxel. In certain alternative embodiments, the antimitotic agent comprises a vinca alkaloid, such as vincristine, binblastine, vinorelbine, or vindesine, or pharmaceutically acceptable salts, acids, or derivatives thereof In some embodiments, the antimitotic agent is an inhibitor of kinesin Eg5 or an inhibitor of a mitotic kinase such as Aurora A or Plk1. In certain embodiments, the additional therapeutic agent is paclitaxel.

In some embodiments, an additional therapeutic agent comprises an agent such as a small molecule. For example, treatment can involve the combined administration of an IgCAM-binding agent of the present invention with a small molecule that acts as an inhibitor against tumor-associated antigens including, but not limited to, EGFR, HER2 (ErbB2), and/or VEGF. In some embodiments, an IgCAM-binding agent of the present invention is administered in combination with a protein kinase inhibitor selected from the group consisting of: gefitinib (IRESSA), erlotinib (TARCEVA), sunitinib (SUTENT), lapatanib, vandetanib (ZACTIMA), AEE788, CI-1033, cediranib (RECENTIN), sorafenib (NEXAVAR), and pazopanib (GW786034B). In some embodiments, an additional therapeutic agent comprises an mTOR inhibitor.

In certain embodiments, the additional therapeutic agent is a small molecule that inhibits a cancer stem cell pathway. In some embodiments, the additional therapeutic agent is an inhibitor of the Notch pathway. In some embodiments, the additional therapeutic agent is an inhibitor of the Wnt pathway. In some embodiments, the additional therapeutic agent is an inhibitor of the BMP pathway. In some embodiments, the additional therapeutic agent is an inhibitor of the mTOR/AKR pathway.

In some embodiments, an additional therapeutic agent comprises a biological molecule, such as an antibody. For example, treatment can involve the combined administration of an IgCAM-binding agent of the present invention with other antibodies against tumor-associated antigens including, but not limited to, antibodies that bind EGFR, HER2/ErbB2, and/or VEGF. In certain embodiments, the additional therapeutic agent is an antibody specific for a cancer stem cell marker. In some embodiments, the additional therapeutic agent is an antibody that binds a component of the Notch pathway. In some embodiments, the additional therapeutic agent is an antibody that binds a component of the Wnt pathway. In certain embodiments, the additional therapeutic agent is an antibody that inhibits a cancer stem cell pathway. In some embodiments, the additional therapeutic agent is an inhibitor of the Notch pathway. In some embodiments, the additional therapeutic agent is an inhibitor of the Wnt pathway. In some embodiments, the additional therapeutic agent is an inhibitor of the BMP pathway. In some embodiments, the additional therapeutic agent is an antibody that inhibits β-catenin signaling. In certain embodiments, the additional therapeutic agent is an antibody that is an angiogenesis inhibitor (e.g., an anti-VEGF or VEGF receptor antibody). In certain embodiments, the additional therapeutic agent is bevacizumab (AVASTIN), ramucirumab, trastuzumab (HERCEPTIN), pertuzumab (OMNITARG), panitumumab (VECTIBIX), nimotuzumab, zalutumumab, or cetuximab (ERBITUX).

Furthermore, treatment with an IgCAM-binding agent described herein can include combination treatment with other biologic molecules, such as one or more cytokines (e.g., lymphokines, interleukins, tumor necrosis factors, and/or growth factors) or can be accompanied by surgical removal of tumors, removal of cancer cells, or any other therapy deemed necessary by a treating physician.

In some embodiments, for example, for tissue regeneration, the IgCAM-binding agent can be combined with a growth factor selected from the group consisting of: adrenomedullin (AM), angiopoietin (Ang), BMPs, BDNF, EOF, erythropoietin (EPO), FGF, GDNF, G-CSF, GM-CSF, GDF9, HGF, HDGF, IGF, migration-stimulating factor, myostatin (GDF-8), NGF, neurotrophins, PDGF, thrombopoietin, TGF-a, TGF-β, TNF-α, VEGF, PIGF, IL-1, IL-2, IL-3, IL-4, IL-5, IL-6 and IL-7.

In certain embodiments, the treatment involves the administration of an IgCAM-binding agent of the present invention in combination with radiation therapy. Treatment with an IgCAM-binding agent can occur prior to, concurrently with, or subsequent to administration of radiation therapy. Dosing schedules for such radiation therapy can be determined by the skilled medical practitioner.

Combined administration can include co-administration, either in a single pharmaceutical formulation or using separate formulations, or consecutive administration in either order but generally within a time period such that all active agents can exert their biological activities simultaneously.

It will be appreciated that the combination of an IgCAM-binding agent and at least one additional therapeutic agent may be administered in any order or concurrently. In some embodiments, the IgCAM-binding agent will be administered to patients that have previously undergone treatment with a second therapeutic agent. In certain other embodiments, the IgCAM-binding agent and a second therapeutic agent will be administered substantially simultaneously or concurrently. For example, a subject may be given an IgCAM-binding agent (e.g., an antibody) while undergoing a course of treatment with a second therapeutic agent (e.g., chemotherapy). In certain embodiments, an IgCAM-binding agent will be administered within 1 year of the treatment with a second therapeutic agent. In certain alternative embodiments, an IgCAM-binding agent will be administered within 10, 8, 6, 4, or 2 months of any treatment with a second therapeutic agent. In certain other embodiments, an IgCAM-binding agent will be administered within 4, 3, 2, or 1 weeks of any treatment with a second therapeutic agent. In some embodiments, an IgCAM-binding agent will be administered within 5,4, 3, 2, or 1 days of any treatment with a second therapeutic agent. It will further be appreciated that the two (or more) agents or treatments may be administered to the subject within a matter of hours or minutes (i.e., substantially simultaneously).

For the treatment of a disease, the appropriate dosage of an IgCAM-binding agent of the present invention depends on the type of disease to be treated, the severity and course of the disease, the responsiveness of the disease, whether the IgCAM-binding agent is administered for therapeutic or preventative purposes, previous therapy, the patient's clinical history, and so on, all at the discretion of the treating physician. The IgCAM -binding agent can be administered one time or over a series of treatments lasting from several days to several months, or until a cure is effected or a diminution of the disease state is achieved (e.g., reduction in tumor size). Optimal dosing schedules can be calculated from measurements of drug accumulation in the body of the patient and will vary depending on the relative potency of an individual agent. The administering physician can easily determine optimum dosages, dosing methodologies, and repetition rates. In certain embodiments, dosage is from 0.01µg to 100mg/kg of body weight, from 0.1µg to 100mg/kg of body weight, from 1µg to 100mg/kg of body weight, from 1mg to 100mg/kg of body weight, 1mg to 80mg/kg of body weight from 10mg to 100mg/kg of body weight, from 10mg to 75mg/kg of body weight, or from 10mg to 50mg/kg of body weight. In certain embodiments, the dosage of the IgCAM-binding agent is from about 0.1mg to about 20mg/kg of body weight. In certain embodiments, dosage can be given once or more daily, weekly, monthly, or yearly. In certain embodiments, the IgCAM-binding agent is given once every week, once every two weeks or once every three weeks.

In some embodiments, an IgCAM-binding agent may be administered at an initial higher "loading" dose, followed by one or more lower doses. In some embodiments, the frequency of administration may also change. In some embodiments, a dosing regimen may comprise administering an initial dose, followed by additional doses (or "maintenance" doses) once a week, once every two weeks, once every three weeks, or once every month. For example, a dosing regimen may comprise administering an initial loading dose, followed by a weekly maintenance dose of, for example, one-half of the initial dose. Or a dosing regimen may comprise administering an initial loading dose, followed by maintenance doses of, for example one-half of the initial dose every other week. Or a dosing regimen may comprise administering three initial doses for 3 weeks, followed by maintenance doses of, for example, the same amount every other week.

As is known to those of skill in the art, administration of any therapeutic agent may lead to side effects and/or toxicities. In some cases, the side effects and/or toxicities are so severe as to preclude administration of the particular agent at a therapeutically effective dose. In some cases, drug therapy must be discontinued, and other agents may be tried. However, many agents in the same therapeutic class often display similar side effects and/or toxicities, meaning that the patient either has to stop therapy, or if possible, suffer from the unpleasant side effects associated with the therapeutic agent.

Thus, the present invention provides methods of treating cancer in a subject comprising using an intermittent dosing strategy for administering one or more agents, which may reduce side effects and/or toxicities associated with administration of an IgCAM-binding agent, chemotherapeutic agent, etc. In some embodiments, a method for treating cancer in a human subject comprises administering to the subject a therapeutically effective dose of an IgCAM-binding agent in combination with a therapeutically effective dose of a chemotherapeutic agent, wherein one or both of the agents are administered according to an intermittent dosing strategy. In some embodiments, the intermittent dosing strategy comprises administering an initial dose of an IgCAM-binding agent to the subject, and administering subsequent doses of the IgCAM-binding agent about once every 2 weeks. In some embodiments, the intermittent dosing strategy comprises administering an initial dose of an IgCAM-binding agent to the subject, and administering subsequent doses of the IgCAM-binding agent about once every 3 weeks. In some embodiments, the intermittent dosing strategy comprises administering an initial dose of an IgCAM-binding agent to the subject, and administering subsequent doses of the IgCAM-binding agent about once every 4 weeks. In some embodiments, the IgCAM-binding agent is administered using an intermittent dosing strategy and the chemotherapeutic agent is administered weekly.

### V. Screening

The present invention provides screening methods to identify agents that modulate the Hippo pathway. In some embodiments, the present invention provides methods for screening candidate agents, including but not limited to, proteins, antibodies, soluble receptors, peptides, peptidomimetics, small molecules, compounds, or other drugs, which modulate Hippo pathway activity.

In some embodiments, a method of screening for and/or identifying a candidate or test agent that modulates the Hippo pathway comprises determining if the agent has an effect on Hippo pathway components. In some embodiments, a method of screening for an agent that modulates the Hippo pathway comprises: screening for an agent that specifically binds the extracellular domain of an IgCAM, wherein the agent modulates the Hippo pathway. In some embodiments, a method of screening for a candidate or test agent that modulates the Hippo pathway comprises determining if the agent has an effect on YAP activity. In some embodiments, a method of screening for a candidate or test agent that modulates the Hippo pathway comprises determining if the agent has an effect on TAZ activity. In some embodiments, a method of screening for a candidate or test agent that modulates the Hippo pathway comprises determining if the agent decreases YAP and/or TAZ activity. In some embodiments, a method of screening for a candidate or test agent that modulates the Hippo pathway comprises determining if the agent increases YAP and/or TAZ activity. In some embodiments, a method of screening for a candidate or test agent that modulates the Hippo pathway comprises determining if the agent decreases expression of YAP-dependent genes or TAZ-dependent genes. In some embodiments, a method of screening for a candidate or test agent that modulates the Hippo pathway comprises determining if the agent increases expression of YAP-dependent genes or TAZ-dependent genes. In some embodiments, a method of screening for a candidate or test agent that modulates the Hippo pathway comprises determining if the agent decreases or increases expression of at least one gene selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GL12, BIRC2, BIRC5 (survivin), FGF1, IL33, GRB2, IGFBP3, AREG, FN1, ADAMTS1, AXL, ADAPTS5, MET, CYR61, IL8, ZEB1, FOXC2, N-cadherin, and SNAIL. In some embodiments, a method of screening for a candidate or test agent that modulates the Hippo pathway comprises determining if the agent has an effect on YAP and/or TAZ phosphorylation. In some embodiments, a method of screening for a candidate or test agent that modulates the Hippo pathway comprises determining if the agent increases YAP and/or TAZ phosphorylation. In some embodiments, a method of screening for a candidate or test agent that modulates the Hippo pathway comprises determining if the agent decreases YAP and/or TAZ phosphorylation. In some embodiments, a method of screening for a candidate or test agent that modulates the Hippo pathway comprises determining if the agent decreases non-phosphorylated YAP and/or non-phosphorylated TAZ. In some embodiments, a method of screening for a candidate or test agent that modulates the Hippo pathway comprises determining if the agent increases non-phosphorylated YAP and/or non-phosphorylated TAZ. In some embodiments, a method of screening for a candidate or test agent that modulates the Hippo pathway comprises determining if the agent increases the level of YAP and/or TAZ in the cytoplasm. In some embodiments, a method of screening for a candidate or test agent that modulates the Hippo pathway comprises determining if the agent decreases the level of YAP and/or TAZ in the cytoplasm. In some embodiments, a method of screening for a candidate or test agent that modulates the Hippo pathway comprises determining if the agent increases YAP and/or TAZ degradation. In some embodiments, a method of screening for a candidate or test agent that modulates the Hippo pathway comprises determining if the agent decreases YAP and/or TAZ degradation. In some embodiments, a method of screening for a candidate or test agent that modulates the Hippo pathway comprises determining if the agent decreases the level of YAP and/or TAZ in the nucleus. In some embodiments, a method of screening for a candidate or test agent that modulates the Hippo pathway comprises determining if the agent increases the level of YAP and/or TAZ in the nucleus.

In some embodiments, a method of screening for a candidate or test agent that modulates the Hippo pathway comprises: contacting a first set of cells, but not a second set of cells, with a candidate or test agent, and comparing the expression of YAP-dependent genes and/or TAZ-dependent genes in the first set of cells to expression of YAP-dependent genes and/or TAZ-dependent genes in the second set of cells. In some embodiments, a method of screening for a candidate or test agent that modulates the Hippo pathway comprises: contacting a first set of cells, but not a second set of cells, with a candidate or test agent, and determining the expression of at least one YAP-dependent gene and/or at least one TAZ-dependent gene in the first set of cells as compared to expression of the YAP-dependent gene and/or the TAZ-dependent gene in the second set of cells. In some embodiments, the at least one YAP-dependent gene or the at least one TAZ-dependent gene is selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GLI2, BIRC2, BIRC5 (survivin), FGF1, IL33, GRB2, IGFBP3, AREG, FN1, ADAMTS1, AXL, ADAMTS5, MET, CYR61, IL8, ZEB1, FOXC2, N-cadherin, and SNAIL. In some embodiments, if the expression of at least one YAP-dependent gene and/or at least one TAZ-dependent gene in the first set of cells has increased as compared to the expression of the YAP-dependent gene(s) and/or the TAZ-dependent gene(s) in the second set of cells, then the agent inhibited the Hippo pathway. In some embodiments, if the expression of at least one YAP-dependent gene and/or at least one TAZ-dependent gene in the first set of cells has decreased as compared to the expression of the YAP-dependent gene(s) and/or the TAZ-dependent gene(s) in the second set of cells, then the agent activated the Hippo pathway.

In some embodiments, a method of screening for a candidate or test agent that the modulates the Hippo pathway comprises: contacting a first set of cells, but not a second set of cells, with a candidate or test agent, and comparing the phosphorylation state of Mst1, Mst2, Lats1, Lats2, YAP, and/or TAZ in the first set of cells to the phosphorylation state of Mst1, Mst2, Lats1, Lats2, YAP, and/or TAZ in the second set of cells. In some embodiments, a method of screening for a candidate or test agent that the modulates the Hippo pathway comprises: contacting a first set of cells, but not a second set of cells, with a candidate or test agent, and determining the phosphorylation state of Mst1, Mst2, Lats1, Lats2, YAP, and/or TAZ in the first set of cells as compared to the phosphorylation state of Mst1, Mst2, Lats1, Lats2, YAP, and/or TAZ in the second set of cells.

In some embodiments, a method of screening for a candidate or test agent that the modulates the Hippo pathway comprises: contacting a first set of cells, but not a second set of cells, with a candidate or test agent, and determining the amount of phosphorylated YAP and/or phosphorylated TAZ in the first set of cells as compared to the second set of cells. In some embodiments, if the amount of phosphorylated YAP and/or phosphorylated TAZ in the first set of cells has increased as compared to the second set of cells, then the agent has activated the Hippo pathway. In some embodiments, if the amount of phosphorylated YAP and/or phosphorylated TAZ in the first set of cells has decreased as compared to the second set of cells, then the agent has inhibited the Hippo pathway.

In some embodiments, a method of screening for a candidate or test agent that the modulates the Hippo pathway comprises: contacting a first set of cells, but not a second set of cells, with a candidate or test agent, and determining the amount of non-phosphorylated YAP and/or non-phosphorylated TAZ in the first set of cells as compared to the second set of cells. In some embodiments, if the amount of non-phosphorylated YAP and/or non-phosphorylated TAZ in the first set of cells has increased as compared to the second set of cells, then the agent has inhibited the Hippo pathway. In some embodiments, if the amount of non-phosphorylated YAP and/or non-phosphorylated TAZ in the first set of cells has decreased as compared to the second set of cells, then the agent has activated the Hippo pathway.

In some embodiments, a method of screening for a candidate or test agent that the modulates the Hippo pathway comprises: contacting a first set of cells, but not a second set of cells, with a candidate or test agent, and comparing the location (nuclear or cytoplasmic) of YAP and/or TAZ in the first set of cells to the second set of cells. In some embodiments, if the candidate agent stimulates or activates the Hippo pathway, the first set of cells has a higher level of YAP and/or TAZ in the cytoplasm than the second set of cells.

In some embodiments, a method of screening for a candidate or test agent that modulates the Hippo pathway comprises: (a) contacting cells with a candidate or test agent, and (b) determining the cellular localization of YAP and/or TAZ using a bimolecular fluorescence complementation (BiFC) assay. In some embodiments of the method, if YAP and/or TAZ is predominantly localized in the cytoplasm of the cell, then the Hippo pathway is activated. In some embodiments of the method, if YAP and/or TAZ is predominantly localized in the nucleus of the cell, then the Hippo pathway is inhibited.

The invention also provides agents that modulate the Hippo pathway identified by any of the screening methods described herein.

### VI. Kits comprising IgCAM-binding agents

The present invention provides kits that comprise the IgCAM-binding agents described herein and that can be used to perform the methods described herein. In certain embodiments, a kit comprises at least one purified IgCAM-binding agent in one or more containers. In some embodiments, the kits contain all of the components necessary and/or sufficient to perform a detection assay, including all controls, directions for performing assays, and any necessary software for analysis and presentation of results. One skilled in the art will readily recognize that the disclosed IgCAM-binding agents of the present invention can be readily incorporated into one of the established kit formats which are well known in the art.

Further provided are kits comprising an IgCAM-binding agent as well as at least one additional therapeutic agent. In certain embodiments, the second (or more) therapeutic agent is a chemotherapeutic agent. In certain embodiments, the second (or more) therapeutic agent is an angiogenesis inhibitor.

Embodiments of the present disclosure can be further defined by reference to the following nonlimiting examples, which describe in detail preparation of certain antibodies of the present disclosure and methods for using antibodies of the present disclosure. It will be apparent to those skilled in the art that many modifications, both to materials and methods, may be practiced without departing from the scope of the present disclosure.

### EXAMPLES

### Example 1

### Expression of YAP in OMP tumors

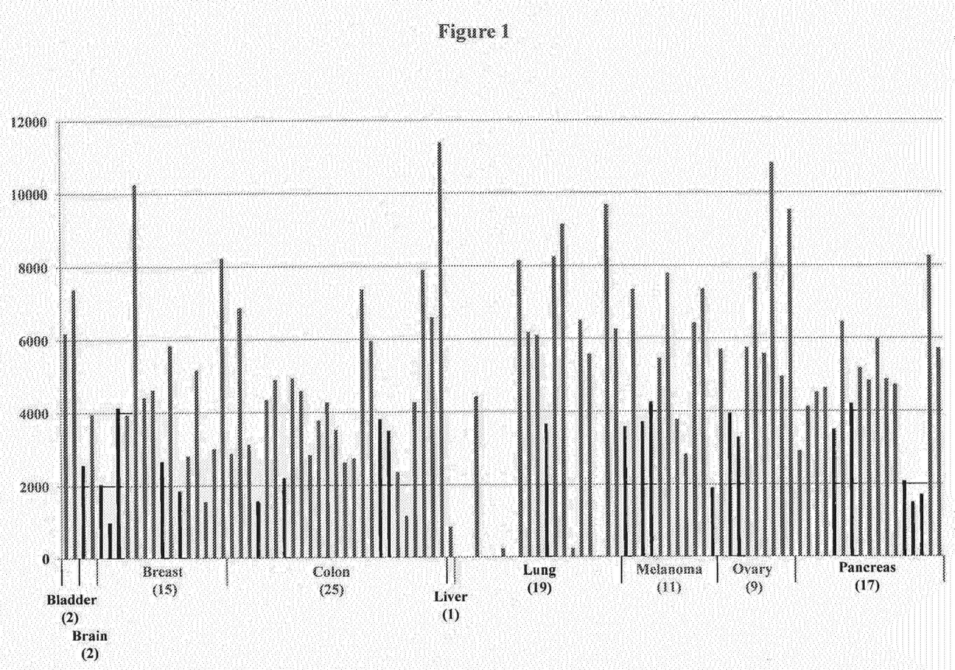

Microarray gene expression analyses of patient-derived tumors in the OncoMed tumor bank were done to determine the levels of YAP expression. 101 tumors including bladder (2), brain (2), breast (15), colon (25), liver (1), lung (19), melanoma (11), ovarian (9) and pancreas (17) were evaluated. Total RNA was isolated from these tumors that had been serially transplanted in NOD/SCID mice. RNA quality was monitored using a Bioanalyzer (Agilent, Santa Clara CA). The purified RNA was processed using established Affymetrix protocols. The samples were hybridized to Affymetrix HG-U133 plus 2.0 microarrays (Affymetrix, Santa Clara, CA) as outlined in the manufacturer's technical manuals. After hybridization, the microarrays were washed, scanned, and analyzed. Scanned array background adjustment and signal intensity normalization were performed using the GCRMA algorithm (Bioconductor, www.bioconductor.org). As shown in Figure 1, approximate 80% of the tumors demonstrated medium to high levels of YAP expression.

### Example 2

### Inhibition of tumor growth by dominant negative YAP

A mutant YAP gene was designed and synthesized that encodes for a dominant negative human YAP (dnYAP). The dnYAP protein comprises the YAP TEAD binding domain, the two WW domains, and the PDZ domain (SEQ ID NO:68). The YAP activation domain was deleted and the serine at amino acid 127 was replaced with an alanine residue (S127A). This substitution eliminates a key phosphorylation site and allows dnYAP to translocate from the cytoplasm to the nucleus.

To express dnYAP in tumor cells both *in vitro* and *in vivo*, the dnYAP gene was cloned into a lentiviral vector containing an immediate early CMV promoter. The lentiviral vector also contains an IRES-GFP portion which allows for visualization and selection of transduced cells. The lentiviral vector is a HIV-1-derived, replication deficient, tat-independent, self-inactivating '3rd generation' vector (see e.g., Durand et al., Viruses, 2011, 3:132-159). The dnYAP-expressing lentiviral vector was named pOM420. The dnYAP-expressing lentiviral vector and 3 helper plasmids were transiently transfected into HEK-293T cells to produce dnYAP lentivirus. The three helper plasmids provided the complementing functions necessary for virus production. To mediate viral entry into a wide variety of human tumor cells, the viruses were pseudotyped with the vesicular stomatitis virus envelope G protein (VSV-G).

Dissociated melanoma OMP-M6 cells (5 x 10⁵ cells/well) were seeded into 6-well Primaria™ plates (BD Biosciences, San Jose CA) and transduced with dnYAP-lentivirus (LOM420, CMV-dnYAP-IRES-GFP) or GFP-lentivirus (LOM92, CMV-IRES-GFP) as a control. Lentiviruses were added to the cells at time of plating (t = 0) at a multiplicity of infection (MOI) of 2.5 infectious particles per cell. Cells were incubated overnight with the lentiviruses, washed and re-incubated in culture media. The culture medium was DMEM Low glucose, F12 medium, B27 medium, insulin-transferrin-selenium supplement, heparin, rhEGF, rhFGF-2, and antibiotics. After 72 hours, cells were collected, trypsinized, washed, and resuspended in HBSS containing 2% FBS and 4',6-diamidino-2-phenylindole (DAPI, Molecular Probes/Invitrogen, Grand Island NY). Transduced melanoma OMP-M6 cells, identified as GFP positive and DAPI negative (GFP⁺/DNPI⁻), were sorted by flow cytometry using a FACSAria flow cytometer (BD Biosciences, San Jose CA). Cell population purity was confirmed by reanalysis of a fraction of the sorted cells and the cells were mixed with 50% Matrigel™ (BD Biosciences, San Jose CA). Control lentivirus-transduced GFP⁺/DAPI⁻ cells were subcutaneously injected into the flanks of NOD/SCID mice (200 cells/mouse, n = 10). dnYAP lentivirus-transduced GFP⁺/DAPI⁻ cells were subcutaneously injected into the flanks of NOD/SCID mice (200 cells/mouse, n = 8). Tumor growth was monitored for 3 months.

Mice injected with melanoma OMP-M6 cells expressing only GFP had palpable tumors at the site of injection approximately 40 days after injection. These tumors reached an average volume of 2000mm³ in 2.5 months. In contrast, mice injected with melanoma OMP-M6 cells expressing dnYAP had tumors that grew extremely slowly or not at all. The growth rate of the dnYAP-expressing melanoma OMP-M6 cells was approximately 17 times slower as compared with the control group (2.8mm³/day vs 47.5mm³/day, respectively). This effect was noticeable and statistically significant at all measurements. As shown in Fig. 2A, on day 75 post-injection the growth of melanoma OMP-M6 tumors expressing dnYAP was significantly reduced as compared to OMP-M6 tumors expressing the GFP control (93% reduction, average tumor volume of 186mm³ compared to an average tumor volume of 2669mm³, respectively). These results suggest that YAP may be acting as an oncogene in melanoma, and that dnYAP was very efficient at inhibiting tumor growth.

Similar experiments were undertaken with additional tumors, including colon tumors OMP-C18, OMP-C11, OMP-C37, pancreatic tumor OMP-PN7, lung tumors OMP-LU52 and OMP-LU2, and ovarian tumor OMP-OV22. Transduced tumor cells and mouse numbers varied based on each sample's tumorigenic properties. As shown in Figure 2B, colon tumor OMP-C18 cells transduced with dnYAP did not produce any tumors (0/5), while cells expressing the GFP control produced tumors in 9/10 mice with an average volume of 323mm³ at day 47. As shown in Figure 2C, colon tumor OMP-C37 cells transduced with dnYAP produced only one tumor (1/10) and this tumor was shown to be GFP negative, thus the tumor arose from cells not expressing dnYAP. In contrast, OMP-C37 tumor cells expressing the GFP control produced tumors in 9/10 mice with an average volume of 342mm³ at day 67. As shown in Figure 2D, pancreatic tumor OMP-PN7 cells transduced with dnYAP produced tumors in 4/10 mice. As seen with OMP-C37, the largest tumor was shown to be GFP negative, while the other 3 tumors were shown to be only 7% GFP positive, 15% GFP positive, and 41% GFP positive, indicating limited amounts of dnYAP expression. In contrast, OMP-PN7 tumor cells expressing the GFP control produced tumors in 8/9 mice with an average volume of 766mm³ at day 80. As shown in Figure 2E, colon tumor OMP-C11 cells transduced with dnYAP produced tumors (7/10), but all were extremely small with an average volume of 65mm³ at day 53. In contrast, OMP-C11 tumor cells expressing the GFP control produced tumors in 10/10 mice with an average volume of 945mm³ at day 53. As shown in Figure 2F, lung tumor OMP-LU2 cells transduced with dnYAP produced tumors in 4/10 mice with an average tumor volume of 251mm³ at day 87, while cells expressing the GFP control produced tumors in 9/10 mice with an average volume of 1023mm³ at day 87. As shown in Figure 2G, ovarian tumor OMP-OV22 cells transduced with dnYAP produced tumors in 8/9 mice, however the tumors were smaller than the control with an average volume 268mm³. The OMP-OV22 tumor cells expressing the GFP control produced tumors in 8/8 mice with an average volume of 416mm³ at day 108. As shown in Figure 2H, the growth ofOMP-LU52 tumors expressing dnYAP was reduced as compared to OMP-LU52 tumors expressing the GFP control at day 62 (53% reduction in tumor volume, average tumor volume of 490mm³ compared to an average tumor volume of 1041mm³).

These results were similar to the results observed with melanoma tumor OMP-M6, i.e., inhibition of YAP activity by dnYAP reduced or prevented tumor growth, and demonstrated that YAP may be acting as an oncogene in colon cancer, pancreatic cancer and lung cancer, as well as in melanoma. The inhibition of tumor growth by expression of dnYAP in lung tumor OMP-LU52 and ovarian tumor OMP-OV22 was less than in the other tumors studied. Interestingly, as shown by microarray analysis OMP-LU52 has a very low level of YAP expression when compared to the other tumors (Figure 2I). This supports the idea that dnYAP is having a direct effect on YAP activity in most of the tumors studied, and that in lung tumor LU52 dnYAP had a more limited effect because there was only a small amount of YAP in the LU52 cells.

### Example 3

### Microarray analysis of tumor cells expressing dnYAP

As described in Example 2, tumor cells from colon tumor OMP-C18, colon tumor OMP-C37, pancreatic tumor OMP-PN7, and lung tumor OMP-LU52 were transduced with dnYAP-lentivirus (LOM420, CMV-dnYAP-IRES-GFP) or GFP-lentivirus (LOM92, CMV-IRES-GFP) as a control. Transduced cells were cultured for 3 days and were FACS sorted for GFP expression. The gene expression profiles of these transduced tumor cells were determined by microarray analysis. RNA was isolated from the transduced cells and processed using established Affymetrix protocols. The samples were hybridized to Affymetrix HG-U133 plus 2.0 microarrays (Affymetrix, Santa Clara, CA) as outlined in the manufacturer's technical manuals. After hybridization, the microarrays were washed, scanned, and analyzed. Scanned array background adjustment and signal intensity normalization were performed using the GCRMA algorithm (Bioconductor, www.bioconductor.org). The gene expression profiles established for tumor cells expressing dnYAP were compared to gene expression profiles established for GFP-expressing tumor cells and analyzed using IPA software (Ingenuity Systems, Redwood City CA). The gene expression profiles from colon tumors OMP-C18 and OMP-C37 and pancreatic tumor OMP-PN7 expressing dnYAP showed an overlap of 208 genes that were significantly down-regulated at least 2-fold (p value ≤ 0.05) as compared to control tumor cells. Analysis showed that the genes down-regulated by expression of dnYAP are involved in many cellular pathways and/or functions, including cell adhesion, polarity, cancer stem cells, membrane and secretory trafficking, DNA repair, RNA metabolism, mitosis, cell death, and ubiquitination. Some membrane proteins that were down-regulated in at least two of the tumors included CA12, CLDN2, PAG1, SEMA4D, GPC4, CD44, CD47, CD133, RHEB, MAGI1, ITPR3, CD168, NRP2, EPHB2 and TDGF1 and some growth factors and cytokines that were down-regulated in at least two of the tumors were AREG-B, IL33, GRB2 and IGFBP3 (Table 1). Some of the genes identified have been reported to be cancer stem cell genes and/or encode for cancer stem cell markers, for example, CD133, CD44, TDGF1, CD47, and EPHB2.

**Table 1**

| | OMP-C18 | OMP-C37 | OMP-PN7 | Lu52 |
|---|---|---|---|---|
| CD133 | -7.1 | -3.5 | -2.3 | -1.4 |
| TDGF1 | -9.2 | -3.9 | 1.0 | 1.0 |
| CD44 | -6.5 | -5.1 | -2.0 | 1.1 |
| EPHB2 | -1.9 | -1.5 | -3.8 | -1.3 |
| CD47 | -2.1 | -2.7 | -1.9 | 1.1 |
| CA12 | -3.6 | -2.2 | -4.0 | -1.2 |
| LRP4 | -4.9 | -3.7 | 1.1 | 1.1 |
| GPC4 | -2.3 | -3.3 | 1.0 | -1.0 |
| CLDN2 | -4.7 | -5.1 | -7.0 | -1.0 |
| SEMA4D | -2.2 | -2.1 | -4.6 | 1.2 |
| NRP2 | -1.5 | -2.0 | -1.8 | -1.0 |
| AREG-B | -2.3 | -2.8 | -2.6 | 1.7 |
| RHEB | -2.3 | -2.4 | 1.23 | -2.0 |
| IGFBP3 | -3.9 | -2.6 | -1.23 | 1.2 |
| CD168 | -2.0 | -2.1 | 1.77 | -1.4 |
| MAGI1 | -2.6 | -2.4 | -1.74 | 1.0 |

### Example 4

### Effect of dnYAP on YAP activity

HEK-293T cells were transiently transfected with different combinations of plasmids to evaluate the effect of dnYAP on YAP activity. Cells were transfected with a luciferase reporter comprising a promoter containing 6 copies of the GT-IIC motif of the SV40 enhancer which serves as a TEAD binding site (TBS-Luc reporter). In addition, cells were transfected with various combinations of plasmids expressing TEAD, YAP or GFP-YAP, and/or dnYAP or GFP-dnYAP. All transfections included a plasmid expressing Renilla luciferase as an internal control. 1.5 x 10⁵ HEK-293T cells/well were seeded into 96-well plates (BD Biosciences, San Jose CA) and transfected with 250ng of an equimolar mix of the plasmids using FuGENE 6 transfection reagent (Roche Applied Science, Indianapolis IN) following the manufacturer's instructions. The transfected cells were incubated for 48-72 hours and luciferase activity was measured using Dual-Glo® Luciferase Assay System according to the manufacturer's instructions (Promega, Madison WI). Activity was expressed as a ratio of the firefly luciferase activity to the Renilla luciferase activity.

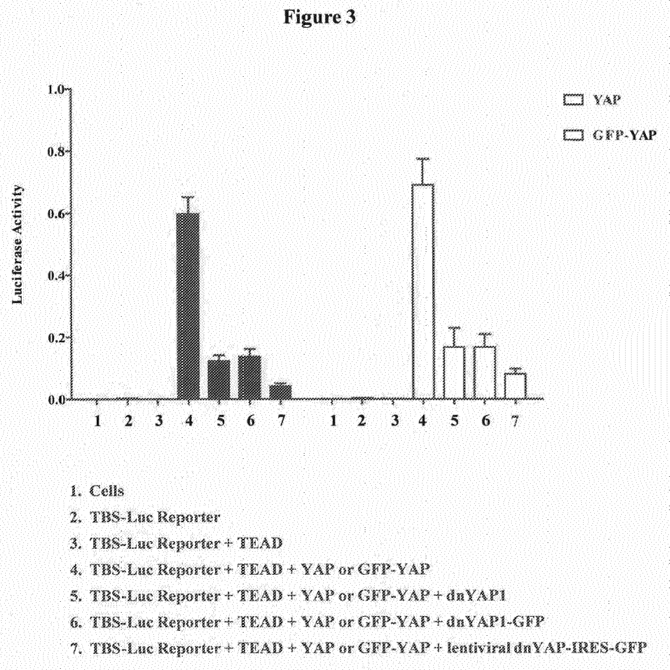

Both YAP and GFP-YAP strongly induced TEAD-mediated transcription of the TBS-Luc reporter (Figure 3, #4). Both dnYAP and GFP-dnYAP strongly inhibited the YAP activity as demonstrated by a significant reduction in luciferase activity (Figure 3, #5 and #6). In addition, it was demonstrated that the lentiviral vector expressing dnYAP (pOM420), which was used in *in vivo* tumor studies, also strongly inhibited YAP activity as demonstrated by a significant reduction in luciferase activity (Figure 3, #7). These results demonstrated that a luciferase-based assay can be used to evaluate and/or screen molecules for their effect on YAP activity. Thus, this assay can be used to evaluate and/or screen molecules for their effect on the Hippo pathway.

To simplify the assay and to avoid transfection with multiple plasmids, HEK-293T cells were stably transfected with the TBS-Luc reporter vector and clones were selected using hygromycin B. Clones were transiently transfected with YAP and luciferase activity was measured. Two clones were identified that showed strong induction of luciferase activity after YAP expression. These stable reporter cell lines can be used to evaluate and/or screen molecules for their effect on the Hippo pathway.

### Example 5

### Effect of cell detachment on Hippo pathway components

It has been reported that cell detachment induces changes in the phosphorylation of Hippo pathway components in the MCF10A breast cell line (Zhao et al., 2012, Genes Dev., 26:54-68). A study was conducted to investigate whether cell detachment had similar effects in HEK-293T cells. HEK-293T cells were grown in culture to approximately 80% confluence, trypsinized, washed with PBS, and divided into three experimental groups. The cells in group 1 were directly lysed with tissue extraction reagent buffer (Invitrogen, Grand Island NY). The cells in groups 2 and 3 were seeded into 6-well Primaria™ plates (BD Biosciences, San Jose CA) at a cell density of 1 x 10⁶ cells/well and incubated for 1 hour and 20 hours, respectively. After incubation, the attached cells were washed with PBS and directly lysed in the plate with tissue extraction reagent buffer. Proteins Lats1, Lats2, and YAP were analyzed by Western blot analysis using antibodies that recognized phosphorylated forms of the proteins and all forms of the proteins. Whole cell protein extracts were prepared with tissue extraction reagent buffer containing protease and phosphatase inhibitor cocktail (Roche Molecular, Pleasanton CA). Proteins were separated by SDS-polyacrylamide gel electrophoresis (PAGE) and were transferred to nitrocellulose membranes. The membranes were incubated in TBS-T (Tris-Buffered Saline plus 0.1% Tween 20) containing 5% nonfat milk to block non-specific binding. The membranes were incubated with a primary antibody overnight at 4°C in TBS-T containing 3% BSA. Bound primary antibody was detected with an appropriate secondary HRP conjugate antibody, incubated for 1 hour at room temperature in TBS-T with 5% milk, and visualized with SuperSignal West Chemiluminescent substrate (Pierce-Thermo Fisher Scientific, Rockford IL). The primary antibodies used were a rabbit anti-phosphorylated YAP (pYAP) (Ser127) antibody, a rabbit anti-YAP antibody, a rabbit anti-phosphorylated Lats1 (pLats1) (Thr1079) antibody, a rabbit anti-pLats1 (Ser909) antibody, a rabbit anti-Lats1 antibody (all from Cell Signaling, Danvers MA), and a rabbit anti-Lats2 antibody (abcam, Cambridge MA). The secondary antibody used was a HRP-conjugated Affinipure donkey anti-rabbit antibody (Jackson ImmunoResearch Laboratories, West Grove PA). Subsequently, the membranes were stripped (Restore Plus Stripping Buffer, Pierce-Thermo Fisher Scientific, Rockford IL) and reprobed with a mouse anti-actin antibody (Millipore, Billerica MA) and a HRP-conjugated Affinipure donkey anti-mouse antibody (Jackson ImmunoResearch Laboratories, West Grove PA) to evaluate and confirm equivalent protein load for each sample.

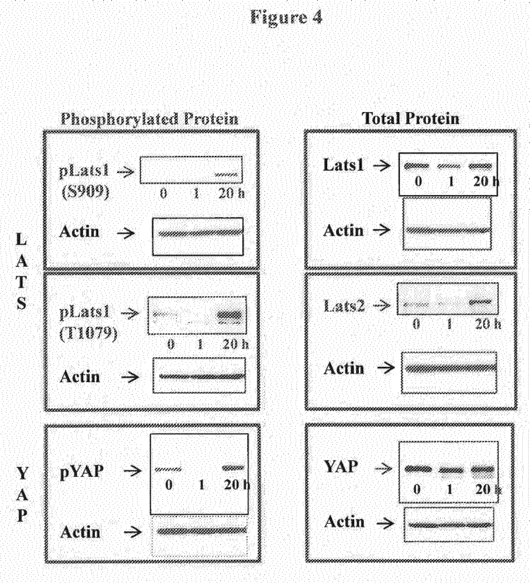

As shown in Figure 4, the amount of pLats1 (S909 or T1079) and Lats2 proteins were significantly lower in Group 1 cells (t = 0) and in Group 2 cells (t = 1 hr incubation/attachment) as compared to Group 3 cells (t = 20 hr incubation/attachment). No detectable reduction in total Lats1 protein was observed in any of the groups. In addition, the amount of pYAP protein was reduced in Group 1 cells (t = 0) and was undetectable in Group 2 cells (t = 1 hr incubation/attachment) as compared to Group 3 cells (t = 20 hr incubation/attachment). Similar to results observed with Lats1, there was no reduction in total YAP in any of the groups. These results demonstrate that the Hippo pathway could be experimentally modulated *in vitro* in HEK-293T cells and that modification in the phosphorylation state of components of the Hippo pathway (e.g., pLats1, pYAP) can be detected.

### Example 6

### IgCAM constructs

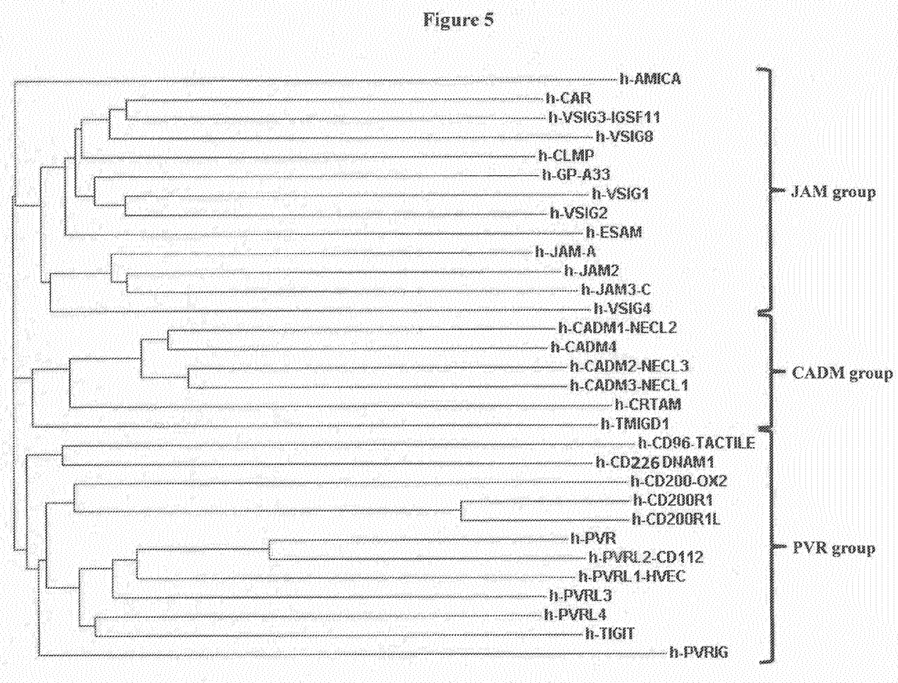

To identify potential Hippo pathway receptors a homology-based bioinformatics analysis of IgCAM proteins encoded by the human genome was undertaken. A family tree or dendrogram, of candidate Hippo pathway receptors is shown in Figure 5. The proteins fall generally into three groups: 1) a "JAM-like" family containing JAM1, JAM2, JAM3, CAR, CLMP, AMICA, ESAM, GPA33, VSIG1, VSIG2, VSIG3, VSIG4, and VSIG8; 2) a "CADM-like" family containing CADM1, CADM2, CADM3, CADM4, CRTAM, and TMIGD1; and 3) a "PVR-like" family containing PVR, PVRL1, PVRL2, PVRL3, PVRL4, PVRIG, CD200, CD200R1, CD200R1L, CD226, CD96, and TIGIT.

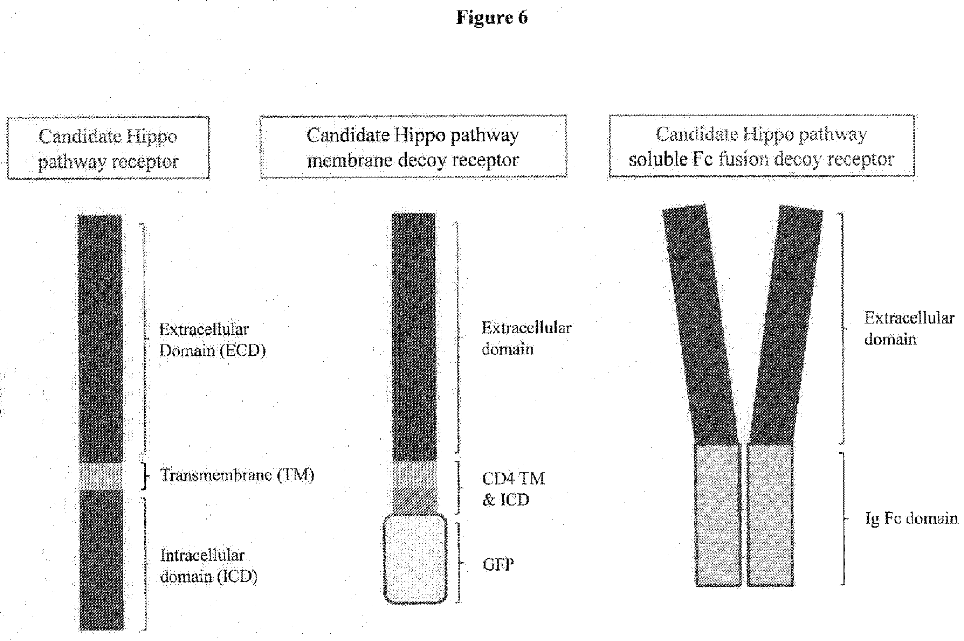

In order to investigate the possible function of these molecules in Hippo pathway signaling, IgCAM constructs were prepared including membrane-anchored proteins and soluble "decoy" receptors (Figure 6). Each membrane-anchored decoy receptor was designed to be non-functional in regard to signaling, as the Transmembrane and cytoplasmic domains were replaced with the transmembrane and intracellular domain of human CD4 protein. The membrane-anchored IgCAM protein constructs were generated by ligating the extracellular domain (ECD) region of human IgCAM proteins to the transmembrane domain and intracellular domain of CD4 and a C-terminal GFP protein tag using standard recombinant DNA techniques. These constructs are referred to as "IgCAM"-CD4TM-GFP, for example CAR-CD4TM-GFP. The soluble decoy receptors were designed to include the ECD of an IgCAM linked to an immunoglobulin Fc domain. The soluble receptor IgCAM protein constructs were generated by ligating the ECD region of human IgCAM proteins to the Fc domain of human IgG1 using standard recombinant DNA techniques. These constructs are referred to as "IgCAM"-Fc, for example CAR-Fc. As known to those of skill in the art, the ECD region of any given IgCAM protein used in the constructs may comprise the ECD or comprise a portion or fragment of the ECD. Also, what is considered to be the ECD may vary by one, two, three, or more amino acids at the amino end, the carboxyl end, or both ends of the ECD. These fusion proteins may be used to examine the role of IgCAM proteins in the Hippo pathway.

The constructs generated include ECD regions, or a portion or fragment thereof, from the IgCAM proteins in Table 2.

**Table 2**

| Name | Full name | Other names | UniProtKB No. | SEQ ID NO |
|---|---|---|---|---|
| JAM Family | | | | |
| AMICA | Junctional adhesion molecule-like | JAML | Q86YT9 | 1, 33 |
| CAR | Coxsackievirus and adenovirus receptor | CXADR | P78310 | 2, 34 |
| CLMP | CXADR-like membrane protein | ACAM, ASAM | Q9H6B4 | 3, 35 |
| ESAM | Endothelial cell-selective adhesion molecule | | Q96AP7 | 4, 36 |
| GPA33 | Cell surface A33 antigen | | Q99795 | 5, 37 |
| VSIG1 | V-set and immunoglobulin domain-containing protein 1 | GPA34 | Q86XK7 | 9, 41 |
| VSIG2 | V-set and immunoglobulin domain-containing protein 2 | CTH, CTXL | Q96IQ7 | 10, 42 |
| VSIG3 | Immunoglobulin superfamily member 11 | IGSF11, BTIGSF, CXADRL1 | Q5DX21 | 11, 43 |
| VSIG4 | V-set immunoglobulin domain-containing 4 | CRIg, Z39IG | Q9Y279 | 12, 44 |
| VSIG8 | V-set and immunoglobulin domain-containing protein 8 | C1orf204 | Q5VU13 | 13, 45 |
| JAM1 | Junctional adhesion molecule A | F11R, JAM-A, JCAM | Q9Y624 | 6, 38 |
| JAM2 | Junctional adhesion molecule B | JAM-B, C21orf43, VEJAM | P57087 | 7, 39 |
| JAM3 | Junctional adhesion molecule C | JAM-C | Q9BX67 | 8, 40 |

| CADM Family | | | | |
|---|---|---|---|---|
| CADM1 | Cell adhesion molecule 1 | IGSF4, IGSF4A, NECL2, SYNCAM, TSLC1 | Q9BY67 | 14, 46 |
| CADM2 | cell adhesion molecule 2 | IGSF4D, NECL3 | Q8N3J6 | 15, 47 |
| CADM3 | Cell adhesion molecule 3 | IGSF4B, NECL1, SYNCAM3, TSLL1 | Q8N126 | 16, 48 |
| CADM4 | Cell adhesion molecule 4 | IGSF4C, NECL4, TSLL2 | Q8NFZ8 | 17, 49 |
| CRTAM | Cytotoxic and regulatory T-cell molecule | CD355 | Q95727 | 18, 50 |
| TMIGD1 | Transmembrane and immunoglobulin domain-containing protein 1 | TMIGD | Q6UXZ0 | 19, 51 |

| PVR Family | | | | |
|---|---|---|---|---|
| PVR | Poliovirus receptor | NECL-5, CD155, PVS | P15151 | 26, 58 |
| PVRL1 | Poliovirus receptor-related protein 1 | HVEC, HLGR, Nectin-1, CD111, PRR1 | Q15223 | 27, 59 |
| PVRL2 | Poliovirus receptor-related protein 2 | HVEB, PRR2, CD112, Nectin-2 | Q92692 | 28, 60 |
| PVRL3 | Poliovirus receptor-related protein 3 | Nectin-3, CD113 | Q9NQS3 | 29, 61 |
| PVRL4 | Poliovirus receptor-related protein 4 | Nectin-4, LNIR, PRR4 | Q96NY8 | 30, 62 |
| PVRIG | transmembrane protein PVRIG | C7orf15 | Q6DKI7 | 25, 57 |
| CD200 | OX-2 membrane glycoprotein | OX-2, MOX1, MOX2, | P41217 | 21, 53 |
| CD200R1 | Cell surface glycoprotein CD200 receptor 1 | CD200R, CRTR2, MOX2R, OX2R | Q8TD46 | 22, 54 |
| CD200R1L | Cell surface glycoprotein CD200 receptor 2 | CD200R2 | Q6Q8B3 | 23, 55 |
| CD226 | CD226 antigen | DNAM1, PTA-1, TLiSA1 | Q15762 | 24, 56 |
| CD96 | T-cell surface protein tactile | | P40200 | 20, 52 |
| TIGIT | T-cell immunoreceptor with Ig and ITIM domains | VSIG9, Vstm3, WUCAM | Q495A1 | 31, 63 |

| Other | | | | |
|---|---|---|---|---|
| TMEM25 | Transmembrane protein 25 | | Q86YD3 | 32, 64 |

### Example 7

### Effect of IgCAM proteins on Hippo pathway components

HEK-293T cells were transiently transfected with IgCAM constructs described in Example 6. 3.5 x 10⁵ HEK-293T cells/well were seeded into 6-well Primaria™ plates (BD Biosciences, San Jose CA). Approximately 18 hours later, 1ug of an IgCAM-CD4TM-GFP construct (described above) was transfected into cells using FuGENE 6 transfection reagent (Roche Applied Science, Indianapolis IN) following the manufacturer's instructions. The IgCAM-CD4TM-GFP constructs used included CAR-CD4TM-GFP, CLMP-CD4TM-GFP, VSIG1-CD4TM-GFP, VSIG2-CD4TM-GFP, VSIG3-CD4TM-GFP, VSIG8-CD4TM-GFP, ESAM-CD4TM-GFP, JAM1-CD4TM-GFP, JAM2-CD4TM-GFP, JAM3-CD4TM-GFP, CADM1-CD4TM-GFP, CADM2-CD4TM-GFP, CADM3-CD4TM-GFP, CADM4-CD4TM-GFP, CD226-CD4TM-GFP, CD200-CD4TM-GFP, CD200R1-CD4TM-GFP, CD200R1L-CD4TM-GFP, PVR-CD4TM-GFP, PVRLI-CD4TM-GFP, PVRL2-CD4TM-GFP, PVRL3-CD4TM-GFP, PVRL4-CD4TM-GFP, TIGIT-CD4TM-GFP, TMIGD1-CD4TM-GFP, and TMEM25-CD4TM-GFP. As controls FLAG-mIgG1-CD4TM-GFP, LGR5-CD4TM, CD4TM, and RSPO4-CD4TM-GFP were used.

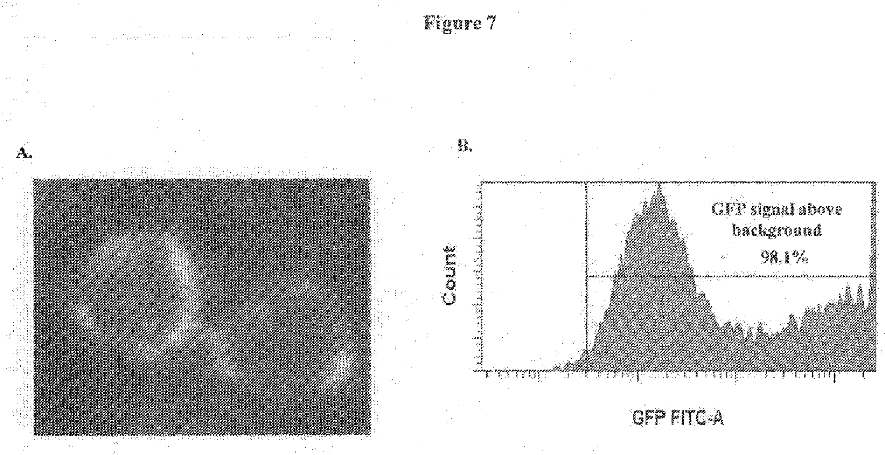

To analyze cell culture growth and GFP localization cell cultures were monitored during transient transfection using a Nikon Eclipse TS100 inverted tissue culture microscope attached to a Nikon mercury-vapor lamp and a camera. As demonstrated in Figure 7A, which shows a representative example of transfected cells, the IgCAM-CD4TM-GFP proteins were expressed at the cell surface and were detectable by fluorescent microscopy.

After 28 hours, some transfected cells were trypsinized to obtain a single cell suspension, washed, and resuspended in HBSS containing FBS and DAPI (Molecular Probes/Invitrogen, Grand Island, NY). Transfection efficiency, as determined by GFP expression levels, and viability, as determined by DAPI staining, were analyzed in a FACSCanto™ flow cytometer (BD Biosciences, San Jose CA). Figure 7B shows a representative flow cytometry histogram of IgCAM-CD4TM-GFP transfected cells, demonstrating that 98% of the cells were GFP positive. For cells transfected with 26 of the IgCAM-CD4TM-GFP constructs the percentage of GFP positive cells ranged from approximately 75% to 99.5% (average 93% ± 6.8%). These results demonstrated that transfections using the IgCAM-CD4TM-GFP constructs were very efficient.

Other cells were directly lysed in the tissue culture plates. Whole cell protein extracts were prepared with tissue extraction reagent buffer (Invitrogen, Grand Island NY) containing protease and phosphatase inhibitor cocktail (Roche Molecular, Pleasanton CA). Proteins were separated by SDS-PAGE and were transferred to nitrocellulose membranes. The membranes were incubated in TBS-T containing 5% nonfat milk to block non-specific binding. The membranes were incubated with primary antibodies overnight at 4°C in TBS-T containing 3% BSA. Bound primary antibodies were detected with an appropriate secondary HRP conjugate antibody, incubated for 1 hour at room temperature in TBS-T with 5% milk, and visualized with SuperSignal West Chemiluminescent substrate (Pierce-Thermo Fisher Scientific Inc. Rockford, IL). The primary antibodies used were a rabbit anti-pYAP (Ser127) antibody, a rabbit anti-YAP antibody, a rabbit anti-pLats1 (Thr1079) antibody, a rabbit anti-pLats1 (Ser909) antibody, and a rabbit anti-Lats1 antibody (all from Cell Signaling, Danvers MA). The secondary antibody used was a HRP-conjugated Affinipure donkey anti-rabbit antibody (Jackson ImmunoResearch Laboratories, West Grove PA). Subsequently, the membranes were stripped using Restore Plus Stripping Buffer (Pierce-Thermo Fisher Scientific, Rockford IL) and reprobed with a mouse anti-actin antibody (Millipore, Billerica MA) and a HRP-conjugated Affinipure donkey anti-mouse antibody (Jackson ImmunoResearch Laboratories, West Grove PA) to evaluate and confirm equivalent protein load for each sample.

For protein expression quantification, the Western blots were scanned and the resulting images were analyzed for band intensities using densitometry software (ImageJ software, NIH). The intensity signal of each protein band was normalized to the actin signal of the same sample. Numbers in Table 3 correspond to relative protein quantity with respect to the FLAG-mIgG1 control. Positive results indicate an increased fold change with respect to control and negative results indicate a decreased fold change with respect to control.

**Table 3**

| | pLATS1 S909 | pLATS1 T1079 | LATS1 | pYAP | YAP |
|---|---|---|---|---|---|
| JAM Family | | | | | |
| CAR | -1.2 | -3.2 | -1.9 | -1.5 | -2.9 |
| CLMP | 2.3 | 1.3 | -2.4 | 1.3 | -10.8 |
| VSIG1 | 1.6 | -1.9 | -1.2 | 1.0 | -1.6 |
| VSIG2 | 1.3 | -1.2 | 1.3 | 1.1 | -1.1 |
| VSIG3 | -1.0 | -2.2 | -1.1 | -1.1 | -1.4 |
| VSIG8 | 1.4 | -1.2 | 1.1 | -1.3 | -1.2 |
| ESAM | 3.1 | -1.8 | -9.3 | 1.3 | -27.2 |
| JAM1 | -2.2 | -4.3 | -2.0 | -1.4 | -1.8 |
| JAM2 | -2.2 | -4.5 | -2.1 | -1.2 | -1.8 |
| JAM3 | -1.7 | -3.0 | -2.1 | -1.6 | -2.2 |

| CADM Family | | | | | |
|---|---|---|---|---|---|
| CADM1 | -1.3 | -1.3 | -1.4 | -1.1 | -1.0 |
| CADM2 | -1.0 | -1.5 | -1.2 | -1.4 | -1.0 |
| CADM3 | -4.2 | -1.8 | -1.3 | -2.0 | -1.1 |
| CADM4 | -1.7 | -1.7 | -1.7 | -1.3 | -1.5 |
| TMIGD1 | 1.0 | -1.4 | -3.6 | -1.4 | -1.4 |

| PVR Family | | | | | |
|---|---|---|---|---|---|
| CD226 | 3.1 | -1.2 | -2.3 | 2.1 | -3.3 |
| CD200 | 2.5 | 1.9 | -1.2 | 2.4 | 1.5 |
| CD200R1 | 1.7 | 1.3 | -1.1 | 1.6 | 1.2 |
| CD200R1L | -2.5 | -1.1 | -1.6 | 1.8 | 1.1 |
| PVR | 2.1 | 1.2 | -1.2 | 1.8 | 1.1 |
| PVRL1 | -1.2 | -1.2 | -1.3 | -1.5 | -3.6 |
| PVRL2 | -1.4 | -1.4 | -1.2 | -1.1 | -2.9 |
| PVRL3 | 3.5 | 1.4 | 1.0 | 1.4 | -1.4 |
| PVRL4 | 2.9 | 1.2 | -1.2 | 1.9 | -1.6 |
| TIGIT | 3.0 | 1.4 | -1.2 | 1.7 | -3.9 |

| Other | | | | | |
|---|---|---|---|---|---|
| TMEM25 | 1.2 | -1.5 | -2.3 | 1.0 | -1.6 |

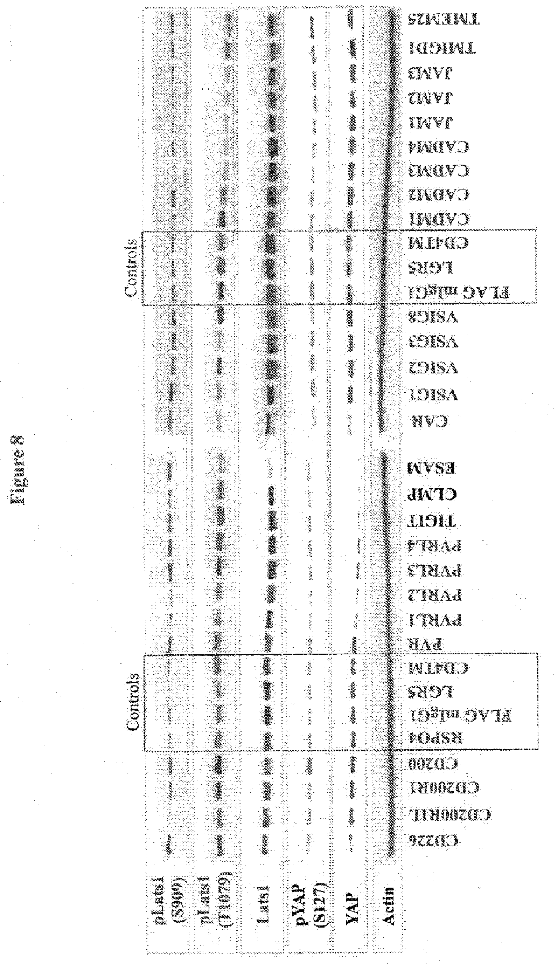

As shown in Figure 8 and Table 3, expression of IgCAM-ECD constructs appeared to affect some components of the Hippo pathway. For example, pLats1 and pYAP were increased by expression of CD200-ECD as compared to controls. In contrast, pLats1 and pYAP were decreased by expression of CADM3-ECD as compared to controls. These results demonstrate that over-expression of the ECD of some IgCAM proteins, such as CD200 and CADM3, could affect components of the Hippo pathway (Lats1 and YAP) *in vitro* and suggest that IgCAM-binding agents could be used to modulate the Hippo pathway.

### Example 8

### Bimolecular fluorescence complementation assay for determination of YAP nuclear translocation

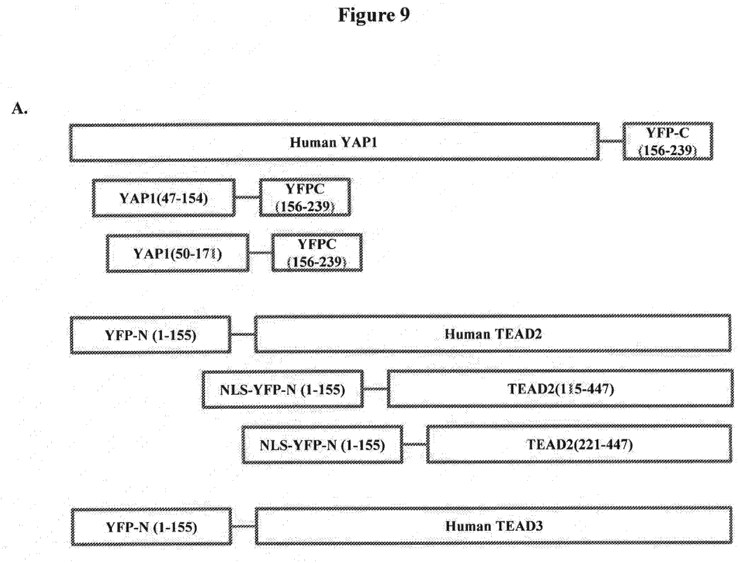

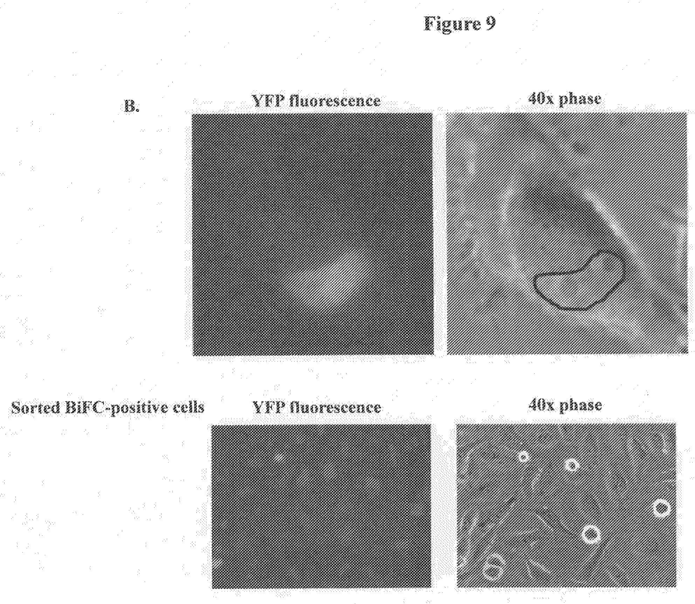

YAP localization can serve as a read-out for the activity of the Hippo pathway. Bimolecular fluorescence complementation (BiFC) constructs with human YAP1, TEAD2, and TEAD3 were generated (Figure 9A). Specifically, the C-terminus of yellow fluorescent protein (YFP, amino acids 156-239) was fused to the C-terminus of YAP1 with a linker sequence (RPACKIPNDLKQKVMNH, SEQ ID NO:72) to generate the construct YAP1-YFPC in a pcDNA3.1(+) vector. The N-terminus of YFP (amino acids 1-155) was fused to the N-terminus of TEAD2 or TEAD3 with a linker sequence (RPACKIPNDLKQKVMNH, SEQ ID NO:72) to generate the constructs YFPN-TEAD2 and YFPN-TEAD3 in a pcDNA3.1/hygro vector. The YAP1-YFPC construct and YFPN-TEAD2 or the YAP1-YFPC construct and YFPN-TEAD3 were stably transfected into HeLa cells and double transfectants were selected using G-418 and hygromycin B. Activation of YAP should lead to predominant nuclear localization of the YAP-YFPC protein allowing for interaction with the YFPN-TEAD protein and giving rise to YFP fluorescence. Trypsinized cells from the stable clones were re-plated at low density (YAP is activated) and YFP fluorescence was evaluated by widefield epifluorescence microscopy (Figure 9B). An additional cell line was generated, the YAP1-YFPC construct was stably transfected into HeLa cells and transfectants were selected using G-418. The HeLa YAP1-YFPC cell line can be transduced with lentiviruses expressing YFPN-TEAD2 or YFPN-TEAD3.

A variation of the BiFC assay using truncation constructs constituting the cognate binding domains of YAP1 and TEAD2 was developed based upon assays from Vassilev et al., 2001, Genes and Development, 15:1229-1241 and Li et al., 2010, Genes and Development, 24:235-240. In particular, amino acids 47-154 and 50-171 of human YAP1 were fused at their C-termini to YFPC (aa 156-239) to generate YAP1(47-154)-YFPC and YAP1(50-171)-YFPC, respectively. Furthermore, amino acids 115-447 and 221-447 of human TEAD2 were fused at their N-termini to YFPN (aa 1-115), with the YFPN domain preceded by a nuclear localization sequence, to generate NLS-YFPN-TEAD2(115-447) and NLS-YFPN-TEAD2(221-447) (Figure 9A). As described above, these constructs are stably transfected or stably transduced by lentiviral infection into HeLa cells or MCF10A cells, and the resulting cell lines are used in BiFC assays to observe YAP1 localization by widefield epifluorescence microscopy or flow cytometry. YAP1 localization is used to determine Hippo pathway signaling status.

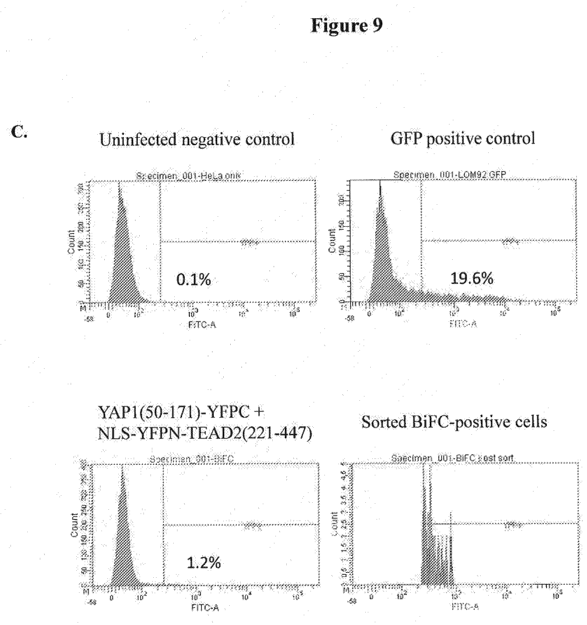

HeLa cells infected with lentivirus to transduce expression of YAP1(50-171)-YFPC and NLS-YFPN-TEAD2(221-447) were sorted by FACS for YFP-positive (BiFC-positive) cells and subsequently imaged by widefield microscopy (Fig. 9B and 9C).

These cell lines are used to screen and identify molecules (e.g., antibodies, proteins, or small molecules) that affect YAP activation/inactivation and potentially the Hippo pathway.

### Example 9

### Assay for determination of YAP nuclear translocation

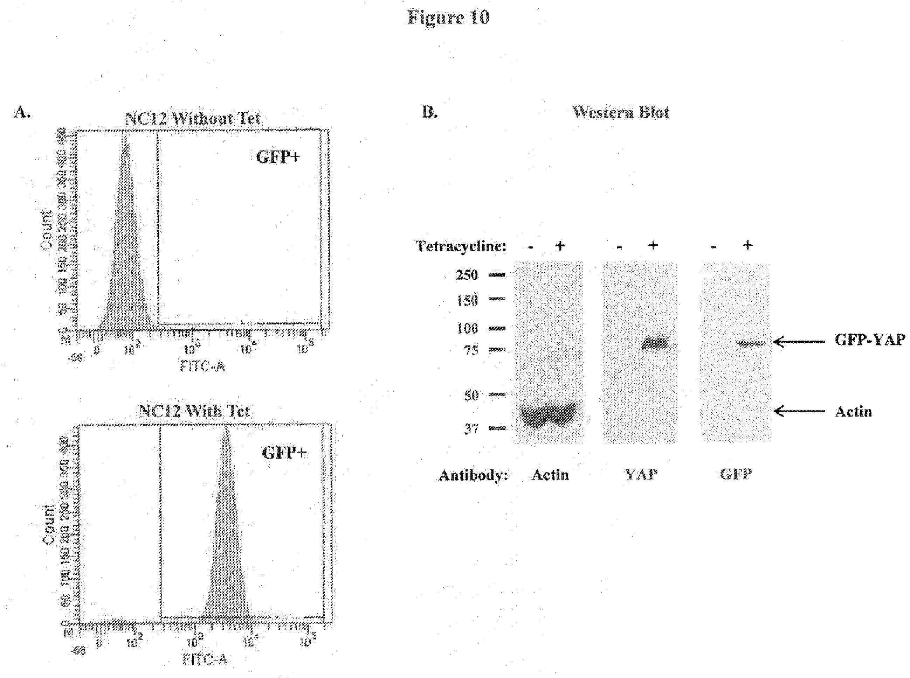

YAP localization in individual cells can also be assessed using a YAP-GFP fusion protein. The GFP allows for direct monitoring of YAP, since activation of YAP should lead to predominant nuclear localization of YAP-GFP. A YAP-GFP construct was cloned into a pLenti6.3/TO/V5/DEST vector (Invitrogen/Life Technologies, Grand Island NY) generating pLenti6.3-TO-YAPGFP-DEST (pOM1017). This vector allows for tetracycline (Tet)-regulated expression of the YAP-GFP fusion protein. HeLa cells were transfected with pLenti3.3/TR repressor plasmid (Invitrogen/Life Technologies, Grand Island NY) that constitutively expresses high levels of the Tet repressor under the control of a CMV promoter. Stably transfected cells were selected with 500µg/ml of G418. Clones were screened by transiently transfecting the Tet-inducible YAP-GFP construct pOM1017 into the transfected HeLa cells and treating the cells with tetracycline. Several clones were identified that demonstrated YAP-GFP expression only in the presence of 1µg/ml tetracycline. One of the clones, HeLaB5, was stably transfected with YAP-GFP construct pOM1017. Double transfectants were selected with 500µg/ml G418 and 4µg/ml of blasticidin. Clones were analyzed by FACS for the expression of GFP in the absence and presence of tetracycline. One clone, NC12, was obtained that showed tight regulation of GFP-YAP expression upon addition of 1µg/ml tetracycline (Figure 10A). The expression of YAP-GFP, upon Tet induction, was confirmed by Western blot analysis (Figure 10B). The NC12 cell line can be used in assays run on a BD Pathway system which allows for real-time imaging of live cells. The assays are used to screen and identify molecules (e.g., antibodies, proteins, or small molecules) that affect YAP activation/inactivation and potentially the Hippo pathway.

### Example 10

### Assay for determination of YAP nuclear translocation

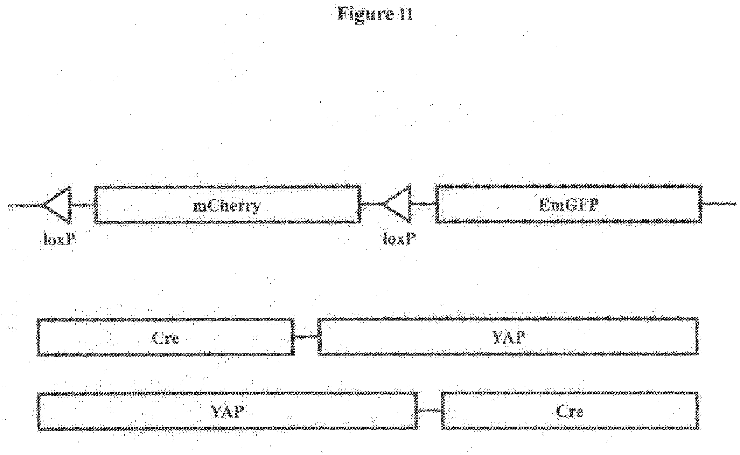

YAP localization in individual cells can be assessed using a YAP-Cre fusion protein co-expressed with a Cre-dependent Emerald green fluorescent protein (EmGFP) expression construct (Figure 11). Specifically, a monomeric form of fluorescent protein DsRed, mCherry, was flanked by loxP sites 5' to EmGFP to generate the construct floxed mCherry/EmGFP in the vector pcDNA3.1(+) (Life Technologies, Grand Island NY). YAP, under the control of the Tet operator in the vector pLenti6.3/TO/V5-DEST (Life Technologies, Grand Island NY), is fused either N- or C-terminally to the coding region of Cre to generate the constructs Cre-YAP or YAP-Cre, respectively. The mCherry/EmGFP construct and Cre-YAP or mCherry/EmGFP construct and YAP-Cre were stably transfected into HeLa cells and double transfectants were selected with G-418 and blasticidin. mCherry fluorescence, arising from inactive cytoplasmic YAP-Cre or Cre-YAP, or EmGFP fluorescence, arising from active nuclear YAP-Cre or Cre-YAP, is assayed by widefield epifluorescence microscopy or flow cytometry. These cell lines are used to screen and identify molecules (e.g., antibodies, proteins, or small molecules) that affect YAP activation/inactivation and activity of the Hippo pathway.

### Example 11

### Effect on tumor growth by over-expression of membrane-anchored IgCAM decoy receptors in colon tumor OMP-C18

Dissociated colon OMP-C18 cells (1 x 10⁶ cells/well) were seeded into 6-well Primaria™ plates (BD Biosciences, San Jose CA) and transduced with RRL-based lentiviruses expressing membrane-anchored decoy receptors, CADM2-CD4TM-GFP, CADM4-CD4TM-GFP, CD226-CD4TM-GFP, PVR-CD4TM-GFP, or GFP only as a control. RRL-based lentiviruses express the GFP gene under the control of a CMV promoter. In addition, a second control was a group of cells that were not transduced. Lentiviruses were added to the cells at time of plating (t = 0) at a MOI which resulted in the infection of 20% to 60% of the OMP-C18 cells. The culture medium was DMEM Low glucose, F12 medium, B27 medium, insulin-transferrin-selenium supplement, heparin, rhEGF, rhFGF-2, and antibiotics. After 72 hours, cells were collected, trypsinized, washed, and resuspended in HBSS containing 2% FBS and DAPI (Molecular Probes/Invitrogen, Grand Island NY). Transduced colon OMP-C18 cells (GFP⁺/DAPI⁻) were sorted by flow cytometry using a FACSAria flow cytometer (BD Biosciences, San Jose CA). Cell population purity was confirmed by reanalysis of a fraction of the sorted cells. The sorted cells were mixed with 50% Matrigel™ (BD Biosciences, San Jose CA). Control lentivirus-transduced GFP⁺/DAPI⁻ cells were subcutaneously injected into the flanks of NOD/SCID mice (300 cells/mouse, n = 10). IgCAM decoy receptor lentivirus-transduced GFP⁺/DAPI⁻ cells were subcutaneously injected into the flanks of NOD/SCID mice (300 cells/mouse, n = 10). Non-transduced cells were subcutaneously injected into the flanks of NOD/SCID mice (300 cells/mouse, n = 5). Tumor growth was monitored weekly, and the experiment was terminated when the fastest growing tumor reached ∼ 1500mm³.

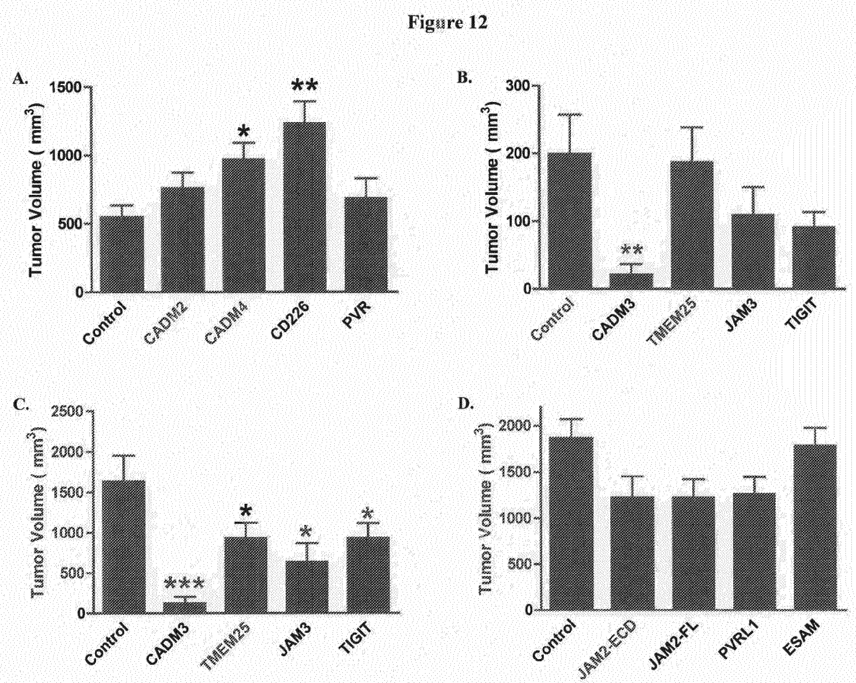

As shown in Figure 12A, at day 45 over-expression of CADM4 and CD226 decoy receptors in OMP-C18 tumors appeared to increase tumor growth as compared to control. Over-expression of CADM2 decoy receptor also appeared to increase tumor growth as compared to control, but to a less pronounced level than CADM4 and CD226.

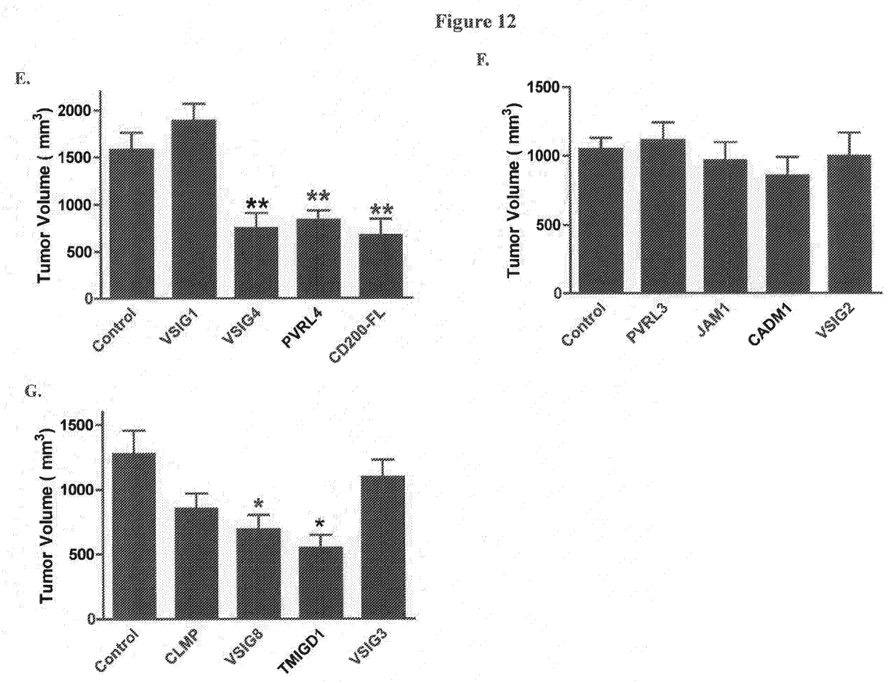

Similar studies were performed with OMP-C18 colon tumors and RRL-based lentiviruses expressing membrane-anchored decoy receptors, CADM3-CD4TM-GFP, TMEM25-CD4TM-GFP, JAM3-CD4TM-GFP, and TIGIT-CD4TM-GFP, (Fig. 12B and 12C), JAM2-CD4TM-GFP, JAM2FL-GFP, PVRL1-CD4TM-GFP, and ESAM-CD4TM-GFP (Fig. 12D), VSIG1-CD4TM-GFP, VSIG4-CD4TM-GFP, PVRL4-CD4TM-GFP, and CD200-FL-GFP (Fig. 12E), PVRL3-CD4TM-GFP, JAM1-CD4TM-GFP, CADM1-CD4TM-GFP and VSIG2-CD4TM-GFP (Fig. 12F), and CLMP-CD4TM-GFP, VSIG8-CD4TM-GFP, TMIGD1-CD4TM-GFP and VSIG3-CD4TM-GFP (Fig. 12G).

As shown in Figure 12B, at day 34 over-expression of CADM3 decoy receptor significantly inhibited growth of OMP-C18 tumor cells as compared to control. In addition, JAM3 and TIGIT appeared to inhibit tumor growth to some degree. At day 48, the inhibition of tumor growth by CADM3 as compared to control was even greater than at day 34 (Fig. 12C). Furthermore, at day 48 over-expression of TEMEM25, JAM3 and TIGIT decoy receptors significantly inhibited tumor growth as compared to control, although not to the extent observed with CADM3. At day 49, over-expression of VSIG4, PVRL4 and CD200 decoy receptors significantly inhibited growth of OMP-018 tumor cells as compared to control (Fig. 12E). At day 49, over-expression of VSIG8 and TIMIGD1 significantly inhibited growth of OMP-018 tumor cells as compared to control (Fig. 12G).

### Example 12

### Effect on tumor growth by over-expression of membrane-anchored IgCAM decoy receptors in lung tumors OMP-LU2 and OMP-LU40

Dissociated colon OMP-LU2 cells (1 x 10⁶ cells/well) were seeded into 6-well Primaria™ plates (BD Biosciences, San Jose CA) and transduced with RRL-based lentiviruses expressing membrane-anchored decoy receptors, CADM2-CD4TM-GFP, CADM4-CD4TM-GFP, PVRL4-CD4TM-GFP, VSIG4-CD4TM-GFP, CADM1-CD4TM-GFP, CD226-CD4TM-GFP, JAM3-CD4TM-GFP, JAM2-CD4TM-GFP, JAM2FL-GFP, CADM4-CD4TM-GFP, TIGIT-CD4TM-GFP, PVRL1-CD4TM-GFP, PVRL3-CD4TM-GFP, dnYAP as a positive control, or GFP only as a negative control. RRL-based lentiviruses express the GFP gene under the control of a CMV promoter. In addition, a second control was a group of cells that were not transduced. Lentiviruses were added to the cells at time of plating (t = 0) at a MOI which resulted in the infection of 20% to 60% of the OMP-LU2 cells. The culture medium was DMEM Low glucose, F12 medium, B27 medium, insulin-transferrin-selenium supplement, heparin, rhEGF, rhFGF-2, and antibiotics. After 72 hours, cells were collected, trypsinized, washed, and resuspended in HBSS containing 2% FBS and DAPI (Molecular Probes/Invitrogen, Grand Island NY). Transduced lung OMP-LU2 cells (GFP⁺/DAPI⁻) were sorted by flow cytometry using a FACSAria flow cytometer (BD Biosciences, San Jose CA). Cell population purity was confirmed by reanalysis of a fraction of the sorted cells. The sorted cells were mixed with 50% Matrigel™ (BD Biosciences, San Jose CA). Control lentivirus-transduced GFP⁺/DAPI⁻ cells were subcutaneously injected into the flanks of NOD/SCID mice (300 cells/mouse, n = 10). IgCAM decoy receptor lentivirus-transduced GFP⁺/DAPI⁻ cells were subcutaneously injected into the flanks of NOD/SCID mice (300 cells/mouse, n = 10). Non-transduced cells were subcutaneously injected into the flanks of NOD/SCID mice (300 cells/mouse, n = 5). Tumor growth was monitored weekly, and the experiment was terminated when the fastest growing tumor reached ∼1500mm³.

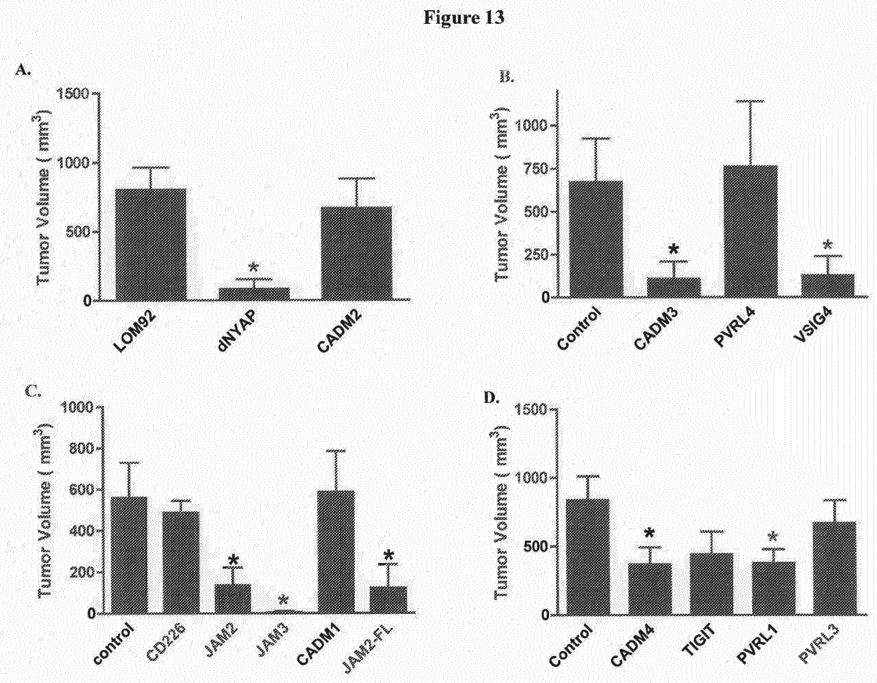

At day 108, over-expression of CADM3 and VSIG4 decoy receptors significantly inhibited growth of OMP-LU2 lung tumor cells as compared to control (Fig. 13B). At day 71, over-expression of JAM3 decoy receptor significantly inhibited growth of OMP-LU2 lung tumor cells as compared to control (Fig. 13C). In addition, at day 70 or 71, over-expression of JAM2, CADM4, and PVRL1 decoy receptors inhibited growth of OMP-LU2 tumor cells as compared to control (Fig. 13C and 13D).

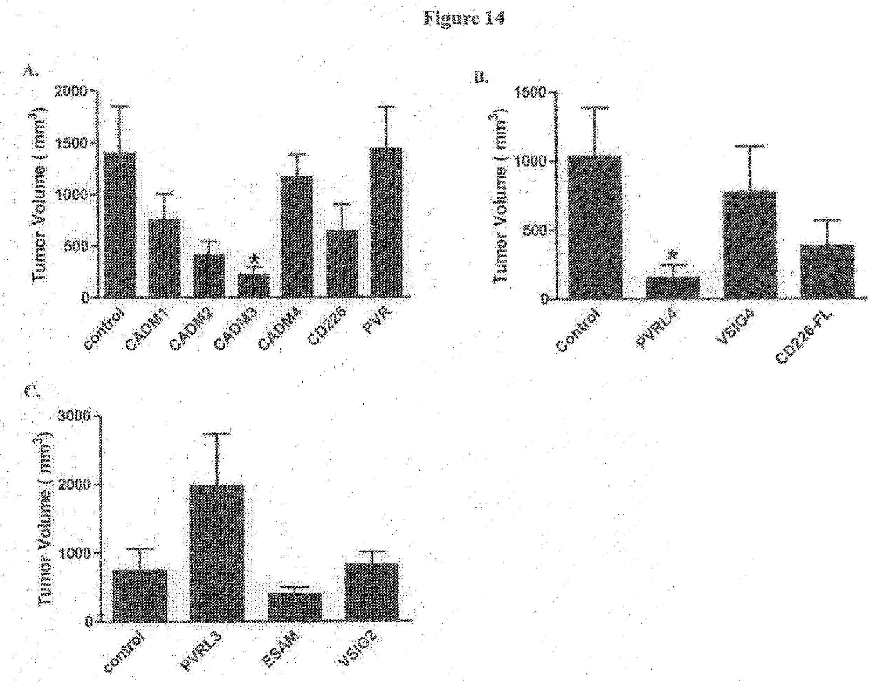

Similar studies were performed with OMP-LU40 lung tumors and RRL-based lentiviruses expressing membrane-anchored decoy receptors, CADM1-CD4TM-GFP, CADM2-CD4TM-GFP, CADM3-CD4TM-GFP, CADM4-CD4TM-GFP, CD226-CD4TM-GFP, and PVR-CD4TM-GFP (Fig. 14A), PVRL4-CD4TM-GFP, VSIG4-CD4TM-GFP, and CD226FL-GFP (Fig. 14B), PVRL3-CD4TM-GFP, ESAM-CD4TM-GFP, and VSIG2-CD4TM-GFP (Fig. 14C).

At day 118, over-expression of CADM2 and CADM3 decoy receptors significantly inhibited growth of OMP-LU40 lung tumor cells as compared to control (Fig. 13A). At day 120, over-expression of PVRL4 decoy receptor significantly inhibited growth of OMP-LU2 lung tumor cells as compared to control (Fig. 13B).

Inhibition by the decoy receptors in the studies described in Examples 11 and 12 is summarized in Table 4. The numbers represent a fold change decrease in tumor size as compared to control tumors.

**Table 4**

| | OMP-C18 | OMP-LU2 | OMP-LU40 |
|---|---|---|---|
| CLMP | 2.0 | ND | ND |
| VSIG4 | 2.4 | 3.7 | 1 |
| VSIG8 | 2.6 | ND | ND |
| JAM2 | 1.0 | 5.1 | ND |
| JAM3 | 1.7 | 56 | ND |
| CADM3 | 7.2 | 6.4 | 6.5 |
| CADM4 | Increased | 2.3 | 1 |
| PVRL1 | 1.5 | 2.2 | ND |
| PVRL4 | 1.9 | ND | 16 |
| TIGIT | 1.9 | 1.9 | ND |
| TMIGD1 | 2.3 | ND | ND |
| TMEM25 | 1.9 | ND | ND |

| | | | |
|---|---|---|---|
| ND = Not determined | | | |

Fourteen IgCAMs used as membrane-anchored decoys reduced tumor growth in colon and/or lung tumors, with inhibition ranging from 1.5 to 56-fold. Among the 14 IgCAMs, CADM3, JAM3 and PVRL4 showed the greatest effect.

### Example 13

### Proteomic studies

To investigate the signaling pathway(s) downstream of IgCAMs, a proteomic approach was taken to identify protein complexes containing a set of candidate IgCAMs implicated in either tumor growth and/or the Hippo pathway. HEK-293T cells were independently transiently transfected with FLAG-tagged full length IgCAMs, CADM1, CADM3, CD200, JAM2, PVR, PVRL1, and PVRL3. A FLAG-tagged YFP (yellow fluorescent protein) was used as a control. Four days post-transfection, lysates generated from the harvested cell pellets were clarified by centrifugation, pre-cleared with mouse IgG-agarose (Sigma-Aldrich, St. Louis, MO), and then incubated with anti-FLAG agarose (Sigma-Aldrich). Anti-FLAG immunoprecipitates were eluted with (2x) non-reducing Tris-Glycine SDS Sample Buffer (Life Technologies/Invitrogen, Grand Island, NY). Eluates were submitted to MSBioworks (Michigan, USA, IP-Works service) for mass spectrometry to identify proteins that co-immunoprecipitate with the transfected "bait" IgCAMs.

The proteins that co-immunoprecipitated with the transfected "bait" IgCAMs were first analyzed for heterotypic interactions with endogenous IgCAMs. For example, endogenous JAM3 co-immunoprecipitates with JAM2, which corroborates results observed with FACS-binding assays. While the FACS-binding assays detected some interactions that were not found by mass spectrometry (i.e., the interaction between JAM2 and CADM3), the inverse is also true (i.e., the interaction between PVR and CD200).

Next, the list of 1039 identified proteins was annotated for function; categories of interest included 1) cytoskeleton/motors, 2) transcription, 3) kinase/phosphatase/signaling cascade, 4) miscellaneous enzymatic activity, 5) small/heterotrimeric G proteins, 6) scaffolding/protein complexes, and 7) receptor/surface protein/adhesion/IgCAM-related. The g:Profiler database (http://biit.cs.ut.ee/gprofiler) was used to analyze the protein-protein interaction networks between the selected list of 125 proteins identified in the 7 categories above and the protein components of the Hippo pathway. The 125 proteins were also analyzed by Pearson correlation to identify clusters of the "bait" IgCAMs that form complexes with the same set of proteins. Based on these two analyses, CADM1, CADM3, PVRL1, and PVRL3 were identified as IgCAMs that co-immunoprecipitated with a set of proteins localized to the membrane and/or cell-cell junctions. These proteins, some of which can interact with Hippo components such as Angiomotin or Merlin, include DCAF7, DDB1, DLG1, INADL, LIN7C, MPDZ, MPP5, MPP7, PARD3, and PRKCI.

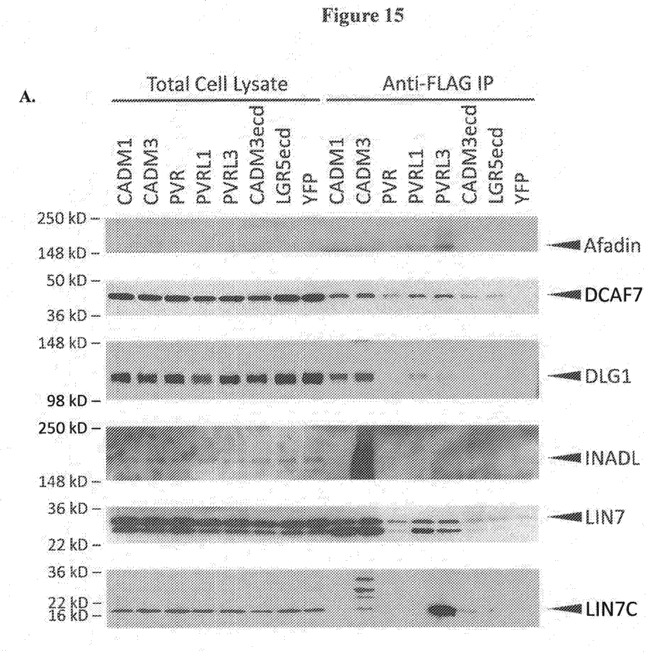

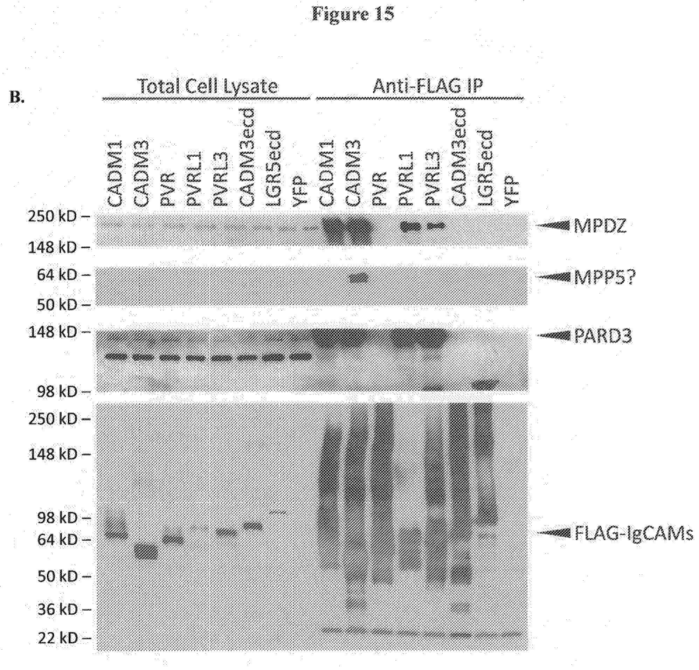

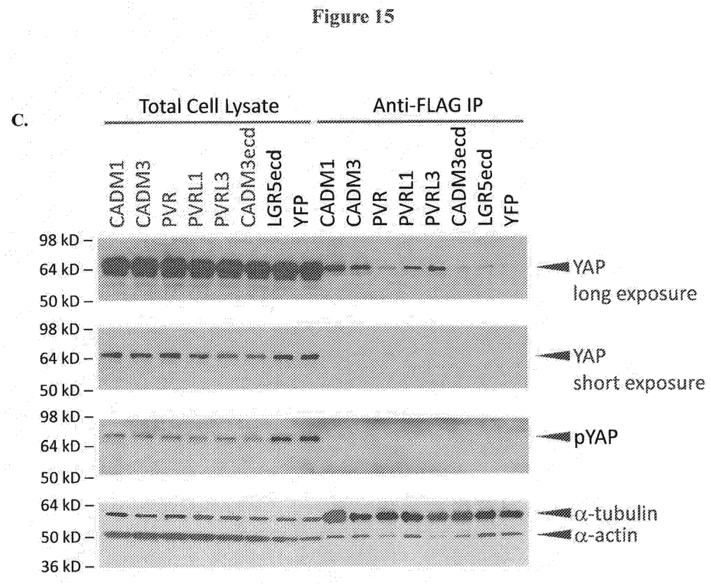

To validate these protein complexes, immunoprecipitates (see method described above) were prepared from HEK-293T cells transiently transfected with FLAG-tagged full-length CADM1, CADM3, PVR, PVRL1, and PVRL3, with CADM3-CD4TM-GFP and LGR5-CD4TM-GFP constructs as internal negative controls. Briefly, four days post-transfection, lysates generated from the harvested cell pellets were clarified by centrifugation, pre-cleared with mouse IgG-agarose (Sigma-Aldrich, St. Louis, MO), and then incubated with anti-FLAG agarose (Sigma-Aldrich). Anti-FLAG immunoprecipitates were eluted with (2x) non-reducing Tris-Glycine SDS Sample Buffer (Life Technologies/Invitrogen, Grand Island, NY). Total cell lysates and the immunoprecipitate eluates were run on 4-12% Tris-Glycine Midi Gels (Life Technologies/Invitrogen, Grand Island, NY) and transferred by iBlot onto PVDF membranes (Life Technologies/Invitrogen). Blots were probed with antibodies against the following proteins: Afadin/MLLT4 (#6492; Cell Signaling Technology, Danvers, MA); DCAF7 (# ab138490; Abeam, Cambridge, MA); DLG1 (# abl34156); INADL (# ab151257; Abcam); LIN7 (Abcam, ab127049; Abcam); LIN7C (# ab82646; Abcam); MPDZ (# ab101277; Abeam); MPP5 (# ab155132; Abcam); PARD3 (# ab64646; Abeam); YAP1 (# ab52771; Abcam); phospho-YAP1 (# 4911; Cell Signaling Technology), and tubulin and actin as controls. Western blots are shown in Figures 15A-15C and the positively-identified complexes containing IgCAMs are summarized in Table 5.

**Table 5**

| | CADM1 | CADM3 | PVR | PVRL1 | PVRL3 |
|---|---|---|---|---|---|
| Afadin | - | - | - | + | ++ |
| DCAF7 | +++ | ++++ | + | + | ++ |
| DLGI | +++ | ++++ | - | ++ | + |
| INADL | - | + | - | - | - |
| LIN7 | +++ | +++ | + | ++ | ++ |
| LIN7C | - | + | - | - | ++ |
| MPDZ | +++ | +++ | - | ++ | + |
| MPP5 | - | + | - | - | - |
| PARD3 | ++ | + | - | + | ++ |
| YAP1 | +++ | ++ | - | + | ++ |

Of particular note, YAP1 co-immunoprecipitated with CADM1, CADM3, PVRL1, and PVRL3 supporting the hypothesis that IgCAMs are involved in the Hippo pathway.

Additional experiments were done, wherein HEK-293T cells were transiently transfected with FLAG-tagged full-length CADM1 and c-myc-tagged YAP. Four days post-transfection, lysates generated from the harvested cell pellets were clarified by centrifugation and incubated with anti-c-myc agarose (Sigma-Aldrich). Anti-c-myc immunoprecipitates were eluted with (2x) non-reducing Tris-Glycine SDS Sample Buffer (Life Technologies/Invitrogen, Grand Island, NY). Total cell lysates and the immunoprecipitate eluates were run on 4-12% Tris-Glycine Midi Gels (Life Technologies/Invitrogen, Grand Island, NY) and transferred by iBlot onto PVDF membranes (Life Technologies/Invitrogen). Blots were probed with anti-myc antibody or anti-FLAG antibody.

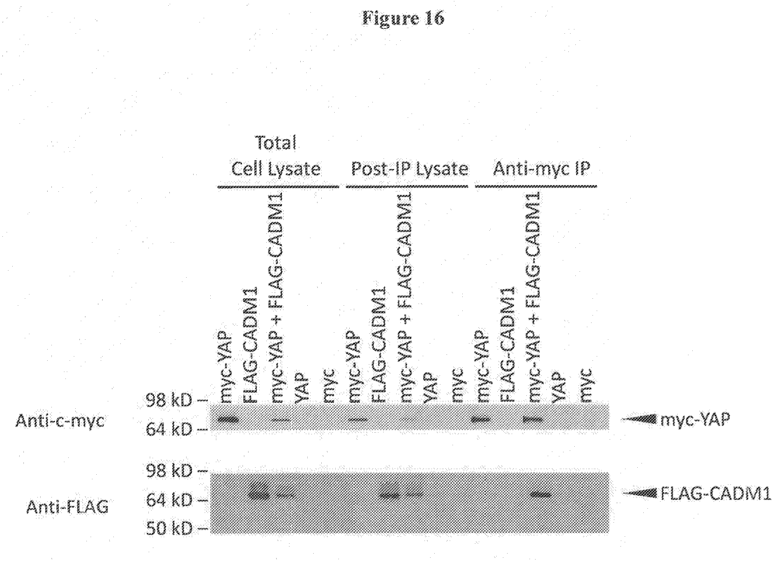

As shown in Figure 16, YAP1 co-immunoprecipitated with CADM1.

It is understood that the examples and embodiments described herein are for illustrative purposes only and that various modifications or changes in light thereof will be suggested to person skilled in the art and are to be included within the spirit and purview of this application.

All publications, patents, patent applications, internet sites, and accession numbers/database sequences including both polynucleotide and polypeptide sequences cited herein are hereby incorporated by reference herein in their entirety for all purposes to the same extent as if each individual publication, patent, patent application, internet site, or accession number/database sequence were specifically and individually indicated to be so incorporated by reference.

The sequences disclosed in this application are as follows:
JAM Family
   Human AMICA with predicted signal sequence underlined (SEQ ID NO:1) 
MFCPLKLILLPVLLDYSLGLNDLNVSPPELTVHVGDSA[?]MGCVFQSTEDKCIFKIDWTLS
PGEHAKDEYVLYYYSNLSVPIGRFQNRVHLMGDILCNDGSLLLQDVQEADQGTYICEIRL
KGESQVFKKAVVLHVLPEEPKELMVHVGGLIQMGCVFQS[?]EVKHVTKVEWIFSGRRAKEE
IVFRYYHKLRMSVEYSQSWGHFQNRVNLVGDIFRNDGS[?]MLQGVRESDGGNYTCSIHLGN
LVFKKTIVLHVSPEEPRTLVTPAALRPLVLGGNQLVIIVGIVCATILLLPVLILIVKKTC
GNKSSVNSTVLVKNTKKTNPEIKEKPCHFERCEG[?]KHIYSPIIVREVIEEEEPSEKSEAT
YMTMHPVWPSLRSDRNNSLEKKSGGGMPKTQQAF
   Human CAR with predicted signal sequence underlined (SEQ ID NO:2) 
MALLLCFVLLCGVVDFARSLSITTPEEMIEKAKGETAYLPCKFTLSPEDQGPLDIEWLIS
PADNQKVDQVIILYSGDKIYDDYYPDLKGRVHFTSNDLKSGDASINVTNLQLSDIGTYQC
KVKKAPGVANKK[?]HI[?]VLVKPSGARCYVDGSEEIGSDFKIKCEPKEGSLPLQYEWQKLSD
SQKMPTSWLAEMTSSVISVKNASS[?]YSGTYSCTVRNRVGSDQCLLRLNVVPPSNKAGLIA
GAIIGTLLALALIGLIIFCCRKKRREEKYEKEVHHDIREDVPPPKSRTSTARSYIGSNHS
SLGSMSPSNMEGYSKTQYNQVPSED[?]ERTPQSPTLPPAKFKYAY
 Human CLMP with predicted signal sequence underlined (SEQ ID NO:3) 
MSLLLLLLLVSYYVGTLGTHTEIKRVAEEKVTLPCHHQLGLPEKDTLDIEWLLTDNEGNQ
KVVITYSSRHVYNNLTEEQKGRVAFASNFLAGDASLQIEPLKPSDEGRYTCKVKNSGRYV
WSHVILKVLVRPSKPKCELEGELTEGSDLTLQCESSSGTEPIVYYWQRIREKEGEDERLP
PKSRIDYNHPGRVLLQNLTMSYSGLYQCTAGNEAGKESCVVRVTVQYVQSIGMVAGAVTG
IVAGALLIFLLVWLLIRRKDKERYEEEERPNEIREDAEAPKARLVKPSSSSSGSRSSRSG
SSSTRSTANSASRSQRTLSTDAAPQPGLATQAYSLVGPEVRGSEPKKVHHANLTKAETTP
SMIPSQSRAFQTV
   Human ESAM with predicted signal sequence underlined (SEQ ID NO:4) 
MISLPGPLVTNLLRFLFLGLSALAPPSRAQLQLHLPANRLQAVEGGEVVLPAWYTLHGEV
SSSQPWEVPFVMWFFKQKEKEDQVLSYINGVTTSKPGVSLVYSMPSRNLSL[?]LEGLQEKD
SGPYSCSVNVQDKQGKSRGHSIKTLELNVLVPPAPPSCRLQGVPHVGANVTLSCQSPRSK
PAVQYQWDRQLPSFQTFFAPALDVIRGSLSLTNLSSSMAGVYVCKAHNEVGTAQCNVTLE
VSTGPGAAVVAGAVVGTLVGLGLLAGLVLLYHRRGKALEEPANDIKEDAIAPR[?][?]PWPKS
SDTISKNGTLSSVTSARALRPPHGPPRPGALTPTPSLSSQALPSPRLPTTDGAHPQPISP
[?]PGGVSSSGLSRMGAVPVMVPAQSQAGSLV
   Human GP A33 with predicted signal sequence underlined (SEQ ID NO:5) 
MVGKMWPVLWTLCAVRVTVDAISVETPQDVLRASQGKSVTLPCTYHTSTSSREGLIQWDK
LLLTHTERVVIWPFSNKNYIHGELYKNRVSISNNAEQSDASITIDQLTMADNGTYECSVS
LMSDLEGNTKSRVRLLVLVPPSKPECGIEGETIIGNNIQLTCQSKEGSPTPQYSWKRYNI
LNQEQPLAQPASGQPVSLKNISTDTSGYYICTSSNEEGTQFCNITVAVRSPSMNVALYVG
IAVGVVAALIIIGIIIYCCCCRGKDDNTEDKEDARPNREAYEEPPEQLRELSREREEEDD
YRQEEQRSTGRESPDHLDQ
   Human JAM1 with predicted signal sequence underlined (SEQ ID NO:6) 
MGTKAQVERKLLCLFILAILLCSLALGSVTVHSSEPEVRIPENNPVKLSCAYSGFSSPRV
EWKFDQGDTTRLVCYNNKITASYEDRVTFLPTGITFKSVTREDTGTYTCMVSEEGGNSYG
LVKVKLIVLVPPSKPTVNIPSSATIGNRAVLTCSEQDGSPPSEYTWFKDGIVMPTNPKST
RAFSNSSYVLNPTTGELVFDPLSASDTGEYSCEARNGYGTPMTSNAVRMEAVERNVGVIV
AAVLVTLILLGILVFGIWFAYSRGHFDRTKKGTSSKKVIYSQPSARSEGEFKQTSSFLV
   Human JAM2 with predicted signal sequence underlined (SEQ ID NO:7) 
MARRSRHRLLLLLLLRYLVVALGYHKAYGFSAPKDQQVVTAVEYQEAILACKTPKKTVSSR
LEWKKLGRSVSFVYYQQTLQGDFKNRAEMIDFNIRIKNVTRSDAGKYRCEVSAPSEQGQN
 
LEEDTVTLEVLVAPAVPSCEVPSSALSGTVVELRCQDKEGNPAPEYTWFKDGIRLLENP[?]
LGSQSTNSSYTMNTKTGTLQFNTVSKLDTGEYSCEARNSVGYRRCPGKRMQVDDLN[?]SGI
IAAVVVVALVISVCGLGVCYAQRKGYFSKETSFQKSNSSSKATTMSENDFKHTKSF[?]I
   Human JAM3 with predicted signal sequence underlined (SEQ ID NO:8) 
MALRRPPRLRLCARLPDFFLLLLFRGCLIGAVNLKSSNRTPVVQEFESVELSCIITDSQT
SDPRIEWKKIQDEQTTYVFFDNKIQGDLAGRAEILGKTSLKIWNVTRRDSALYRCEVVAR
NDRKEIDEIVIELTVQVKPV[?]PVCRVPKAVPVGKMATLHCQESEGHPRPHYSWYRNDVPL
PTDSRANPRFRNSSFHLNSETGTLVFTAVHKDDSGQYYCIASNDAGSARCEEQEMEVYDL
NIGGIIGGVLVVLAVLALITLGICCAYRRGYFINNKQDGESYKNPGKPDGVNYIRTDEEG
DFRHKSSFVI
   Human VSIG1 with predicted signal sequence underlined (SEQ ID NO:9) 
MVFAFWKVFLILSCLAGQVSVVQVTIPDGFVNVTVGSNVTLICIYTTTVASREQLSIQWS
FFHKKEMEPISHSSCLSTEGMEEKAVSQCLKMTHARDARGRCSWTSEIYFSQGGQAVAIG
QFKDRITGSNDPGNASITISHMQPADSGIYICDVNNPPDFLGQNQGILNVSVLVKPSKPL
CSVQGRPETGHTISLSCLSALGTPSPVYYWHKLEGRDIVPVKENFNPTTGILVIGNLTNF
EQGYYQCTAINRLGNSSCEIDLTSSHPEVGIIVGALIGSLVGAAIIISVVCFARNKAKAK
AKERNSKTIAELEPMTK[?]N[?]RGESEAMPREDATQLEVTLPSSIHETGPDTIQEPDYEPKP
TQEPAPEPAPGSEPMAVPDLDIELELEPETQSELEPEPEPEPESEPGVVVEPLSEDEKGV
VKA
   Human VSIG2 with predicted signal sequence underlined (SEQ ID NO:10) 
MAELPGPFLCGALLGFLCLSGLAVEVKVPTEPLSTPLGKTAELTCTYSTSVGDSFALEWS
FVQPGKPISESHPILYFTNGHLYPTGSKSKRVSLLQNPPTVGVATLKLTDVHPSDTGTYL
CQVNNPPDFYTNGLGLINLTVLVPPSNPLCSQSGQTSVGGSTALRCSSSEGAPKPVYNWV
RLGTFPTPSPGSMVQDEVSGQLILTNLSLTSSGTYRCVATNQMGSASCELTLSVTEPSQG
RVAGALIGVLLGVLLLSVAAFCLVRFQKERGKKPKETYGGSDLREDAIAPGISEHTCMRA
DSSKGFLERPSSASTVTTTKSKLPMVV
   Human VSIG3 with predicted signal sequence underlined (SEQ ID NO:11) 
MSLVELLLWWNCFSRTGVAASLEVSESPGSIQVARGQPAVLPCTFTTSAAL[?]NLNVIWMV
TPLSNANQPEQVILYQGGQMFDGAPRFHGRVGFTGTMPATNVSIFINNTQLSDTGTYQCL
VNNLPDIGGRNIGVTGLTVLVPPSAPHCQIQGSQDIGSDVILLCSSEEGIPRPTYLWEKL
DNTLKLPPTATQDQVQGTVTIRNISALSSGLYQCVASNAIGTSTCLLDLQVISPQPRN[?]G
LIAGAIGTGAVIIIFCIALILGAFFYWRSKNKEEEEEEEIPNEIREDDLPPKCSSAKAFHT
EISSSDNNTLTSSNAYNSRYWSNNPKVHRNTESVSHFSDLGQSFSFHSGNANIPSIYANG
THLVPGQHKTLVVTANRGSSPQVMSRSNGSVSRKPRPPHTHSYTISHATLERIGAVPVMV
PAQSRAGSLV
   Human VSIG4 with predicted signal sequence underlined (SEQ ID NO:12) 
MGILLGLLLLGHLTVDTYGRPILEVPESVTGPWKGDVNLPCTYDPLQGYTQVLVKWLVQR
GSDPVTIFLRDSSGDHIQQAKYQGRLHVSHKVPGDVSLQLSTLEMDDRSHYTCEV[?]WQTP
DGNQVVRDKITELRVQKLSVSKPTVTTGSGYGFTVPQGMRISLQCQARGSPPISYIWYKQ
QTNNQEPIKVATLSTLLFKPAVIADSGSYFCTAKGQVGSEQHSDIVKFVVKDSSKLLKTK
TEAPTTMTYPLKATSTVKQSWDWTTDMDGYLGETSAGPGKSLPVFAIILIISLCCMVVFT
MAYIMLCRKTSQQEHVYEAARAHAREANDSGETMRVAIFASGCSSDEPTSQNLGNNYSDE
PCIGQEYQIIAQINGNYARLLDTVPLDYEFLATEGKSVC
   Human VSIG8 with predicted signal sequence underlined (SEQ ID NO:13) 
MRVGGAFHLLLVCLSPALLSAVRINGDGQEVLYLAEGDNVRLGCPYVLDPEDYGPNGLDI
EWMQVNSDPAHHRENVFLSYQDKRINHGSLPHLQQRVRFAASDPSQYDASINLMNLQVSD
TATYECRVKKTTMATRKVIVTVQARPAVPMCWTEGHMTYGNDVVLKCYASGGSQPLSYKW
AKISGHHYPYRAGSYTSQHSYHSELSYQESFHSSINQGLNNGDLVLKDISRADDGLYQCT
 
VANNVGYSVCVVEVKVSDSRRIGVIIGIVLGSLLALGCLAVGIWGLVCCCCGGSGAGGAR
GAFGYGNGGGVGGGACGDLASEIREDAVAPGCKASGRGSRVTHLLGYPTQNVSRSLRRKY
APPPCGGPEDVALAPCTAAAACEAGPSPVYVKVKSAEPADCAEGPVQCKNGLLV
CADM Family
   Human CADM1 with predicted signal sequence underlined (SEQ ID NO:14) 
MASVVLPSGSQCAAAAAAAAPPGLRLRLLLLLFSAAALIPTGDGQNLFTKDVTVIEGEVA
TISCQVNKSDDSVIQLLNPNRQTIYFRDFRPLKDSRFQLLNFSSSELKVSLTNVSISDEG
RYFCQLYTDPPQESYTTITVLVPPRNLMIDIQKDTAVEGEEIEVNCTAMASKPATTIRWF
KGNTELKGKSEVEEWSDMYTVTSQLMLKVHKEDDGVPVICQVEHPAVTGNLQTQRYLEVQ
YKPQVHIQMTYPLQGLTREGDALELTCEAIGKPQPVMVTWVRVDDEMPQHAVLSGPNLFI
NNLNKTDNGTYRCEASNIVGKAHSDYMLYVYDPPTTIPPPTTTTTTTTTTTTILTIITD
SRAGEEGSIRAVDHAVIGGVVAVVVFAMLCLLIILGRYFARHKGTYFTHEAKGADDAADA
DTAIINAEGGQNNSEEKKEYFI
   Human CADM2 with predicted signal sequence underlined (SEQ ID NO:15) 
MIWKRSAVLRFYSVCGLLLQGSQGQFPLTQNVTVVEGGTAILTCRVDQNDNTSLQWSNPA
QQTLYFDDKKALRDNRIELVRASWHELSISVSDVSLSDEGQYTCSLFTMPVKTSKAYLTV
LGVPEKPQISGFSSPVMEGDLMQLTCKTSGSKPAADIRWFKNDKEIKDVKYLKEEDANRK
TFTVSSTLDFRVDRSDDGVAVICRVDHESLNATPQVAMQVLEIHYTPSVKIIPSTPFPQE
GQPLILTCESKGKPLPEPVLWTKDGGELPDPDRMVVSGRELNILFLNKTDNGTYRCEATN
TIGQSSAEYVLIVHDVPNTLLPTTIIPSLTTATVTTTVAITTSPTTSATTSSIRDPNALA
GQNGPDHALIGGIVAVVVFVTLCSIFLLGRYLARHKGTYLTNEAKGAEDAPDADTAIINA
EGSQVNAEEKKEYFI
   Human CADM3 with predicted signal sequence underlined (SEQ ID NO:16) 
MGAPAASLLLLLLLFACCWAPGGANLSQDGYWQEQDLELGTLAPLDEAISSTVWSSPDML
ASQDSQPWTSDETVVAGGTVVLKCQVKDHEDSSLQWSNPAQQTLYFGEKRALRDNRIQLV
TSTPHELSISISNVALADEGEYTCSIFTMPVRTAKSLVTVLGIPQKPIITGYKSSLREKD
TATLNCQSSGSKPAARLTWRKGDQELHGEPTRIQEDPNGKTFTVSSSVTFQVTREDDGAS
IVCSVNHESLKGADRSTSQRIEVLYTPTAMIRPDPPHPREGQKLLLHCEGRGNPVPQQYL
WEKEGSVPPLKMTQESALIFPFLNKSDSGTYGCTATSNMGSYKAYYTLNVNDPSPVPSSS
STYHAIIGGIVAFIVFLLLIMLIFLGHYLIRHKGTYLTHEAKGSDDAPDADTAIINAEGG
QSGGDDKKEYFI
   Human CADM4 with predicted signal sequence underlined (SEQ ID NO:17) 
MGRARRFQWPLLLLWAAAAGPGAGQEVQTENVTVAEGGVAEITCRLHQYDGSIVVIQNPA
RQTLFFNGTRALKDERFQLEEFSPRRVRIRLSDARLEDEGGYFCQLYTEDTHHQIATLTV
LVAPENPVVEVREQAVEGGEVELSCLVPRSRPAATLRWYRDRKELKGVSSSQENGKVWSV
ASTVRFRVDRKDDGGIIICEAQNQALPSGHSKQTQYVLDVQYSPTARIHASQAVVREGDT
LVLTCAVTGNPRPNQIRWNRGNESLPERAEAVGETLTLPGLVSADNGTYTCEASNKHGHA
RALYVLVVYDPGAVVEAQTSVPYAIVGGILALLVFLIICVLVGMVWCSVRQKGSYLTHEA
SGLDEQGEAREAFLNGSDGHKRKEEFFI
   Human CRTAM with predicted signal sequence underlined (SEQ ID NO:18) 
MWWRVLSLLAWFPLQEASLTNHTETITVEEGQTLTLKCVTSLRKNSSLQWLTPSGFTIFL
NEYPALKNSKYQLLHHSANQLSITVPNVTLQDEGVYKCLHYSDSVSTKEVKVIVLATPFK
PILEASVIRKQNGEEHVVLMCSTMRSKPPPQITWLLGNSMEVSGGTLHEFETDGKKCNTT
STLIIHTYGKNSTVDCIIRHRGLQGRKLVAPFRFEDLVTDEETASDALERNSLSSQDPQQ
PTSTVSVTEDSSTSEIDKEEKEQTTQDPDLTTEANPQYLGLARKKSGILLLTLVSFLIFI
LFIIVQLFIMKLRKAHVIWKKENEVSEHTLESYRSRSNNEETSSEEKNGQSSHPMRCMNY
ITKLYSEAKTKRKENVQHSKLEEKHIQVPESIV
   Human TMIGD1 with predicted signal sequence underlined (SEQ ID NO:19) 
MAWKSSVIMQMGRFLLLVILFLPREMTSSVLTVNGKTENYILDTTPGSQASLICAVQNHT
REEELLWYREEGRVDLKSGNKINSSSVCVSSISENDNGISFTCRLGRDQSVSVSVVLNVT
FPPLLSGNDFQTVEEGSNVKLVCNVKANPQAQMMWYKNSSLLDLEKSRHQIQQTSESFQL
SITKVEKPDNGTYSCIAKSSLKTESLDFHLIVKDKTVGVPIEPIIAACVVIFLTLCFGLI
ARRKKIMKLCMKDKDPHSETAL
PVR Family
   Human CD96 with predicted signal sequence underlined (SEQ ID NO:20) 
MEKKWKYCAVYYIIQIHFVKGVWEKTVNTEENVYATLGSDVNLTCQTQTVGFFVQMQWSK
VTNKIDLIAVYHPQYGFYCAYGRPCESLVTFTETPENGSKWTLHLRNMSCSVSGRYECML
VLYPEGIQTKIYNLLIQTHVTADEWNSNHTIEIEINQTLEIPCFQNSSSKISSEFTYAWS
VENSSTDSWVLLSKGIKEDNGTQETLISQNHLISNSTLLKDRVKLGTDYRLHLSPVQIFD
DGRKFSCHIRVGPNKILRSSTTVKVFAKPEIPVIVENNSTDVLVERRFTCLLKNVFPKAN
ITWFIDGSFLHDEKEGIYITNEERKGKDGFLELKSVLTRVHSNKPAQSDNLTIWCMALSP
VPGNKVWNISSEKITFLLGSEISSTDPPLSVTESTLDTQPSPASSVSPARYPATSSVTLV
DVSALRPNTTPQPSNSSMTTRGFNYPWTSSGTDTKKSVSRIPSETYSSSPSGAGSTLHDN
VFTSTARAFSEVPTTANGSTKTNHVHITGIVVNKPKDGMSWPVIVAALLFCCMILFGLGV
RKWCQYQKEIMERPPPFKPPPPPIKYTCIQEPNESDLPYHEMETL
   Human CD200 with predicted signal sequence underlined (SEQ ID NO:21) 
MERLVIRMPFSHLSTYSLVWVMAAVVLCTAQVQVVTQDEREQLYTPASLKCSLQNAQEAL
IVTWQKKKAVSPENMVTFSENHGVVIQPAYKDKINITQLGLQNSTITFWNITLEDEGCYM
CLFNTFGFGKISGTACLTVYVQPIVSLHYKFSEDHLNITCSATARPAPMVFWKVPRSGIE
NSTVTLSHPNGTTSVTSILHIKDPKNQVGKEVICQVLHLGTVTDFKQTVNKGYWFSVPLL
LSIVSLVILLVLISILLYWKRHRNQDREP
   Human CD200R1 with predicted signal sequence underlined (SEQ ID NO:22) 
MLCPWRTANLGLLLILTIFLVAEAEGAAQPNNSLMLQTSKENHALASSSLCMDEKQITQN
YSKVLAEVNTSWPVKMATNAVLCCPPIALRNLIIITWEIILRGQPSCTKAYKKETNETKE
TNCTDERITWVSRPDQNSDLQIRTVAITHDGYYRCIMVTPDGNFHRGYHLQVLVTPEVTL
FQNRNRTAVCKAVAGKPAAHISWIPEGDCATKQEYWSNGTVTVKSTCHWEVHNVSTVTCH
VSH[?]TGNKSLYIELLPVPGAKKSAKLYIPYIILTIIILTIVGFIWLLKVNGCRKYKLNKT
ESTPVVEEDEMQPYASYTEKNNPLYDTTNKVKASEALQSEVDTDLHTL
   Human CD200R1L with predicted signal sequence underlined (SEQ ID NO:23) 
MSAPRLLISIIIMVSASSSSCMGGKQMTQNYSTIFAEGNISQPVLMDINAVLCCPPIALR
NLIIIITWEIILRGQPSCTKAYKKETNETKETNCTVERITWVSRPDQNSDLQIRPVDTTHD
GYYRGIVVTPDGNFHRGYHLQVLVTPEVNLFQSRNITAVCKAVTGKPAAQISWIPEGSIL
ATKQEYWGNGTVTVKSTCPWEGHKSTVTCHVSHLTGNKSLSVKLNSGLRTSGSPALSLLI
ILYVKLSLFVVILVTTGFVFFQRINHVRKVL
   Human CD226 with predicted signal sequence underlined (SEQ ID NO:24) 
MDYPTLLLALLHVYRALCEEVLWHTSVPFAENMSLECVYPSMGILTQVEWFKIGTQQDSI
AIFSPTHGMVIRKPYAERVYFLNSTMASNNMTLFFRNASEDDVGYYSCSLYTYPQGTWQK
VIQVVQSDSFEAAVPSNSHIVSEPGKNVTLTCQPQMTWPVQAVRWEK[?]QPRQIDLLTYCN
LVHGRNFTSKFPRQIVSNCSHGRWSVIVIPDVTVSDSGLYRCYLQASAGENETFVMRLTV
AEGKTDNQYTLFVAGGTVLLLLFVISITTIIVIFLNRRRRRERRDLFTESWDTQKAPNNY
RSPISTSQPTNQSMDDTREDIYVNYPTFSRRPKTRV
   Human PVRIG with predicted signal sequence underlined (SEQ ID NO:25) 
MRTEAQVPALQPPEPGLEGAMGHRTLVLPWVLLTLCVTAGTPEVWVQVRMEATELSSFTI
RCGFLGSGSISLVTVSWGGPNGAGGTTLAVLHPERGIRQWAPARQARWETQSSISLILEG
SGASSPCANTTFCCKFASFPEGSWEACGSLPPSSDPGLSAPPTPAPILRADLAGILGVSG
VLLFGCVYLLHLLRRHKHRPAPRLQPSRTSPQAPRARAWAPSQASQAALHVPYATINTSC
 
RPATLDTAHPHGGPSWWASLPTHAAHRPQGPAAWASTPIPARGSFVSVENGLYAQAGERP
PHTGPGLTLFPDPRGPRAMEGPLGVR
   Human PVR with predicted signal sequence underlined (SEQ ID NO:26) 
MARAMAAAWPLLLVAL[?]VLSWPPPGTGDVVVQAPTQVPGF[?]GDSVTLPCYLQVPNMEVTH
VSQLTWARHGESGSMAVFHQTQGPSYSESKRLEFVAAR[?]GAELRNAS[?]RMFGLRVEDEGN
YTCLFVTFPQGSRSVD[?]W[?]RVLAKPQNTAEVQKVQ[?]TGEPVPMARCVSTGGRPPAQITWH
SDLGGMPNTSQVPGFLSGTVTVTSLWILVPSSQVDGKNVTCKVEHESFEKPQLLTV[?]LTV
YYPPEVSISGYDNNWYLGQNEATLTCDARSNPEPTGYNWSTTMGPLPPFAVAQGAQLLIR
PVDKPINTTLICNVTNALGARQAELTVQVKEGPPSEHSGMSRNAIIFLVLGILVF[?]ILLG
IGIYFYWSKCSREVLWHCHLCPSSTEHASASANGHVSYSAVSRENSSSQDPQTEGTR
   Human PVRL1 with predicted signal sequence underlined (SEQ ID NO:27) 
MARMGLAGAAGRWWGLALGLTAFFLPGVHSQVVQVNDSMYGFIGTDVVLHCSFANPLPSV
KITQVTWQKSTNGSKQNVAIYNPSMGVSVLAPYRERVEFLRPSFTDGTIRLSRLELEDEG
VYICEFATFPTGNRESQLNLTVMAKPTNWIEGTQAVLRAKKGQDDKVLVATCTSANGKPP
SVVSWETRLKGEAEYQEIRNPNGTVTVISRYRLVPSREAHQQSLACIVNYHMDRFKESLT
LNVQYEPEVTIEGFDGNWYLQRMDVKLTCKADANPPATEYHWTTLNGSLPKGVEAQNRTL
FFKGPINYSLAGTYICEATNPIGTRSGQVEVNITEFPYTPSPPEHGRRAGPVPTAIIGGV
AGSILLVLIVVGGIVVALRRRRHTFKGDYSTKKHVYGNGYSKAGIPQHHPPMAQNLQYPD
DSDDEKKAGPLGGSSYEEEEEEEEGGGGGERKVGGPHPKYDEDAKRPYFTVDEAEARQDG
YGDRTLGYQYDPEQLDLAENMVSQNDGSFISKKEWYV
   Human PVRL2 with predicted signal sequence underlined (SEQ ID NO:28) 
MARAAALLPSRSPPTPLLWPLLLLLLLETGAQDVRVQVLPEVRGQLGGTVELPCHLLPPV
PGLYISLVTWQRPDAPANHQNVAAFHPKMGPSFPSPKPGSERLSFVSAKQSTGQDTEAEL
QDATLALHGLTVEDEGNYTCEFATFPKGSVRGMTWLRVIAKPKNQAEAQKVTFSQDPTTV
ALCISKEGRPPARISWLSSLDWEAKETQVSGTLAGTVTVTSRFTLVPSGRADGVTVTCKV
EHESFEEPALIPVTLSVRYPPEVSISGYDDNWYLGRTDATLSCDVRSNPEPTGYDWSTTS
GT[?]PTSAVAQGSQLVIHAVDSLFNTTFVCTVTNAVGMGRAEQVIFVRETPNTAGAGATGG
IIGGIIAAIIATAVAATGILICRQQRKEQTLQGAEEDEDLEGPPSYKPPTPKAKLEAQEM
PSQLFTLGASEHSPLKTPYFDAGASCTEQEMPRYHELPTLEERSGPLHPGATSLGSPIPV
PPGPPAVEDVSLDLEDEEGEEEEEYLDKINPIYDALSYSSPSDSYQGKGFVMSRAMYV
   Human PVRL3 with predicted signal sequence underlined (SEQ ID NO:29) 
MARTLRPSPLCPGGGKAQLSSASLLGAGLLLQPPTPPPLLLLLFPLLLFSRLCGALAGPI
IVEPHVTAVWGKNVSLKCLIEVNETITQISWEKIHGKSSQTVAVHHPQYGFSVQGEYQGR
VLFKNYSLNDATITLHNIGFSDSGKYICKAVTFPLGNAQSSTTVTVLVEPTVSLIKGPDS
LIDGGNETVAAICIAATGKPVAHIDWEGDLGEMESTTTSFPNETATIISQYKLFPTRFAR
GRRITCVVKHPALEKDIRYSFILDIQYAPEVSVTGYDGNWFVGRKGVNLKCNADANPPPF
KSVWSRLDGQWPDGLLASDNTLHFVHPLTFNYSGVYICKVTNSLGQRSDQKVIYISDPPT
TTTLQPTIQWHPSTADIEDLATEPKKLPFPLSTLATIKDDTIATIIASVVGGALFIVLVS
VLAGIFCYRRRRTFRGDYFAKNYIPPSDMQKESQIDVLQQDELDSYPDSVKKENKNPVNN
LIRKDYLEEPEKTQWNNVENLNRFERPMDYYEDLKMGMKFVSDEHYDENEDDLVSHVDGS
VISRREWYV
   Human PVRL4 with predicted signal sequence underlined (SEQ ID NO:30) 
MPLSLGAEMWGPEAWLLLLLLLASFTGRCPAGELETSDVVTVVLGQDAKLPCFYRGDSGE
QVGQVAWARVDAGEGAQELALLHSKYGLHVSPAYEGRVEQPPPPRNPLDGSVLLRNAVQA
DEGEYECRVSTFPAGSFQARLRLRVLVPPLPSLNPGPALEEGQGLTLAASCTAEGSPAPS
VTWDTEVKGTTSSRSFKHSRSAAVTSEFHLVPSRSMNGQPLTCVVSHPGLLQDQRITHIL
HVSFLAEASVRGLEDQNLWHIGREGAMLKCLSEGQPPPSYNWTRLDGPLPSGVRVDGDTL
GFPPLTTEHSGIYVCHVSNEFSSRDSQVTVDVLDPQEDSGKQVDLVSASVVVVGVIAALL
FCLLVVVVVLMSRYHRRKAQQMTQKYEEELTLTRENSIRRLHSHHTDPRSQPEESVGLRA
 
EGHPDSLKDNSSCSVMSEEPEGRSYSTLTTVREIETQTELLSPGSGRAEEEEDQDEGIKQ
AMNHFVQENGTLRAKPTGNGIYINGRGHLV
   Human TIGIT with predicted signal sequence underlined (SEQ ID NO:31) 
MRWCLLLIWAQGLRQAPLASGMMTGTIETTGNISAEKGGSIILQCHLSSTTAQVTQVNWE
QQDQLLAICNADLGWHISPSFKDRVAPGPGLGLTLQSLTVNDTGEYFCIYHTYPDGTYTG
RIFLEVLESSVAEHGARFQIPLLGAMAATLVVICTAVIVVVALTRKKKALRIHSVEGDLR
RKSAGQEEWSPSAPSPPGSCVQAEAAPAGLCGEQRGEDCAELHDYFNVLSYRSLGNCSFF
TETG
   Human TMEM25 with predicted signal sequence underlined (SEQ ID NO:32) 
MALPPGPAALRHTLLLLPALLSSGWGELEPQIDGQTWAERALRENERHAFTCRVAGGPGT
PRLAWYLDGQLQEASTSRLLSVGGEAFSGGTSTFTVTAHRAQHELNCSLQDPRSGRSANA
SVILNVQFKPEIAQVGAKYQEAQG[?]GLLVVLFALVRANPPANVTWIDQDGPVTVNTSDFL
VLDAQNYPWLTNHTVQLQLRSLAHNLSVVATNDVGTSASLPAPGLLATRVEVPLLGIVV
AAGLALGTLVGFSTLVACLVCRKEKKTKGPSRHPSLISSDSNNLKLNNVRLPRENMSLPS
NLQLNDLTPDSRAVKPADRQMAQNNSRPELLDPEPGGLLTSQGFIRLPVLGYIYRVSSVS
SDEIWL
JAM Family
   Human AMICA - ECD without predicted signal sequence (SEQ ID NO:33) 
LNDLNVSPPELTVHVGDSALMGCVFQSTEDKCIFKIDWTLSPGEHAKDEYVLYYYSNLSV
PIGRFQNRV[?]LMGDILCNDGSLLLQDVQEADQGTYICEIRLKGESQVFKKAVVLHVLPEE
PKELMVHVGGLIQMGCVFQSTEVKHVTKVEWIFSGRRAKEEIVFRYYHKLRMSVEYSQSW
GHFQNRVNLVGDIFRNDGSIMLQGVRESDGGNYTCSIHLGNLVFKKTIVLHVSPEEPRTL
VTPAALRPLVLGGNQLV
   Human CAR - ECD without predicted signal sequence (SEQ ID NO:34) 
LSITTPEEMIEKAKGETAYLPCK[?]TLSPEDQGPLDIEWLISPADNQKVDQVIILYSGDKI
YDDYYPDLKGRVHFTSNDLKSGDASINVTNLQLSDIGTYQCKVKKAPGVANKKIHLVVLV
KPSGARCYVDGSEEIGSDFKIKCEPKEGSLPLQYEWQKLSDSQKMPTSWLAEMTSSVISV
KNASSEYSGTYSCTVRNRVGSDQCLLRLNVVPPSNKAG
   Human CLMP - ECD without predicted signal sequence (SEQ ID NO:35) 
THTEIKRVAEEKVTLPCHHQLGLPEKDTLDIEWLLTDNEGNQKVVITYSSRHVYNNLTEE
QKGRVAFASNFLAGDASLQIEPLKPSDEGRYTCKVKNSGRYVWSHVILKVLVRPSKPKCE
LEGELTEGSDLTLQCESSSGTEPIVYYWQRIREKEGEDERLPPKSRIDYNHPGRVLLQNL
TMSYSGLYQCTAGNEAGKESCVVRVTVQYVQSIGMVA
   Human ESAM - ECD without predicted signal sequence (SEQ ID NO:36) 
QLQLHLPANRLQAVEGGEVVLPAWYTLHGEVSSSQPWEVPFVMWFFKQKEKEDQVLSYIN
GVTTSKPGVSLVYSMPSRNLSLRLEGLQEKDSGPYSCSVNVQDKQGKSRGHSIKTLELNV
LVPPAPPSCRLQGVPHVGANVTLSCQSPRSKPAVQYQWDRQLPSFQTFFAPALDVIRGSL
SLTNLSSSMAGVYVCKAHNEVGTAQCNVTLEVSTGPGAA
   Human GP A33 - ECD without predicted signal sequence (SEQ ID NO:37) 
ISVETPQDVLRASQGKSVTLPCTYHTSTSSREGLIQWDKLLLTHTERVVIWPFSNKNYIH
GELYKNRVSISNNAEQSDASITIDQLTMADNGTYECSVSLMSDLEGNTKSRVRLLVLVPP
SKPECGIEGETIIGNNIQLTCQSKEGSPTPQYSWKRYNILNQEQPLAQPASGQPVSLKNI
STDTSGYYICTSSNEEGTQFCNITVAVRSPSMNV
   Human JAM1 - ECD without predicted signal sequence (SEQ ID NO:38) 
SVTVHSSEPEVRIPENNPVKLSCAYSGFSSPRVEWKFDQGDTTRLVCYNNKITASYEDRV
TFLPTGITFKSVTREDTGTYTCMVSEEGGNSYGEVKVKLIVLVPPSKPTVNIPSSATIGN
 
RAVLTCSEQDGSPPSEYTWFKDGIVMPTNPKSTRAFSNSSYVLNPTTGELVFDPLSASDT
GEYSCEARNGYGTPMTSNAVRMEAVERNVGV
   Human JAM2 - ECD without predicted signal sequence (SEQ ID NO:39) 
FSAPKDQQVVTAVEYQEAILACKTPKKTVSSRLEWKKLGRSVSFVYYQQTLQGDFKNRAE
MIDFNIRIKNVTRSDAGKYRCEVSAPSEQGQNLEEDTVTLEVLVAPAVPSCEVPSSALSG
TVVELRCQDKEGNPAPEYTWFKDGIRLLENPRLGSQSTNSSYTMNTKTGTLQFNTVSKLD
TGEYSCEARNSVGYRRCPGKRMQVDDLNIS
   Human JAM3 - ECD without predicted signal sequence (SEQ ID NO:40) 
VNLKSSNRTPVVQEFESVELSCIITDSQTSDPRIEWKKIQDEQTTYVFFDNKIQGDLAGR
AEILGKTSLKIWNVTRRDSALYRCEVVARNDRKEIDEIVIELTVQVKPVTPVCRVPKAVP
VGKMATLHCQESEGHPRPHYSWYRNDVPLPTDSRANPRFRNSSFHLNSETGTLVFTAVHK
DDSGQYYCIASNDAGSARCEEQEMEVYDLN
   Human VSIG1 - ECD without predicted signal sequence (SEQ ID NO:41) 
QVSVVQVTIPDGFVNVTVGSNVTLICIYTTTVASREQLSIQWSFFHKKEMEPISHSSCLS
TEGMEEKAVSQCLKMTHARDARGRCSWTSEIYFSQGGQAVAIGQFKDRITGSNDPGNASI
TISHMQPADSGIYICDVNNPPDFLGQNQGILNVSVLVKPSKPLCSVQGRPETGHTISLSC
LSALGTPSPVYYWHKLEGRDIVPVKENFNPTTGIL
   Human VSIG2 - ECD without predicted signal sequence (SEQ ID NO:42) 
VEVKVPTEPLSTPLGKTAELTCTYSTSVGDSFALEWSFVQPGKPISESHPILYFTNGHLY
PTGSKS[?]RVSLLQNPPTVGVATLKLTDVHPSDTGTYLCQVNNPPDFYTNGLGLINLTVLV
PPSNPLCSQSGQTSVGGSTALRCSSSEGAPKPVYNWVRLGTFPTPSPGSMVQDEVSGQLI
LTNLSLTSSGTYRCVATNQMGSASC[?]LTLSVTEPSQGRVA
   Human VSIG3 - ECD without predicted signal sequence (SEQ ID NO:43) 
ASLEVSESPGSIQVARGQPAVLPCTFTTSAALIN[?]NVIWMVTPLSNANQPEQVILYQGGQ
MFDGAPRFHGRVGFTGTMPATNVSIFINNT[?]LSDTGTYQCLVNNLPDIGGRNIGVTGLTV
LVPPSAPHCQIQGSQDIGSDVILLCSSEEGIPRPTYLWEKLDNTLKLPPTATQDQVQGTV
TIRNISALSSGLYQCVASNAIGTSTCLLDLQVISPQPRNIGL
   Human VSIG4 - ECD without predicted signal sequence (SEQ ID NO:44) 
RPILEVPESVTGPWKGDVNLPCTYDPLQGYTQVLVKWLVQRGSDPVTIFLRDSSGDHIQQ
AKYQGRLHVSHKVPGDVSLQLSTLEMDDRSHYTCEVTWQTPDGNQVVRDKITELRVQKLS
VSKPTVTTGSGYGFTVPQGMRISLQCQARGSPPISYIWYKQQTNNQEPIKVATLSTLLFK
PAVIADSGSYFCTAKGQVGSEQHSDIVKFVVKDSSKLLKTKTEAPTTMTYPLKATSTVKQ
SWDWTTDMDGYLGETSAGPGKSLP
   Human VSIG8 - ECD without predicted signal sequence (SEQ ID NO:45) 
VRINGDGQEVLYLAEGDNVRLGCPYVLDPEDYGPNGLDIEWMQVNSDPAHHRENVFLSYQ
DKRINHGSLPHLQQRVRFAASDPSQYDASINLMNLQVSDTATYECRVKKTTMATRKVIVT
VQARPAVPMCWTEGHMTYGNDVVLKCYASGGSQPLSYKWAKISGHHYPYRAGSYTSQHSY
HSELSYQESFHSSINQGLNNGDLVLKDISRADDGLYQCTVANNVGYSVCVVEVKVSDSRR
IG
CADM Family
   Human CADM1 - ECD without predicted signal sequence (SEQ 10 NO:46) 
QNLFTKDVTVIEGEVATISCQVNKSDDSVIQLLNPNRQTIYFRDFRPLKDSRFQLLNFSS
SELKVSLTNVSISDEGRYFCQLYTDPPQESYTTITVLVPPRNLMIDIQKDTAVEGEEIEV
NCTAMASKPATTIRWFKGNTELKGKSEVEEWSDMYTVTSQLMLKVHKEDDGVPVICQVEH
PAVTGNLQTQRYLEVQYKPQVHIQMTYPLQGLTREGDALELTCEAIGKPQPVMVTWVRVD
DEMPQHAVLSGPNLFINNLNKTDNGTYRCEASNIVGKAHSDYMLYVYDPPTTIPPPTTTT
 TTTTTTTTTILTIITDSRAGEEGSIRAVDH
   Human CADM2 - ECD without predicted signal sequence (SEQ ID NO:47) 
QFPLTQNVTVVEGGTAILTCRVDQNDNTSLQWSNPAQQTLYFDDKKALRDNRIELVRASW
HELSISVSDVSLSDEGQYTCSLFTMPVKTSKAYLTVLGVPEKPQISGFSSPVMEGDLMQL
TCKTSGSKPAADIRWFKNDKEIKDVKYLKEEDANRKTFTVSSTLDFRVDRSDDGVAVICR
VDHESLNATPQVAMQVLEIHYTPSVKIIPSTPFPQEGQPLILTCESKGKPLPEPVLWTKD
GGELPDPDRMVVSGRELNILFLNKTDNGTYRCEATNTIGQSSAEYVLIVHDVPNTLLPTT
IIPSLTTATVTTTVAITTSPTTSATTSSIRDPNALAGQNGPDH
   Human CADM3 - ECD without predicted signal sequence (SEQ ID NO:48) 
NLSQDGYWQEQDLELGTLAPLDEAISSTVWSSPDMLASQDSQPWTSDETVVAGGTVVLKC
QVKDHEDSSLQWSNPAQQTLYFGEKRALRDNRIQLVTSTPHELSISISNVALADEGEYTC
SIFTMPVRTAKSLVTVLGIPQKPIITGYKSSLREKDTATLNCQSSGSKPAARLTWRKGDQ
ELHGEPTRIQEDPNGKTFTVSSSVTFQVTREDDGASIVCSVNHESLKGADRSTSQ[?]IEVL
YTPTAMIRPDPPHPREGQKLLLHCEGRGNPVPQQYLWEKEGSVPPLKMTQESALIFPFLN
KSDSGT
   Human CADM4 - ECD without predicted signal sequence (SEQ ID NO:49) 
QEVQTENVTVAEGGVAEITCRLHQYDGSIVVIQNPARQTLFFNGTRALKDERFQLEEFSP
RRVRIRLSDARLEDEGGYFCQLYTEDTHHQIATLTVLVAPENPVVEVREQAVEGGEVELS
CLVPRSRPAATLRWYRDRKELKGVSSSQENGKVWSVASTVRFRVDRKDDGG[?]IICEAQNQ
ALPSGHSKQTQYVLDVQYSPTARIHASQAVVREGDTLVLTCAVTGNPRPNQIRWNRGNES
LPERAEAVGETLTLPGLVSADNGTYTCEASNKHGHARALYVLVVYDPGAVVEAQTSVPYA
   Human CRTAM - ECD without predicted signal sequence (SEQ ID NO:50) 
SLTNHTETITVEEGQTLTLKCVTSLRKNSSLQWLTPSGFTIFLNEYPALKNSKYQLLHHS
ANQLSITVPNVTLQDEGVYKCLHYSDSVSTKEVKVIVLATPFKPILEASVIRKQNGEEHV
VLMCSTMRSKPPPQITWLLGNSMEVSGGT[?]HEFETDGKKCNTTSTLIIHTYGKNSTVDCI
IRHRGLQGRKLVAPFRFEDLVTDEETASDALERNSLSSQDPQQPTSTVSVTEDSSTSEID
KEEKEQTTQD[?]DLTT[?]ANPQYLGLARKKSG
   Human TMIGD1 - ECD without predicted signal sequence (SEQ ID NO:51) 
VLTVNGKTENYILDTTPGSQASLICAVQNHTREEELLWYREEGRVDLKSGNKINSSSVCV
SSISENDNGISFTCRLGRDQSVSVSVVLNVTFPPLLSGNDFQTVEEGSNVKLVCNVKANP
QAQMMWYKNSSLLDLEKSRHQIQQTSESFQLSITKVEKPDNGTYSC[?]AKSSLKTESLDFH
LIVKDKTVGVP
PVR Family
   Human CD96 - ECD without predicted signal sequence (SEQ ID NO:52) 
KTVNTEENVYATLGSDVNLTCQTQTVGFFVQMQWSKVTNKIDLIAVYHPQYGFYCAYGRP
CESLVTFTETPENGSKWTLHLRNMSCSVSGRYECMLVLYPEGIQTKIYNLLIQTHVTADE
WNSNHTIEIEINQTLEIPCFQNSSSKISSEFTYAWSVENSSTDSWVLLSKGIKEDNGTQE
TLISQNHLISNSTLLKDRVKLGTDYRLHLSPVQIFDDGRKFSCHIRVGPNKILRSSTTVK
VFAKPEIPVIVENNSTDVLVERRFTCLLKNVFPKANITWFIDGSFLHDEKEGIYITNEER
KGKDGFLELKSVLTRVHSNKPAQSDNLTIWCMALSPVPGNKVWNISSEKITFLLGSEISS
TDPPLSVTESTLDTQPSPASSVSPARYPATSSVTLVDVSALRPNTTPQPSNSSMTTRGFN
YPWTSSGTDTKKSVSRIPSETYSSSPSGAGSTLHDNVFTSTARAFSEVPTTANGSTKTNH
VHITGIVVNKPKDGMS
   Human CD200 - ECD without predicted signal sequence (SEQ ID NO:53) 
QVQVVTQDEREQLYTPASLKCSLQNAQEALIVTWQKKKAVSPENMVTFSENHGVVIQPAY
KDKINITQLGLQNSTITFWNITLEDEGCYMCLFNTFGFGKISGTACLTVYVQPIVSLHYK
FSEDHLNITCSATARPAPMVFWKVPRSGIENSTVTLSHPNGTTSVTSILHIKDPKNQVGK
 EVICQVLHLGTVTDFKQTVNKG
   Human CD200R1 - ECD without predicted signal sequence (SEQ ID NO:54) 
EGAAQPNNSLMLQTSKENHALASSSLCMDEKQITQNYSKVLAEVNTSWPVKMATNAVLCC
PPIALRNLIIIITWEIILRGQPSCTKAYKKETNETKETNCTDERITWVSRPDQNSDLQIRT
VAITHDGYYRCIMVTPDGNFHRGYHLQVLV[?]PEVTLFQNRNRTAVCKAVAGKPAAHISWI
PEGDCATKQEYWSNGTVTVKSTCHWEVHNVSTVTCHVSH
   Human CD200R1L - ECD without predicted signal sequence (SEQ ID NO:55) 
SCMGGKQMTQNYSTIFAEGNISQPVLMDINAVLCCPPIALRNLIIITWEIILRGQPSCTK
AYKKETNETKETNCTVERITWVSRPDQNSDLQIRPVDTTHDGYYRGIVVTPDGNFHRGYH
LQVLVTPEVNLFQSRNITAVCKAVTGKPAAQISWIPEGSILATKQEYWGNGTVTVKSTCP
WEGHKSTVTCHVSHLTGNKSLSVKLNSGLRTSGSPALSLL
 Human CD226 - ECD without predicted signal sequence (SEQ ID NO:56) 




   Human PVRIG - ECD without predicted signal sequence (SEQ ID NO:57) 
TPEVWVQVRMEATELSSFTIRCGFLGSGSISLVTVSWGGPNGAGGTTLAVLHPERGIRQW
APARQARWETQSSISLILEGSGASSPCANTTFCCKFASFPEGSWEACGSLPPSSDPGLSA
PPTPAPILRADL
   Human PVR - ECD without predicted signal sequence (SEQ ID NO:58) 
DVVVQAPTQVPGFLGDSVTLPCYLQVPNMEVTHVSQLTWARHGESGSMAVFHQTQGPSYS
ESKRLEFVAARLGAELRNASLRMFGLRVEDEGNYTCLFVTFPQGSRSVDIWLRVLAKPQN
TAEVQKVQLTGEPVPMARCVSTGGRPPAQITWHSDLGGMPNTSQVPGFLSGTVTVTSLWI
LVPSSQVDGK[?]VTCKVEHESFEKPQLLTVNLTVYYPPEVSISGYDNNWYLGQNEATLTCD
ARSNPEPTGYNWSTTMGPLPPFAVAQGAQLLIRPVDKPINTTLICNVTNALGARQAELTV
QVKEGPPSEHSGMSRN
   Human PVRL1 - ECD without predicted signal sequence (SEQ ID NO:59) 
QVVQVNDSMYGFIGTDVVLHCSFANPLPSVKITQVTWQKSTNGSKQNVAIYNPSMGVSVL
APYRERVEFLRPSFTDGTIRLSRLELEDEGVYICEFATFPTGNRESQLNLTVMAKPTNWI
EGTQAVLRAKKGQDDKVLVATCTSANGKPPSVVSWETRLKGEAEYQEIRNPNGTVTVISR
YRLVPSREAHQQSLACIVNYHMDRFKESLTLNVQYEPEVTIEGFDGNWYLQRMDVKLTCK
ADANPPATEYHWTTLNGSLPKGVEAQNRTLFFKGPINYSLAGTYICEATNPIGTRSGQVE
VNITEFPYTPSPPEHGRRAGPVPTA
   Human PVRL2 - ECD without predicted signal sequence (SEQ ID NO:60) 
QDVRVQVLPEVRGQLGGTV[?]LPCHLLPPVPGLYISLVTWQRPDAPANHQNVAAFH[?]KMGP
SFPSPKPGSERLSFVSAKQSTGQDTEAELQDATLALHGLTVEDEGNYTCEFATFPKGSVR
GMTWLRVIAKPKNQAEAQKVTFSQDPTTVALCISKEGRPPARISWLSSLDWEAKETQVSG
TLAGTVTVTSRFTLVPSGRADGVTVTCKVEHESFEEPALIPVTLSVRYPPEVSISGYDDN
WYLGRTDATLSCDVRSNPEPTGYDWSTTSGTFPTSAVAQGSQLVIHAVDSLFNTTFVCTV
TNAVGMGRAEQV[?]FVRETPNTAGAGATGG
   Human PVRL3 - ECD without predicted signal sequence (SEQ ID NO:61) 
GP[?][?]VEPHVTAVWGKNVSLKCLIEVNETITQISWEKIHGKSSQTVAVHHPQYGFSVQGEY
QGRVLFKNYSLNDATITLHNIGFSDSGKYICKAVTFPLGNAQSSTTVTVLVEPTVSLIKG
PDSLIDGGNETVAAICIAATGKPVAHIDWEGDLGEMESTTTSFPNETATIISQYKLFPTR
FARGRRITCVVKHPALEKDIRYSFILD[?]QYAPEVSVTGYDGNWFVGRKGVNLKCNADANP
 
PPFKSVWSRLDGQWPDGLLASDNTLHFVHPLTFNYSGVYICKVTNSLGQRSDQKVIYISD
PPTTTTLQPTIQWHPSTADIEDLATEPKKLPFPLSTLATIKDDTIAT
   Human PVRL4 - ECD without predicted signal sequence (SEQ ID NO:62) 
GELETSDVVTVVLGQDAKLPCFYRGDSGEQVGQVAWARVDAGEGAQELALLHSKYGLHVS
PAYEGRVEQPPPPRNPLDGSVLLRNAVQADEGEYECRVSTFPAGSFQARLRLRVLVPPLP
SLNPGPALEEGQGLTLAASCTAEGSPAPSVTWDTEVKGTTSSRSFKHSRSAAVTSEFHLV
PSRSMNGQPLTCVVSHPGLLQDQRITHILHVSFLAEASVRGLEDQNLWHIGREGAMLKCL
SEGQPPPSYNWTRLDGPLPSGVRVDGDTLGFPPLTTEHSGIYVCHVSNEFSSRDSQVTVD
VLDPQEDSGKQVDLVSAS
   Human TIGIT - ECD without predicted signal sequence (SEQ ID NO:63) 
MMTGTIETTGNISAEKGGSIILQCHLSSTTAQVTQVNWEQQDQLLAICNADLGWHISPSF
KDRVAPGPGLGLTLQSLTVNDTGEYFCIYHTYPDGTYTGRIFLEVLESSVAEHGARFQIP
LLGAMAATLVVICTAVIVVVA
   TMEM25 - ECD without predicted signal sequence (SEQ ID NO:64) 
ELEPQIDGQTWAERALRENERHAFTCRVAGGPGTPRLAWYLDGQLQEASTSRLLSVGGEA
FSGGTSTFTVTAHRAQHELNCSLQDPRSGRSANASVILNVQFKPEIAQVGAKYQEAQGPG
LLVVLFALVRANPPANVTWIDQDGPVTVNTSDFLVLDAQNYPWLTNHTVQLQLRSLAHNL
SVVATNDVGVTSASLPAPGLLATRVE
   Human IgG₁ Fc region (SEQ ID NO:65) 
DKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK
   Human IgG₁ Fc region (SEQ ID NO:66) 
KSSDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSD[?]AVEWESNGQPENNYKTTPPV
LDSDGSFFLYSKLTVDKSRWQQGNVFSCSVMHEALHNHYTQKSLSLSPGK
   Human IgG₁ Fc region (SEQ ID NO:67) 
EPKSSDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPGK
   dnYAP (SEQ ID NO:68) 
MDPGQQPPPQPAPQGQGQPPSQPPQGQGPPSGPGQPAPAATQAAPQAPPAGHQIVHVRGD
S[?]T[?]LEALFNAVMNPKTANVPQTVPMRLRKLPDSFFKPPEPKSHSRQASTDAGTAGALTP
QHVRAHASPASLQLGAVSPGTLTPTGVVSGPAATPTAQHLRQSSFEIPDDVPLPAGWEMA
KTSSGQRYFLNHIDQTTTWQDPRKAMLSQMNVTAPTSPPVQQNMMNSASGPLPDGWEQAM
TQDGEIYYINHKNKTTSWLDPRLDPRFAMNQRISQSAPVKQPPPLAPQSPQGGVMGGSNL
NDMESVLAATKLDKESFLTWL
   YFP (SEQ ID NO:69) 
MVSKGEELFTGVVPILVELDGDVNGHKFSVSGEGEGDATYGKLTLKFICTTGKLPVPWPT
LVTTFGYGLQCFARYPDHMKQHDFFKSAMPEGYVQERTIFFKDDGNYKTRAEVKFEGDTL
VNRIELKGIDFKEDGNILGHKLEYNYNSHNVYIMADKQKNGIKVNFKIRHNIEDGSVQLA
DHYQQNTPIGDGPVLLPDNHYLSYQSALSKDPNEKRDHMVLLEFVTAAGITLGMDELYK
   Human TEAD2 (SEQ ID NO:70) 
MGEPRAGAALDDGSGWTGSEEGSEEGTGGSEGAGGDGGPDAEGVWSPDIEQSFQEALAIY
PPCGRRKIILSDEGKMYGRNELIARYIKLRTGKTRTRKQVSSHIQVLARRKSREIQSKLK
DQVSKDKAFQTMA[?]MSSAQLISAPSLQAKLGPTGPQASELFQFWSGGSGPPWNVPDVKPF
SQTPFTLSLTPPSTDLPGYEPPQALSPLPPPTPSPPAWQARGLGTARLQLVEFSAFVEPP
DAVDSYQRHLFVHISQHCPSPGAPPLESVDVRQIYDKFPEKKGGLRELYD[?]GPPHAFFLV
KFWADLNWGPSGEEAGAGGSISSGGFYGVSSQYESLEHMTLTCSSKVCSFGKQVVEKVET
ERAQLEDGRFVYRLLRSPMCEYLVNFLHKLRQLPERYMMNSVLENFTILQVVTNRDTQEL
LLCTAYVFEVSTSERGAQHHIYRLVRD
   Human TEAD3 (SEQ ID NO:71) 
MASNSWNASSSPGEAREDGPEGLDKGLDNDAEGVWSPDIEQSFQEALAIYPPCGRRKIIL
SDEGKMYGRNELIARYIKLRTGKTRTRKQVSSHIQVLARKKVREYQVGIKAMNLDQVSKD
KALQSMASMSSAQIVSASVLQNKFSPPSPLPQAVFSTSSRFWSSPPLLGQQPGPSQDIKP
FAQPAYPIQPPLPPTLSSYEPLAPLPSAAASVPVWQDRTIASSRLRLLEYSAFMEVQRDP
DTYSKHLFVHIGQTNPAFSDPPLEAVDVRQIYDKFPEKKGGLKELYEKGPPNAFFLVKFW
ADLNSTIQEGPGAFYGVSSQYSSADSMTISVSTKVCSFGKQVVEKVETEYARLENGRFVY
RIHRSPMCEYMINFIHKLKHLPEKYMMNSVLENFTILQVVTSRDSQETLLVIAFVFEVST
SEHGAQHHVYKLVKD
   Linker (SEQ ID NO:72)
      RPACKIPNDLKQKVMNH
   FLAG Tag (SEQ ID NO:73)
      DYKDDDDK
   Linker (SEQ ID NO:74)
      ESGGGGVT
   Linker (SEQ ID NO:75)
      LESGGGGVT
   Linker (SEQ ID NO:76)
      GRAQVT
   Linker (SEQ ID NO:77)
      WRAQVT
   Linker (SEQ ID NO:78)
      ARGRAQVT
   Human IgG2 Heavy chain constant region (SEQ ID NO:79)

ASTKGPSVFP[?]APCSRSTSESTAALGCLVKDYFPEPVTVSWNSGALTSGVHTFPAVLQSS
GLYSLSSVVTVPSSNFGTQTYTCNVDHKPSNTKVDKTVERKCCVECPPCPAPPVAGPSVF
LFPPKPKDTLMISRTPEVTCVVVDVSHEDPEVQFNWYVDGVEVHNAKTKPREEQFNSTFR
VVSVLTVVHQDWLNGKEYKCKVSNKGLPAPIEKTISKTKGQPREPQVYTLPPSREEMTKN
QVSLTCLVKGFYPSDIAVEWESNGQPENNYKTT[?]PMLDSDGSFFLYSKLTVDKSRWQQGN
VFSCSVMHEALHNHYTQKSLSLSPGK

The following numbered clauses, describing aspects of the invention, are part of the description.
1. An agent that specifically binds the extracellular domain of a cell adhesion molecule of the immunoglobulin superfamily (IgCAM), wherein the agent modulates the Hippo pathway.
2. The agent of clause 1, wherein the IgCAM is selected from the JAM family, the PVR family, or the CADM family.
3. The agent of clause 1 or clause 2, wherein the IgCAM is selected from the group consisting of AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25.
4. The agent of any one of clauses 1-3, which specifically binds more than one IgCAM.
5. The agent of any one of clauses 1-4, which is an antibody.
6. The agent of clause 5, wherein the antibody is a monoclonal antibody, a recombinant antibody, a chimeric antibody, a humanized antibody, a human antibody, or a antibody fragment comprising an antigen-binding site.
7. The agent of clause 5, wherein the antibody is a bispecific antibody.
8. The agent of clause 5, wherein the antibody is an IgG1 antibody or an IgG2 antibody.
9. The agent of any one of clauses 1-8, wherein the agent is conjugated to a cytotoxin.
10. The agent of any one of clauses 1-4, which is a soluble receptor.
11. The agent of clause 10, wherein the soluble receptor comprises the extracellular domain of an IgCAM or a fragment thereof.
12. The agent of clause 10, wherein the soluble receptor comprises a sequence selected from the group consisting of: SEQ ID NOs:33-64.
13. The agent of clause 10, wherein the soluble receptor comprises a fragment of a sequence selected from the group consisting of: SEQ ID NOs:33-64.
14. The agent of any one of clauses 11-13, wherein the extracellular domain is linked to a Fc region.
15. The agent of clause 14, wherein the Fc region is a human Fc region.
16. The agent of clause 14, wherein the Fc region comprises SEQ ID NO:65, SEQ ID NO:66, or SEQ ID NO:67.
17. The agent of any one of clauses 11-16, wherein the extracellular domain is directly linked to a Fc region.
18. The agent of any one of clauses 11-16, wherein the extracellular domain is linked to a Fc region by a linker.
19. The agent of clause 18, wherein the linker component is an intervening peptide linker.
20. The agent of any one of clauses 1-19, which activates or increases Hippo pathway signaling.
21. The agent of any one of clauses 1-20, wherein the modulation of the Hippo pathway comprises a reduction in YAP activity.
22. The agent of any one of clauses 1-20, wherein the modulation of the Hippo pathway comprises an increase in phosphorylated YAP.
23. The agent of any one of clauses 1-20, wherein the modulation of the Hippo pathway comprises a decrease in the expression of YAP-dependent genes.
24. The agent of any one of clauses 1-20, wherein the modulation of the Hippo pathway comprises an decrease in the expression of at least one of the genes selected from the group consisting of: CD44, CD47, CD133, TDGF1, EPHB2, CA12, LRP4, GPC4, CLDN2, CTGF, PAG1, SEMA4D, RHEb, MAGI1, ITPR3, CD168, NRP2, Gli2, Birc2, Birc5, FGF1, IL33, GRB2, IGFBP3, and AREG.
25. The agent of any one of clauses 1-24, which modulates apoptosis.
26. The agent of clause 25, wherein apoptosis is increased.
27. The agent of any one of clauses 1-24, which modulates cellular proliferation.
28. The agent of clause 27, wherein cellular proliferation is inhibited.
29. The agent of any one of clauses 1-24, which modulates epithelial mesenchymal transition (EMT).
30. The agent of clause 29, wherein EMT is inhibited.
31. The agent of any one of clauses 1-24, which inhibits the growth of a tumor or tumor cells.
32. The agent of clause 31, wherein the tumor is a tumor selected from the group consisting of colorectal tumor, colon tumor, pancreatic tumor, lung tumor, ovarian tumor, liver tumor, breast tumor, kidney tumor, prostate tumor, melanoma, cervical tumor, bladder tumor, brain tumor, gastrointestinal tumor, and head and neck tumor.
33. A polypeptide comprising: (a) an amino acid sequence selected from the group consisting of: SEQ ID NOs:33-64, and (b) an immunoglobulin Fc region.
34. A polypeptide comprising: (a) a fragment of an amino acid sequence selected from the group consisting of: SEQ ID NOs:33-64, and an immunoglobulin Fc region.
35. The polypeptide of clause 33 or clause 34, wherein the Fc region is a human Fc region.
36. The polypeptide of clause 34 or clause 35, wherein the Fc region comprises SEQ ID NO:65, SEQ ID NO:66, or SEQ ID NO:67.
37. The polypeptide of any one of clauses 33-36, wherein the amino acid sequence is directly linked to the Fc region.
38. The polypeptide of any one of clauses 33-36, wherein the amino acid sequence is linked to the Fc region by a linker.
39. The polypeptide of clause38, wherein the linker component is an intervening peptide linker.
40. The polypeptide of any one of clauses 33-39, which modulates the Hippo pathway.
41. The polypeptide of any one of clauses 33-39, which inhibits the Hippo pathway.
42. The polypeptide of any one of clauses 33-39, which activates the Hippo pathway.
43. A cell producing the agent or polypeptide of any one of clauses 1-42.
44. A composition comprising the agent or polypeptide of any one of clauses 1-42.
45. A pharmaceutical composition comprising the agent or polypeptide of any one of clauses 1-42 and a pharmaceutically acceptable carrier.
46. A polynucleotide comprising a polynucleotide that encodes the agent or polypeptide of any one of clauses 1-42.
47. A vector comprising the polynucleotide of clause 46.
48. An isolated cell comprising the polynucleotide of clause 46 or the vector of clause 47.
49. A method of modulating the Hippo pathway in a cell, comprising contacting the cell with an agent or polypeptide of any one of clauses 1-42.
50. A method of inhibiting tumor growth comprising contacting a tumor or tumor cell with an agent or polypeptide of any one of clauses 1-42.
51. The method of clause 50, wherein the tumor is a tumor selected from the group consisting of colorectal tumor, colon tumor, pancreatic tumor, lung tumor, ovarian tumor, liver tumor, breast tumor, kidney tumor, prostate tumor, melanoma, cervical tumor, bladder tumor, brain tumor, gastrointestinal tumor, and head and neck tumor.
52. A method of treating cancer in a subject, comprising administering a therapeutically effective amount of an agent or polypeptide of any one of clauses 1-42 to the subject.
53. The method of clause 52, wherein the cancer is selected from the group consisting of: colorectal cancer, colon cancer, pancreatic cancer, lung cancer, ovarian cancer, liver cancer, breast cancer, kidney cancer, prostate cancer, melanoma, cervical cancer, bladder cancer, brain cancer, gastrointestinal cancer, and head and neck cancer.
54. The method of clause 52 or clause 53, which further comprises administering a therapeutically effective amount of a second therapeutic agent to the subject.
55. The method of clause 54, wherein the second therapeutic agent is a chemotherapeutic agent.
56. The method of clause 54, wherein the second therapeutic agent is an antibody.
57. The method of clause 56, wherein the antibody is an antibody that targets a cancer stem cell pathway.
58. A method of modulating the Hippo pathway comprising contacting a cell with an agent that specifically binds the extracellular domain of an IgCAM.
59. A method of inhibiting tumor growth comprising contacting the tumor with an agent that specifically binds the extracellular domain of an IgCAM.
60. A method of treating cancer in a subject, comprising administering to the subject a therapeutically effective amount of an agent that specifically binds the extracellular domain of an IgCAM.
61. A method of targeting tumor cells in a subject, comprising administering to the subject a therapeutically effective amount of an agent that specifically binds the extracellular domain of an IgCAM.
62. The method of any one of clauses 58-61, wherein the IgCAM is selected from the JAM family, the PVR family, or the CADM family.
63. The method of any one of clauses 58-61, wherein the IgCAM is selected from the group consisting of AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25.
64. The method of any one of clauses 58-63, wherein the agent is an antibody.
65. The method of any one of clauses 58-63, wherein the agent is a soluble receptor.
66. The method of any one of clauses58-65, wherein the agent delivers a cytotoxin.
67. The method of any one of clauses 58-65, wherein the agent delivers an angiogenesis inhibitor.
68. The method of any one of clauses 58-65, wherein the agent delivers a molecule which modulates immune responses.
69. The method of any one of clauses 58 or 62-68, wherein the modulation comprises activating the Hippo pathway.
70. The method of any one of clauses 58-69, wherein YAP activity is inhibited.
71. The method of any one of clauses 58-69, wherein phosphorylated YAP is increased.
72. The method of any one of clauses 58-69, wherein non-phosphorylated YAP is decreased.
73. The agent of any one of clauses 1-32, wherein the IgCAM is selected from the group consisting of: CLMP, VSIG4, VSIG8, JAM2, JAM3, CADM1, CADM3, CADM4, PVRL1, PVRL3, PVRL4, TIGIT, TMIGD1, and TMEM25.
74. The agent of any one of clauses 1-32, wherein the IgCAM is selected from the group consisting of: CADM1, CADM3, PVRL1, and PVRL3.
75. The agent of any one of clauses 1-32, wherein the IgCAM is selected from the group consisting of: CADM3, PVRL1, and PVRL3.
76. The agent of any one of clauses 1-32, wherein the IgCAM is CADM3.
77. The agent of any one of clauses 1-32, which does not bind CADM1.
78. The method of any one of clauses 49-72, wherein the IgCAM is selected from the group consisting of: CLMP, VSIG4, VSIG8, JAM2, JAM3, CADM1, CADM3, CADM4, PVRL1, PVRL3, PVRL4, TIGIT, TMIGD1, and TMEM25.
79. The method of any one of clauses 49-72, wherein the IgCAM is selected from the group consisting of: CADM1, CADM3, PVRL1, and PVRL3.
80. The method of any one of clauses 49-72, wherein the IgCAM is selected from the group consisting of: CADM3, PVRL1, and PVRL3.
81. The method of any one of clauses 49-72, wherein the IgCAM is CADM3.
82. The method of any one of clauses 49-72, wherein the IgCAM does not bind CADM1.
83. A method of screening for an agent that modulates the Hippo pathway comprising: screening for an agent that specifically binds the extracellular domain of an IgCAM, wherein the agent modulates the Hippo pathway.
84. A method of screening for an agent that modulates the Hippo pathway comprising:
   (a) contacting cells with a test agent, and
   (b) determining the cellular localization of YAP and/or TAZ using a bimolecular fluorescence complementation (BiFC) assay.
85. A method of screening for an agent that modulates the Hippo pathway comprising:
   (a) contacting cells with a test agent that specifically binds the extracellular domain of an IgCAM, and
   (b) determining the cellular localization of YAP and/or TAZ using a bimolecular fluorescence complementation (BiFC) assay.
86. The method of clause 84 or clause 85, wherein if YAP and/or TAZ is predominantly localized in the cytoplasm of the cell, then the Hippo pathway is activated.
87. The method of clause 84 or clause 85, wherein if YAP and/or TAZ is predominantly localized in the nucleus of the cell, then the Hippo pathway is inhibited.
88. A method of screening for an agent that modulates the Hippo pathway comprising:
   (a) contacting a first set of cells, but not a second set of cells, with a test agent that specifically binds the extracellular domain of an IgCAM, and
   (b) determining the amount of phosphorylated YAP and/or phosphorylated TAZ in the first set of cells as compared to the second set of cells.
89. The method of clause 88, wherein if the amount of phosphorylated YAP and/or phosphorylated TAZ in the first set of cells has increased as compared to the second set of cells, then the agent has activated the Hippo pathway.
90. The method of clause 88, wherein if the amount of phosphorylated YAP and/or phosphorylated TAZ in the first set of cells has decreased as compared to the second set of cells, then the agent has inhibited the Hippo pathway.
91. A method of screening for an agent that modulates the Hippo pathway comprising:
   (a) contacting a first set of cells, but not a second set of cells, with a test agent that specifically binds the extracellular domain of an IgCAM, and
   (b) determining the amount of non-phosphorylated YAP and/or non-phosphorylated TAZ in the first set of cells as compared to the second set of cells.
92. The method of clause 91, wherein if the amount of non-phosphorylated YAP and/or non-phosphorylated TAZ in the first set of cells has increased as compared to the second set of cells, then the agent inhibited the Hippo pathway.
93. The method of clause 91, wherein if the amount of non-phosphorylated YAP and/or non-phosphorylated TAZ in the first set of cells has decreased as compared to the second set of cells, then the agent activated the Hippo pathway.
94. A method of screening for an agent that modulates the Hippo pathway comprising:
   (a) contacting a first set of cells, but not a second set of cells, with a test agent that specifically binds the extracellular domain of an IgCAM, and
   (b) determining the expression of at least one YAP-dependent gene and/or at least one TAZ-dependent gene in the first set of cells as compared to the expression of the YAP-dependent gene and/or the TAZ-dependent gene in the second set of cells.
95. The method of clause 94, wherein the at least one YAP-dependent gene or the at least one TAZ-dependent gene is selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, GLI2, BIRC2, BIRC5 (survivin), FGF1, IL33, GRB2, IGFBP3, AREG, FN1, ADAMTS1, AXL, ADAMTS5, MET, CYR61, IL8, ZEB1, FOXC2, N-cadherin, and SNAIL.
96. The method of clause 94 or clause 95, wherein if the expression of at least one YAP-dependent gene and/or at least one TAZ-dependent gene in the first set of cells has increased as compared to the expression of the YAP-dependent gene(s) and/or the TAZ-dependent gene(s) in the second set of cells, then the agent inhibited the Hippo pathway.
97. The method of clause 94 or clause 95, wherein if the expression of at least one YAP-dependent gene and/or at least one TAZ-dependent gene in the first set of cells has decreased as compared to the expression of the YAP-dependent gene(s) and/or the TAZ-dependent gene(s) in the second set of cells, then the agent activated the Hippo pathway.
98. The method of any one of clauses 83 or 85-97, wherein the IgCAM is selected from the group consisting of AMICA, CAR, CLMP, ESAM, GPA33, VSIG1, VSIG2, VSIG3, VSIG4, VSIG8, JAM1, JAM2, JAM3, CADM1, CADM2, CADM3, CADM4, CRTAM, TMIGD1, PVR, PVRL1, PVRL2, PVRL3, PVRL4, PVRIG, CD200, CD200R1, CD200R1L, CD226, CD96, TIGIT, and TMEM25.
99. The method of any one of clauses 83 or 85-97, wherein the IgCAM is selected from the group consisting of: CLMP, VSIG4, VSIG8, JAM2, JAM3, CADM1, CADM3, CADM4, PVRL1, PVRL3, PVRL4, TIGIT, TMIGD1, and TMEM25.
100. The method of any one of clauses 83 or 85-97, wherein the IgCAM is selected from the group consisting of: CADM1, CADM3, PVRL1 and PVRL3.
101. The method of any one of clauses 83 or 85-97, wherein the IgCAM is selected from the group consisting of: CADM3, PVRL1 and/or PVRL3.
102. The method of any one of clauses 83 or 85-97, wherein the IgCAM is CADM3.
103. The method of any one of clauses 83 or 85-97, wherein the IgCAM does not bind CADM1.
104. The method of any one of clauses 83-103, wherein the agent is an antibody, a soluble receptor, a small molecule, a polypeptide, a peptide, a peptidomimetic, or a drug compound.
105. The method of any one of clauses 83-104, wherein the modulation of the Hippo pathway comprises a reduction in YAP activity.
106. The method of any one of clauses 83-104, wherein the modulation of the Hippo pathway comprises an increase in phosphorylated YAP.
107. The method of any one of clauses 83-104, wherein the modulation of the Hippo pathway comprises a decrease in the expression of YAP-dependent genes.
108. The method of any one of clauses 83-104, wherein the modulation of the Hippo pathway comprises an decrease in the expression of at least one of the genes selected from the group consisting of: CD44, CD47, CD133, TDGF1, EPHB2, CA12, LRP4, GPC4, CLDN2, CTGF, PAG1, SEMA4D, RHEB, MAGI1, ITPR3, CD168, NRP2, Gli2, Birc2, Birc5, FGF1, IL33, GRB2, IGFBP3, and AREG.
109. An agent identified by the method of any one of clauses 83-108.

## Claims

1. An agent that specifically binds the extracellular domain of PVRIG for use in a method of treating cancer in a subject comprising administering to the subject an effective amount of the agent.

2. The agent for use according to claim 1, wherein the cancer is selected from the group consisting of colorectal cancer, pancreatic cancer, lung cancer, ovarian cancer, liver cancer, breast cancer, kidney cancer, prostate cancer, gastrointestinal cancer, melanoma, cervical cancer, bladder cancer, glioblastoma, and head and neck cancer.

3. The agent for use according to any of claims 1 to 2, wherein the agent is an antibody.

4. The agent for use according to claim 3, wherein the antibody is a monoclonal antibody.

5. The agent for use according to any of claims 1 to 4, wherein the PVRIG is human PVRIG.

6. An agent for use according to any of claims 1 to 5 , wherein the method further comprises administering a therapeutically effective amount of a second therapeutic agent to the subject.

7. An agent for use according to claim 6, wherein the second therapeutic agent is a chemotherapeutic agent or antibody.

8. An antibody that specifically binds PVRIG.

9. The antibody according to claim 8 that specifically binds to the extracellular domain of PVRIG.

10. The antibody according to claim 8 or 9, wherein the antibody is a monoclonal antibody.

11. The antibody according to any of claims 8 to 10, wherein the PVRIG is human PVRIG.

12. The agent for use according to claim 3 or claim 4 or the antibody according to claim 10 or 11, wherein the antibody is a monospecific antibody, a bispecific antibody, or an antibody fragment comprising an antigen binding site.

13. The agent for use according to claim 3 or claim 4 or the antibody according to claim 10 or claim 11, wherein the antibody is an IgG1 antibody or an IgG2 antibody.

14. The agent for use according to any one of claims 3, 4 and 11-13 or the antibody of any of claims 8 to 13, wherein the antibody is an antagonistic antibody.

15. A pharmaceutical composition comprising the antibody of any one of claims 8-14 and a pharmaceutically acceptable carrier.


SEQUENCE LISTING

<110>  ONCOMED PHARMACEUTICALS, INC.

<120>  BINDING AGENTS THAT MODULATE THE HIPPO PATHWAY AND USES THEREOF

<130>  NRS/FP7245251

<140>  EP
<141>  2013-06-06

<150>  EP 13801023.6
<151>  2013-06-06

<140>  PCT/US2013/044503
<141>  2013-06-06

<150>  US 61/783,190
<151>  2013-03-14

<150>  US 61/737,390
<151>  2012-12-14

<150>  US 61/656,249
<151>  2012-06-06

<160>  79

<170>  PatentIn version 3.5

<210>  1
<211>  394
<212>  PRT
<213>  Homo sapiens

<400>  1

Met Phe Cys Pro Leu Lys Leu Ile Leu Leu Pro Val Leu Leu Asp Tyr
1               5                   10                  15

Ser Leu Gly Leu Asn Asp Leu Asn Val Ser Pro Pro Glu Leu Thr Val
            20                  25                  30

His Val Gly Asp Ser Ala Leu Met Gly Cys Val Phe Gln Ser Thr Glu
        35                  40                  45

Asp Lys Cys Ile Phe Lys Ile Asp Trp Thr Leu Ser Pro Gly Glu His
    50                  55                  60

Ala Lys Asp Glu Tyr Val Leu Tyr Tyr Tyr Ser Asn Leu Ser Val Pro
65                  70                  75                  80

Ile Gly Arg Phe Gln Asn Arg Val His Leu Met Gly Asp Ile Leu Cys
                85                  90                  95

Asn Asp Gly Ser Leu Leu Leu Gln Asp Val Gln Glu Ala Asp Gln Gly
            100                 105                 110

Thr Tyr Ile Cys Glu Ile Arg Leu Lys Gly Glu Ser Gln Val Phe Lys
        115                 120                 125

Lys Ala Val Val Leu His Val Leu Pro Glu Glu Pro Lys Glu Leu Met
    130                 135                 140

Val His Val Gly Gly Leu Ile Gln Met Gly Cys Val Phe Gln Ser Thr
145                 150                 155                 160

Glu Val Lys His Val Thr Lys Val Glu Trp Ile Phe Ser Gly Arg Arg
                165                 170                 175

Ala Lys Glu Glu Ile Val Phe Arg Tyr Tyr His Lys Leu Arg Met Ser
            180                 185                 190

Val Glu Tyr Ser Gln Ser Trp Gly His Phe Gln Asn Arg Val Asn Leu
        195                 200                 205

Val Gly Asp Ile Phe Arg Asn Asp Gly Ser Ile Met Leu Gln Gly Val
    210                 215                 220

Arg Glu Ser Asp Gly Gly Asn Tyr Thr Cys Ser Ile His Leu Gly Asn
225                 230                 235                 240

Leu Val Phe Lys Lys Thr Ile Val Leu His Val Ser Pro Glu Glu Pro
                245                 250                 255

Arg Thr Leu Val Thr Pro Ala Ala Leu Arg Pro Leu Val Leu Gly Gly
            260                 265                 270

Asn Gln Leu Val Ile Ile Val Gly Ile Val Cys Ala Thr Ile Leu Leu
        275                 280                 285

Leu Pro Val Leu Ile Leu Ile Val Lys Lys Thr Cys Gly Asn Lys Ser
    290                 295                 300

Ser Val Asn Ser Thr Val Leu Val Lys Asn Thr Lys Lys Thr Asn Pro
305                 310                 315                 320

Glu Ile Lys Glu Lys Pro Cys His Phe Glu Arg Cys Glu Gly Glu Lys
                325                 330                 335

His Ile Tyr Ser Pro Ile Ile Val Arg Glu Val Ile Glu Glu Glu Glu
            340                 345                 350

Pro Ser Glu Lys Ser Glu Ala Thr Tyr Met Thr Met His Pro Val Trp

355                 360                 365

Pro Ser Leu Arg Ser Asp Arg Asn Asn Ser Leu Glu Lys Lys Ser Gly
    370                 375                 380

Gly Gly Met Pro Lys Thr Gln Gln Ala Phe
385                 390

<210>  2
<211>  344
<212>  PRT
<213>  Homo sapiens

<400>  2

Met Ala Leu Leu Leu Cys Phe Val Leu Leu Cys Gly Val Val Asp Phe
1               5                   10                  15

Ala Arg Ser Leu Ser Ile Thr Thr Pro Glu Glu Met Ile Glu Lys Ala
            20                  25                  30

Lys Gly Glu Thr Ala Tyr Leu Pro Cys Lys Phe Thr Leu Ser Pro Glu
        35                  40                  45

Asp Gln Gly Pro Leu Asp Ile Glu Trp Leu Ile Ser Pro Ala Asp Asn
    50                  55                  60

Gln Lys Val Asp Gln Val Ile Ile Leu Tyr Ser Gly Asp Lys Ile Tyr
65                  70                  75                  80

Asp Asp Tyr Tyr Pro Asp Leu Lys Gly Arg Val His Phe Thr Ser Asn
                85                  90                  95

Asp Leu Lys Ser Gly Asp Ala Ser Ile Asn Val Thr Asn Leu Gln Leu
            100                 105                 110

Ser Asp Ile Gly Thr Tyr Gln Cys Lys Val Lys Lys Ala Pro Gly Val
        115                 120                 125

Ala Asn Lys Lys Ile His Leu Val Val Leu Val Lys Pro Ser Gly Ala
    130                 135                 140

Arg Cys Tyr Val Asp Gly Ser Glu Glu Ile Gly Ser Asp Phe Lys Ile
145                 150                 155                 160

Lys Cys Glu Pro Lys Glu Gly Ser Leu Pro Leu Gln Tyr Glu Trp Gln
                165                 170                 175

Lys Leu Ser Asp Ser Gln Lys Met Pro Thr Ser Trp Leu Ala Glu Met

180                 185                 190

Thr Ser Ser Val Ile Ser Val Lys Asn Ala Ser Ser Glu Tyr Ser Gly
        195                 200                 205

Thr Tyr Ser Cys Thr Val Arg Asn Arg Val Gly Ser Asp Gln Cys Leu
    210                 215                 220

Leu Arg Leu Asn Val Val Pro Pro Ser Asn Lys Ala Gly Leu Ile Ala
225                 230                 235                 240

Gly Ala Ile Ile Gly Thr Leu Leu Ala Leu Ala Leu Ile Gly Leu Ile
                245                 250                 255

Ile Phe Cys Cys Arg Lys Lys Arg Arg Glu Glu Lys Tyr Glu Lys Glu
            260                 265                 270

Val His His Asp Ile Arg Glu Asp Val Pro Pro Pro Lys Ser Arg Thr
        275                 280                 285

Ser Thr Ala Arg Ser Tyr Ile Gly Ser Asn His Ser Ser Leu Gly Ser
    290                 295                 300

Met Ser Pro Ser Asn Met Glu Gly Tyr Ser Lys Thr Gln Tyr Asn Gln
305                 310                 315                 320

Val Pro Ser Glu Asp Phe Glu Arg Thr Pro Gln Ser Pro Thr Leu Pro
                325                 330                 335

Pro Ala Lys Phe Lys Tyr Ala Tyr
            340

<210>  3
<211>  373
<212>  PRT
<213>  Homo sapiens

<400>  3

Met Ser Leu Leu Leu Leu Leu Leu Leu Val Ser Tyr Tyr Val Gly Thr
1               5                   10                  15

Leu Gly Thr His Thr Glu Ile Lys Arg Val Ala Glu Glu Lys Val Thr
            20                  25                  30

Leu Pro Cys His His Gln Leu Gly Leu Pro Glu Lys Asp Thr Leu Asp
        35                  40                  45

Ile Glu Trp Leu Leu Thr Asp Asn Glu Gly Asn Gln Lys Val Val Ile

50                  55                  60

Thr Tyr Ser Ser Arg His Val Tyr Asn Asn Leu Thr Glu Glu Gln Lys
65                  70                  75                  80

Gly Arg Val Ala Phe Ala Ser Asn Phe Leu Ala Gly Asp Ala Ser Leu
                85                  90                  95

Gln Ile Glu Pro Leu Lys Pro Ser Asp Glu Gly Arg Tyr Thr Cys Lys
            100                 105                 110

Val Lys Asn Ser Gly Arg Tyr Val Trp Ser His Val Ile Leu Lys Val
        115                 120                 125

Leu Val Arg Pro Ser Lys Pro Lys Cys Glu Leu Glu Gly Glu Leu Thr
    130                 135                 140

Glu Gly Ser Asp Leu Thr Leu Gln Cys Glu Ser Ser Ser Gly Thr Glu
145                 150                 155                 160

Pro Ile Val Tyr Tyr Trp Gln Arg Ile Arg Glu Lys Glu Gly Glu Asp
                165                 170                 175

Glu Arg Leu Pro Pro Lys Ser Arg Ile Asp Tyr Asn His Pro Gly Arg
            180                 185                 190

Val Leu Leu Gln Asn Leu Thr Met Ser Tyr Ser Gly Leu Tyr Gln Cys
        195                 200                 205

Thr Ala Gly Asn Glu Ala Gly Lys Glu Ser Cys Val Val Arg Val Thr
    210                 215                 220

Val Gln Tyr Val Gln Ser Ile Gly Met Val Ala Gly Ala Val Thr Gly
225                 230                 235                 240

Ile Val Ala Gly Ala Leu Leu Ile Phe Leu Leu Val Trp Leu Leu Ile
                245                 250                 255

Arg Arg Lys Asp Lys Glu Arg Tyr Glu Glu Glu Glu Arg Pro Asn Glu
            260                 265                 270

Ile Arg Glu Asp Ala Glu Ala Pro Lys Ala Arg Leu Val Lys Pro Ser
        275                 280                 285

Ser Ser Ser Ser Gly Ser Arg Ser Ser Arg Ser Gly Ser Ser Ser Thr
    290                 295                 300

Arg Ser Thr Ala Asn Ser Ala Ser Arg Ser Gln Arg Thr Leu Ser Thr
305                 310                 315                 320

Asp Ala Ala Pro Gln Pro Gly Leu Ala Thr Gln Ala Tyr Ser Leu Val
                325                 330                 335

Gly Pro Glu Val Arg Gly Ser Glu Pro Lys Lys Val His His Ala Asn
            340                 345                 350

Leu Thr Lys Ala Glu Thr Thr Pro Ser Met Ile Pro Ser Gln Ser Arg
        355                 360                 365

Ala Phe Gln Thr Val
    370

<210>  4
<211>  390
<212>  PRT
<213>  Homo sapiens

<400>  4

Met Ile Ser Leu Pro Gly Pro Leu Val Thr Asn Leu Leu Arg Phe Leu
1               5                   10                  15

Phe Leu Gly Leu Ser Ala Leu Ala Pro Pro Ser Arg Ala Gln Leu Gln
            20                  25                  30

Leu His Leu Pro Ala Asn Arg Leu Gln Ala Val Glu Gly Gly Glu Val
        35                  40                  45

Val Leu Pro Ala Trp Tyr Thr Leu His Gly Glu Val Ser Ser Ser Gln
    50                  55                  60

Pro Trp Glu Val Pro Phe Val Met Trp Phe Phe Lys Gln Lys Glu Lys
65                  70                  75                  80

Glu Asp Gln Val Leu Ser Tyr Ile Asn Gly Val Thr Thr Ser Lys Pro
                85                  90                  95

Gly Val Ser Leu Val Tyr Ser Met Pro Ser Arg Asn Leu Ser Leu Arg
            100                 105                 110

Leu Glu Gly Leu Gln Glu Lys Asp Ser Gly Pro Tyr Ser Cys Ser Val
        115                 120                 125

Asn Val Gln Asp Lys Gln Gly Lys Ser Arg Gly His Ser Ile Lys Thr
    130                 135                 140

Leu Glu Leu Asn Val Leu Val Pro Pro Ala Pro Pro Ser Cys Arg Leu
145                 150                 155                 160

Gln Gly Val Pro His Val Gly Ala Asn Val Thr Leu Ser Cys Gln Ser
                165                 170                 175

Pro Arg Ser Lys Pro Ala Val Gln Tyr Gln Trp Asp Arg Gln Leu Pro
            180                 185                 190

Ser Phe Gln Thr Phe Phe Ala Pro Ala Leu Asp Val Ile Arg Gly Ser
        195                 200                 205

Leu Ser Leu Thr Asn Leu Ser Ser Ser Met Ala Gly Val Tyr Val Cys
    210                 215                 220

Lys Ala His Asn Glu Val Gly Thr Ala Gln Cys Asn Val Thr Leu Glu
225                 230                 235                 240

Val Ser Thr Gly Pro Gly Ala Ala Val Val Ala Gly Ala Val Val Gly
                245                 250                 255

Thr Leu Val Gly Leu Gly Leu Leu Ala Gly Leu Val Leu Leu Tyr His
            260                 265                 270

Arg Arg Gly Lys Ala Leu Glu Glu Pro Ala Asn Asp Ile Lys Glu Asp
        275                 280                 285

Ala Ile Ala Pro Arg Thr Leu Pro Trp Pro Lys Ser Ser Asp Thr Ile
    290                 295                 300

Ser Lys Asn Gly Thr Leu Ser Ser Val Thr Ser Ala Arg Ala Leu Arg
305                 310                 315                 320

Pro Pro His Gly Pro Pro Arg Pro Gly Ala Leu Thr Pro Thr Pro Ser
                325                 330                 335

Leu Ser Ser Gln Ala Leu Pro Ser Pro Arg Leu Pro Thr Thr Asp Gly
            340                 345                 350

Ala His Pro Gln Pro Ile Ser Pro Ile Pro Gly Gly Val Ser Ser Ser
        355                 360                 365

Gly Leu Ser Arg Met Gly Ala Val Pro Val Met Val Pro Ala Gln Ser
    370                 375                 380

Gln Ala Gly Ser Leu Val
385                 390

<210>  5
<211>  319
<212>  PRT
<213>  Homo sapiens

<400>  5

Met Val Gly Lys Met Trp Pro Val Leu Trp Thr Leu Cys Ala Val Arg
1               5                   10                  15

Val Thr Val Asp Ala Ile Ser Val Glu Thr Pro Gln Asp Val Leu Arg
            20                  25                  30

Ala Ser Gln Gly Lys Ser Val Thr Leu Pro Cys Thr Tyr His Thr Ser
        35                  40                  45

Thr Ser Ser Arg Glu Gly Leu Ile Gln Trp Asp Lys Leu Leu Leu Thr
    50                  55                  60

His Thr Glu Arg Val Val Ile Trp Pro Phe Ser Asn Lys Asn Tyr Ile
65                  70                  75                  80

His Gly Glu Leu Tyr Lys Asn Arg Val Ser Ile Ser Asn Asn Ala Glu
                85                  90                  95

Gln Ser Asp Ala Ser Ile Thr Ile Asp Gln Leu Thr Met Ala Asp Asn
            100                 105                 110

Gly Thr Tyr Glu Cys Ser Val Ser Leu Met Ser Asp Leu Glu Gly Asn
        115                 120                 125

Thr Lys Ser Arg Val Arg Leu Leu Val Leu Val Pro Pro Ser Lys Pro
    130                 135                 140

Glu Cys Gly Ile Glu Gly Glu Thr Ile Ile Gly Asn Asn Ile Gln Leu
145                 150                 155                 160

Thr Cys Gln Ser Lys Glu Gly Ser Pro Thr Pro Gln Tyr Ser Trp Lys
                165                 170                 175

Arg Tyr Asn Ile Leu Asn Gln Glu Gln Pro Leu Ala Gln Pro Ala Ser
            180                 185                 190

Gly Gln Pro Val Ser Leu Lys Asn Ile Ser Thr Asp Thr Ser Gly Tyr
        195                 200                 205

Tyr Ile Cys Thr Ser Ser Asn Glu Glu Gly Thr Gln Phe Cys Asn Ile
    210                 215                 220

Thr Val Ala Val Arg Ser Pro Ser Met Asn Val Ala Leu Tyr Val Gly
225                 230                 235                 240

Ile Ala Val Gly Val Val Ala Ala Leu Ile Ile Ile Gly Ile Ile Ile
                245                 250                 255

Tyr Cys Cys Cys Cys Arg Gly Lys Asp Asp Asn Thr Glu Asp Lys Glu
            260                 265                 270

Asp Ala Arg Pro Asn Arg Glu Ala Tyr Glu Glu Pro Pro Glu Gln Leu
        275                 280                 285

Arg Glu Leu Ser Arg Glu Arg Glu Glu Glu Asp Asp Tyr Arg Gln Glu
    290                 295                 300

Glu Gln Arg Ser Thr Gly Arg Glu Ser Pro Asp His Leu Asp Gln
305                 310                 315

<210>  6
<211>  299
<212>  PRT
<213>  Homo sapiens

<400>  6

Met Gly Thr Lys Ala Gln Val Glu Arg Lys Leu Leu Cys Leu Phe Ile
1               5                   10                  15

Leu Ala Ile Leu Leu Cys Ser Leu Ala Leu Gly Ser Val Thr Val His
            20                  25                  30

Ser Ser Glu Pro Glu Val Arg Ile Pro Glu Asn Asn Pro Val Lys Leu
        35                  40                  45

Ser Cys Ala Tyr Ser Gly Phe Ser Ser Pro Arg Val Glu Trp Lys Phe
    50                  55                  60

Asp Gln Gly Asp Thr Thr Arg Leu Val Cys Tyr Asn Asn Lys Ile Thr
65                  70                  75                  80

Ala Ser Tyr Glu Asp Arg Val Thr Phe Leu Pro Thr Gly Ile Thr Phe
                85                  90                  95

Lys Ser Val Thr Arg Glu Asp Thr Gly Thr Tyr Thr Cys Met Val Ser
            100                 105                 110

Glu Glu Gly Gly Asn Ser Tyr Gly Glu Val Lys Val Lys Leu Ile Val
        115                 120                 125

Leu Val Pro Pro Ser Lys Pro Thr Val Asn Ile Pro Ser Ser Ala Thr
    130                 135                 140

Ile Gly Asn Arg Ala Val Leu Thr Cys Ser Glu Gln Asp Gly Ser Pro
145                 150                 155                 160

Pro Ser Glu Tyr Thr Trp Phe Lys Asp Gly Ile Val Met Pro Thr Asn
                165                 170                 175

Pro Lys Ser Thr Arg Ala Phe Ser Asn Ser Ser Tyr Val Leu Asn Pro
            180                 185                 190

Thr Thr Gly Glu Leu Val Phe Asp Pro Leu Ser Ala Ser Asp Thr Gly
        195                 200                 205

Glu Tyr Ser Cys Glu Ala Arg Asn Gly Tyr Gly Thr Pro Met Thr Ser
    210                 215                 220

Asn Ala Val Arg Met Glu Ala Val Glu Arg Asn Val Gly Val Ile Val
225                 230                 235                 240

Ala Ala Val Leu Val Thr Leu Ile Leu Leu Gly Ile Leu Val Phe Gly
                245                 250                 255

Ile Trp Phe Ala Tyr Ser Arg Gly His Phe Asp Arg Thr Lys Lys Gly
            260                 265                 270

Thr Ser Ser Lys Lys Val Ile Tyr Ser Gln Pro Ser Ala Arg Ser Glu
        275                 280                 285

Gly Glu Phe Lys Gln Thr Ser Ser Phe Leu Val
    290                 295

<210>  7
<211>  298
<212>  PRT
<213>  Homo sapiens

<400>  7

Met Ala Arg Arg Ser Arg His Arg Leu Leu Leu Leu Leu Leu Arg Tyr
1               5                   10                  15

Leu Val Val Ala Leu Gly Tyr His Lys Ala Tyr Gly Phe Ser Ala Pro
            20                  25                  30

Lys Asp Gln Gln Val Val Thr Ala Val Glu Tyr Gln Glu Ala Ile Leu
        35                  40                  45

Ala Cys Lys Thr Pro Lys Lys Thr Val Ser Ser Arg Leu Glu Trp Lys
    50                  55                  60

Lys Leu Gly Arg Ser Val Ser Phe Val Tyr Tyr Gln Gln Thr Leu Gln
65                  70                  75                  80

Gly Asp Phe Lys Asn Arg Ala Glu Met Ile Asp Phe Asn Ile Arg Ile
                85                  90                  95

Lys Asn Val Thr Arg Ser Asp Ala Gly Lys Tyr Arg Cys Glu Val Ser
            100                 105                 110

Ala Pro Ser Glu Gln Gly Gln Asn Leu Glu Glu Asp Thr Val Thr Leu
        115                 120                 125

Glu Val Leu Val Ala Pro Ala Val Pro Ser Cys Glu Val Pro Ser Ser
    130                 135                 140

Ala Leu Ser Gly Thr Val Val Glu Leu Arg Cys Gln Asp Lys Glu Gly
145                 150                 155                 160

Asn Pro Ala Pro Glu Tyr Thr Trp Phe Lys Asp Gly Ile Arg Leu Leu
                165                 170                 175

Glu Asn Pro Arg Leu Gly Ser Gln Ser Thr Asn Ser Ser Tyr Thr Met
            180                 185                 190

Asn Thr Lys Thr Gly Thr Leu Gln Phe Asn Thr Val Ser Lys Leu Asp
        195                 200                 205

Thr Gly Glu Tyr Ser Cys Glu Ala Arg Asn Ser Val Gly Tyr Arg Arg
    210                 215                 220

Cys Pro Gly Lys Arg Met Gln Val Asp Asp Leu Asn Ile Ser Gly Ile
225                 230                 235                 240

Ile Ala Ala Val Val Val Val Ala Leu Val Ile Ser Val Cys Gly Leu
                245                 250                 255

Gly Val Cys Tyr Ala Gln Arg Lys Gly Tyr Phe Ser Lys Glu Thr Ser
            260                 265                 270

Phe Gln Lys Ser Asn Ser Ser Ser Lys Ala Thr Thr Met Ser Glu Asn
        275                 280                 285

Asp Phe Lys His Thr Lys Ser Phe Ile Ile
    290                 295

<210>  8
<211>  310
<212>  PRT
<213>  Homo sapiens

<400>  8

<210>  9
<211>  423
<212>  PRT
<213>  Homo sapiens

<400>  9

Met Val Phe Ala Phe Trp Lys Val Phe Leu Ile Leu Ser Cys Leu Ala
1               5                   10                  15

Gly Gln Val Ser Val Val Gln Val Thr Ile Pro Asp Gly Phe Val Asn
            20                  25                  30

Val Thr Val Gly Ser Asn Val Thr Leu Ile Cys Ile Tyr Thr Thr Thr
        35                  40                  45

Val Ala Ser Arg Glu Gln Leu Ser Ile Gln Trp Ser Phe Phe His Lys
    50                  55                  60

Lys Glu Met Glu Pro Ile Ser His Ser Ser Cys Leu Ser Thr Glu Gly
65                  70                  75                  80

Met Glu Glu Lys Ala Val Ser Gln Cys Leu Lys Met Thr His Ala Arg
                85                  90                  95

Asp Ala Arg Gly Arg Cys Ser Trp Thr Ser Glu Ile Tyr Phe Ser Gln
            100                 105                 110

Gly Gly Gln Ala Val Ala Ile Gly Gln Phe Lys Asp Arg Ile Thr Gly
        115                 120                 125

Ser Asn Asp Pro Gly Asn Ala Ser Ile Thr Ile Ser His Met Gln Pro
    130                 135                 140

Ala Asp Ser Gly Ile Tyr Ile Cys Asp Val Asn Asn Pro Pro Asp Phe
145                 150                 155                 160

Leu Gly Gln Asn Gln Gly Ile Leu Asn Val Ser Val Leu Val Lys Pro
                165                 170                 175

Ser Lys Pro Leu Cys Ser Val Gln Gly Arg Pro Glu Thr Gly His Thr
            180                 185                 190

Ile Ser Leu Ser Cys Leu Ser Ala Leu Gly Thr Pro Ser Pro Val Tyr
        195                 200                 205

Tyr Trp His Lys Leu Glu Gly Arg Asp Ile Val Pro Val Lys Glu Asn
    210                 215                 220

Phe Asn Pro Thr Thr Gly Ile Leu Val Ile Gly Asn Leu Thr Asn Phe
225                 230                 235                 240

Glu Gln Gly Tyr Tyr Gln Cys Thr Ala Ile Asn Arg Leu Gly Asn Ser
                245                 250                 255

Ser Cys Glu Ile Asp Leu Thr Ser Ser His Pro Glu Val Gly Ile Ile
            260                 265                 270

Val Gly Ala Leu Ile Gly Ser Leu Val Gly Ala Ala Ile Ile Ile Ser
        275                 280                 285

Val Val Cys Phe Ala Arg Asn Lys Ala Lys Ala Lys Ala Lys Glu Arg
    290                 295                 300

Asn Ser Lys Thr Ile Ala Glu Leu Glu Pro Met Thr Lys Ile Asn Pro
305                 310                 315                 320

Arg Gly Glu Ser Glu Ala Met Pro Arg Glu Asp Ala Thr Gln Leu Glu
                325                 330                 335

Val Thr Leu Pro Ser Ser Ile His Glu Thr Gly Pro Asp Thr Ile Gln
            340                 345                 350

Glu Pro Asp Tyr Glu Pro Lys Pro Thr Gln Glu Pro Ala Pro Glu Pro
        355                 360                 365

Ala Pro Gly Ser Glu Pro Met Ala Val Pro Asp Leu Asp Ile Glu Leu

370                 375                 380

Glu Leu Glu Pro Glu Thr Gln Ser Glu Leu Glu Pro Glu Pro Glu Pro
385                 390                 395                 400

Glu Pro Glu Ser Glu Pro Gly Val Val Val Glu Pro Leu Ser Glu Asp
                405                 410                 415

Glu Lys Gly Val Val Lys Ala
            420

<210>  10
<211>  327
<212>  PRT
<213>  Homo sapiens

<400>  10

Met Ala Glu Leu Pro Gly Pro Phe Leu Cys Gly Ala Leu Leu Gly Phe
1               5                   10                  15

Leu Cys Leu Ser Gly Leu Ala Val Glu Val Lys Val Pro Thr Glu Pro
            20                  25                  30

Leu Ser Thr Pro Leu Gly Lys Thr Ala Glu Leu Thr Cys Thr Tyr Ser
        35                  40                  45

Thr Ser Val Gly Asp Ser Phe Ala Leu Glu Trp Ser Phe Val Gln Pro
    50                  55                  60

Gly Lys Pro Ile Ser Glu Ser His Pro Ile Leu Tyr Phe Thr Asn Gly
65                  70                  75                  80

His Leu Tyr Pro Thr Gly Ser Lys Ser Lys Arg Val Ser Leu Leu Gln
                85                  90                  95

Asn Pro Pro Thr Val Gly Val Ala Thr Leu Lys Leu Thr Asp Val His
            100                 105                 110

Pro Ser Asp Thr Gly Thr Tyr Leu Cys Gln Val Asn Asn Pro Pro Asp
        115                 120                 125

Phe Tyr Thr Asn Gly Leu Gly Leu Ile Asn Leu Thr Val Leu Val Pro
    130                 135                 140

Pro Ser Asn Pro Leu Cys Ser Gln Ser Gly Gln Thr Ser Val Gly Gly
145                 150                 155                 160

Ser Thr Ala Leu Arg Cys Ser Ser Ser Glu Gly Ala Pro Lys Pro Val

165                 170                 175

Tyr Asn Trp Val Arg Leu Gly Thr Phe Pro Thr Pro Ser Pro Gly Ser
            180                 185                 190

Met Val Gln Asp Glu Val Ser Gly Gln Leu Ile Leu Thr Asn Leu Ser
        195                 200                 205

Leu Thr Ser Ser Gly Thr Tyr Arg Cys Val Ala Thr Asn Gln Met Gly
    210                 215                 220

Ser Ala Ser Cys Glu Leu Thr Leu Ser Val Thr Glu Pro Ser Gln Gly
225                 230                 235                 240

Arg Val Ala Gly Ala Leu Ile Gly Val Leu Leu Gly Val Leu Leu Leu
                245                 250                 255

Ser Val Ala Ala Phe Cys Leu Val Arg Phe Gln Lys Glu Arg Gly Lys
            260                 265                 270

Lys Pro Lys Glu Thr Tyr Gly Gly Ser Asp Leu Arg Glu Asp Ala Ile
        275                 280                 285

Ala Pro Gly Ile Ser Glu His Thr Cys Met Arg Ala Asp Ser Ser Lys
    290                 295                 300

Gly Phe Leu Glu Arg Pro Ser Ser Ala Ser Thr Val Thr Thr Thr Lys
305                 310                 315                 320

Ser Lys Leu Pro Met Val Val
                325

<210>  11
<211>  430
<212>  PRT
<213>  Homo sapiens

<400>  11

Met Ser Leu Val Glu Leu Leu Leu Trp Trp Asn Cys Phe Ser Arg Thr
1               5                   10                  15

Gly Val Ala Ala Ser Leu Glu Val Ser Glu Ser Pro Gly Ser Ile Gln
            20                  25                  30

Val Ala Arg Gly Gln Pro Ala Val Leu Pro Cys Thr Phe Thr Thr Ser
        35                  40                  45

Ala Ala Leu Ile Asn Leu Asn Val Ile Trp Met Val Thr Pro Leu Ser

50                  55                  60

Asn Ala Asn Gln Pro Glu Gln Val Ile Leu Tyr Gln Gly Gly Gln Met
65                  70                  75                  80

Phe Asp Gly Ala Pro Arg Phe His Gly Arg Val Gly Phe Thr Gly Thr
                85                  90                  95

Met Pro Ala Thr Asn Val Ser Ile Phe Ile Asn Asn Thr Gln Leu Ser
            100                 105                 110

Asp Thr Gly Thr Tyr Gln Cys Leu Val Asn Asn Leu Pro Asp Ile Gly
        115                 120                 125

Gly Arg Asn Ile Gly Val Thr Gly Leu Thr Val Leu Val Pro Pro Ser
    130                 135                 140

Ala Pro His Cys Gln Ile Gln Gly Ser Gln Asp Ile Gly Ser Asp Val
145                 150                 155                 160

Ile Leu Leu Cys Ser Ser Glu Glu Gly Ile Pro Arg Pro Thr Tyr Leu
                165                 170                 175

Trp Glu Lys Leu Asp Asn Thr Leu Lys Leu Pro Pro Thr Ala Thr Gln
            180                 185                 190

Asp Gln Val Gln Gly Thr Val Thr Ile Arg Asn Ile Ser Ala Leu Ser
        195                 200                 205

Ser Gly Leu Tyr Gln Cys Val Ala Ser Asn Ala Ile Gly Thr Ser Thr
    210                 215                 220

Cys Leu Leu Asp Leu Gln Val Ile Ser Pro Gln Pro Arg Asn Ile Gly
225                 230                 235                 240

Leu Ile Ala Gly Ala Ile Gly Thr Gly Ala Val Ile Ile Ile Phe Cys
                245                 250                 255

Ile Ala Leu Ile Leu Gly Ala Phe Phe Tyr Trp Arg Ser Lys Asn Lys
            260                 265                 270

Glu Glu Glu Glu Glu Glu Ile Pro Asn Glu Ile Arg Glu Asp Asp Leu
        275                 280                 285

Pro Pro Lys Cys Ser Ser Ala Lys Ala Phe His Thr Glu Ile Ser Ser
    290                 295                 300

Ser Asp Asn Asn Thr Leu Thr Ser Ser Asn Ala Tyr Asn Ser Arg Tyr
305                 310                 315                 320

Trp Ser Asn Asn Pro Lys Val His Arg Asn Thr Glu Ser Val Ser His
                325                 330                 335

Phe Ser Asp Leu Gly Gln Ser Phe Ser Phe His Ser Gly Asn Ala Asn
            340                 345                 350

Ile Pro Ser Ile Tyr Ala Asn Gly Thr His Leu Val Pro Gly Gln His
        355                 360                 365

Lys Thr Leu Val Val Thr Ala Asn Arg Gly Ser Ser Pro Gln Val Met
    370                 375                 380

Ser Arg Ser Asn Gly Ser Val Ser Arg Lys Pro Arg Pro Pro His Thr
385                 390                 395                 400

His Ser Tyr Thr Ile Ser His Ala Thr Leu Glu Arg Ile Gly Ala Val
                405                 410                 415

Pro Val Met Val Pro Ala Gln Ser Arg Ala Gly Ser Leu Val
            420                 425                 430

<210>  12
<211>  399
<212>  PRT
<213>  Homo sapiens

<400>  12

Met Gly Ile Leu Leu Gly Leu Leu Leu Leu Gly His Leu Thr Val Asp
1               5                   10                  15

Thr Tyr Gly Arg Pro Ile Leu Glu Val Pro Glu Ser Val Thr Gly Pro
            20                  25                  30

Trp Lys Gly Asp Val Asn Leu Pro Cys Thr Tyr Asp Pro Leu Gln Gly
        35                  40                  45

Tyr Thr Gln Val Leu Val Lys Trp Leu Val Gln Arg Gly Ser Asp Pro
    50                  55                  60

Val Thr Ile Phe Leu Arg Asp Ser Ser Gly Asp His Ile Gln Gln Ala
65                  70                  75                  80

Lys Tyr Gln Gly Arg Leu His Val Ser His Lys Val Pro Gly Asp Val
                85                  90                  95

Ser Leu Gln Leu Ser Thr Leu Glu Met Asp Asp Arg Ser His Tyr Thr
        100                 105                 110

Cys Glu Val Thr Trp Gln Thr Pro Asp Gly Asn Gln Val Val Arg Asp
    115                 120                 125

Lys Ile Thr Glu Leu Arg Val Gln Lys Leu Ser Val Ser Lys Pro Thr
    130                 135                 140

Val Thr Thr Gly Ser Gly Tyr Gly Phe Thr Val Pro Gln Gly Met Arg
145                 150                 155                 160

Ile Ser Leu Gln Cys Gln Ala Arg Gly Ser Pro Pro Ile Ser Tyr Ile
                165                 170                 175

Trp Tyr Lys Gln Gln Thr Asn Asn Gln Glu Pro Ile Lys Val Ala Thr
            180                 185                 190

Leu Ser Thr Leu Leu Phe Lys Pro Ala Val Ile Ala Asp Ser Gly Ser
        195                 200                 205

Tyr Phe Cys Thr Ala Lys Gly Gln Val Gly Ser Glu Gln His Ser Asp
    210                 215                 220

Ile Val Lys Phe Val Val Lys Asp Ser Ser Lys Leu Leu Lys Thr Lys
225                 230                 235                 240

Thr Glu Ala Pro Thr Thr Met Thr Tyr Pro Leu Lys Ala Thr Ser Thr
                245                 250                 255

Val Lys Gln Ser Trp Asp Trp Thr Thr Asp Met Asp Gly Tyr Leu Gly
            260                 265                 270

Glu Thr Ser Ala Gly Pro Gly Lys Ser Leu Pro Val Phe Ala Ile Ile
        275                 280                 285

Leu Ile Ile Ser Leu Cys Cys Met Val Val Phe Thr Met Ala Tyr Ile
    290                 295                 300

Met Leu Cys Arg Lys Thr Ser Gln Gln Glu His Val Tyr Glu Ala Ala
305                 310                 315                 320

Arg Ala His Ala Arg Glu Ala Asn Asp Ser Gly Glu Thr Met Arg Val
                325                 330                 335

Ala Ile Phe Ala Ser Gly Cys Ser Ser Asp Glu Pro Thr Ser Gln Asn
            340                 345                 350

Leu Gly Asn Asn Tyr Ser Asp Glu Pro Cys Ile Gly Gln Glu Tyr Gln
        355                 360                 365

Ile Ile Ala Gln Ile Asn Gly Asn Tyr Ala Arg Leu Leu Asp Thr Val
    370                 375                 380

Pro Leu Asp Tyr Glu Phe Leu Ala Thr Glu Gly Lys Ser Val Cys
385                 390                 395

<210>  13
<211>  414
<212>  PRT
<213>  Homo sapiens

<400>  13

Met Arg Val Gly Gly Ala Phe His Leu Leu Leu Val Cys Leu Ser Pro
1               5                   10                  15

Ala Leu Leu Ser Ala Val Arg Ile Asn Gly Asp Gly Gln Glu Val Leu
            20                  25                  30

Tyr Leu Ala Glu Gly Asp Asn Val Arg Leu Gly Cys Pro Tyr Val Leu
        35                  40                  45

Asp Pro Glu Asp Tyr Gly Pro Asn Gly Leu Asp Ile Glu Trp Met Gln
    50                  55                  60

Val Asn Ser Asp Pro Ala His His Arg Glu Asn Val Phe Leu Ser Tyr
65                  70                  75                  80

Gln Asp Lys Arg Ile Asn His Gly Ser Leu Pro His Leu Gln Gln Arg
                85                  90                  95

Val Arg Phe Ala Ala Ser Asp Pro Ser Gln Tyr Asp Ala Ser Ile Asn
            100                 105                 110

Leu Met Asn Leu Gln Val Ser Asp Thr Ala Thr Tyr Glu Cys Arg Val
        115                 120                 125

Lys Lys Thr Thr Met Ala Thr Arg Lys Val Ile Val Thr Val Gln Ala
    130                 135                 140

Arg Pro Ala Val Pro Met Cys Trp Thr Glu Gly His Met Thr Tyr Gly
145                 150                 155                 160

Asn Asp Val Val Leu Lys Cys Tyr Ala Ser Gly Gly Ser Gln Pro Leu
                165                 170                 175

Ser Tyr Lys Trp Ala Lys Ile Ser Gly His His Tyr Pro Tyr Arg Ala
            180                 185                 190

Gly Ser Tyr Thr Ser Gln His Ser Tyr His Ser Glu Leu Ser Tyr Gln
        195                 200                 205

Glu Ser Phe His Ser Ser Ile Asn Gln Gly Leu Asn Asn Gly Asp Leu
    210                 215                 220

Val Leu Lys Asp Ile Ser Arg Ala Asp Asp Gly Leu Tyr Gln Cys Thr
225                 230                 235                 240

Val Ala Asn Asn Val Gly Tyr Ser Val Cys Val Val Glu Val Lys Val
                245                 250                 255

Ser Asp Ser Arg Arg Ile Gly Val Ile Ile Gly Ile Val Leu Gly Ser
            260                 265                 270

Leu Leu Ala Leu Gly Cys Leu Ala Val Gly Ile Trp Gly Leu Val Cys
        275                 280                 285

Cys Cys Cys Gly Gly Ser Gly Ala Gly Gly Ala Arg Gly Ala Phe Gly
    290                 295                 300

Tyr Gly Asn Gly Gly Gly Val Gly Gly Gly Ala Cys Gly Asp Leu Ala
305                 310                 315                 320

Ser Glu Ile Arg Glu Asp Ala Val Ala Pro Gly Cys Lys Ala Ser Gly
                325                 330                 335

Arg Gly Ser Arg Val Thr His Leu Leu Gly Tyr Pro Thr Gln Asn Val
            340                 345                 350

Ser Arg Ser Leu Arg Arg Lys Tyr Ala Pro Pro Pro Cys Gly Gly Pro
        355                 360                 365

Glu Asp Val Ala Leu Ala Pro Cys Thr Ala Ala Ala Ala Cys Glu Ala
    370                 375                 380

Gly Pro Ser Pro Val Tyr Val Lys Val Lys Ser Ala Glu Pro Ala Asp
385                 390                 395                 400

Cys Ala Glu Gly Pro Val Gln Cys Lys Asn Gly Leu Leu Val
                405                 410

<210>  14
<211>  442

<212>  PRT
<213>  Homo sapiens

<400>  14

Met Ala Ser Val Val Leu Pro Ser Gly Ser Gln Cys Ala Ala Ala Ala
1               5                   10                  15

Ala Ala Ala Ala Pro Pro Gly Leu Arg Leu Arg Leu Leu Leu Leu Leu
            20                  25                  30

Phe Ser Ala Ala Ala Leu Ile Pro Thr Gly Asp Gly Gln Asn Leu Phe
        35                  40                  45

Thr Lys Asp Val Thr Val Ile Glu Gly Glu Val Ala Thr Ile Ser Cys
    50                  55                  60

Gln Val Asn Lys Ser Asp Asp Ser Val Ile Gln Leu Leu Asn Pro Asn
65                  70                  75                  80

Arg Gln Thr Ile Tyr Phe Arg Asp Phe Arg Pro Leu Lys Asp Ser Arg
                85                  90                  95

Phe Gln Leu Leu Asn Phe Ser Ser Ser Glu Leu Lys Val Ser Leu Thr
            100                 105                 110

Asn Val Ser Ile Ser Asp Glu Gly Arg Tyr Phe Cys Gln Leu Tyr Thr
        115                 120                 125

Asp Pro Pro Gln Glu Ser Tyr Thr Thr Ile Thr Val Leu Val Pro Pro
    130                 135                 140

Arg Asn Leu Met Ile Asp Ile Gln Lys Asp Thr Ala Val Glu Gly Glu
145                 150                 155                 160

Glu Ile Glu Val Asn Cys Thr Ala Met Ala Ser Lys Pro Ala Thr Thr
                165                 170                 175

Ile Arg Trp Phe Lys Gly Asn Thr Glu Leu Lys Gly Lys Ser Glu Val
            180                 185                 190

Glu Glu Trp Ser Asp Met Tyr Thr Val Thr Ser Gln Leu Met Leu Lys
        195                 200                 205

Val His Lys Glu Asp Asp Gly Val Pro Val Ile Cys Gln Val Glu His
    210                 215                 220

Pro Ala Val Thr Gly Asn Leu Gln Thr Gln Arg Tyr Leu Glu Val Gln
225                 230                 235                 240

Tyr Lys Pro Gln Val His Ile Gln Met Thr Tyr Pro Leu Gln Gly Leu
                245                 250                 255

Thr Arg Glu Gly Asp Ala Leu Glu Leu Thr Cys Glu Ala Ile Gly Lys
            260                 265                 270

Pro Gln Pro Val Met Val Thr Trp Val Arg Val Asp Asp Glu Met Pro
        275                 280                 285

Gln His Ala Val Leu Ser Gly Pro Asn Leu Phe Ile Asn Asn Leu Asn
    290                 295                 300

Lys Thr Asp Asn Gly Thr Tyr Arg Cys Glu Ala Ser Asn Ile Val Gly
305                 310                 315                 320

Lys Ala His Ser Asp Tyr Met Leu Tyr Val Tyr Asp Pro Pro Thr Thr
                325                 330                 335

Ile Pro Pro Pro Thr Thr Thr Thr Thr Thr Thr Thr Thr Thr Thr Thr
            340                 345                 350

Thr Ile Leu Thr Ile Ile Thr Asp Ser Arg Ala Gly Glu Glu Gly Ser
        355                 360                 365

Ile Arg Ala Val Asp His Ala Val Ile Gly Gly Val Val Ala Val Val
    370                 375                 380

Val Phe Ala Met Leu Cys Leu Leu Ile Ile Leu Gly Arg Tyr Phe Ala
385                 390                 395                 400

Arg His Lys Gly Thr Tyr Phe Thr His Glu Ala Lys Gly Ala Asp Asp
                405                 410                 415

Ala Ala Asp Ala Asp Thr Ala Ile Ile Asn Ala Glu Gly Gly Gln Asn
            420                 425                 430

Asn Ser Glu Glu Lys Lys Glu Tyr Phe Ile
        435                 440


<210>  15
<211>  435
<212>  PRT
<213>  Homo sapiens

<400>  15

Met Ile Trp Lys Arg Ser Ala Val Leu Arg Phe Tyr Ser Val Cys Gly
1               5                   10                  15

Leu Leu Leu Gln Gly Ser Gln Gly Gln Phe Pro Leu Thr Gln Asn Val
            20                  25                  30

Thr Val Val Glu Gly Gly Thr Ala Ile Leu Thr Cys Arg Val Asp Gln
        35                  40                  45

Asn Asp Asn Thr Ser Leu Gln Trp Ser Asn Pro Ala Gln Gln Thr Leu
    50                  55                  60

Tyr Phe Asp Asp Lys Lys Ala Leu Arg Asp Asn Arg Ile Glu Leu Val
65                  70                  75                  80

Arg Ala Ser Trp His Glu Leu Ser Ile Ser Val Ser Asp Val Ser Leu
                85                  90                  95

Ser Asp Glu Gly Gln Tyr Thr Cys Ser Leu Phe Thr Met Pro Val Lys
            100                 105                 110

Thr Ser Lys Ala Tyr Leu Thr Val Leu Gly Val Pro Glu Lys Pro Gln
        115                 120                 125

Ile Ser Gly Phe Ser Ser Pro Val Met Glu Gly Asp Leu Met Gln Leu
    130                 135                 140

Thr Cys Lys Thr Ser Gly Ser Lys Pro Ala Ala Asp Ile Arg Trp Phe
145                 150                 155                 160

Lys Asn Asp Lys Glu Ile Lys Asp Val Lys Tyr Leu Lys Glu Glu Asp
                165                 170                 175

Ala Asn Arg Lys Thr Phe Thr Val Ser Ser Thr Leu Asp Phe Arg Val
            180                 185                 190

Asp Arg Ser Asp Asp Gly Val Ala Val Ile Cys Arg Val Asp His Glu
        195                 200                 205

Ser Leu Asn Ala Thr Pro Gln Val Ala Met Gln Val Leu Glu Ile His
    210                 215                 220

Tyr Thr Pro Ser Val Lys Ile Ile Pro Ser Thr Pro Phe Pro Gln Glu
225                 230                 235                 240

Gly Gln Pro Leu Ile Leu Thr Cys Glu Ser Lys Gly Lys Pro Leu Pro
                245                 250                 255

Glu Pro Val Leu Trp Thr Lys Asp Gly Gly Glu Leu Pro Asp Pro Asp

260                 265                 270

Arg Met Val Val Ser Gly Arg Glu Leu Asn Ile Leu Phe Leu Asn Lys
        275                 280                 285

Thr Asp Asn Gly Thr Tyr Arg Cys Glu Ala Thr Asn Thr Ile Gly Gln
    290                 295                 300

Ser Ser Ala Glu Tyr Val Leu Ile Val His Asp Val Pro Asn Thr Leu
305                 310                 315                 320

Leu Pro Thr Thr Ile Ile Pro Ser Leu Thr Thr Ala Thr Val Thr Thr
                325                 330                 335

Thr Val Ala Ile Thr Thr Ser Pro Thr Thr Ser Ala Thr Thr Ser Ser
            340                 345                 350

Ile Arg Asp Pro Asn Ala Leu Ala Gly Gln Asn Gly Pro Asp His Ala
        355                 360                 365

Leu Ile Gly Gly Ile Val Ala Val Val Val Phe Val Thr Leu Cys Ser
    370                 375                 380

Ile Phe Leu Leu Gly Arg Tyr Leu Ala Arg His Lys Gly Thr Tyr Leu
385                 390                 395                 400

Thr Asn Glu Ala Lys Gly Ala Glu Asp Ala Pro Asp Ala Asp Thr Ala
                405                 410                 415

Ile Ile Asn Ala Glu Gly Ser Gln Val Asn Ala Glu Glu Lys Lys Glu
            420                 425                 430

Tyr Phe Ile
        435

<210>  16
<211>  432
<212>  PRT
<213>  Homo sapiens

<400>  16

Met Gly Ala Pro Ala Ala Ser Leu Leu Leu Leu Leu Leu Leu Phe Ala
1               5                   10                  15

Cys Cys Trp Ala Pro Gly Gly Ala Asn Leu Ser Gln Asp Gly Tyr Trp
            20                  25                  30

Gln Glu Gln Asp Leu Glu Leu Gly Thr Leu Ala Pro Leu Asp Glu Ala

35                  40                  45

Ile Ser Ser Thr Val Trp Ser Ser Pro Asp Met Leu Ala Ser Gln Asp
    50                  55                  60

Ser Gln Pro Trp Thr Ser Asp Glu Thr Val Val Ala Gly Gly Thr Val
65                  70                  75                  80

Val Leu Lys Cys Gln Val Lys Asp His Glu Asp Ser Ser Leu Gln Trp
                85                  90                  95

Ser Asn Pro Ala Gln Gln Thr Leu Tyr Phe Gly Glu Lys Arg Ala Leu
            100                 105                 110

Arg Asp Asn Arg Ile Gln Leu Val Thr Ser Thr Pro His Glu Leu Ser
        115                 120                 125

Ile Ser Ile Ser Asn Val Ala Leu Ala Asp Glu Gly Glu Tyr Thr Cys
    130                 135                 140

Ser Ile Phe Thr Met Pro Val Arg Thr Ala Lys Ser Leu Val Thr Val
145                 150                 155                 160

Leu Gly Ile Pro Gln Lys Pro Ile Ile Thr Gly Tyr Lys Ser Ser Leu
                165                 170                 175

Arg Glu Lys Asp Thr Ala Thr Leu Asn Cys Gln Ser Ser Gly Ser Lys
            180                 185                 190

Pro Ala Ala Arg Leu Thr Trp Arg Lys Gly Asp Gln Glu Leu His Gly
        195                 200                 205

Glu Pro Thr Arg Ile Gln Glu Asp Pro Asn Gly Lys Thr Phe Thr Val
    210                 215                 220

Ser Ser Ser Val Thr Phe Gln Val Thr Arg Glu Asp Asp Gly Ala Ser
225                 230                 235                 240

Ile Val Cys Ser Val Asn His Glu Ser Leu Lys Gly Ala Asp Arg Ser
                245                 250                 255

Thr Ser Gln Arg Ile Glu Val Leu Tyr Thr Pro Thr Ala Met Ile Arg
            260                 265                 270

Pro Asp Pro Pro His Pro Arg Glu Gly Gln Lys Leu Leu Leu His Cys
        275                 280                 285

Glu Gly Arg Gly Asn Pro Val Pro Gln Gln Tyr Leu Trp Glu Lys Glu
    290                 295                 300

Gly Ser Val Pro Pro Leu Lys Met Thr Gln Glu Ser Ala Leu Ile Phe
305                 310                 315                 320

Pro Phe Leu Asn Lys Ser Asp Ser Gly Thr Tyr Gly Cys Thr Ala Thr
                325                 330                 335

Ser Asn Met Gly Ser Tyr Lys Ala Tyr Tyr Thr Leu Asn Val Asn Asp
            340                 345                 350

Pro Ser Pro Val Pro Ser Ser Ser Ser Thr Tyr His Ala Ile Ile Gly
        355                 360                 365

Gly Ile Val Ala Phe Ile Val Phe Leu Leu Leu Ile Met Leu Ile Phe
    370                 375                 380

Leu Gly His Tyr Leu Ile Arg His Lys Gly Thr Tyr Leu Thr His Glu
385                 390                 395                 400

Ala Lys Gly Ser Asp Asp Ala Pro Asp Ala Asp Thr Ala Ile Ile Asn
                405                 410                 415

Ala Glu Gly Gly Gln Ser Gly Gly Asp Asp Lys Lys Glu Tyr Phe Ile
            420                 425                 430

<210>  17
<211>  388
<212>  PRT
<213>  Homo sapiens

<400>  17

Met Gly Arg Ala Arg Arg Phe Gln Trp Pro Leu Leu Leu Leu Trp Ala
1               5                   10                  15

Ala Ala Ala Gly Pro Gly Ala Gly Gln Glu Val Gln Thr Glu Asn Val
            20                  25                  30

Thr Val Ala Glu Gly Gly Val Ala Glu Ile Thr Cys Arg Leu His Gln
        35                  40                  45

Tyr Asp Gly Ser Ile Val Val Ile Gln Asn Pro Ala Arg Gln Thr Leu
    50                  55                  60

Phe Phe Asn Gly Thr Arg Ala Leu Lys Asp Glu Arg Phe Gln Leu Glu
65                  70                  75                  80

Glu Phe Ser Pro Arg Arg Val Arg Ile Arg Leu Ser Asp Ala Arg Leu
                85                  90                  95

Glu Asp Glu Gly Gly Tyr Phe Cys Gln Leu Tyr Thr Glu Asp Thr His
            100                 105                 110

His Gln Ile Ala Thr Leu Thr Val Leu Val Ala Pro Glu Asn Pro Val
        115                 120                 125

Val Glu Val Arg Glu Gln Ala Val Glu Gly Gly Glu Val Glu Leu Ser
    130                 135                 140

Cys Leu Val Pro Arg Ser Arg Pro Ala Ala Thr Leu Arg Trp Tyr Arg
145                 150                 155                 160

Asp Arg Lys Glu Leu Lys Gly Val Ser Ser Ser Gln Glu Asn Gly Lys
                165                 170                 175

Val Trp Ser Val Ala Ser Thr Val Arg Phe Arg Val Asp Arg Lys Asp
            180                 185                 190

Asp Gly Gly Ile Ile Ile Cys Glu Ala Gln Asn Gln Ala Leu Pro Ser
        195                 200                 205

Gly His Ser Lys Gln Thr Gln Tyr Val Leu Asp Val Gln Tyr Ser Pro
    210                 215                 220

Thr Ala Arg Ile His Ala Ser Gln Ala Val Val Arg Glu Gly Asp Thr
225                 230                 235                 240

Leu Val Leu Thr Cys Ala Val Thr Gly Asn Pro Arg Pro Asn Gln Ile
                245                 250                 255

Arg Trp Asn Arg Gly Asn Glu Ser Leu Pro Glu Arg Ala Glu Ala Val
            260                 265                 270

Gly Glu Thr Leu Thr Leu Pro Gly Leu Val Ser Ala Asp Asn Gly Thr
        275                 280                 285

Tyr Thr Cys Glu Ala Ser Asn Lys His Gly His Ala Arg Ala Leu Tyr
    290                 295                 300

Val Leu Val Val Tyr Asp Pro Gly Ala Val Val Glu Ala Gln Thr Ser
305                 310                 315                 320

Val Pro Tyr Ala Ile Val Gly Gly Ile Leu Ala Leu Leu Val Phe Leu
                325                 330                 335

Ile Ile Cys Val Leu Val Gly Met Val Trp Cys Ser Val Arg Gln Lys
        340                 345                 350

Gly Ser Tyr Leu Thr His Glu Ala Ser Gly Leu Asp Glu Gln Gly Glu
    355                 360                 365

Ala Arg Glu Ala Phe Leu Asn Gly Ser Asp Gly His Lys Arg Lys Glu
    370                 375                 380

Glu Phe Phe Ile
385

<210>  18
<211>  393
<212>  PRT
<213>  Homo sapiens

<400>  18

Met Trp Trp Arg Val Leu Ser Leu Leu Ala Trp Phe Pro Leu Gln Glu
1               5                   10                  15

Ala Ser Leu Thr Asn His Thr Glu Thr Ile Thr Val Glu Glu Gly Gln
            20                  25                  30

Thr Leu Thr Leu Lys Cys Val Thr Ser Leu Arg Lys Asn Ser Ser Leu
        35                  40                  45

Gln Trp Leu Thr Pro Ser Gly Phe Thr Ile Phe Leu Asn Glu Tyr Pro
    50                  55                  60

Ala Leu Lys Asn Ser Lys Tyr Gln Leu Leu His His Ser Ala Asn Gln
65                  70                  75                  80

Leu Ser Ile Thr Val Pro Asn Val Thr Leu Gln Asp Glu Gly Val Tyr
                85                  90                  95

Lys Cys Leu His Tyr Ser Asp Ser Val Ser Thr Lys Glu Val Lys Val
            100                 105                 110

Ile Val Leu Ala Thr Pro Phe Lys Pro Ile Leu Glu Ala Ser Val Ile
        115                 120                 125

Arg Lys Gln Asn Gly Glu Glu His Val Val Leu Met Cys Ser Thr Met
    130                 135                 140

Arg Ser Lys Pro Pro Pro Gln Ile Thr Trp Leu Leu Gly Asn Ser Met
145                 150                 155                 160

Glu Val Ser Gly Gly Thr Leu His Glu Phe Glu Thr Asp Gly Lys Lys
                165                 170                 175

Cys Asn Thr Thr Ser Thr Leu Ile Ile His Thr Tyr Gly Lys Asn Ser
            180                 185                 190

Thr Val Asp Cys Ile Ile Arg His Arg Gly Leu Gln Gly Arg Lys Leu
        195                 200                 205

Val Ala Pro Phe Arg Phe Glu Asp Leu Val Thr Asp Glu Glu Thr Ala
    210                 215                 220

Ser Asp Ala Leu Glu Arg Asn Ser Leu Ser Ser Gln Asp Pro Gln Gln
225                 230                 235                 240

Pro Thr Ser Thr Val Ser Val Thr Glu Asp Ser Ser Thr Ser Glu Ile
                245                 250                 255

Asp Lys Glu Glu Lys Glu Gln Thr Thr Gln Asp Pro Asp Leu Thr Thr
            260                 265                 270

Glu Ala Asn Pro Gln Tyr Leu Gly Leu Ala Arg Lys Lys Ser Gly Ile
        275                 280                 285

Leu Leu Leu Thr Leu Val Ser Phe Leu Ile Phe Ile Leu Phe Ile Ile
    290                 295                 300

Val Gln Leu Phe Ile Met Lys Leu Arg Lys Ala His Val Ile Trp Lys
305                 310                 315                 320

Lys Glu Asn Glu Val Ser Glu His Thr Leu Glu Ser Tyr Arg Ser Arg
                325                 330                 335

Ser Asn Asn Glu Glu Thr Ser Ser Glu Glu Lys Asn Gly Gln Ser Ser
            340                 345                 350

His Pro Met Arg Cys Met Asn Tyr Ile Thr Lys Leu Tyr Ser Glu Ala
        355                 360                 365

Lys Thr Lys Arg Lys Glu Asn Val Gln His Ser Lys Leu Glu Glu Lys
    370                 375                 380

His Ile Gln Val Pro Glu Ser Ile Val
385                 390

<210>  19
<211>  262

<212>  PRT
<213>  Homo sapiens

<400>  19

Met Ala Trp Lys Ser Ser Val Ile Met Gln Met Gly Arg Phe Leu Leu
1               5                   10                  15

Leu Val Ile Leu Phe Leu Pro Arg Glu Met Thr Ser Ser Val Leu Thr
            20                  25                  30

Val Asn Gly Lys Thr Glu Asn Tyr Ile Leu Asp Thr Thr Pro Gly Ser
        35                  40                  45

Gln Ala Ser Leu Ile Cys Ala Val Gln Asn His Thr Arg Glu Glu Glu
    50                  55                  60

Leu Leu Trp Tyr Arg Glu Glu Gly Arg Val Asp Leu Lys Ser Gly Asn
65                  70                  75                  80

Lys Ile Asn Ser Ser Ser Val Cys Val Ser Ser Ile Ser Glu Asn Asp
                85                  90                  95

Asn Gly Ile Ser Phe Thr Cys Arg Leu Gly Arg Asp Gln Ser Val Ser
            100                 105                 110

Val Ser Val Val Leu Asn Val Thr Phe Pro Pro Leu Leu Ser Gly Asn
        115                 120                 125

Asp Phe Gln Thr Val Glu Glu Gly Ser Asn Val Lys Leu Val Cys Asn
    130                 135                 140

Val Lys Ala Asn Pro Gln Ala Gln Met Met Trp Tyr Lys Asn Ser Ser
145                 150                 155                 160

Leu Leu Asp Leu Glu Lys Ser Arg His Gln Ile Gln Gln Thr Ser Glu
                165                 170                 175

Ser Phe Gln Leu Ser Ile Thr Lys Val Glu Lys Pro Asp Asn Gly Thr
            180                 185                 190

Tyr Ser Cys Ile Ala Lys Ser Ser Leu Lys Thr Glu Ser Leu Asp Phe
        195                 200                 205

His Leu Ile Val Lys Asp Lys Thr Val Gly Val Pro Ile Glu Pro Ile
    210                 215                 220

Ile Ala Ala Cys Val Val Ile Phe Leu Thr Leu Cys Phe Gly Leu Ile
225                 230                 235                 240

Ala Arg Arg Lys Lys Ile Met Lys Leu Cys Met Lys Asp Lys Asp Pro
                245                 250                 255

His Ser Glu Thr Ala Leu
            260

<210>  20
<211>  585
<212>  PRT
<213>  Homo sapiens

<400>  20

Met Glu Lys Lys Trp Lys Tyr Cys Ala Val Tyr Tyr Ile Ile Gln Ile
1               5                   10                  15

His Phe Val Lys Gly Val Trp Glu Lys Thr Val Asn Thr Glu Glu Asn
            20                  25                  30

Val Tyr Ala Thr Leu Gly Ser Asp Val Asn Leu Thr Cys Gln Thr Gln
        35                  40                  45

Thr Val Gly Phe Phe Val Gln Met Gln Trp Ser Lys Val Thr Asn Lys
    50                  55                  60

Ile Asp Leu Ile Ala Val Tyr His Pro Gln Tyr Gly Phe Tyr Cys Ala
65                  70                  75                  80

Tyr Gly Arg Pro Cys Glu Ser Leu Val Thr Phe Thr Glu Thr Pro Glu
                85                  90                  95

Asn Gly Ser Lys Trp Thr Leu His Leu Arg Asn Met Ser Cys Ser Val
            100                 105                 110

Ser Gly Arg Tyr Glu Cys Met Leu Val Leu Tyr Pro Glu Gly Ile Gln
        115                 120                 125

Thr Lys Ile Tyr Asn Leu Leu Ile Gln Thr His Val Thr Ala Asp Glu
    130                 135                 140

Trp Asn Ser Asn His Thr Ile Glu Ile Glu Ile Asn Gln Thr Leu Glu
145                 150                 155                 160

Ile Pro Cys Phe Gln Asn Ser Ser Ser Lys Ile Ser Ser Glu Phe Thr
                165                 170                 175

Tyr Ala Trp Ser Val Glu Asn Ser Ser Thr Asp Ser Trp Val Leu Leu
            180                 185                 190

Ser Lys Gly Ile Lys Glu Asp Asn Gly Thr Gln Glu Thr Leu Ile Ser
        195                 200                 205

Gln Asn His Leu Ile Ser Asn Ser Thr Leu Leu Lys Asp Arg Val Lys
    210                 215                 220

Leu Gly Thr Asp Tyr Arg Leu His Leu Ser Pro Val Gln Ile Phe Asp
225                 230                 235                 240

Asp Gly Arg Lys Phe Ser Cys His Ile Arg Val Gly Pro Asn Lys Ile
                245                 250                 255

Leu Arg Ser Ser Thr Thr Val Lys Val Phe Ala Lys Pro Glu Ile Pro
            260                 265                 270

Val Ile Val Glu Asn Asn Ser Thr Asp Val Leu Val Glu Arg Arg Phe
        275                 280                 285

Thr Cys Leu Leu Lys Asn Val Phe Pro Lys Ala Asn Ile Thr Trp Phe
    290                 295                 300

Ile Asp Gly Ser Phe Leu His Asp Glu Lys Glu Gly Ile Tyr Ile Thr
305                 310                 315                 320

Asn Glu Glu Arg Lys Gly Lys Asp Gly Phe Leu Glu Leu Lys Ser Val
                325                 330                 335

Leu Thr Arg Val His Ser Asn Lys Pro Ala Gln Ser Asp Asn Leu Thr
            340                 345                 350

Ile Trp Cys Met Ala Leu Ser Pro Val Pro Gly Asn Lys Val Trp Asn
        355                 360                 365

Ile Ser Ser Glu Lys Ile Thr Phe Leu Leu Gly Ser Glu Ile Ser Ser
    370                 375                 380

Thr Asp Pro Pro Leu Ser Val Thr Glu Ser Thr Leu Asp Thr Gln Pro
385                 390                 395                 400

Ser Pro Ala Ser Ser Val Ser Pro Ala Arg Tyr Pro Ala Thr Ser Ser
                405                 410                 415

Val Thr Leu Val Asp Val Ser Ala Leu Arg Pro Asn Thr Thr Pro Gln
            420                 425                 430

Pro Ser Asn Ser Ser Met Thr Thr Arg Gly Phe Asn Tyr Pro Trp Thr

435                 440                 445

Ser Ser Gly Thr Asp Thr Lys Lys Ser Val Ser Arg Ile Pro Ser Glu
    450                 455                 460

Thr Tyr Ser Ser Ser Pro Ser Gly Ala Gly Ser Thr Leu His Asp Asn
465                 470                 475                 480

Val Phe Thr Ser Thr Ala Arg Ala Phe Ser Glu Val Pro Thr Thr Ala
                485                 490                 495

Asn Gly Ser Thr Lys Thr Asn His Val His Ile Thr Gly Ile Val Val
            500                 505                 510

Asn Lys Pro Lys Asp Gly Met Ser Trp Pro Val Ile Val Ala Ala Leu
        515                 520                 525

Leu Phe Cys Cys Met Ile Leu Phe Gly Leu Gly Val Arg Lys Trp Cys
    530                 535                 540

Gln Tyr Gln Lys Glu Ile Met Glu Arg Pro Pro Pro Phe Lys Pro Pro
545                 550                 555                 560

Pro Pro Pro Ile Lys Tyr Thr Cys Ile Gln Glu Pro Asn Glu Ser Asp
                565                 570                 575

Leu Pro Tyr His Glu Met Glu Thr Leu
            580                 585

<210>  21
<211>  269
<212>  PRT
<213>  Homo sapiens

<400>  21

Met Glu Arg Leu Val Ile Arg Met Pro Phe Ser His Leu Ser Thr Tyr
1               5                   10                  15

Ser Leu Val Trp Val Met Ala Ala Val Val Leu Cys Thr Ala Gln Val
            20                  25                  30

Gln Val Val Thr Gln Asp Glu Arg Glu Gln Leu Tyr Thr Pro Ala Ser
        35                  40                  45

Leu Lys Cys Ser Leu Gln Asn Ala Gln Glu Ala Leu Ile Val Thr Trp
    50                  55                  60

Gln Lys Lys Lys Ala Val Ser Pro Glu Asn Met Val Thr Phe Ser Glu

65                  70                  75                  80

Asn His Gly Val Val Ile Gln Pro Ala Tyr Lys Asp Lys Ile Asn Ile
                85                  90                  95

Thr Gln Leu Gly Leu Gln Asn Ser Thr Ile Thr Phe Trp Asn Ile Thr
            100                 105                 110

Leu Glu Asp Glu Gly Cys Tyr Met Cys Leu Phe Asn Thr Phe Gly Phe
        115                 120                 125

Gly Lys Ile Ser Gly Thr Ala Cys Leu Thr Val Tyr Val Gln Pro Ile
    130                 135                 140

Val Ser Leu His Tyr Lys Phe Ser Glu Asp His Leu Asn Ile Thr Cys
145                 150                 155                 160

Ser Ala Thr Ala Arg Pro Ala Pro Met Val Phe Trp Lys Val Pro Arg
                165                 170                 175

Ser Gly Ile Glu Asn Ser Thr Val Thr Leu Ser His Pro Asn Gly Thr
            180                 185                 190

Thr Ser Val Thr Ser Ile Leu His Ile Lys Asp Pro Lys Asn Gln Val
        195                 200                 205

Gly Lys Glu Val Ile Cys Gln Val Leu His Leu Gly Thr Val Thr Asp
    210                 215                 220

Phe Lys Gln Thr Val Asn Lys Gly Tyr Trp Phe Ser Val Pro Leu Leu
225                 230                 235                 240

Leu Ser Ile Val Ser Leu Val Ile Leu Leu Val Leu Ile Ser Ile Leu
                245                 250                 255

Leu Tyr Trp Lys Arg His Arg Asn Gln Asp Arg Glu Pro
            260                 265


<210>  22
<211>  348
<212>  PRT
<213>  Homo sapiens

<400>  22

Met Leu Cys Pro Trp Arg Thr Ala Asn Leu Gly Leu Leu Leu Ile Leu
1               5                   10                  15

Thr Ile Phe Leu Val Ala Glu Ala Glu Gly Ala Ala Gln Pro Asn Asn

20                  25                  30

Ser Leu Met Leu Gln Thr Ser Lys Glu Asn His Ala Leu Ala Ser Ser
        35                  40                  45

Ser Leu Cys Met Asp Glu Lys Gln Ile Thr Gln Asn Tyr Ser Lys Val
    50                  55                  60

Leu Ala Glu Val Asn Thr Ser Trp Pro Val Lys Met Ala Thr Asn Ala
65                  70                  75                  80

Val Leu Cys Cys Pro Pro Ile Ala Leu Arg Asn Leu Ile Ile Ile Thr
                85                  90                  95

Trp Glu Ile Ile Leu Arg Gly Gln Pro Ser Cys Thr Lys Ala Tyr Lys
            100                 105                 110

Lys Glu Thr Asn Glu Thr Lys Glu Thr Asn Cys Thr Asp Glu Arg Ile
        115                 120                 125

Thr Trp Val Ser Arg Pro Asp Gln Asn Ser Asp Leu Gln Ile Arg Thr
    130                 135                 140

Val Ala Ile Thr His Asp Gly Tyr Tyr Arg Cys Ile Met Val Thr Pro
145                 150                 155                 160

Asp Gly Asn Phe His Arg Gly Tyr His Leu Gln Val Leu Val Thr Pro
                165                 170                 175

Glu Val Thr Leu Phe Gln Asn Arg Asn Arg Thr Ala Val Cys Lys Ala
            180                 185                 190

Val Ala Gly Lys Pro Ala Ala His Ile Ser Trp Ile Pro Glu Gly Asp
        195                 200                 205

Cys Ala Thr Lys Gln Glu Tyr Trp Ser Asn Gly Thr Val Thr Val Lys
    210                 215                 220

Ser Thr Cys His Trp Glu Val His Asn Val Ser Thr Val Thr Cys His
225                 230                 235                 240

Val Ser His Leu Thr Gly Asn Lys Ser Leu Tyr Ile Glu Leu Leu Pro
                245                 250                 255

Val Pro Gly Ala Lys Lys Ser Ala Lys Leu Tyr Ile Pro Tyr Ile Ile
            260                 265                 270

Leu Thr Ile Ile Ile Leu Thr Ile Val Gly Phe Ile Trp Leu Leu Lys
        275                 280                 285

Val Asn Gly Cys Arg Lys Tyr Lys Leu Asn Lys Thr Glu Ser Thr Pro
    290                 295                 300

Val Val Glu Glu Asp Glu Met Gln Pro Tyr Ala Ser Tyr Thr Glu Lys
305                 310                 315                 320

Asn Asn Pro Leu Tyr Asp Thr Thr Asn Lys Val Lys Ala Ser Glu Ala
                325                 330                 335

Leu Gln Ser Glu Val Asp Thr Asp Leu His Thr Leu
            340                 345

<210>  23
<211>  271
<212>  PRT
<213>  Homo sapiens

<400>  23

Met Ser Ala Pro Arg Leu Leu Ile Ser Ile Ile Ile Met Val Ser Ala
1               5                   10                  15

Ser Ser Ser Ser Cys Met Gly Gly Lys Gln Met Thr Gln Asn Tyr Ser
            20                  25                  30

Thr Ile Phe Ala Glu Gly Asn Ile Ser Gln Pro Val Leu Met Asp Ile
        35                  40                  45

Asn Ala Val Leu Cys Cys Pro Pro Ile Ala Leu Arg Asn Leu Ile Ile
    50                  55                  60

Ile Thr Trp Glu Ile Ile Leu Arg Gly Gln Pro Ser Cys Thr Lys Ala
65                  70                  75                  80

Tyr Lys Lys Glu Thr Asn Glu Thr Lys Glu Thr Asn Cys Thr Val Glu
                85                  90                  95

Arg Ile Thr Trp Val Ser Arg Pro Asp Gln Asn Ser Asp Leu Gln Ile
            100                 105                 110

Arg Pro Val Asp Thr Thr His Asp Gly Tyr Tyr Arg Gly Ile Val Val
        115                 120                 125

Thr Pro Asp Gly Asn Phe His Arg Gly Tyr His Leu Gln Val Leu Val
    130                 135                 140

Thr Pro Glu Val Asn Leu Phe Gln Ser Arg Asn Ile Thr Ala Val Cys
145                 150                 155                 160

Lys Ala Val Thr Gly Lys Pro Ala Ala Gln Ile Ser Trp Ile Pro Glu
                165                 170                 175

Gly Ser Ile Leu Ala Thr Lys Gln Glu Tyr Trp Gly Asn Gly Thr Val
            180                 185                 190

Thr Val Lys Ser Thr Cys Pro Trp Glu Gly His Lys Ser Thr Val Thr
        195                 200                 205

Cys His Val Ser His Leu Thr Gly Asn Lys Ser Leu Ser Val Lys Leu
    210                 215                 220

Asn Ser Gly Leu Arg Thr Ser Gly Ser Pro Ala Leu Ser Leu Leu Ile
225                 230                 235                 240

Ile Leu Tyr Val Lys Leu Ser Leu Phe Val Val Ile Leu Val Thr Thr
                245                 250                 255

Gly Phe Val Phe Phe Gln Arg Ile Asn His Val Arg Lys Val Leu
            260                 265                 270


<210>  24
<211>  336
<212>  PRT
<213>  Homo sapiens

<400>  24

Met Asp Tyr Pro Thr Leu Leu Leu Ala Leu Leu His Val Tyr Arg Ala
1               5                   10                  15

Leu Cys Glu Glu Val Leu Trp His Thr Ser Val Pro Phe Ala Glu Asn
            20                  25                  30

Met Ser Leu Glu Cys Val Tyr Pro Ser Met Gly Ile Leu Thr Gln Val
        35                  40                  45

Glu Trp Phe Lys Ile Gly Thr Gln Gln Asp Ser Ile Ala Ile Phe Ser
    50                  55                  60

Pro Thr His Gly Met Val Ile Arg Lys Pro Tyr Ala Glu Arg Val Tyr
65                  70                  75                  80

Phe Leu Asn Ser Thr Met Ala Ser Asn Asn Met Thr Leu Phe Phe Arg
                85                  90                  95

Asn Ala Ser Glu Asp Asp Val Gly Tyr Tyr Ser Cys Ser Leu Tyr Thr
            100                 105                 110

Tyr Pro Gln Gly Thr Trp Gln Lys Val Ile Gln Val Val Gln Ser Asp
        115                 120                 125

Ser Phe Glu Ala Ala Val Pro Ser Asn Ser His Ile Val Ser Glu Pro
    130                 135                 140

Gly Lys Asn Val Thr Leu Thr Cys Gln Pro Gln Met Thr Trp Pro Val
145                 150                 155                 160

Gln Ala Val Arg Trp Glu Lys Ile Gln Pro Arg Gln Ile Asp Leu Leu
                165                 170                 175

Thr Tyr Cys Asn Leu Val His Gly Arg Asn Phe Thr Ser Lys Phe Pro
            180                 185                 190

Arg Gln Ile Val Ser Asn Cys Ser His Gly Arg Trp Ser Val Ile Val
        195                 200                 205

Ile Pro Asp Val Thr Val Ser Asp Ser Gly Leu Tyr Arg Cys Tyr Leu
    210                 215                 220

Gln Ala Ser Ala Gly Glu Asn Glu Thr Phe Val Met Arg Leu Thr Val
225                 230                 235                 240

Ala Glu Gly Lys Thr Asp Asn Gln Tyr Thr Leu Phe Val Ala Gly Gly
                245                 250                 255

Thr Val Leu Leu Leu Leu Phe Val Ile Ser Ile Thr Thr Ile Ile Val
            260                 265                 270

Ile Phe Leu Asn Arg Arg Arg Arg Arg Glu Arg Arg Asp Leu Phe Thr
        275                 280                 285

Glu Ser Trp Asp Thr Gln Lys Ala Pro Asn Asn Tyr Arg Ser Pro Ile
    290                 295                 300

Ser Thr Ser Gln Pro Thr Asn Gln Ser Met Asp Asp Thr Arg Glu Asp
305                 310                 315                 320

Ile Tyr Val Asn Tyr Pro Thr Phe Ser Arg Arg Pro Lys Thr Arg Val
                325                 330                 335

<210>  25
<211>  326
<212>  PRT

<213>  Homo sapiens

<400>  25

Met Arg Thr Glu Ala Gln Val Pro Ala Leu Gln Pro Pro Glu Pro Gly
1               5                   10                  15

Leu Glu Gly Ala Met Gly His Arg Thr Leu Val Leu Pro Trp Val Leu
            20                  25                  30

Leu Thr Leu Cys Val Thr Ala Gly Thr Pro Glu Val Trp Val Gln Val
        35                  40                  45

Arg Met Glu Ala Thr Glu Leu Ser Ser Phe Thr Ile Arg Cys Gly Phe
    50                  55                  60

Leu Gly Ser Gly Ser Ile Ser Leu Val Thr Val Ser Trp Gly Gly Pro
65                  70                  75                  80

Asn Gly Ala Gly Gly Thr Thr Leu Ala Val Leu His Pro Glu Arg Gly
                85                  90                  95

Ile Arg Gln Trp Ala Pro Ala Arg Gln Ala Arg Trp Glu Thr Gln Ser
            100                 105                 110

Ser Ile Ser Leu Ile Leu Glu Gly Ser Gly Ala Ser Ser Pro Cys Ala
        115                 120                 125

Asn Thr Thr Phe Cys Cys Lys Phe Ala Ser Phe Pro Glu Gly Ser Trp
    130                 135                 140

Glu Ala Cys Gly Ser Leu Pro Pro Ser Ser Asp Pro Gly Leu Ser Ala
145                 150                 155                 160

Pro Pro Thr Pro Ala Pro Ile Leu Arg Ala Asp Leu Ala Gly Ile Leu
                165                 170                 175

Gly Val Ser Gly Val Leu Leu Phe Gly Cys Val Tyr Leu Leu His Leu
            180                 185                 190

Leu Arg Arg His Lys His Arg Pro Ala Pro Arg Leu Gln Pro Ser Arg
        195                 200                 205

Thr Ser Pro Gln Ala Pro Arg Ala Arg Ala Trp Ala Pro Ser Gln Ala
    210                 215                 220

Ser Gln Ala Ala Leu His Val Pro Tyr Ala Thr Ile Asn Thr Ser Cys
225                 230                 235                 240

Arg Pro Ala Thr Leu Asp Thr Ala His Pro His Gly Gly Pro Ser Trp
                245                 250                 255

Trp Ala Ser Leu Pro Thr His Ala Ala His Arg Pro Gln Gly Pro Ala
            260                 265                 270

Ala Trp Ala Ser Thr Pro Ile Pro Ala Arg Gly Ser Phe Val Ser Val
        275                 280                 285

Glu Asn Gly Leu Tyr Ala Gln Ala Gly Glu Arg Pro Pro His Thr Gly
    290                 295                 300

Pro Gly Leu Thr Leu Phe Pro Asp Pro Arg Gly Pro Arg Ala Met Glu
305                 310                 315                 320

Gly Pro Leu Gly Val Arg
                325

<210>  26
<211>  417
<212>  PRT
<213>  Homo sapiens

<400>  26

Met Ala Arg Ala Met Ala Ala Ala Trp Pro Leu Leu Leu Val Ala Leu
1               5                   10                  15

Leu Val Leu Ser Trp Pro Pro Pro Gly Thr Gly Asp Val Val Val Gln
            20                  25                  30

Ala Pro Thr Gln Val Pro Gly Phe Leu Gly Asp Ser Val Thr Leu Pro
        35                  40                  45

Cys Tyr Leu Gln Val Pro Asn Met Glu Val Thr His Val Ser Gln Leu
    50                  55                  60

Thr Trp Ala Arg His Gly Glu Ser Gly Ser Met Ala Val Phe His Gln
65                  70                  75                  80

Thr Gln Gly Pro Ser Tyr Ser Glu Ser Lys Arg Leu Glu Phe Val Ala
                85                  90                  95

Ala Arg Leu Gly Ala Glu Leu Arg Asn Ala Ser Leu Arg Met Phe Gly
            100                 105                 110

Leu Arg Val Glu Asp Glu Gly Asn Tyr Thr Cys Leu Phe Val Thr Phe
        115                 120                 125

Pro Gln Gly Ser Arg Ser Val Asp Ile Trp Leu Arg Val Leu Ala Lys
    130                 135                 140

Pro Gln Asn Thr Ala Glu Val Gln Lys Val Gln Leu Thr Gly Glu Pro
145                 150                 155                 160

Val Pro Met Ala Arg Cys Val Ser Thr Gly Gly Arg Pro Pro Ala Gln
                165                 170                 175

Ile Thr Trp His Ser Asp Leu Gly Gly Met Pro Asn Thr Ser Gln Val
            180                 185                 190

Pro Gly Phe Leu Ser Gly Thr Val Thr Val Thr Ser Leu Trp Ile Leu
        195                 200                 205

Val Pro Ser Ser Gln Val Asp Gly Lys Asn Val Thr Cys Lys Val Glu
    210                 215                 220

His Glu Ser Phe Glu Lys Pro Gln Leu Leu Thr Val Asn Leu Thr Val
225                 230                 235                 240

Tyr Tyr Pro Pro Glu Val Ser Ile Ser Gly Tyr Asp Asn Asn Trp Tyr
                245                 250                 255

Leu Gly Gln Asn Glu Ala Thr Leu Thr Cys Asp Ala Arg Ser Asn Pro
            260                 265                 270

Glu Pro Thr Gly Tyr Asn Trp Ser Thr Thr Met Gly Pro Leu Pro Pro
        275                 280                 285

Phe Ala Val Ala Gln Gly Ala Gln Leu Leu Ile Arg Pro Val Asp Lys
    290                 295                 300

Pro Ile Asn Thr Thr Leu Ile Cys Asn Val Thr Asn Ala Leu Gly Ala
305                 310                 315                 320

Arg Gln Ala Glu Leu Thr Val Gln Val Lys Glu Gly Pro Pro Ser Glu
                325                 330                 335

His Ser Gly Met Ser Arg Asn Ala Ile Ile Phe Leu Val Leu Gly Ile
            340                 345                 350

Leu Val Phe Leu Ile Leu Leu Gly Ile Gly Ile Tyr Phe Tyr Trp Ser
        355                 360                 365

Lys Cys Ser Arg Glu Val Leu Trp His Cys His Leu Cys Pro Ser Ser
    370                 375                 380

Thr Glu His Ala Ser Ala Ser Ala Asn Gly His Val Ser Tyr Ser Ala
385                 390                 395                 400

Val Ser Arg Glu Asn Ser Ser Ser Gln Asp Pro Gln Thr Glu Gly Thr
                405                 410                 415

Arg

<210>  27
<211>  517
<212>  PRT
<213>  Homo sapiens

<400>  27

Met Ala Arg Met Gly Leu Ala Gly Ala Ala Gly Arg Trp Trp Gly Leu
1               5                   10                  15

Ala Leu Gly Leu Thr Ala Phe Phe Leu Pro Gly Val His Ser Gln Val
            20                  25                  30

Val Gln Val Asn Asp Ser Met Tyr Gly Phe Ile Gly Thr Asp Val Val
        35                  40                  45

Leu His Cys Ser Phe Ala Asn Pro Leu Pro Ser Val Lys Ile Thr Gln
    50                  55                  60

Val Thr Trp Gln Lys Ser Thr Asn Gly Ser Lys Gln Asn Val Ala Ile
65                  70                  75                  80

Tyr Asn Pro Ser Met Gly Val Ser Val Leu Ala Pro Tyr Arg Glu Arg
                85                  90                  95

Val Glu Phe Leu Arg Pro Ser Phe Thr Asp Gly Thr Ile Arg Leu Ser
            100                 105                 110

Arg Leu Glu Leu Glu Asp Glu Gly Val Tyr Ile Cys Glu Phe Ala Thr
        115                 120                 125

Phe Pro Thr Gly Asn Arg Glu Ser Gln Leu Asn Leu Thr Val Met Ala
    130                 135                 140

Lys Pro Thr Asn Trp Ile Glu Gly Thr Gln Ala Val Leu Arg Ala Lys
145                 150                 155                 160

Lys Gly Gln Asp Asp Lys Val Leu Val Ala Thr Cys Thr Ser Ala Asn
                165                 170                 175

Gly Lys Pro Pro Ser Val Val Ser Trp Glu Thr Arg Leu Lys Gly Glu
            180                 185                 190

Ala Glu Tyr Gln Glu Ile Arg Asn Pro Asn Gly Thr Val Thr Val Ile
        195                 200                 205

Ser Arg Tyr Arg Leu Val Pro Ser Arg Glu Ala His Gln Gln Ser Leu
    210                 215                 220

Ala Cys Ile Val Asn Tyr His Met Asp Arg Phe Lys Glu Ser Leu Thr
225                 230                 235                 240

Leu Asn Val Gln Tyr Glu Pro Glu Val Thr Ile Glu Gly Phe Asp Gly
                245                 250                 255

Asn Trp Tyr Leu Gln Arg Met Asp Val Lys Leu Thr Cys Lys Ala Asp
            260                 265                 270

Ala Asn Pro Pro Ala Thr Glu Tyr His Trp Thr Thr Leu Asn Gly Ser
        275                 280                 285

Leu Pro Lys Gly Val Glu Ala Gln Asn Arg Thr Leu Phe Phe Lys Gly
    290                 295                 300

Pro Ile Asn Tyr Ser Leu Ala Gly Thr Tyr Ile Cys Glu Ala Thr Asn
305                 310                 315                 320

Pro Ile Gly Thr Arg Ser Gly Gln Val Glu Val Asn Ile Thr Glu Phe
                325                 330                 335

Pro Tyr Thr Pro Ser Pro Pro Glu His Gly Arg Arg Ala Gly Pro Val
            340                 345                 350

Pro Thr Ala Ile Ile Gly Gly Val Ala Gly Ser Ile Leu Leu Val Leu
        355                 360                 365

Ile Val Val Gly Gly Ile Val Val Ala Leu Arg Arg Arg Arg His Thr
    370                 375                 380

Phe Lys Gly Asp Tyr Ser Thr Lys Lys His Val Tyr Gly Asn Gly Tyr
385                 390                 395                 400

Ser Lys Ala Gly Ile Pro Gln His His Pro Pro Met Ala Gln Asn Leu
                405                 410                 415

Gln Tyr Pro Asp Asp Ser Asp Asp Glu Lys Lys Ala Gly Pro Leu Gly

420                 425                 430

Gly Ser Ser Tyr Glu Glu Glu Glu Glu Glu Glu Glu Gly Gly Gly Gly
        435                 440                 445

Gly Glu Arg Lys Val Gly Gly Pro His Pro Lys Tyr Asp Glu Asp Ala
    450                 455                 460

Lys Arg Pro Tyr Phe Thr Val Asp Glu Ala Glu Ala Arg Gln Asp Gly
465                 470                 475                 480

Tyr Gly Asp Arg Thr Leu Gly Tyr Gln Tyr Asp Pro Glu Gln Leu Asp
                485                 490                 495

Leu Ala Glu Asn Met Val Ser Gln Asn Asp Gly Ser Phe Ile Ser Lys
            500                 505                 510

Lys Glu Trp Tyr Val
        515

<210>  28
<211>  538
<212>  PRT
<213>  Homo sapiens

<400>  28

Met Ala Arg Ala Ala Ala Leu Leu Pro Ser Arg Ser Pro Pro Thr Pro
1               5                   10                  15

Leu Leu Trp Pro Leu Leu Leu Leu Leu Leu Leu Glu Thr Gly Ala Gln
            20                  25                  30

Asp Val Arg Val Gln Val Leu Pro Glu Val Arg Gly Gln Leu Gly Gly
        35                  40                  45

Thr Val Glu Leu Pro Cys His Leu Leu Pro Pro Val Pro Gly Leu Tyr
    50                  55                  60

Ile Ser Leu Val Thr Trp Gln Arg Pro Asp Ala Pro Ala Asn His Gln
65                  70                  75                  80

Asn Val Ala Ala Phe His Pro Lys Met Gly Pro Ser Phe Pro Ser Pro
                85                  90                  95

Lys Pro Gly Ser Glu Arg Leu Ser Phe Val Ser Ala Lys Gln Ser Thr
            100                 105                 110

Gly Gln Asp Thr Glu Ala Glu Leu Gln Asp Ala Thr Leu Ala Leu His

115                 120                 125

Gly Leu Thr Val Glu Asp Glu Gly Asn Tyr Thr Cys Glu Phe Ala Thr
    130                 135                 140

Phe Pro Lys Gly Ser Val Arg Gly Met Thr Trp Leu Arg Val Ile Ala
145                 150                 155                 160

Lys Pro Lys Asn Gln Ala Glu Ala Gln Lys Val Thr Phe Ser Gln Asp
                165                 170                 175

Pro Thr Thr Val Ala Leu Cys Ile Ser Lys Glu Gly Arg Pro Pro Ala
            180                 185                 190

Arg Ile Ser Trp Leu Ser Ser Leu Asp Trp Glu Ala Lys Glu Thr Gln
        195                 200                 205

Val Ser Gly Thr Leu Ala Gly Thr Val Thr Val Thr Ser Arg Phe Thr
    210                 215                 220

Leu Val Pro Ser Gly Arg Ala Asp Gly Val Thr Val Thr Cys Lys Val
225                 230                 235                 240

Glu His Glu Ser Phe Glu Glu Pro Ala Leu Ile Pro Val Thr Leu Ser
                245                 250                 255

Val Arg Tyr Pro Pro Glu Val Ser Ile Ser Gly Tyr Asp Asp Asn Trp
            260                 265                 270

Tyr Leu Gly Arg Thr Asp Ala Thr Leu Ser Cys Asp Val Arg Ser Asn
        275                 280                 285

Pro Glu Pro Thr Gly Tyr Asp Trp Ser Thr Thr Ser Gly Thr Phe Pro
    290                 295                 300

Thr Ser Ala Val Ala Gln Gly Ser Gln Leu Val Ile His Ala Val Asp
305                 310                 315                 320

Ser Leu Phe Asn Thr Thr Phe Val Cys Thr Val Thr Asn Ala Val Gly
                325                 330                 335

Met Gly Arg Ala Glu Gln Val Ile Phe Val Arg Glu Thr Pro Asn Thr
            340                 345                 350

Ala Gly Ala Gly Ala Thr Gly Gly Ile Ile Gly Gly Ile Ile Ala Ala
        355                 360                 365

Ile Ile Ala Thr Ala Val Ala Ala Thr Gly Ile Leu Ile Cys Arg Gln
    370                 375                 380

Gln Arg Lys Glu Gln Thr Leu Gln Gly Ala Glu Glu Asp Glu Asp Leu
385                 390                 395                 400

Glu Gly Pro Pro Ser Tyr Lys Pro Pro Thr Pro Lys Ala Lys Leu Glu
                405                 410                 415

Ala Gln Glu Met Pro Ser Gln Leu Phe Thr Leu Gly Ala Ser Glu His
            420                 425                 430

Ser Pro Leu Lys Thr Pro Tyr Phe Asp Ala Gly Ala Ser Cys Thr Glu
        435                 440                 445

Gln Glu Met Pro Arg Tyr His Glu Leu Pro Thr Leu Glu Glu Arg Ser
    450                 455                 460

Gly Pro Leu His Pro Gly Ala Thr Ser Leu Gly Ser Pro Ile Pro Val
465                 470                 475                 480

Pro Pro Gly Pro Pro Ala Val Glu Asp Val Ser Leu Asp Leu Glu Asp
                485                 490                 495

Glu Glu Gly Glu Glu Glu Glu Glu Tyr Leu Asp Lys Ile Asn Pro Ile
            500                 505                 510

Tyr Asp Ala Leu Ser Tyr Ser Ser Pro Ser Asp Ser Tyr Gln Gly Lys
        515                 520                 525

Gly Phe Val Met Ser Arg Ala Met Tyr Val
    530                 535

<210>  29
<211>  549
<212>  PRT
<213>  Homo sapiens

<400>  29

Met Ala Arg Thr Leu Arg Pro Ser Pro Leu Cys Pro Gly Gly Gly Lys
1               5                   10                  15

Ala Gln Leu Ser Ser Ala Ser Leu Leu Gly Ala Gly Leu Leu Leu Gln
            20                  25                  30

Pro Pro Thr Pro Pro Pro Leu Leu Leu Leu Leu Phe Pro Leu Leu Leu
        35                  40                  45

Phe Ser Arg Leu Cys Gly Ala Leu Ala Gly Pro Ile Ile Val Glu Pro
    50                  55                  60

His Val Thr Ala Val Trp Gly Lys Asn Val Ser Leu Lys Cys Leu Ile
65                  70                  75                  80

Glu Val Asn Glu Thr Ile Thr Gln Ile Ser Trp Glu Lys Ile His Gly
                85                  90                  95

Lys Ser Ser Gln Thr Val Ala Val His His Pro Gln Tyr Gly Phe Ser
            100                 105                 110

Val Gln Gly Glu Tyr Gln Gly Arg Val Leu Phe Lys Asn Tyr Ser Leu
        115                 120                 125

Asn Asp Ala Thr Ile Thr Leu His Asn Ile Gly Phe Ser Asp Ser Gly
    130                 135                 140

Lys Tyr Ile Cys Lys Ala Val Thr Phe Pro Leu Gly Asn Ala Gln Ser
145                 150                 155                 160

Ser Thr Thr Val Thr Val Leu Val Glu Pro Thr Val Ser Leu Ile Lys
                165                 170                 175

Gly Pro Asp Ser Leu Ile Asp Gly Gly Asn Glu Thr Val Ala Ala Ile
            180                 185                 190

Cys Ile Ala Ala Thr Gly Lys Pro Val Ala His Ile Asp Trp Glu Gly
        195                 200                 205

Asp Leu Gly Glu Met Glu Ser Thr Thr Thr Ser Phe Pro Asn Glu Thr
    210                 215                 220

Ala Thr Ile Ile Ser Gln Tyr Lys Leu Phe Pro Thr Arg Phe Ala Arg
225                 230                 235                 240

Gly Arg Arg Ile Thr Cys Val Val Lys His Pro Ala Leu Glu Lys Asp
                245                 250                 255

Ile Arg Tyr Ser Phe Ile Leu Asp Ile Gln Tyr Ala Pro Glu Val Ser
            260                 265                 270

Val Thr Gly Tyr Asp Gly Asn Trp Phe Val Gly Arg Lys Gly Val Asn
        275                 280                 285

Leu Lys Cys Asn Ala Asp Ala Asn Pro Pro Pro Phe Lys Ser Val Trp
    290                 295                 300

Ser Arg Leu Asp Gly Gln Trp Pro Asp Gly Leu Leu Ala Ser Asp Asn
305                 310                 315                 320

Thr Leu His Phe Val His Pro Leu Thr Phe Asn Tyr Ser Gly Val Tyr
                325                 330                 335

Ile Cys Lys Val Thr Asn Ser Leu Gly Gln Arg Ser Asp Gln Lys Val
            340                 345                 350

Ile Tyr Ile Ser Asp Pro Pro Thr Thr Thr Thr Leu Gln Pro Thr Ile
        355                 360                 365

Gln Trp His Pro Ser Thr Ala Asp Ile Glu Asp Leu Ala Thr Glu Pro
    370                 375                 380

Lys Lys Leu Pro Phe Pro Leu Ser Thr Leu Ala Thr Ile Lys Asp Asp
385                 390                 395                 400

Thr Ile Ala Thr Ile Ile Ala Ser Val Val Gly Gly Ala Leu Phe Ile
                405                 410                 415

Val Leu Val Ser Val Leu Ala Gly Ile Phe Cys Tyr Arg Arg Arg Arg
            420                 425                 430

Thr Phe Arg Gly Asp Tyr Phe Ala Lys Asn Tyr Ile Pro Pro Ser Asp
        435                 440                 445

Met Gln Lys Glu Ser Gln Ile Asp Val Leu Gln Gln Asp Glu Leu Asp
    450                 455                 460

Ser Tyr Pro Asp Ser Val Lys Lys Glu Asn Lys Asn Pro Val Asn Asn
465                 470                 475                 480

Leu Ile Arg Lys Asp Tyr Leu Glu Glu Pro Glu Lys Thr Gln Trp Asn
                485                 490                 495

Asn Val Glu Asn Leu Asn Arg Phe Glu Arg Pro Met Asp Tyr Tyr Glu
            500                 505                 510

Asp Leu Lys Met Gly Met Lys Phe Val Ser Asp Glu His Tyr Asp Glu
        515                 520                 525

Asn Glu Asp Asp Leu Val Ser His Val Asp Gly Ser Val Ile Ser Arg
    530                 535                 540

Arg Glu Trp Tyr Val
545

<210>  30
<211>  510
<212>  PRT
<213>  Homo sapiens

<400>  30

Met Pro Leu Ser Leu Gly Ala Glu Met Trp Gly Pro Glu Ala Trp Leu
1               5                   10                  15

Leu Leu Leu Leu Leu Leu Ala Ser Phe Thr Gly Arg Cys Pro Ala Gly
            20                  25                  30

Glu Leu Glu Thr Ser Asp Val Val Thr Val Val Leu Gly Gln Asp Ala
        35                  40                  45

Lys Leu Pro Cys Phe Tyr Arg Gly Asp Ser Gly Glu Gln Val Gly Gln
    50                  55                  60

Val Ala Trp Ala Arg Val Asp Ala Gly Glu Gly Ala Gln Glu Leu Ala
65                  70                  75                  80

Leu Leu His Ser Lys Tyr Gly Leu His Val Ser Pro Ala Tyr Glu Gly
                85                  90                  95

Arg Val Glu Gln Pro Pro Pro Pro Arg Asn Pro Leu Asp Gly Ser Val
            100                 105                 110

Leu Leu Arg Asn Ala Val Gln Ala Asp Glu Gly Glu Tyr Glu Cys Arg
        115                 120                 125

Val Ser Thr Phe Pro Ala Gly Ser Phe Gln Ala Arg Leu Arg Leu Arg
    130                 135                 140

Val Leu Val Pro Pro Leu Pro Ser Leu Asn Pro Gly Pro Ala Leu Glu
145                 150                 155                 160

Glu Gly Gln Gly Leu Thr Leu Ala Ala Ser Cys Thr Ala Glu Gly Ser
                165                 170                 175

Pro Ala Pro Ser Val Thr Trp Asp Thr Glu Val Lys Gly Thr Thr Ser
            180                 185                 190

Ser Arg Ser Phe Lys His Ser Arg Ser Ala Ala Val Thr Ser Glu Phe
        195                 200                 205

His Leu Val Pro Ser Arg Ser Met Asn Gly Gln Pro Leu Thr Cys Val
    210                 215                 220

Val Ser His Pro Gly Leu Leu Gln Asp Gln Arg Ile Thr His Ile Leu
225                 230                 235                 240

His Val Ser Phe Leu Ala Glu Ala Ser Val Arg Gly Leu Glu Asp Gln
                245                 250                 255

Asn Leu Trp His Ile Gly Arg Glu Gly Ala Met Leu Lys Cys Leu Ser
            260                 265                 270

Glu Gly Gln Pro Pro Pro Ser Tyr Asn Trp Thr Arg Leu Asp Gly Pro
        275                 280                 285

Leu Pro Ser Gly Val Arg Val Asp Gly Asp Thr Leu Gly Phe Pro Pro
    290                 295                 300

Leu Thr Thr Glu His Ser Gly Ile Tyr Val Cys His Val Ser Asn Glu
305                 310                 315                 320

Phe Ser Ser Arg Asp Ser Gln Val Thr Val Asp Val Leu Asp Pro Gln
                325                 330                 335

Glu Asp Ser Gly Lys Gln Val Asp Leu Val Ser Ala Ser Val Val Val
            340                 345                 350

Val Gly Val Ile Ala Ala Leu Leu Phe Cys Leu Leu Val Val Val Val
        355                 360                 365

Val Leu Met Ser Arg Tyr His Arg Arg Lys Ala Gln Gln Met Thr Gln
    370                 375                 380

Lys Tyr Glu Glu Glu Leu Thr Leu Thr Arg Glu Asn Ser Ile Arg Arg
385                 390                 395                 400

Leu His Ser His His Thr Asp Pro Arg Ser Gln Pro Glu Glu Ser Val
                405                 410                 415

Gly Leu Arg Ala Glu Gly His Pro Asp Ser Leu Lys Asp Asn Ser Ser
            420                 425                 430

Cys Ser Val Met Ser Glu Glu Pro Glu Gly Arg Ser Tyr Ser Thr Leu
        435                 440                 445

Thr Thr Val Arg Glu Ile Glu Thr Gln Thr Glu Leu Leu Ser Pro Gly
    450                 455                 460

Ser Gly Arg Ala Glu Glu Glu Glu Asp Gln Asp Glu Gly Ile Lys Gln

465                 470                 475                 480

Ala Met Asn His Phe Val Gln Glu Asn Gly Thr Leu Arg Ala Lys Pro
                485                 490                 495

Thr Gly Asn Gly Ile Tyr Ile Asn Gly Arg Gly His Leu Val
            500                 505                 510

<210>  31
<211>  244
<212>  PRT
<213>  Homo sapiens

<400>  31

Met Arg Trp Cys Leu Leu Leu Ile Trp Ala Gln Gly Leu Arg Gln Ala
1               5                   10                  15

Pro Leu Ala Ser Gly Met Met Thr Gly Thr Ile Glu Thr Thr Gly Asn
            20                  25                  30

Ile Ser Ala Glu Lys Gly Gly Ser Ile Ile Leu Gln Cys His Leu Ser
        35                  40                  45

Ser Thr Thr Ala Gln Val Thr Gln Val Asn Trp Glu Gln Gln Asp Gln
    50                  55                  60

Leu Leu Ala Ile Cys Asn Ala Asp Leu Gly Trp His Ile Ser Pro Ser
65                  70                  75                  80

Phe Lys Asp Arg Val Ala Pro Gly Pro Gly Leu Gly Leu Thr Leu Gln
                85                  90                  95

Ser Leu Thr Val Asn Asp Thr Gly Glu Tyr Phe Cys Ile Tyr His Thr
            100                 105                 110

Tyr Pro Asp Gly Thr Tyr Thr Gly Arg Ile Phe Leu Glu Val Leu Glu
        115                 120                 125

Ser Ser Val Ala Glu His Gly Ala Arg Phe Gln Ile Pro Leu Leu Gly
    130                 135                 140

Ala Met Ala Ala Thr Leu Val Val Ile Cys Thr Ala Val Ile Val Val
145                 150                 155                 160

Val Ala Leu Thr Arg Lys Lys Lys Ala Leu Arg Ile His Ser Val Glu
                165                 170                 175

Gly Asp Leu Arg Arg Lys Ser Ala Gly Gln Glu Glu Trp Ser Pro Ser

180                 185                 190

Ala Pro Ser Pro Pro Gly Ser Cys Val Gln Ala Glu Ala Ala Pro Ala
        195                 200                 205

Gly Leu Cys Gly Glu Gln Arg Gly Glu Asp Cys Ala Glu Leu His Asp
    210                 215                 220

Tyr Phe Asn Val Leu Ser Tyr Arg Ser Leu Gly Asn Cys Ser Phe Phe
225                 230                 235                 240

Thr Glu Thr Gly

<210>  32
<211>  366
<212>  PRT
<213>  Homo sapiens

<400>  32

Met Ala Leu Pro Pro Gly Pro Ala Ala Leu Arg His Thr Leu Leu Leu
1               5                   10                  15

Leu Pro Ala Leu Leu Ser Ser Gly Trp Gly Glu Leu Glu Pro Gln Ile
            20                  25                  30

Asp Gly Gln Thr Trp Ala Glu Arg Ala Leu Arg Glu Asn Glu Arg His
        35                  40                  45

Ala Phe Thr Cys Arg Val Ala Gly Gly Pro Gly Thr Pro Arg Leu Ala
    50                  55                  60

Trp Tyr Leu Asp Gly Gln Leu Gln Glu Ala Ser Thr Ser Arg Leu Leu
65                  70                  75                  80

Ser Val Gly Gly Glu Ala Phe Ser Gly Gly Thr Ser Thr Phe Thr Val
                85                  90                  95

Thr Ala His Arg Ala Gln His Glu Leu Asn Cys Ser Leu Gln Asp Pro
            100                 105                 110

Arg Ser Gly Arg Ser Ala Asn Ala Ser Val Ile Leu Asn Val Gln Phe
        115                 120                 125

Lys Pro Glu Ile Ala Gln Val Gly Ala Lys Tyr Gln Glu Ala Gln Gly
    130                 135                 140

Pro Gly Leu Leu Val Val Leu Phe Ala Leu Val Arg Ala Asn Pro Pro

145                 150                 155                 160

Ala Asn Val Thr Trp Ile Asp Gln Asp Gly Pro Val Thr Val Asn Thr
                165                 170                 175

Ser Asp Phe Leu Val Leu Asp Ala Gln Asn Tyr Pro Trp Leu Thr Asn
            180                 185                 190

His Thr Val Gln Leu Gln Leu Arg Ser Leu Ala His Asn Leu Ser Val
        195                 200                 205

Val Ala Thr Asn Asp Val Gly Val Thr Ser Ala Ser Leu Pro Ala Pro
    210                 215                 220

Gly Leu Leu Ala Thr Arg Val Glu Val Pro Leu Leu Gly Ile Val Val
225                 230                 235                 240

Ala Ala Gly Leu Ala Leu Gly Thr Leu Val Gly Phe Ser Thr Leu Val
                245                 250                 255

Ala Cys Leu Val Cys Arg Lys Glu Lys Lys Thr Lys Gly Pro Ser Arg
            260                 265                 270

His Pro Ser Leu Ile Ser Ser Asp Ser Asn Asn Leu Lys Leu Asn Asn
        275                 280                 285

Val Arg Leu Pro Arg Glu Asn Met Ser Leu Pro Ser Asn Leu Gln Leu
    290                 295                 300

Asn Asp Leu Thr Pro Asp Ser Arg Ala Val Lys Pro Ala Asp Arg Gln
305                 310                 315                 320

Met Ala Gln Asn Asn Ser Arg Pro Glu Leu Leu Asp Pro Glu Pro Gly
                325                 330                 335

Gly Leu Leu Thr Ser Gln Gly Phe Ile Arg Leu Pro Val Leu Gly Tyr
            340                 345                 350

Ile Tyr Arg Val Ser Ser Val Ser Ser Asp Glu Ile Trp Leu
        355                 360                 365

<210>  33
<211>  257
<212>  PRT
<213>  Homo sapiens

<400>  33

Leu Asn Asp Leu Asn Val Ser Pro Pro Glu Leu Thr Val His Val Gly

1                   5                   10                  15

Asp Ser Ala Leu Met Gly Cys Val Phe Gln Ser Thr Glu Asp Lys Cys
            20                  25                  30

Ile Phe Lys Ile Asp Trp Thr Leu Ser Pro Gly Glu His Ala Lys Asp
        35                  40                  45

Glu Tyr Val Leu Tyr Tyr Tyr Ser Asn Leu Ser Val Pro Ile Gly Arg
    50                  55                  60

Phe Gln Asn Arg Val His Leu Met Gly Asp Ile Leu Cys Asn Asp Gly
65                  70                  75                  80

Ser Leu Leu Leu Gln Asp Val Gln Glu Ala Asp Gln Gly Thr Tyr Ile
                85                  90                  95

Cys Glu Ile Arg Leu Lys Gly Glu Ser Gln Val Phe Lys Lys Ala Val
            100                 105                 110

Val Leu His Val Leu Pro Glu Glu Pro Lys Glu Leu Met Val His Val
        115                 120                 125

Gly Gly Leu Ile Gln Met Gly Cys Val Phe Gln Ser Thr Glu Val Lys
    130                 135                 140

His Val Thr Lys Val Glu Trp Ile Phe Ser Gly Arg Arg Ala Lys Glu
145                 150                 155                 160

Glu Ile Val Phe Arg Tyr Tyr His Lys Leu Arg Met Ser Val Glu Tyr
                165                 170                 175

Ser Gln Ser Trp Gly His Phe Gln Asn Arg Val Asn Leu Val Gly Asp
            180                 185                 190

Ile Phe Arg Asn Asp Gly Ser Ile Met Leu Gln Gly Val Arg Glu Ser
        195                 200                 205

Asp Gly Gly Asn Tyr Thr Cys Ser Ile His Leu Gly Asn Leu Val Phe
    210                 215                 220

Lys Lys Thr Ile Val Leu His Val Ser Pro Glu Glu Pro Arg Thr Leu
225                 230                 235                 240

Val Thr Pro Ala Ala Leu Arg Pro Leu Val Leu Gly Gly Asn Gln Leu
                245                 250                 255

Val

<210>  34
<211>  218
<212>  PRT
<213>  Homo sapiens

<400>  34

<210>  35
<211>  217
<212>  PRT
<213>  Homo sapiens

<400>  35

Thr His Thr Glu Ile Lys Arg Val Ala Glu Glu Lys Val Thr Leu Pro
1               5                   10                  15

Cys His His Gln Leu Gly Leu Pro Glu Lys Asp Thr Leu Asp Ile Glu
            20                  25                  30

Trp Leu Leu Thr Asp Asn Glu Gly Asn Gln Lys Val Val Ile Thr Tyr
        35                  40                  45

Ser Ser Arg His Val Tyr Asn Asn Leu Thr Glu Glu Gln Lys Gly Arg
    50                  55                  60

Val Ala Phe Ala Ser Asn Phe Leu Ala Gly Asp Ala Ser Leu Gln Ile
65                  70                  75                  80

Glu Pro Leu Lys Pro Ser Asp Glu Gly Arg Tyr Thr Cys Lys Val Lys
                85                  90                  95

Asn Ser Gly Arg Tyr Val Trp Ser His Val Ile Leu Lys Val Leu Val
            100                 105                 110

Arg Pro Ser Lys Pro Lys Cys Glu Leu Glu Gly Glu Leu Thr Glu Gly
        115                 120                 125

Ser Asp Leu Thr Leu Gln Cys Glu Ser Ser Ser Gly Thr Glu Pro Ile
    130                 135                 140

Val Tyr Tyr Trp Gln Arg Ile Arg Glu Lys Glu Gly Glu Asp Glu Arg
145                 150                 155                 160

Leu Pro Pro Lys Ser Arg Ile Asp Tyr Asn His Pro Gly Arg Val Leu
                165                 170                 175

Leu Gln Asn Leu Thr Met Ser Tyr Ser Gly Leu Tyr Gln Cys Thr Ala
            180                 185                 190

Gly Asn Glu Ala Gly Lys Glu Ser Cys Val Val Arg Val Thr Val Gln
        195                 200                 205

Tyr Val Gln Ser Ile Gly Met Val Ala
    210                 215

<210>  36
<211>  219
<212>  PRT
<213>  Homo sapiens

<400>  36

Gln Leu Gln Leu His Leu Pro Ala Asn Arg Leu Gln Ala Val Glu Gly
1               5                   10                  15

Gly Glu Val Val Leu Pro Ala Trp Tyr Thr Leu His Gly Glu Val Ser
            20                  25                  30

Ser Ser Gln Pro Trp Glu Val Pro Phe Val Met Trp Phe Phe Lys Gln
        35                  40                  45

Lys Glu Lys Glu Asp Gln Val Leu Ser Tyr Ile Asn Gly Val Thr Thr
    50                  55                  60

Ser Lys Pro Gly Val Ser Leu Val Tyr Ser Met Pro Ser Arg Asn Leu
65                  70                  75                  80

Ser Leu Arg Leu Glu Gly Leu Gln Glu Lys Asp Ser Gly Pro Tyr Ser
                85                  90                  95

Cys Ser Val Asn Val Gln Asp Lys Gln Gly Lys Ser Arg Gly His Ser
            100                 105                 110

Ile Lys Thr Leu Glu Leu Asn Val Leu Val Pro Pro Ala Pro Pro Ser
        115                 120                 125

Cys Arg Leu Gln Gly Val Pro His Val Gly Ala Asn Val Thr Leu Ser
    130                 135                 140

Cys Gln Ser Pro Arg Ser Lys Pro Ala Val Gln Tyr Gln Trp Asp Arg
145                 150                 155                 160

Gln Leu Pro Ser Phe Gln Thr Phe Phe Ala Pro Ala Leu Asp Val Ile
                165                 170                 175

Arg Gly Ser Leu Ser Leu Thr Asn Leu Ser Ser Ser Met Ala Gly Val
            180                 185                 190

Tyr Val Cys Lys Ala His Asn Glu Val Gly Thr Ala Gln Cys Asn Val
        195                 200                 205

Thr Leu Glu Val Ser Thr Gly Pro Gly Ala Ala
    210                 215

<210>  37
<211>  214
<212>  PRT
<213>  Homo sapiens

<400>  37

Ile Ser Val Glu Thr Pro Gln Asp Val Leu Arg Ala Ser Gln Gly Lys
1               5                   10                  15

Ser Val Thr Leu Pro Cys Thr Tyr His Thr Ser Thr Ser Ser Arg Glu
            20                  25                  30

Gly Leu Ile Gln Trp Asp Lys Leu Leu Leu Thr His Thr Glu Arg Val
        35                  40                  45

Val Ile Trp Pro Phe Ser Asn Lys Asn Tyr Ile His Gly Glu Leu Tyr
    50                  55                  60

Lys Asn Arg Val Ser Ile Ser Asn Asn Ala Glu Gln Ser Asp Ala Ser
65                  70                  75                  80

Ile Thr Ile Asp Gln Leu Thr Met Ala Asp Asn Gly Thr Tyr Glu Cys
                85                  90                  95

Ser Val Ser Leu Met Ser Asp Leu Glu Gly Asn Thr Lys Ser Arg Val
            100                 105                 110

Arg Leu Leu Val Leu Val Pro Pro Ser Lys Pro Glu Cys Gly Ile Glu
        115                 120                 125

Gly Glu Thr Ile Ile Gly Asn Asn Ile Gln Leu Thr Cys Gln Ser Lys
    130                 135                 140

Glu Gly Ser Pro Thr Pro Gln Tyr Ser Trp Lys Arg Tyr Asn Ile Leu
145                 150                 155                 160

Asn Gln Glu Gln Pro Leu Ala Gln Pro Ala Ser Gly Gln Pro Val Ser
                165                 170                 175

Leu Lys Asn Ile Ser Thr Asp Thr Ser Gly Tyr Tyr Ile Cys Thr Ser
            180                 185                 190

Ser Asn Glu Glu Gly Thr Gln Phe Cys Asn Ile Thr Val Ala Val Arg
        195                 200                 205

Ser Pro Ser Met Asn Val
    210

<210>  38
<211>  211
<212>  PRT
<213>  Homo sapiens

<400>  38

Ser Val Thr Val His Ser Ser Glu Pro Glu Val Arg Ile Pro Glu Asn
1               5                   10                  15

Asn Pro Val Lys Leu Ser Cys Ala Tyr Ser Gly Phe Ser Ser Pro Arg
            20                  25                  30

Val Glu Trp Lys Phe Asp Gln Gly Asp Thr Thr Arg Leu Val Cys Tyr
        35                  40                  45

Asn Asn Lys Ile Thr Ala Ser Tyr Glu Asp Arg Val Thr Phe Leu Pro
    50                  55                  60

Thr Gly Ile Thr Phe Lys Ser Val Thr Arg Glu Asp Thr Gly Thr Tyr
65                  70                  75                  80

Thr Cys Met Val Ser Glu Glu Gly Gly Asn Ser Tyr Gly Glu Val Lys
                85                  90                  95

Val Lys Leu Ile Val Leu Val Pro Pro Ser Lys Pro Thr Val Asn Ile
            100                 105                 110

Pro Ser Ser Ala Thr Ile Gly Asn Arg Ala Val Leu Thr Cys Ser Glu
        115                 120                 125

Gln Asp Gly Ser Pro Pro Ser Glu Tyr Thr Trp Phe Lys Asp Gly Ile
    130                 135                 140

Val Met Pro Thr Asn Pro Lys Ser Thr Arg Ala Phe Ser Asn Ser Ser
145                 150                 155                 160

Tyr Val Leu Asn Pro Thr Thr Gly Glu Leu Val Phe Asp Pro Leu Ser
                165                 170                 175

Ala Ser Asp Thr Gly Glu Tyr Ser Cys Glu Ala Arg Asn Gly Tyr Gly
            180                 185                 190

Thr Pro Met Thr Ser Asn Ala Val Arg Met Glu Ala Val Glu Arg Asn
        195                 200                 205

Val Gly Val
    210

<210>  39
<211>  210
<212>  PRT
<213>  Homo sapiens

<400>  39

Phe Ser Ala Pro Lys Asp Gln Gln Val Val Thr Ala Val Glu Tyr Gln
1               5                   10                  15

Glu Ala Ile Leu Ala Cys Lys Thr Pro Lys Lys Thr Val Ser Ser Arg
            20                  25                  30

Leu Glu Trp Lys Lys Leu Gly Arg Ser Val Ser Phe Val Tyr Tyr Gln
        35                  40                  45

Gln Thr Leu Gln Gly Asp Phe Lys Asn Arg Ala Glu Met Ile Asp Phe
    50                  55                  60

Asn Ile Arg Ile Lys Asn Val Thr Arg Ser Asp Ala Gly Lys Tyr Arg
65                  70                  75                  80

Cys Glu Val Ser Ala Pro Ser Glu Gln Gly Gln Asn Leu Glu Glu Asp
                85                  90                  95

Thr Val Thr Leu Glu Val Leu Val Ala Pro Ala Val Pro Ser Cys Glu
            100                 105                 110

Val Pro Ser Ser Ala Leu Ser Gly Thr Val Val Glu Leu Arg Cys Gln
        115                 120                 125

Asp Lys Glu Gly Asn Pro Ala Pro Glu Tyr Thr Trp Phe Lys Asp Gly
    130                 135                 140

Ile Arg Leu Leu Glu Asn Pro Arg Leu Gly Ser Gln Ser Thr Asn Ser
145                 150                 155                 160

Ser Tyr Thr Met Asn Thr Lys Thr Gly Thr Leu Gln Phe Asn Thr Val
                165                 170                 175

Ser Lys Leu Asp Thr Gly Glu Tyr Ser Cys Glu Ala Arg Asn Ser Val
            180                 185                 190

Gly Tyr Arg Arg Cys Pro Gly Lys Arg Met Gln Val Asp Asp Leu Asn
        195                 200                 205

Ile Ser
    210

<210>  40
<211>  210
<212>  PRT
<213>  Homo sapiens

<400>  40

Val Asn Leu Lys Ser Ser Asn Arg Thr Pro Val Val Gln Glu Phe Glu
1               5                   10                  15

Ser Val Glu Leu Ser Cys Ile Ile Thr Asp Ser Gln Thr Ser Asp Pro
            20                  25                  30

Arg Ile Glu Trp Lys Lys Ile Gln Asp Glu Gln Thr Thr Tyr Val Phe
        35                  40                  45

Phe Asp Asn Lys Ile Gln Gly Asp Leu Ala Gly Arg Ala Glu Ile Leu
    50                  55                  60

Gly Lys Thr Ser Leu Lys Ile Trp Asn Val Thr Arg Arg Asp Ser Ala
65                  70                  75                  80

Leu Tyr Arg Cys Glu Val Val Ala Arg Asn Asp Arg Lys Glu Ile Asp
                85                  90                  95

Glu Ile Val Ile Glu Leu Thr Val Gln Val Lys Pro Val Thr Pro Val
            100                 105                 110

Cys Arg Val Pro Lys Ala Val Pro Val Gly Lys Met Ala Thr Leu His
        115                 120                 125

Cys Gln Glu Ser Glu Gly His Pro Arg Pro His Tyr Ser Trp Tyr Arg
    130                 135                 140

Asn Asp Val Pro Leu Pro Thr Asp Ser Arg Ala Asn Pro Arg Phe Arg
145                 150                 155                 160

Asn Ser Ser Phe His Leu Asn Ser Glu Thr Gly Thr Leu Val Phe Thr
                165                 170                 175

Ala Val His Lys Asp Asp Ser Gly Gln Tyr Tyr Cys Ile Ala Ser Asn
            180                 185                 190

Asp Ala Gly Ser Ala Arg Cys Glu Glu Gln Glu Met Glu Val Tyr Asp
        195                 200                 205

Leu Asn
    210

<210>  41
<211>  215
<212>  PRT
<213>  Homo sapiens

<400>  41

Gln Val Ser Val Val Gln Val Thr Ile Pro Asp Gly Phe Val Asn Val
1               5                   10                  15

Thr Val Gly Ser Asn Val Thr Leu Ile Cys Ile Tyr Thr Thr Thr Val
            20                  25                  30

Ala Ser Arg Glu Gln Leu Ser Ile Gln Trp Ser Phe Phe His Lys Lys
        35                  40                  45

Glu Met Glu Pro Ile Ser His Ser Ser Cys Leu Ser Thr Glu Gly Met
    50                  55                  60

Glu Glu Lys Ala Val Ser Gln Cys Leu Lys Met Thr His Ala Arg Asp
65                  70                  75                  80

Ala Arg Gly Arg Cys Ser Trp Thr Ser Glu Ile Tyr Phe Ser Gln Gly
                85                  90                  95

Gly Gln Ala Val Ala Ile Gly Gln Phe Lys Asp Arg Ile Thr Gly Ser
            100                 105                 110

Asn Asp Pro Gly Asn Ala Ser Ile Thr Ile Ser His Met Gln Pro Ala
        115                 120                 125

Asp Ser Gly Ile Tyr Ile Cys Asp Val Asn Asn Pro Pro Asp Phe Leu
    130                 135                 140

Gly Gln Asn Gln Gly Ile Leu Asn Val Ser Val Leu Val Lys Pro Ser
145                 150                 155                 160

Lys Pro Leu Cys Ser Val Gln Gly Arg Pro Glu Thr Gly His Thr Ile
                165                 170                 175

Ser Leu Ser Cys Leu Ser Ala Leu Gly Thr Pro Ser Pro Val Tyr Tyr
            180                 185                 190

Trp His Lys Leu Glu Gly Arg Asp Ile Val Pro Val Lys Glu Asn Phe
        195                 200                 205

Asn Pro Thr Thr Gly Ile Leu
    210                 215

<210>  42
<211>  220
<212>  PRT
<213>  Homo sapiens

<400>  42

Val Glu Val Lys Val Pro Thr Glu Pro Leu Ser Thr Pro Leu Gly Lys
1               5                   10                  15

Thr Ala Glu Leu Thr Cys Thr Tyr Ser Thr Ser Val Gly Asp Ser Phe
            20                  25                  30

Ala Leu Glu Trp Ser Phe Val Gln Pro Gly Lys Pro Ile Ser Glu Ser
        35                  40                  45

His Pro Ile Leu Tyr Phe Thr Asn Gly His Leu Tyr Pro Thr Gly Ser
    50                  55                  60

Lys Ser Lys Arg Val Ser Leu Leu Gln Asn Pro Pro Thr Val Gly Val
65                  70                  75                  80

Ala Thr Leu Lys Leu Thr Asp Val His Pro Ser Asp Thr Gly Thr Tyr
                85                  90                  95

Leu Cys Gln Val Asn Asn Pro Pro Asp Phe Tyr Thr Asn Gly Leu Gly
            100                 105                 110

Leu Ile Asn Leu Thr Val Leu Val Pro Pro Ser Asn Pro Leu Cys Ser
        115                 120                 125

Gln Ser Gly Gln Thr Ser Val Gly Gly Ser Thr Ala Leu Arg Cys Ser
    130                 135                 140

Ser Ser Glu Gly Ala Pro Lys Pro Val Tyr Asn Trp Val Arg Leu Gly
145                 150                 155                 160

Thr Phe Pro Thr Pro Ser Pro Gly Ser Met Val Gln Asp Glu Val Ser
                165                 170                 175

Gly Gln Leu Ile Leu Thr Asn Leu Ser Leu Thr Ser Ser Gly Thr Tyr
            180                 185                 190

Arg Cys Val Ala Thr Asn Gln Met Gly Ser Ala Ser Cys Glu Leu Thr
        195                 200                 205

Leu Ser Val Thr Glu Pro Ser Gln Gly Arg Val Ala
    210                 215                 220

<210>  43
<211>  222
<212>  PRT
<213>  Homo sapiens

<400>  43

Ala Ser Leu Glu Val Ser Glu Ser Pro Gly Ser Ile Gln Val Ala Arg
1               5                   10                  15

Gly Gln Pro Ala Val Leu Pro Cys Thr Phe Thr Thr Ser Ala Ala Leu
            20                  25                  30

Ile Asn Leu Asn Val Ile Trp Met Val Thr Pro Leu Ser Asn Ala Asn
        35                  40                  45

Gln Pro Glu Gln Val Ile Leu Tyr Gln Gly Gly Gln Met Phe Asp Gly
    50                  55                  60

Ala Pro Arg Phe His Gly Arg Val Gly Phe Thr Gly Thr Met Pro Ala
65                  70                  75                  80

Thr Asn Val Ser Ile Phe Ile Asn Asn Thr Gln Leu Ser Asp Thr Gly
                85                  90                  95

Thr Tyr Gln Cys Leu Val Asn Asn Leu Pro Asp Ile Gly Gly Arg Asn
            100                 105                 110

Ile Gly Val Thr Gly Leu Thr Val Leu Val Pro Pro Ser Ala Pro His
        115                 120                 125

Cys Gln Ile Gln Gly Ser Gln Asp Ile Gly Ser Asp Val Ile Leu Leu
    130                 135                 140

Cys Ser Ser Glu Glu Gly Ile Pro Arg Pro Thr Tyr Leu Trp Glu Lys
145                 150                 155                 160

Leu Asp Asn Thr Leu Lys Leu Pro Pro Thr Ala Thr Gln Asp Gln Val
                165                 170                 175

Gln Gly Thr Val Thr Ile Arg Asn Ile Ser Ala Leu Ser Ser Gly Leu
            180                 185                 190

Tyr Gln Cys Val Ala Ser Asn Ala Ile Gly Thr Ser Thr Cys Leu Leu
        195                 200                 205

Asp Leu Gln Val Ile Ser Pro Gln Pro Arg Asn Ile Gly Leu
    210                 215                 220

<210>  44
<211>  264
<212>  PRT
<213>  Homo sapiens

<400>  44

Arg Pro Ile Leu Glu Val Pro Glu Ser Val Thr Gly Pro Trp Lys Gly
1               5                   10                  15

Asp Val Asn Leu Pro Cys Thr Tyr Asp Pro Leu Gln Gly Tyr Thr Gln
            20                  25                  30

Val Leu Val Lys Trp Leu Val Gln Arg Gly Ser Asp Pro Val Thr Ile
        35                  40                  45

Phe Leu Arg Asp Ser Ser Gly Asp His Ile Gln Gln Ala Lys Tyr Gln
    50                  55                  60

Gly Arg Leu His Val Ser His Lys Val Pro Gly Asp Val Ser Leu Gln
65                  70                  75                  80

Leu Ser Thr Leu Glu Met Asp Asp Arg Ser His Tyr Thr Cys Glu Val
                85                  90                  95

Thr Trp Gln Thr Pro Asp Gly Asn Gln Val Val Arg Asp Lys Ile Thr
            100                 105                 110

Glu Leu Arg Val Gln Lys Leu Ser Val Ser Lys Pro Thr Val Thr Thr
        115                 120                 125

Gly Ser Gly Tyr Gly Phe Thr Val Pro Gln Gly Met Arg Ile Ser Leu
    130                 135                 140

Gln Cys Gln Ala Arg Gly Ser Pro Pro Ile Ser Tyr Ile Trp Tyr Lys
145                 150                 155                 160

Gln Gln Thr Asn Asn Gln Glu Pro Ile Lys Val Ala Thr Leu Ser Thr
                165                 170                 175

Leu Leu Phe Lys Pro Ala Val Ile Ala Asp Ser Gly Ser Tyr Phe Cys
            180                 185                 190

Thr Ala Lys Gly Gln Val Gly Ser Glu Gln His Ser Asp Ile Val Lys
        195                 200                 205

Phe Val Val Lys Asp Ser Ser Lys Leu Leu Lys Thr Lys Thr Glu Ala
    210                 215                 220

Pro Thr Thr Met Thr Tyr Pro Leu Lys Ala Thr Ser Thr Val Lys Gln
225                 230                 235                 240

Ser Trp Asp Trp Thr Thr Asp Met Asp Gly Tyr Leu Gly Glu Thr Ser
                245                 250                 255

Ala Gly Pro Gly Lys Ser Leu Pro
            260

<210>  45
<211>  242
<212>  PRT
<213>  Homo sapiens

<400>  45

Val Arg Ile Asn Gly Asp Gly Gln Glu Val Leu Tyr Leu Ala Glu Gly
1               5                   10                  15

Asp Asn Val Arg Leu Gly Cys Pro Tyr Val Leu Asp Pro Glu Asp Tyr
            20                  25                  30

Gly Pro Asn Gly Leu Asp Ile Glu Trp Met Gln Val Asn Ser Asp Pro
        35                  40                  45

Ala His His Arg Glu Asn Val Phe Leu Ser Tyr Gln Asp Lys Arg Ile
    50                  55                  60

Asn His Gly Ser Leu Pro His Leu Gln Gln Arg Val Arg Phe Ala Ala
65                  70                  75                  80

Ser Asp Pro Ser Gln Tyr Asp Ala Ser Ile Asn Leu Met Asn Leu Gln
                85                  90                  95

Val Ser Asp Thr Ala Thr Tyr Glu Cys Arg Val Lys Lys Thr Thr Met
            100                 105                 110

Ala Thr Arg Lys Val Ile Val Thr Val Gln Ala Arg Pro Ala Val Pro
        115                 120                 125

Met Cys Trp Thr Glu Gly His Met Thr Tyr Gly Asn Asp Val Val Leu
    130                 135                 140

Lys Cys Tyr Ala Ser Gly Gly Ser Gln Pro Leu Ser Tyr Lys Trp Ala
145                 150                 155                 160

Lys Ile Ser Gly His His Tyr Pro Tyr Arg Ala Gly Ser Tyr Thr Ser
                165                 170                 175

Gln His Ser Tyr His Ser Glu Leu Ser Tyr Gln Glu Ser Phe His Ser
            180                 185                 190

Ser Ile Asn Gln Gly Leu Asn Asn Gly Asp Leu Val Leu Lys Asp Ile
        195                 200                 205

Ser Arg Ala Asp Asp Gly Leu Tyr Gln Cys Thr Val Ala Asn Asn Val
    210                 215                 220

Gly Tyr Ser Val Cys Val Val Glu Val Lys Val Ser Asp Ser Arg Arg
225                 230                 235                 240

Ile Gly

<210> 46
<211> 330
<212> PRT
<213> Homo sapiens

<400> 46

Gln Asn Leu Phe Thr Lys Asp Val Thr Val Ile Glu Gly Glu Val Ala
1               5                   10                  15

Thr Ile Ser Cys Gln Val Asn Lys Ser Asp Asp Ser Val Ile Gln Leu
            20                  25                  30

Leu Asn Pro Asn Arg Gln Thr Ile Tyr Phe Arg Asp Phe Arg Pro Leu
        35                  40                  45

Lys Asp Ser Arg Phe Gln Leu Leu Asn Phe Ser Ser Ser Glu Leu Lys
    50                  55                  60

Val Ser Leu Thr Asn Val Ser Ile Ser Asp Glu Gly Arg Tyr Phe Cys
65                  70                  75                  80

Gln Leu Tyr Thr Asp Pro Pro Gln Glu Ser Tyr Thr Thr Ile Thr Val
                85                  90                  95

Leu Val Pro Pro Arg Asn Leu Met Ile Asp Ile Gln Lys Asp Thr Ala
            100                 105                 110

Val Glu Gly Glu Glu Ile Glu Val Asn Cys Thr Ala Met Ala Ser Lys
        115                 120                 125

Pro Ala Thr Thr Ile Arg Trp Phe Lys Gly Asn Thr Glu Leu Lys Gly
    130                 135                 140

Lys Ser Glu Val Glu Glu Trp Ser Asp Met Tyr Thr Val Thr Ser Gln
145                 150                 155                 160

Leu Met Leu Lys Val His Lys Glu Asp Asp Gly Val Pro Val Ile Cys
                165                 170                 175

Gln Val Glu His Pro Ala Val Thr Gly Asn Leu Gln Thr Gln Arg Tyr
            180                 185                 190

Leu Glu Val Gln Tyr Lys Pro Gln Val His Ile Gln Met Thr Tyr Pro
        195                 200                 205

Leu Gln Gly Leu Thr Arg Glu Gly Asp Ala Leu Glu Leu Thr Cys Glu
    210                 215                 220

Ala Ile Gly Lys Pro Gln Pro Val Met Val Thr Trp Val Arg Val Asp
225                 230                 235                 240

Asp Glu Met Pro Gln His Ala Val Leu Ser Gly Pro Asn Leu Phe Ile
                245                 250                 255

Asn Asn Leu Asn Lys Thr Asp Asn Gly Thr Tyr Arg Cys Glu Ala Ser
            260                 265                 270

Asn Ile Val Gly Lys Ala His Ser Asp Tyr Met Leu Tyr Val Tyr Asp
        275                 280                 285

Pro Pro Thr Thr Ile Pro Pro Pro Thr Thr Thr Thr Thr Thr Thr Thr
    290                 295                 300

Thr Thr Thr Thr Thr Ile Leu Thr Ile Ile Thr Asp Ser Arg Ala Gly
305                 310                 315                 320

Glu Glu Gly Ser Ile Arg Ala Val Asp His
                325                 330

<210>  47
<211>  343
<212>  PRT
<213>  Homo sapiens

<400>  47

Gln Phe Pro Leu Thr Gln Asn Val Thr Val Val Glu Gly Gly Thr Ala
1               5                   10                  15

Ile Leu Thr Cys Arg Val Asp Gln Asn Asp Asn Thr Ser Leu Gln Trp
            20                  25                  30

Ser Asn Pro Ala Gln Gln Thr Leu Tyr Phe Asp Asp Lys Lys Ala Leu
        35                  40                  45

Arg Asp Asn Arg Ile Glu Leu Val Arg Ala Ser Trp His Glu Leu Ser
    50                  55                  60

Ile Ser Val Ser Asp Val Ser Leu Ser Asp Glu Gly Gln Tyr Thr Cys
65                  70                  75                  80

Ser Leu Phe Thr Met Pro Val Lys Thr Ser Lys Ala Tyr Leu Thr Val
                85                  90                  95

Leu Gly Val Pro Glu Lys Pro Gln Ile Ser Gly Phe Ser Ser Pro Val
            100                 105                 110

Met Glu Gly Asp Leu Met Gln Leu Thr Cys Lys Thr Ser Gly Ser Lys
        115                 120                 125

Pro Ala Ala Asp Ile Arg Trp Phe Lys Asn Asp Lys Glu Ile Lys Asp
    130                 135                 140

Val Lys Tyr Leu Lys Glu Glu Asp Ala Asn Arg Lys Thr Phe Thr Val
145                 150                 155                 160

Ser Ser Thr Leu Asp Phe Arg Val Asp Arg Ser Asp Asp Gly Val Ala
                165                 170                 175

Val Ile Cys Arg Val Asp His Glu Ser Leu Asn Ala Thr Pro Gln Val
            180                 185                 190

Ala Met Gln Val Leu Glu Ile His Tyr Thr Pro Ser Val Lys Ile Ile
        195                 200                 205

Pro Ser Thr Pro Phe Pro Gln Glu Gly Gln Pro Leu Ile Leu Thr Cys
    210                 215                 220

Glu Ser Lys Gly Lys Pro Leu Pro Glu Pro Val Leu Trp Thr Lys Asp
225                 230                 235                 240

Gly Gly Glu Leu Pro Asp Pro Asp Arg Met Val Val Ser Gly Arg Glu
                245                 250                 255

Leu Asn Ile Leu Phe Leu Asn Lys Thr Asp Asn Gly Thr Tyr Arg Cys
            260                 265                 270

Glu Ala Thr Asn Thr Ile Gly Gln Ser Ser Ala Glu Tyr Val Leu Ile
        275                 280                 285

Val His Asp Val Pro Asn Thr Leu Leu Pro Thr Thr Ile Ile Pro Ser
    290                 295                 300

Leu Thr Thr Ala Thr Val Thr Thr Thr Val Ala Ile Thr Thr Ser Pro
305                 310                 315                 320

Thr Thr Ser Ala Thr Thr Ser Ser Ile Arg Asp Pro Asn Ala Leu Ala
                325                 330                 335

Gly Gln Asn Gly Pro Asp His
            340

<210>  48
<211>  306
<212>  PRT
<213>  Homo sapiens

<400>  48

Asn Leu Ser Gln Asp Gly Tyr Trp Gln Glu Gln Asp Leu Glu Leu Gly
1               5                   10                  15

Thr Leu Ala Pro Leu Asp Glu Ala Ile Ser Ser Thr Val Trp Ser Ser
            20                  25                  30

Pro Asp Met Leu Ala Ser Gln Asp Ser Gln Pro Trp Thr Ser Asp Glu
        35                  40                  45

Thr Val Val Ala Gly Gly Thr Val Val Leu Lys Cys Gln Val Lys Asp
    50                  55                  60

His Glu Asp Ser Ser Leu Gln Trp Ser Asn Pro Ala Gln Gln Thr Leu
65                  70                  75                  80

Tyr Phe Gly Glu Lys Arg Ala Leu Arg Asp Asn Arg Ile Gln Leu Val
                85                  90                  95

Thr Ser Thr Pro His Glu Leu Ser Ile Ser Ile Ser Asn Val Ala Leu
            100                 105                 110

Ala Asp Glu Gly Glu Tyr Thr Cys Ser Ile Phe Thr Met Pro Val Arg
        115                 120                 125

Thr Ala Lys Ser Leu Val Thr Val Leu Gly Ile Pro Gln Lys Pro Ile
    130                 135                 140

Ile Thr Gly Tyr Lys Ser Ser Leu Arg Glu Lys Asp Thr Ala Thr Leu
145                 150                 155                 160

Asn Cys Gln Ser Ser Gly Ser Lys Pro Ala Ala Arg Leu Thr Trp Arg
                165                 170                 175

Lys Gly Asp Gln Glu Leu His Gly Glu Pro Thr Arg Ile Gln Glu Asp
            180                 185                 190

Pro Asn Gly Lys Thr Phe Thr Val Ser Ser Ser Val Thr Phe Gln Val
        195                 200                 205

Thr Arg Glu Asp Asp Gly Ala Ser Ile Val Cys Ser Val Asn His Glu
    210                 215                 220

Ser Leu Lys Gly Ala Asp Arg Ser Thr Ser Gln Arg Ile Glu Val Leu
225                 230                 235                 240

Tyr Thr Pro Thr Ala Met Ile Arg Pro Asp Pro Pro His Pro Arg Glu
                245                 250                 255

Gly Gln Lys Leu Leu Leu His Cys Glu Gly Arg Gly Asn Pro Val Pro
            260                 265                 270

Gln Gln Tyr Leu Trp Glu Lys Glu Gly Ser Val Pro Pro Leu Lys Met
        275                 280                 285

Thr Gln Glu Ser Ala Leu Ile Phe Pro Phe Leu Asn Lys Ser Asp Ser
    290                 295                 300

Gly Thr
305

<210>  49
<211>  300
<212>  PRT
<213>  Homo sapiens

<400>  49

Gln Glu Val Gln Thr Glu Asn Val Thr Val Ala Glu Gly Gly Val Ala
1               5                   10                  15

Glu Ile Thr Cys Arg Leu His Gln Tyr Asp Gly Ser Ile Val Val Ile
            20                  25                  30

Gln Asn Pro Ala Arg Gln Thr Leu Phe Phe Asn Gly Thr Arg Ala Leu
        35                  40                  45

Lys Asp Glu Arg Phe Gln Leu Glu Glu Phe Ser Pro Arg Arg Val Arg
    50                  55                  60

Ile Arg Leu Ser Asp Ala Arg Leu Glu Asp Glu Gly Gly Tyr Phe Cys
65                  70                  75                  80

Gln Leu Tyr Thr Glu Asp Thr His His Gln Ile Ala Thr Leu Thr Val
                85                  90                  95

Leu Val Ala Pro Glu Asn Pro Val Val Glu Val Arg Glu Gln Ala Val
            100                 105                 110

Glu Gly Gly Glu Val Glu Leu Ser Cys Leu Val Pro Arg Ser Arg Pro
        115                 120                 125

Ala Ala Thr Leu Arg Trp Tyr Arg Asp Arg Lys Glu Leu Lys Gly Val
    130                 135                 140

Ser Ser Ser Gln Glu Asn Gly Lys Val Trp Ser Val Ala Ser Thr Val
145                 150                 155                 160

Arg Phe Arg Val Asp Arg Lys Asp Asp Gly Gly Ile Ile Ile Cys Glu
                165                 170                 175

Ala Gln Asn Gln Ala Leu Pro Ser Gly His Ser Lys Gln Thr Gln Tyr
            180                 185                 190

Val Leu Asp Val Gln Tyr Ser Pro Thr Ala Arg Ile His Ala Ser Gln
        195                 200                 205

Ala Val Val Arg Glu Gly Asp Thr Leu Val Leu Thr Cys Ala Val Thr
    210                 215                 220

Gly Asn Pro Arg Pro Asn Gln Ile Arg Trp Asn Arg Gly Asn Glu Ser
225                 230                 235                 240

Leu Pro Glu Arg Ala Glu Ala Val Gly Glu Thr Leu Thr Leu Pro Gly
                245                 250                 255

Leu Val Ser Ala Asp Asn Gly Thr Tyr Thr Cys Glu Ala Ser Asn Lys
            260                 265                 270

His Gly His Ala Arg Ala Leu Tyr Val Leu Val Val Tyr Asp Pro Gly
        275                 280                 285

Ala Val Val Glu Ala Gln Thr Ser Val Pro Tyr Ala
    290                 295                 300

<210>  50
<211>  270
<212>  PRT
<213>  Homo sapiens

<400>  50

Ser Leu Thr Asn His Thr Glu Thr Ile Thr Val Glu Glu Gly Gln Thr
1               5                   10                  15

Leu Thr Leu Lys Cys Val Thr Ser Leu Arg Lys Asn Ser Ser Leu Gln
            20                  25                  30

Trp Leu Thr Pro Ser Gly Phe Thr Ile Phe Leu Asn Glu Tyr Pro Ala
        35                  40                  45

Leu Lys Asn Ser Lys Tyr Gln Leu Leu His His Ser Ala Asn Gln Leu
    50                  55                  60

Ser Ile Thr Val Pro Asn Val Thr Leu Gln Asp Glu Gly Val Tyr Lys
65                  70                  75                  80

Cys Leu His Tyr Ser Asp Ser Val Ser Thr Lys Glu Val Lys Val Ile
                85                  90                  95

Val Leu Ala Thr Pro Phe Lys Pro Ile Leu Glu Ala Ser Val Ile Arg
            100                 105                 110

Lys Gln Asn Gly Glu Glu His Val Val Leu Met Cys Ser Thr Met Arg
        115                 120                 125

Ser Lys Pro Pro Pro Gln Ile Thr Trp Leu Leu Gly Asn Ser Met Glu
    130                 135                 140

Val Ser Gly Gly Thr Leu His Glu Phe Glu Thr Asp Gly Lys Lys Cys
145                 150                 155                 160

Asn Thr Thr Ser Thr Leu Ile Ile His Thr Tyr Gly Lys Asn Ser Thr
                165                 170                 175

Val Asp Cys Ile Ile Arg His Arg Gly Leu Gln Gly Arg Lys Leu Val
            180                 185                 190

Ala Pro Phe Arg Phe Glu Asp Leu Val Thr Asp Glu Glu Thr Ala Ser
        195                 200                 205

Asp Ala Leu Glu Arg Asn Ser Leu Ser Ser Gln Asp Pro Gln Gln Pro
    210                 215                 220

Thr Ser Thr Val Ser Val Thr Glu Asp Ser Ser Thr Ser Glu Ile Asp
225                 230                 235                 240

Lys Glu Glu Lys Glu Gln Thr Thr Gln Asp Pro Asp Leu Thr Thr Glu
                245                 250                 255

Ala Asn Pro Gln Tyr Leu Gly Leu Ala Arg Lys Lys Ser Gly
            260                 265                 270

<210>  51
<211>  191
<212>  PRT
<213>  Homo sapiens

<400>  51

Val Leu Thr Val Asn Gly Lys Thr Glu Asn Tyr Ile Leu Asp Thr Thr
1               5                   10                  15

Pro Gly Ser Gln Ala Ser Leu Ile Cys Ala Val Gln Asn His Thr Arg
            20                  25                  30

Glu Glu Glu Leu Leu Trp Tyr Arg Glu Glu Gly Arg Val Asp Leu Lys
        35                  40                  45

Ser Gly Asn Lys Ile Asn Ser Ser Ser Val Cys Val Ser Ser Ile Ser
    50                  55                  60

Glu Asn Asp Asn Gly Ile Ser Phe Thr Cys Arg Leu Gly Arg Asp Gln
65                  70                  75                  80

Ser Val Ser Val Ser Val Val Leu Asn Val Thr Phe Pro Pro Leu Leu
                85                  90                  95

Ser Gly Asn Asp Phe Gln Thr Val Glu Glu Gly Ser Asn Val Lys Leu
            100                 105                 110

Val Cys Asn Val Lys Ala Asn Pro Gln Ala Gln Met Met Trp Tyr Lys
        115                 120                 125

Asn Ser Ser Leu Leu Asp Leu Glu Lys Ser Arg His Gln Ile Gln Gln
    130                 135                 140

Thr Ser Glu Ser Phe Gln Leu Ser Ile Thr Lys Val Glu Lys Pro Asp
145                 150                 155                 160

Asn Gly Thr Tyr Ser Cys Ile Ala Lys Ser Ser Leu Lys Thr Glu Ser
                165                 170                 175

Leu Asp Phe His Leu Ile Val Lys Asp Lys Thr Val Gly Val Pro
            180                 185                 190

<210>  52
<211>  496
<212>  PRT
<213>  Homo sapiens

<400>  52

Lys Thr Val Asn Thr Glu Glu Asn Val Tyr Ala Thr Leu Gly Ser Asp
1               5                   10                  15

Val Asn Leu Thr Cys Gln Thr Gln Thr Val Gly Phe Phe Val Gln Met
            20                  25                  30

Gln Trp Ser Lys Val Thr Asn Lys Ile Asp Leu Ile Ala Val Tyr His
        35                  40                  45

Pro Gln Tyr Gly Phe Tyr Cys Ala Tyr Gly Arg Pro Cys Glu Ser Leu
    50                  55                  60

Val Thr Phe Thr Glu Thr Pro Glu Asn Gly Ser Lys Trp Thr Leu His
65                  70                  75                  80

Leu Arg Asn Met Ser Cys Ser Val Ser Gly Arg Tyr Glu Cys Met Leu
                85                  90                  95

Val Leu Tyr Pro Glu Gly Ile Gln Thr Lys Ile Tyr Asn Leu Leu Ile
            100                 105                 110

Gln Thr His Val Thr Ala Asp Glu Trp Asn Ser Asn His Thr Ile Glu
        115                 120                 125

Ile Glu Ile Asn Gln Thr Leu Glu Ile Pro Cys Phe Gln Asn Ser Ser
    130                 135                 140

Ser Lys Ile Ser Ser Glu Phe Thr Tyr Ala Trp Ser Val Glu Asn Ser
145                 150                 155                 160

Ser Thr Asp Ser Trp Val Leu Leu Ser Lys Gly Ile Lys Glu Asp Asn
                165                 170                 175

Gly Thr Gln Glu Thr Leu Ile Ser Gln Asn His Leu Ile Ser Asn Ser
            180                 185                 190

Thr Leu Leu Lys Asp Arg Val Lys Leu Gly Thr Asp Tyr Arg Leu His
        195                 200                 205

Leu Ser Pro Val Gln Ile Phe Asp Asp Gly Arg Lys Phe Ser Cys His
    210                 215                 220

Ile Arg Val Gly Pro Asn Lys Ile Leu Arg Ser Ser Thr Thr Val Lys
225                 230                 235                 240

Val Phe Ala Lys Pro Glu Ile Pro Val Ile Val Glu Asn Asn Ser Thr
                245                 250                 255

Asp Val Leu Val Glu Arg Arg Phe Thr Cys Leu Leu Lys Asn Val Phe
            260                 265                 270

Pro Lys Ala Asn Ile Thr Trp Phe Ile Asp Gly Ser Phe Leu His Asp
        275                 280                 285

Glu Lys Glu Gly Ile Tyr Ile Thr Asn Glu Glu Arg Lys Gly Lys Asp
    290                 295                 300

Gly Phe Leu Glu Leu Lys Ser Val Leu Thr Arg Val His Ser Asn Lys
305                 310                 315                 320

Pro Ala Gln Ser Asp Asn Leu Thr Ile Trp Cys Met Ala Leu Ser Pro
                325                 330                 335

Val Pro Gly Asn Lys Val Trp Asn Ile Ser Ser Glu Lys Ile Thr Phe
            340                 345                 350

Leu Leu Gly Ser Glu Ile Ser Ser Thr Asp Pro Pro Leu Ser Val Thr
        355                 360                 365

Glu Ser Thr Leu Asp Thr Gln Pro Ser Pro Ala Ser Ser Val Ser Pro
    370                 375                 380

Ala Arg Tyr Pro Ala Thr Ser Ser Val Thr Leu Val Asp Val Ser Ala
385                 390                 395                 400

Leu Arg Pro Asn Thr Thr Pro Gln Pro Ser Asn Ser Ser Met Thr Thr
                405                 410                 415

Arg Gly Phe Asn Tyr Pro Trp Thr Ser Ser Gly Thr Asp Thr Lys Lys
            420                 425                 430

Ser Val Ser Arg Ile Pro Ser Glu Thr Tyr Ser Ser Ser Pro Ser Gly
        435                 440                 445

Ala Gly Ser Thr Leu His Asp Asn Val Phe Thr Ser Thr Ala Arg Ala

450                 455                 460

Phe Ser Glu Val Pro Thr Thr Ala Asn Gly Ser Thr Lys Thr Asn His
465                 470                 475                 480

Val His Ile Thr Gly Ile Val Val Asn Lys Pro Lys Asp Gly Met Ser
                485                 490                 495

<210>  53
<211>  202
<212>  PRT
<213>  Homo sapiens

<400>  53

Gln Val Gln Val Val Thr Gln Asp Glu Arg Glu Gln Leu Tyr Thr Pro
1               5                   10                  15

Ala Ser Leu Lys Cys Ser Leu Gln Asn Ala Gln Glu Ala Leu Ile Val
            20                  25                  30

Thr Trp Gln Lys Lys Lys Ala Val Ser Pro Glu Asn Met Val Thr Phe
        35                  40                  45

Ser Glu Asn His Gly Val Val Ile Gln Pro Ala Tyr Lys Asp Lys Ile
    50                  55                  60

Asn Ile Thr Gln Leu Gly Leu Gln Asn Ser Thr Ile Thr Phe Trp Asn
65                  70                  75                  80

Ile Thr Leu Glu Asp Glu Gly Cys Tyr Met Cys Leu Phe Asn Thr Phe
                85                  90                  95

Gly Phe Gly Lys Ile Ser Gly Thr Ala Cys Leu Thr Val Tyr Val Gln
            100                 105                 110

Pro Ile Val Ser Leu His Tyr Lys Phe Ser Glu Asp His Leu Asn Ile
        115                 120                 125

Thr Cys Ser Ala Thr Ala Arg Pro Ala Pro Met Val Phe Trp Lys Val
    130                 135                 140

Pro Arg Ser Gly Ile Glu Asn Ser Thr Val Thr Leu Ser His Pro Asn
145                 150                 155                 160

Gly Thr Thr Ser Val Thr Ser Ile Leu His Ile Lys Asp Pro Lys Asn
                165                 170                 175

Gln Val Gly Lys Glu Val Ile Cys Gln Val Leu His Leu Gly Thr Val

180                 185                 190

Thr Asp Phe Lys Gln Thr Val Asn Lys Gly
        195                 200

<210>  54
<211>  219
<212>  PRT
<213>  Homo sapiens

<400>  54

Glu Gly Ala Ala Gln Pro Asn Asn Ser Leu Met Leu Gln Thr Ser Lys
1               5                   10                  15

Glu Asn His Ala Leu Ala Ser Ser Ser Leu Cys Met Asp Glu Lys Gln
            20                  25                  30

Ile Thr Gln Asn Tyr Ser Lys Val Leu Ala Glu Val Asn Thr Ser Trp
        35                  40                  45

Pro Val Lys Met Ala Thr Asn Ala Val Leu Cys Cys Pro Pro Ile Ala
    50                  55                  60

Leu Arg Asn Leu Ile Ile Ile Thr Trp Glu Ile Ile Leu Arg Gly Gln
65                  70                  75                  80

Pro Ser Cys Thr Lys Ala Tyr Lys Lys Glu Thr Asn Glu Thr Lys Glu
                85                  90                  95

Thr Asn Cys Thr Asp Glu Arg Ile Thr Trp Val Ser Arg Pro Asp Gln
            100                 105                 110

Asn Ser Asp Leu Gln Ile Arg Thr Val Ala Ile Thr His Asp Gly Tyr
        115                 120                 125

Tyr Arg Cys Ile Met Val Thr Pro Asp Gly Asn Phe His Arg Gly Tyr
    130                 135                 140

His Leu Gln Val Leu Val Thr Pro Glu Val Thr Leu Phe Gln Asn Arg
145                 150                 155                 160

Asn Arg Thr Ala Val Cys Lys Ala Val Ala Gly Lys Pro Ala Ala His
                165                 170                 175

Ile Ser Trp Ile Pro Glu Gly Asp Cys Ala Thr Lys Gln Glu Tyr Trp
            180                 185                 190

Ser Asn Gly Thr Val Thr Val Lys Ser Thr Cys His Trp Glu Val His

195                 200                 205

Asn Val Ser Thr Val Thr Cys His Val Ser His
    210                 215

<210>  55
<211>  220
<212>  PRT
<213>  Homo sapiens

<400>  55

Ser Cys Met Gly Gly Lys Gln Met Thr Gln Asn Tyr Ser Thr Ile Phe
1               5                   10                  15

Ala Glu Gly Asn Ile Ser Gln Pro Val Leu Met Asp Ile Asn Ala Val
            20                  25                  30

Leu Cys Cys Pro Pro Ile Ala Leu Arg Asn Leu Ile Ile Ile Thr Trp
        35                  40                  45

Glu Ile Ile Leu Arg Gly Gln Pro Ser Cys Thr Lys Ala Tyr Lys Lys
    50                  55                  60

Glu Thr Asn Glu Thr Lys Glu Thr Asn Cys Thr Val Glu Arg Ile Thr
65                  70                  75                  80

Trp Val Ser Arg Pro Asp Gln Asn Ser Asp Leu Gln Ile Arg Pro Val
                85                  90                  95

Asp Thr Thr His Asp Gly Tyr Tyr Arg Gly Ile Val Val Thr Pro Asp
            100                 105                 110

Gly Asn Phe His Arg Gly Tyr His Leu Gln Val Leu Val Thr Pro Glu
        115                 120                 125

Val Asn Leu Phe Gln Ser Arg Asn Ile Thr Ala Val Cys Lys Ala Val
    130                 135                 140

Thr Gly Lys Pro Ala Ala Gln Ile Ser Trp Ile Pro Glu Gly Ser Ile
145                 150                 155                 160

Leu Ala Thr Lys Gln Glu Tyr Trp Gly Asn Gly Thr Val Thr Val Lys
                165                 170                 175

Ser Thr Cys Pro Trp Glu Gly His Lys Ser Thr Val Thr Cys His Val
            180                 185                 190

Ser His Leu Thr Gly Asn Lys Ser Leu Ser Val Lys Leu Asn Ser Gly

195                 200                 205

Leu Arg Thr Ser Gly Ser Pro Ala Leu Ser Leu Leu
    210                 215                 220

<210>  56
<211>  236
<212>  PRT
<213>  Homo sapiens

<400>  56

<210>  57
<211>  132
<212>  PRT
<213>  Homo sapiens

<400>  57

Thr Pro Glu Val Trp Val Gln Val Arg Met Glu Ala Thr Glu Leu Ser
1               5                   10                  15

Ser Phe Thr Ile Arg Cys Gly Phe Leu Gly Ser Gly Ser Ile Ser Leu
            20                  25                  30

Val Thr Val Ser Trp Gly Gly Pro Asn Gly Ala Gly Gly Thr Thr Leu
        35                  40                  45

Ala Val Leu His Pro Glu Arg Gly Ile Arg Gln Trp Ala Pro Ala Arg
    50                  55                  60

Gln Ala Arg Trp Glu Thr Gln Ser Ser Ile Ser Leu Ile Leu Glu Gly
65                  70                  75                  80

Ser Gly Ala Ser Ser Pro Cys Ala Asn Thr Thr Phe Cys Cys Lys Phe
                85                  90                  95

Ala Ser Phe Pro Glu Gly Ser Trp Glu Ala Cys Gly Ser Leu Pro Pro
            100                 105                 110

Ser Ser Asp Pro Gly Leu Ser Ala Pro Pro Thr Pro Ala Pro Ile Leu
        115                 120                 125

Arg Ala Asp Leu
    130

<210>  58
<211>  316
<212>  PRT
<213>  Homo sapiens

<400>  58

Asp Val Val Val Gln Ala Pro Thr Gln Val Pro Gly Phe Leu Gly Asp
1               5                   10                  15

Ser Val Thr Leu Pro Cys Tyr Leu Gln Val Pro Asn Met Glu Val Thr
            20                  25                  30

His Val Ser Gln Leu Thr Trp Ala Arg His Gly Glu Ser Gly Ser Met
        35                  40                  45

Ala Val Phe His Gln Thr Gln Gly Pro Ser Tyr Ser Glu Ser Lys Arg
    50                  55                  60

Leu Glu Phe Val Ala Ala Arg Leu Gly Ala Glu Leu Arg Asn Ala Ser
65                  70                  75                  80

Leu Arg Met Phe Gly Leu Arg Val Glu Asp Glu Gly Asn Tyr Thr Cys
                85                  90                  95

Leu Phe Val Thr Phe Pro Gln Gly Ser Arg Ser Val Asp Ile Trp Leu
            100                 105                 110

Arg Val Leu Ala Lys Pro Gln Asn Thr Ala Glu Val Gln Lys Val Gln
        115                 120                 125

Leu Thr Gly Glu Pro Val Pro Met Ala Arg Cys Val Ser Thr Gly Gly
    130                 135                 140

Arg Pro Pro Ala Gln Ile Thr Trp His Ser Asp Leu Gly Gly Met Pro
145                 150                 155                 160

Asn Thr Ser Gln Val Pro Gly Phe Leu Ser Gly Thr Val Thr Val Thr
                165                 170                 175

Ser Leu Trp Ile Leu Val Pro Ser Ser Gln Val Asp Gly Lys Asn Val
            180                 185                 190

Thr Cys Lys Val Glu His Glu Ser Phe Glu Lys Pro Gln Leu Leu Thr
        195                 200                 205

Val Asn Leu Thr Val Tyr Tyr Pro Pro Glu Val Ser Ile Ser Gly Tyr
    210                 215                 220

Asp Asn Asn Trp Tyr Leu Gly Gln Asn Glu Ala Thr Leu Thr Cys Asp
225                 230                 235                 240

Ala Arg Ser Asn Pro Glu Pro Thr Gly Tyr Asn Trp Ser Thr Thr Met
                245                 250                 255

Gly Pro Leu Pro Pro Phe Ala Val Ala Gln Gly Ala Gln Leu Leu Ile

260                 265                 270

Arg Pro Val Asp Lys Pro Ile Asn Thr Thr Leu Ile Cys Asn Val Thr
        275                 280                 285

Asn Ala Leu Gly Ala Arg Gln Ala Glu Leu Thr Val Gln Val Lys Glu
    290                 295                 300

Gly Pro Pro Ser Glu His Ser Gly Met Ser Arg Asn
305                 310                 315

<210>  59
<211>  325
<212>  PRT
<213>  Homo sapiens

<400>  59

Gln Val Val Gln Val Asn Asp Ser Met Tyr Gly Phe Ile Gly Thr Asp
1               5                   10                  15

Val Val Leu His Cys Ser Phe Ala Asn Pro Leu Pro Ser Val Lys Ile
            20                  25                  30

Thr Gln Val Thr Trp Gln Lys Ser Thr Asn Gly Ser Lys Gln Asn Val
        35                  40                  45

Ala Ile Tyr Asn Pro Ser Met Gly Val Ser Val Leu Ala Pro Tyr Arg
    50                  55                  60

Glu Arg Val Glu Phe Leu Arg Pro Ser Phe Thr Asp Gly Thr Ile Arg
65                  70                  75                  80

Leu Ser Arg Leu Glu Leu Glu Asp Glu Gly Val Tyr Ile Cys Glu Phe
                85                  90                  95

Ala Thr Phe Pro Thr Gly Asn Arg Glu Ser Gln Leu Asn Leu Thr Val
            100                 105                 110

Met Ala Lys Pro Thr Asn Trp Ile Glu Gly Thr Gln Ala Val Leu Arg
        115                 120                 125

Ala Lys Lys Gly Gln Asp Asp Lys Val Leu Val Ala Thr Cys Thr Ser
    130                 135                 140

Ala Asn Gly Lys Pro Pro Ser Val Val Ser Trp Glu Thr Arg Leu Lys
145                 150                 155                 160

Gly Glu Ala Glu Tyr Gln Glu Ile Arg Asn Pro Asn Gly Thr Val Thr

165                 170                 175

Val Ile Ser Arg Tyr Arg Leu Val Pro Ser Arg Glu Ala His Gln Gln
            180                 185                 190

Ser Leu Ala Cys Ile Val Asn Tyr His Met Asp Arg Phe Lys Glu Ser
        195                 200                 205

Leu Thr Leu Asn Val Gln Tyr Glu Pro Glu Val Thr Ile Glu Gly Phe
    210                 215                 220

Asp Gly Asn Trp Tyr Leu Gln Arg Met Asp Val Lys Leu Thr Cys Lys
225                 230                 235                 240

Ala Asp Ala Asn Pro Pro Ala Thr Glu Tyr His Trp Thr Thr Leu Asn
                245                 250                 255

Gly Ser Leu Pro Lys Gly Val Glu Ala Gln Asn Arg Thr Leu Phe Phe
            260                 265                 270

Lys Gly Pro Ile Asn Tyr Ser Leu Ala Gly Thr Tyr Ile Cys Glu Ala
        275                 280                 285

Thr Asn Pro Ile Gly Thr Arg Ser Gly Gln Val Glu Val Asn Ile Thr
    290                 295                 300

Glu Phe Pro Tyr Thr Pro Ser Pro Pro Glu His Gly Arg Arg Ala Gly
305                 310                 315                 320

Pro Val Pro Thr Ala
                325

<210>  60
<211>  329
<212>  PRT
<213>  Homo sapiens

<400>  60

Gln Asp Val Arg Val Gln Val Leu Pro Glu Val Arg Gly Gln Leu Gly
1               5                   10                  15

Gly Thr Val Glu Leu Pro Cys His Leu Leu Pro Pro Val Pro Gly Leu
            20                  25                  30

Tyr Ile Ser Leu Val Thr Trp Gln Arg Pro Asp Ala Pro Ala Asn His
        35                  40                  45

Gln Asn Val Ala Ala Phe His Pro Lys Met Gly Pro Ser Phe Pro Ser

50                  55                  60

Pro Lys Pro Gly Ser Glu Arg Leu Ser Phe Val Ser Ala Lys Gln Ser
65                  70                  75                  80

Thr Gly Gln Asp Thr Glu Ala Glu Leu Gln Asp Ala Thr Leu Ala Leu
                85                  90                  95

His Gly Leu Thr Val Glu Asp Glu Gly Asn Tyr Thr Cys Glu Phe Ala
            100                 105                 110

Thr Phe Pro Lys Gly Ser Val Arg Gly Met Thr Trp Leu Arg Val Ile
        115                 120                 125

Ala Lys Pro Lys Asn Gln Ala Glu Ala Gln Lys Val Thr Phe Ser Gln
    130                 135                 140

Asp Pro Thr Thr Val Ala Leu Cys Ile Ser Lys Glu Gly Arg Pro Pro
145                 150                 155                 160

Ala Arg Ile Ser Trp Leu Ser Ser Leu Asp Trp Glu Ala Lys Glu Thr
                165                 170                 175

Gln Val Ser Gly Thr Leu Ala Gly Thr Val Thr Val Thr Ser Arg Phe
            180                 185                 190

Thr Leu Val Pro Ser Gly Arg Ala Asp Gly Val Thr Val Thr Cys Lys
        195                 200                 205

Val Glu His Glu Ser Phe Glu Glu Pro Ala Leu Ile Pro Val Thr Leu
    210                 215                 220

Ser Val Arg Tyr Pro Pro Glu Val Ser Ile Ser Gly Tyr Asp Asp Asn
225                 230                 235                 240

Trp Tyr Leu Gly Arg Thr Asp Ala Thr Leu Ser Cys Asp Val Arg Ser
                245                 250                 255

Asn Pro Glu Pro Thr Gly Tyr Asp Trp Ser Thr Thr Ser Gly Thr Phe
            260                 265                 270

Pro Thr Ser Ala Val Ala Gln Gly Ser Gln Leu Val Ile His Ala Val
        275                 280                 285

Asp Ser Leu Phe Asn Thr Thr Phe Val Cys Thr Val Thr Asn Ala Val
    290                 295                 300

Gly Met Gly Arg Ala Glu Gln Val Ile Phe Val Arg Glu Thr Pro Asn
305                 310                 315                 320

Thr Ala Gly Ala Gly Ala Thr Gly Gly
                325

<210>  61
<211>  347
<212>  PRT
<213>  Homo sapiens

<400>  61

Gly Pro Ile Ile Val Glu Pro His Val Thr Ala Val Trp Gly Lys Asn
1               5                   10                  15

Val Ser Leu Lys Cys Leu Ile Glu Val Asn Glu Thr Ile Thr Gln Ile
            20                  25                  30

Ser Trp Glu Lys Ile His Gly Lys Ser Ser Gln Thr Val Ala Val His
        35                  40                  45

His Pro Gln Tyr Gly Phe Ser Val Gln Gly Glu Tyr Gln Gly Arg Val
    50                  55                  60

Leu Phe Lys Asn Tyr Ser Leu Asn Asp Ala Thr Ile Thr Leu His Asn
65                  70                  75                  80

Ile Gly Phe Ser Asp Ser Gly Lys Tyr Ile Cys Lys Ala Val Thr Phe
                85                  90                  95

Pro Leu Gly Asn Ala Gln Ser Ser Thr Thr Val Thr Val Leu Val Glu
            100                 105                 110

Pro Thr Val Ser Leu Ile Lys Gly Pro Asp Ser Leu Ile Asp Gly Gly
        115                 120                 125

Asn Glu Thr Val Ala Ala Ile Cys Ile Ala Ala Thr Gly Lys Pro Val
    130                 135                 140

Ala His Ile Asp Trp Glu Gly Asp Leu Gly Glu Met Glu Ser Thr Thr
145                 150                 155                 160

Thr Ser Phe Pro Asn Glu Thr Ala Thr Ile Ile Ser Gln Tyr Lys Leu
                165                 170                 175

Phe Pro Thr Arg Phe Ala Arg Gly Arg Arg Ile Thr Cys Val Val Lys
            180                 185                 190

His Pro Ala Leu Glu Lys Asp Ile Arg Tyr Ser Phe Ile Leu Asp Ile
        195                 200                 205

Gln Tyr Ala Pro Glu Val Ser Val Thr Gly Tyr Asp Gly Asn Trp Phe
    210                 215                 220

Val Gly Arg Lys Gly Val Asn Leu Lys Cys Asn Ala Asp Ala Asn Pro
225                 230                 235                 240

Pro Pro Phe Lys Ser Val Trp Ser Arg Leu Asp Gly Gln Trp Pro Asp
                245                 250                 255

Gly Leu Leu Ala Ser Asp Asn Thr Leu His Phe Val His Pro Leu Thr
            260                 265                 270

Phe Asn Tyr Ser Gly Val Tyr Ile Cys Lys Val Thr Asn Ser Leu Gly
        275                 280                 285

Gln Arg Ser Asp Gln Lys Val Ile Tyr Ile Ser Asp Pro Pro Thr Thr
    290                 295                 300

Thr Thr Leu Gln Pro Thr Ile Gln Trp His Pro Ser Thr Ala Asp Ile
305                 310                 315                 320

Glu Asp Leu Ala Thr Glu Pro Lys Lys Leu Pro Phe Pro Leu Ser Thr
                325                 330                 335

Leu Ala Thr Ile Lys Asp Asp Thr Ile Ala Thr
            340                 345

<210>  62
<211>  318
<212>  PRT
<213>  Homo sapiens

<400>  62

Gly Glu Leu Glu Thr Ser Asp Val Val Thr Val Val Leu Gly Gln Asp
1               5                   10                  15

Ala Lys Leu Pro Cys Phe Tyr Arg Gly Asp Ser Gly Glu Gln Val Gly
            20                  25                  30

Gln Val Ala Trp Ala Arg Val Asp Ala Gly Glu Gly Ala Gln Glu Leu
        35                  40                  45

Ala Leu Leu His Ser Lys Tyr Gly Leu His Val Ser Pro Ala Tyr Glu
    50                  55                  60

Gly Arg Val Glu Gln Pro Pro Pro Pro Arg Asn Pro Leu Asp Gly Ser
65                  70                  75                  80

Val Leu Leu Arg Asn Ala Val Gln Ala Asp Glu Gly Glu Tyr Glu Cys
                85                  90                  95

Arg Val Ser Thr Phe Pro Ala Gly Ser Phe Gln Ala Arg Leu Arg Leu
            100                 105                 110

Arg Val Leu Val Pro Pro Leu Pro Ser Leu Asn Pro Gly Pro Ala Leu
        115                 120                 125

Glu Glu Gly Gln Gly Leu Thr Leu Ala Ala Ser Cys Thr Ala Glu Gly
    130                 135                 140

Ser Pro Ala Pro Ser Val Thr Trp Asp Thr Glu Val Lys Gly Thr Thr
145                 150                 155                 160

Ser Ser Arg Ser Phe Lys His Ser Arg Ser Ala Ala Val Thr Ser Glu
                165                 170                 175

Phe His Leu Val Pro Ser Arg Ser Met Asn Gly Gln Pro Leu Thr Cys
            180                 185                 190

Val Val Ser His Pro Gly Leu Leu Gln Asp Gln Arg Ile Thr His Ile
        195                 200                 205

Leu His Val Ser Phe Leu Ala Glu Ala Ser Val Arg Gly Leu Glu Asp
    210                 215                 220

Gln Asn Leu Trp His Ile Gly Arg Glu Gly Ala Met Leu Lys Cys Leu
225                 230                 235                 240

Ser Glu Gly Gln Pro Pro Pro Ser Tyr Asn Trp Thr Arg Leu Asp Gly
                245                 250                 255

Pro Leu Pro Ser Gly Val Arg Val Asp Gly Asp Thr Leu Gly Phe Pro
            260                 265                 270

Pro Leu Thr Thr Glu His Ser Gly Ile Tyr Val Cys His Val Ser Asn
        275                 280                 285

Glu Phe Ser Ser Arg Asp Ser Gln Val Thr Val Asp Val Leu Asp Pro
    290                 295                 300

Gln Glu Asp Ser Gly Lys Gln Val Asp Leu Val Ser Ala Ser
305                 310                 315

<210>  63
<211>  141
<212>  PRT
<213>  Homo sapiens

<400>  63

Met Met Thr Gly Thr Ile Glu Thr Thr Gly Asn Ile Ser Ala Glu Lys
1               5                   10                  15

Gly Gly Ser Ile Ile Leu Gln Cys His Leu Ser Ser Thr Thr Ala Gln
            20                  25                  30

Val Thr Gln Val Asn Trp Glu Gln Gln Asp Gln Leu Leu Ala Ile Cys
        35                  40                  45

Asn Ala Asp Leu Gly Trp His Ile Ser Pro Ser Phe Lys Asp Arg Val
    50                  55                  60

Ala Pro Gly Pro Gly Leu Gly Leu Thr Leu Gln Ser Leu Thr Val Asn
65                  70                  75                  80

Asp Thr Gly Glu Tyr Phe Cys Ile Tyr His Thr Tyr Pro Asp Gly Thr
                85                  90                  95

Tyr Thr Gly Arg Ile Phe Leu Glu Val Leu Glu Ser Ser Val Ala Glu
            100                 105                 110

His Gly Ala Arg Phe Gln Ile Pro Leu Leu Gly Ala Met Ala Ala Thr
        115                 120                 125

Leu Val Val Ile Cys Thr Ala Val Ile Val Val Val Ala
    130                 135                 140

<210>  64
<211>  206
<212>  PRT
<213>  Artificial sequence

<220>
<223>  TMEM25 - ECD without predicted signal sequence

<400>  64

Glu Leu Glu Pro Gln Ile Asp Gly Gln Thr Trp Ala Glu Arg Ala Leu
1               5                   10                  15

Arg Glu Asn Glu Arg His Ala Phe Thr Cys Arg Val Ala Gly Gly Pro
            20                  25                  30

Gly Thr Pro Arg Leu Ala Trp Tyr Leu Asp Gly Gln Leu Gln Glu Ala

35                  40                  45

Ser Thr Ser Arg Leu Leu Ser Val Gly Gly Glu Ala Phe Ser Gly Gly
    50                  55                  60

Thr Ser Thr Phe Thr Val Thr Ala His Arg Ala Gln His Glu Leu Asn
65                  70                  75                  80

Cys Ser Leu Gln Asp Pro Arg Ser Gly Arg Ser Ala Asn Ala Ser Val
                85                  90                  95

Ile Leu Asn Val Gln Phe Lys Pro Glu Ile Ala Gln Val Gly Ala Lys
            100                 105                 110

Tyr Gln Glu Ala Gln Gly Pro Gly Leu Leu Val Val Leu Phe Ala Leu
        115                 120                 125

Val Arg Ala Asn Pro Pro Ala Asn Val Thr Trp Ile Asp Gln Asp Gly
    130                 135                 140

Pro Val Thr Val Asn Thr Ser Asp Phe Leu Val Leu Asp Ala Gln Asn
145                 150                 155                 160

Tyr Pro Trp Leu Thr Asn His Thr Val Gln Leu Gln Leu Arg Ser Leu
                165                 170                 175

Ala His Asn Leu Ser Val Val Ala Thr Asn Asp Val Gly Val Thr Ser
            180                 185                 190

Ala Ser Leu Pro Ala Pro Gly Leu Leu Ala Thr Arg Val Glu
        195                 200                 205

<210>  65
<211>  227
<212>  PRT
<213>  Homo sapiens

<400>  65

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val

50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210>  66
<211>  230
<212>  PRT
<213>  Homo sapiens

<400>  66

Lys Ser Ser Asp Lys Thr His Thr Cys Pro Pro Cys Pro Ala Pro Glu
1               5                   10                  15

Leu Leu Gly Gly Pro Ser Val Phe Leu Phe Pro Pro Lys Pro Lys Asp
            20                  25                  30

Thr Leu Met Ile Ser Arg Thr Pro Glu Val Thr Cys Val Val Val Asp

35                  40                  45

Val Ser His Glu Asp Pro Glu Val Lys Phe Asn Trp Tyr Val Asp Gly
    50                  55                  60

Val Glu Val His Asn Ala Lys Thr Lys Pro Arg Glu Glu Gln Tyr Asn
65                  70                  75                  80

Ser Thr Tyr Arg Val Val Ser Val Leu Thr Val Leu His Gln Asp Trp
                85                  90                  95

Leu Asn Gly Lys Glu Tyr Lys Cys Lys Val Ser Asn Lys Ala Leu Pro
            100                 105                 110

Ala Pro Ile Glu Lys Thr Ile Ser Lys Ala Lys Gly Gln Pro Arg Glu
        115                 120                 125

Pro Gln Val Tyr Thr Leu Pro Pro Ser Arg Asp Glu Leu Thr Lys Asn
    130                 135                 140

Gln Val Ser Leu Thr Cys Leu Val Lys Gly Phe Tyr Pro Ser Asp Ile
145                 150                 155                 160

Ala Val Glu Trp Glu Ser Asn Gly Gln Pro Glu Asn Asn Tyr Lys Thr
                165                 170                 175

Thr Pro Pro Val Leu Asp Ser Asp Gly Ser Phe Phe Leu Tyr Ser Lys
            180                 185                 190

Leu Thr Val Asp Lys Ser Arg Trp Gln Gln Gly Asn Val Phe Ser Cys
        195                 200                 205

Ser Val Met His Glu Ala Leu His Asn His Tyr Thr Gln Lys Ser Leu
    210                 215                 220

Ser Leu Ser Pro Gly Lys
225                 230

<210>  67
<211>  232
<212>  PRT
<213>  Homo sapiens

<400>  67

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro

20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Asp Glu Leu Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  68
<211>  321
<212>  PRT
<213>  Artificial sequence

<220>
<223>  dnYAP

<400>  68

Met Asp Pro Gly Gln Gln Pro Pro Pro Gln Pro Ala Pro Gln Gly Gln
1               5                   10                  15

Gly Gln Pro Pro Ser Gln Pro Pro Gln Gly Gln Gly Pro Pro Ser Gly
            20                  25                  30

Pro Gly Gln Pro Ala Pro Ala Ala Thr Gln Ala Ala Pro Gln Ala Pro
        35                  40                  45

Pro Ala Gly His Gln Ile Val His Val Arg Gly Asp Ser Glu Thr Asp
    50                  55                  60

Leu Glu Ala Leu Phe Asn Ala Val Met Asn Pro Lys Thr Ala Asn Val
65                  70                  75                  80

Pro Gln Thr Val Pro Met Arg Leu Arg Lys Leu Pro Asp Ser Phe Phe
                85                  90                  95

Lys Pro Pro Glu Pro Lys Ser His Ser Arg Gln Ala Ser Thr Asp Ala
            100                 105                 110

Gly Thr Ala Gly Ala Leu Thr Pro Gln His Val Arg Ala His Ala Ser
        115                 120                 125

Pro Ala Ser Leu Gln Leu Gly Ala Val Ser Pro Gly Thr Leu Thr Pro
    130                 135                 140

Thr Gly Val Val Ser Gly Pro Ala Ala Thr Pro Thr Ala Gln His Leu
145                 150                 155                 160

Arg Gln Ser Ser Phe Glu Ile Pro Asp Asp Val Pro Leu Pro Ala Gly
                165                 170                 175

Trp Glu Met Ala Lys Thr Ser Ser Gly Gln Arg Tyr Phe Leu Asn His
            180                 185                 190

Ile Asp Gln Thr Thr Thr Trp Gln Asp Pro Arg Lys Ala Met Leu Ser
        195                 200                 205

Gln Met Asn Val Thr Ala Pro Thr Ser Pro Pro Val Gln Gln Asn Met
    210                 215                 220

Met Asn Ser Ala Ser Gly Pro Leu Pro Asp Gly Trp Glu Gln Ala Met
225                 230                 235                 240

Thr Gln Asp Gly Glu Ile Tyr Tyr Ile Asn His Lys Asn Lys Thr Thr

245                 250                 255

Ser Trp Leu Asp Pro Arg Leu Asp Pro Arg Phe Ala Met Asn Gln Arg
            260                 265                 270

Ile Ser Gln Ser Ala Pro Val Lys Gln Pro Pro Pro Leu Ala Pro Gln
        275                 280                 285

Ser Pro Gln Gly Gly Val Met Gly Gly Ser Asn Leu Asn Asp Met Glu
    290                 295                 300

Ser Val Leu Ala Ala Thr Lys Leu Asp Lys Glu Ser Phe Leu Thr Trp
305                 310                 315                 320

Leu

<210>  69
<211>  239
<212>  PRT
<213>  Artificial sequence

<220>
<223>  YFP

<400>  69

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Phe Gly Tyr Gly Leu Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Tyr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  70
<211>  447
<212>  PRT
<213>  Homo sapiens

<400>  70

Met Gly Glu Pro Arg Ala Gly Ala Ala Leu Asp Asp Gly Ser Gly Trp
1               5                   10                  15

Thr Gly Ser Glu Glu Gly Ser Glu Glu Gly Thr Gly Gly Ser Glu Gly
            20                  25                  30

Ala Gly Gly Asp Gly Gly Pro Asp Ala Glu Gly Val Trp Ser Pro Asp
        35                  40                  45

Ile Glu Gln Ser Phe Gln Glu Ala Leu Ala Ile Tyr Pro Pro Cys Gly
    50                  55                  60

Arg Arg Lys Ile Ile Leu Ser Asp Glu Gly Lys Met Tyr Gly Arg Asn
65                  70                  75                  80

Glu Leu Ile Ala Arg Tyr Ile Lys Leu Arg Thr Gly Lys Thr Arg Thr
                85                  90                  95

Arg Lys Gln Val Ser Ser His Ile Gln Val Leu Ala Arg Arg Lys Ser
            100                 105                 110

Arg Glu Ile Gln Ser Lys Leu Lys Asp Gln Val Ser Lys Asp Lys Ala
        115                 120                 125

Phe Gln Thr Met Ala Thr Met Ser Ser Ala Gln Leu Ile Ser Ala Pro
    130                 135                 140

Ser Leu Gln Ala Lys Leu Gly Pro Thr Gly Pro Gln Ala Ser Glu Leu
145                 150                 155                 160

Phe Gln Phe Trp Ser Gly Gly Ser Gly Pro Pro Trp Asn Val Pro Asp
                165                 170                 175

Val Lys Pro Phe Ser Gln Thr Pro Phe Thr Leu Ser Leu Thr Pro Pro
            180                 185                 190

Ser Thr Asp Leu Pro Gly Tyr Glu Pro Pro Gln Ala Leu Ser Pro Leu
        195                 200                 205

Pro Pro Pro Thr Pro Ser Pro Pro Ala Trp Gln Ala Arg Gly Leu Gly
    210                 215                 220

Thr Ala Arg Leu Gln Leu Val Glu Phe Ser Ala Phe Val Glu Pro Pro
225                 230                 235                 240

Asp Ala Val Asp Ser Tyr Gln Arg His Leu Phe Val His Ile Ser Gln
                245                 250                 255

His Cys Pro Ser Pro Gly Ala Pro Pro Leu Glu Ser Val Asp Val Arg
            260                 265                 270

Gln Ile Tyr Asp Lys Phe Pro Glu Lys Lys Gly Gly Leu Arg Glu Leu
        275                 280                 285

Tyr Asp Arg Gly Pro Pro His Ala Phe Phe Leu Val Lys Phe Trp Ala
    290                 295                 300

Asp Leu Asn Trp Gly Pro Ser Gly Glu Glu Ala Gly Ala Gly Gly Ser
305                 310                 315                 320

Ile Ser Ser Gly Gly Phe Tyr Gly Val Ser Ser Gln Tyr Glu Ser Leu
                325                 330                 335

Glu His Met Thr Leu Thr Cys Ser Ser Lys Val Cys Ser Phe Gly Lys
            340                 345                 350

Gln Val Val Glu Lys Val Glu Thr Glu Arg Ala Gln Leu Glu Asp Gly

355                 360                 365

Arg Phe Val Tyr Arg Leu Leu Arg Ser Pro Met Cys Glu Tyr Leu Val
    370                 375                 380

Asn Phe Leu His Lys Leu Arg Gln Leu Pro Glu Arg Tyr Met Met Asn
385                 390                 395                 400

Ser Val Leu Glu Asn Phe Thr Ile Leu Gln Val Val Thr Asn Arg Asp
                405                 410                 415

Thr Gln Glu Leu Leu Leu Cys Thr Ala Tyr Val Phe Glu Val Ser Thr
            420                 425                 430

Ser Glu Arg Gly Ala Gln His His Ile Tyr Arg Leu Val Arg Asp
        435                 440                 445

<210>  71
<211>  435
<212>  PRT
<213>  Homo sapiens

<400>  71

Met Ala Ser Asn Ser Trp Asn Ala Ser Ser Ser Pro Gly Glu Ala Arg
1               5                   10                  15

Glu Asp Gly Pro Glu Gly Leu Asp Lys Gly Leu Asp Asn Asp Ala Glu
            20                  25                  30

Gly Val Trp Ser Pro Asp Ile Glu Gln Ser Phe Gln Glu Ala Leu Ala
        35                  40                  45

Ile Tyr Pro Pro Cys Gly Arg Arg Lys Ile Ile Leu Ser Asp Glu Gly
    50                  55                  60

Lys Met Tyr Gly Arg Asn Glu Leu Ile Ala Arg Tyr Ile Lys Leu Arg
65                  70                  75                  80

Thr Gly Lys Thr Arg Thr Arg Lys Gln Val Ser Ser His Ile Gln Val
                85                  90                  95

Leu Ala Arg Lys Lys Val Arg Glu Tyr Gln Val Gly Ile Lys Ala Met
            100                 105                 110

Asn Leu Asp Gln Val Ser Lys Asp Lys Ala Leu Gln Ser Met Ala Ser
        115                 120                 125

Met Ser Ser Ala Gln Ile Val Ser Ala Ser Val Leu Gln Asn Lys Phe

130                 135                 140

Ser Pro Pro Ser Pro Leu Pro Gln Ala Val Phe Ser Thr Ser Ser Arg
145                 150                 155                 160

Phe Trp Ser Ser Pro Pro Leu Leu Gly Gln Gln Pro Gly Pro Ser Gln
                165                 170                 175

Asp Ile Lys Pro Phe Ala Gln Pro Ala Tyr Pro Ile Gln Pro Pro Leu
            180                 185                 190

Pro Pro Thr Leu Ser Ser Tyr Glu Pro Leu Ala Pro Leu Pro Ser Ala
        195                 200                 205

Ala Ala Ser Val Pro Val Trp Gln Asp Arg Thr Ile Ala Ser Ser Arg
    210                 215                 220

Leu Arg Leu Leu Glu Tyr Ser Ala Phe Met Glu Val Gln Arg Asp Pro
225                 230                 235                 240

Asp Thr Tyr Ser Lys His Leu Phe Val His Ile Gly Gln Thr Asn Pro
                245                 250                 255

Ala Phe Ser Asp Pro Pro Leu Glu Ala Val Asp Val Arg Gln Ile Tyr
            260                 265                 270

Asp Lys Phe Pro Glu Lys Lys Gly Gly Leu Lys Glu Leu Tyr Glu Lys
        275                 280                 285

Gly Pro Pro Asn Ala Phe Phe Leu Val Lys Phe Trp Ala Asp Leu Asn
    290                 295                 300

Ser Thr Ile Gln Glu Gly Pro Gly Ala Phe Tyr Gly Val Ser Ser Gln
305                 310                 315                 320

Tyr Ser Ser Ala Asp Ser Met Thr Ile Ser Val Ser Thr Lys Val Cys
                325                 330                 335

Ser Phe Gly Lys Gln Val Val Glu Lys Val Glu Thr Glu Tyr Ala Arg
            340                 345                 350

Leu Glu Asn Gly Arg Phe Val Tyr Arg Ile His Arg Ser Pro Met Cys
        355                 360                 365

Glu Tyr Met Ile Asn Phe Ile His Lys Leu Lys His Leu Pro Glu Lys
    370                 375                 380

Tyr Met Met Asn Ser Val Leu Glu Asn Phe Thr Ile Leu Gln Val Val
385                 390                 395                 400

Thr Ser Arg Asp Ser Gln Glu Thr Leu Leu Val Ile Ala Phe Val Phe
                405                 410                 415

Glu Val Ser Thr Ser Glu His Gly Ala Gln His His Val Tyr Lys Leu
            420                 425                 430

Val Lys Asp
        435

<210>  72
<211>  17
<212>  PRT
<213>  Artificial sequence

<220>
<223>  Linker

<400>  72

Arg Pro Ala Cys Lys Ile Pro Asn Asp Leu Lys Gln Lys Val Met Asn
1               5                   10                  15

His

<210>  73
<211>  8
<212>  PRT
<213>  Artificial sequence

<220>
<223>  FLAG Tag

<400>  73

Asp Tyr Lys Asp Asp Asp Asp Lys
1               5

<210>  74
<211>  8
<212>  PRT
<213>  Artificial sequence

<220>
<223>  Linker

<400>  74

Glu Ser Gly Gly Gly Gly Val Thr
1               5

<210>  75

<211>  9
<212>  PRT
<213>  Artificial sequence

<220>
<223>  Linker

<400>  75

Leu Glu Ser Gly Gly Gly Gly Val Thr
1               5


<210>  76
<211>  6
<212>  PRT
<213>  Artificial sequence

<220>
<223>  Linker

<400>  76

Gly Arg Ala Gln Val Thr
1               5


<210>  77
<211>  6
<212>  PRT
<213>  Artificial sequence

<220>
<223>  Linker

<400>  77

Trp Arg Ala Gln Val Thr
1               5


<210>  78
<211>  8
<212>  PRT
<213>  Artificial sequence

<220>
<223>  Linker

<400>  78

Ala Arg Gly Arg Ala Gln Val Thr
1               5


<210>  79
<211>  326
<212>  PRT
<213>  Homo sapiens

<400>  79

Ala Ser Thr Lys Gly Pro Ser Val Phe Pro Leu Ala Pro Cys Ser Arg

1                   5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325